# EUROPEAN PATENT APPLICATION

(11) **EP 4 678 626 A1**
(43) Date of publication of application: **14.01.2026**
(21) Application number: 24767142.3
(22) Date of filing: 05.03.2024
(51) Int. Cl.: C07C 65/40, C07C 69/92, C07C 235/52, C07F 9/09, C07H 19/10, C07H 19/073

(54) **LONG-CHAIN ALKENYLOXY-SUBSTITUTED BENZOYL DERIVATIVE AND OLIGONUCLEOTIDE SYNTHESIS METHOD USING SAME**

(30) Priority: 06.03.2023 JP 2023033426
(71) Applicant: Spera Pharma, Inc., Osaka-shi, Osaka 532-0024 (JP); Tokyo University of Agriculture and Technology, Fuchu-shi, Tokyo 183-8538 (JP)
(72) Inventor: OKADA Yohei, Fuchu-shi, Tokyo 183-8538 (JP); INANAGA Kazato, Osaka-shi, Osaka 532-0024 (JP); SHOJI Koya, Osaka-shi, Osaka 532-0024 (JP); MIZUFUNE Hideya, Osaka-shi, Osaka 532-0024 (JP); SUDO Tatsuya, Fuchu-shi, Tokyo 183-8538 (JP); ADACHI Sota, Fuchu-shi, Tokyo 183-8538 (JP); HOSOI Kazushi, Osaka-shi, Osaka 532-0024 (JP)
(74) Representative: Maiwald GmbH
(86) International application number: PCT/JP2024/008311
(87) International publication number: WO 2024/185775

(57) **Abstract**

The present invention aims to provide a novel pseudo-solid support protecting group that can be used for oligonucleotide synthesis by a liquid-phase method. The present invention also aims to provide a method for synthesizing oligonucleotides using the novel pseudo-solid support protecting group.

According to the present invention, there is provided a compound represented by the following formula (1): 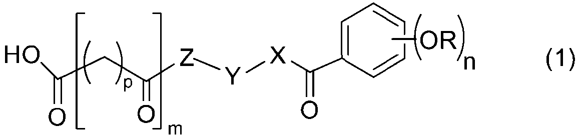 (wherein R represents an alkenyl group having 14 to 60 carbon atoms, n represents 2 or 3, m represents 0 or 1, p represents 1 or 2, X represents C, N, O, or S, Y represents a single bond, B(CH₂)ₜ* or may form a ring together with X, and Z represents a single bond or (CH₂)ₛ (s is an integer of 1 to 5)). The present invention also provides a method for producing oligonucleotides using the compound.

## Description

### [Technical Field]

The present invention relates to a long-chain alkenyloxy-substituted benzoyl derivative, which is a pseudo-solid-phase protecting group used in the synthesis of oligonucleotides by liquid-phase synthesis, an oligonucleotide to which the derivative is bound, and a method for synthesizing oligonucleotides using said derivative.

### [Background Art]

In recent years, oligonucleotides such as DNA probes, siRNA, antisense DNA, and antisense RNA have been actively used in the field of biotechnology. As chemical synthesis methods for oligonucleotides, the phosphoramidite method, H-phosphonate method, and the like are known, and currently, the solid-phase method using the phosphoramidite method is widely used. The solid-phase method has been process-optimized and automated, and is advantageous in terms of speed, but has the disadvantage of being limited in terms of scaling-up. In addition, a method for producing oligonucleotides using a liquid-phase method has also been considered, but the liquid-phase method requires complicated operations and has a low yield, making it difficult to synthesize oligonucleotides on a large scale and in a rapid manner.

Accordingly, in an attempt to overcome the respective shortcomings of the solid-phase method and the liquid-phase method, methods using hydrophobic group-bonded nucleosides (Patent Document 1: JP-A No. 2010-275254) and liquid-phase methods using pseudo-solid-phase protecting groups, wherein nucleosides or oligonucleotides in which the hydroxyl group at the 3'-position is protected with a specific organic group (Patent Documents 2 to 10: WO 2012/157723 (Ajinomoto Co., Inc.), WO 2013/122236 (Ajinomoto Co., Inc.), WO 2017/104836 (Ajinomoto Co., Inc.), WO 2013/179412 (Hokkaido System Science Co., Ltd.), WO 2014/077292 (Takeda Pharmaceutical Co., Ltd.), WO 2017/086397 (Nissan Chemical Corporation), WO 2018/203574 (Nissan Chemical Corporation), JP-A No. 2020-11932 (Fujimoto Chemicals Co., Ltd.), WO 2020/227618 (Biogen Inc. and Ajinomoto Co., Inc.), etc., have been proposed, but in liquid-phase methods using pseudo-solid-phase protecting groups, there are still issues to be addressed in terms of omitting the purification step in each elongation reaction step as much as possible and quickly performing the subsequent elongation reaction.

### [Prior Art Documents]

### [Patent Documents]

[Patent Document 1] JP-A No. 2010-275254
[Patent Document 2] WO 2012/157723
[Patent Document 3] WO 2013/122236
[Patent Document 4] WO 2017/104836
[Patent Document 5] WO 2013/179412
[Patent Document 6] WO 2014/077292
[Patent Document 7] WO 2017/086397
[Patent Document 8] WO 2018/203574
[Patent Document 9] JP-A 2020-11932
[Patent Document 10] WO 2020/227618

### [Summary of the Invention]

### [Technical Problem]

An object of the present invention is to provide a novel pseudo-solid-phase protecting group that can be used in oligonucleotide synthesis by a liquid-phase method. Another object of the present invention is to provide a method for synthesizing oligonucleotides using the novel pseudo-solid-phase protecting group.

### [Solution to Problem]

As a result of intensive research aimed at solving the problems, the present inventors have found a pseudo-solid-phase protecting group characterized by having a benzoyl group substituted with an alkenyloxy group on the benzene ring and having a carboxy group at the terminal, and have completed the present invention. The present invention includes the following:
[1] A compound represented by the following formula (1) or a salt thereof: 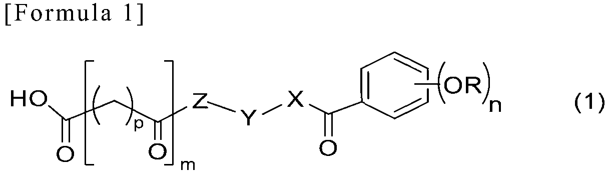 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   X is C, N, O or S, and when X is C, it represents CH₂ or may form, together with Y, a 5- to 7-membered carbocyclic or heterocyclic ring which may have a substituent, and when X is N, it represents N R₁ or may form, together with Y, a 5- to 7-membered heterocyclic ring which may have a substituent, wherein R₁ is H or a C1-6 alkyl group;
   Y is a single bond or B(CH₂)ₜ* (wherein B is a 5- to 7-membered carbocyclic ring which may have a substituent, t is 0, 1, or 2, and * indicates a bond to X), or may form, together with X, a 5- to 7-membered carbocyclic or heterocyclic ring which may have a substituent;
   Z is a single bond or (CH₂)_{S} (wherein s is an integer of 1 to 5, preferably an integer of 1 to 3), provided that when Y is a single bond, Z is not a single bond.).
[2] The compound or a salt thereof according to [1], which is represented by the following formula (2): 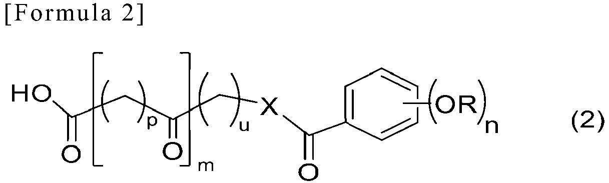 (In the formula,
   R each independently represents an optionally substituted alkenyl group having 14 to 60 carbon atoms (preferably having 14 to 50 carbon atoms, more preferably having 10 to 40 carbon atoms, and still more preferably having 14 to 30 carbon atoms);
   n represents 2 or 3, and the plurality of OR groups may be the same or different;
   m represents 0 or 1;
   p represents 1 or 2;
   u represents an integer of 1 to 5 (preferably an integer of 1 to 3);
   X represents NR₁, O or S, wherein R₁ is H or a C1-6 alkyl group.).
[3] The compound or a salt thereof according to the [2], wherein in the formula (2), m is 0 and X represents NR₁, O or S (wherein R₁ is H or a C1-6 alkyl group).
[4] The compound or a salt thereof according to [2], wherein in the formula (2), m is 1 and X represents NR₁, O or S (wherein R₁ is H or a C1-6 alkyl group).
[5] The compound or a salt thereof according to any one of [2] to [4], wherein in the formula (2), the OR groups on the benzene ring are in a 3,4,5-substitution, 2,4-substitution, or 2,3,4-substitution.
[6] The compound or a salt thereof according to [1], which is represented by the following formula (3): 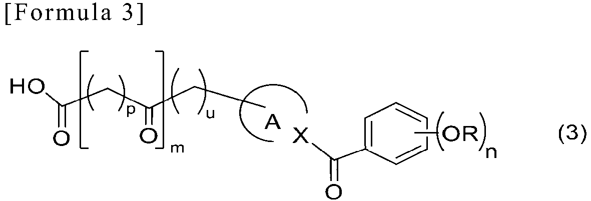 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   u represents 0 or an integer of 1 to 5 (preferably 0 or an integer of 1 to 3, more preferably 0, 1, or 2);
   ring A represents a 5- to 7-membered carbocyclic or heterocyclic ring which may have a substituent;
   X represents C or N.).
[7] The compound or a salt thereof according to [6], wherein in the formula (3), m is 0, u is 0 or an integer of 1 to 3 (preferably 0, 1, or 2), and X represents C.
[8] The compound or a salt thereof according to [7], wherein in the formula (3), ring A is benzene or cyclohexane which may have a substituent, and the substituent is a group selected from the group consisting of an optionally substituted hydroxyl group, an optionally substituted amino group, an optionally substituted alkyl group, a halogen atom, a nitro group, and a carbonyl group.
[9] The compound or a salt thereof according to [6], wherein in the formula (3), u is 0, 1, or 2, X represents N, and ring A containing X represents a 5- to 7-membered heterocyclic ring containing one nitrogen atom which may have a substituent, and the substituent is a group selected from the group consisting of an optionally substituted hydroxyl group, an optionally substituted amino group, an optionally substituted alkyl group, a halogen atom, a nitro group, and an optionally substituted carbonyl group.
[10] The compound or a salt thereof according to [9], wherein in the formula (3), m is 0.
[11] The compound or a salt thereof according to [9], wherein in the formula (3), ring A is pyrrolidine or piperidine which may have a substituent, and the substituent is a group selected from the group consisting of an optionally substituted hydroxyl group, an optionally substituted alkyl group, and an optionally substituted carbonyl group.
[12] The compound or a salt thereof according to any one of [6] to [11], wherein in the formula (3), the OR groups on the benzene ring are in a 3,4,5-substitution, 2,4-substitution, or 2,3,4-substitution.
[13] The compound or a salt thereof according to [1], which is represented by the following formula (4): 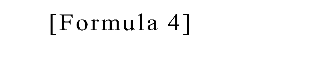 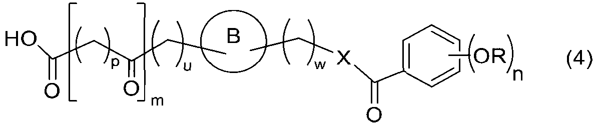 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   u represents 0 or an integer of 1 to 5 (preferably 0 or an integer of 1 to 3, more preferably 0, 1, or 2);
   w represents 0, 1, or 2;
   ring B represents a carbocyclic ring having 5 to 7 carbon atoms which may have a substituent;
   X represents NR₁, O or S, wherein R₁ is H or CH₃.).
[14] The compound or a salt thereof according to [13], wherein in the formula (4), m is 0.
[15] The compound or a salt thereof according to [13], wherein in the formula (4), ring B is benzene or cyclohexane which may have a substituent, and the substituent is a group selected from the group consisting of an optionally substituted hydroxyl group, an optionally substituted amino group, an optionally substituted alkyl group, a halogen atom, a nitro group, and an optionally substituted carbonyl group.
[16] The compound or a salt thereof according to any one of [13] to [15], wherein in the formula (3), the OR groups on the benzene ring are in a 3,4,5-substitution, 2,4-substitution, or 2,3,4-substitution.
[17] The compound or a salt thereof according to [1], wherein the compound represented by the formula (1) is a compound selected from the group consisting of compounds represented by the following formulae: 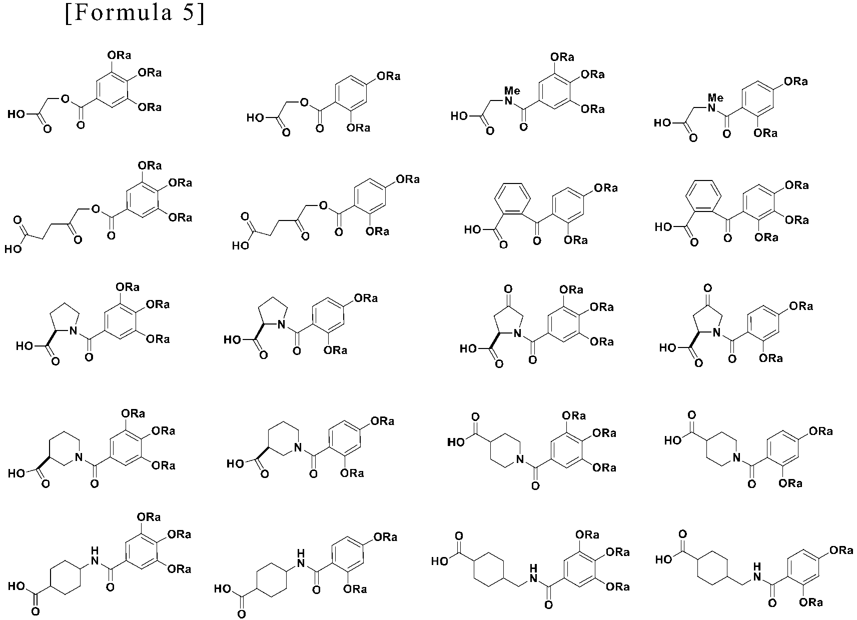 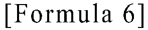 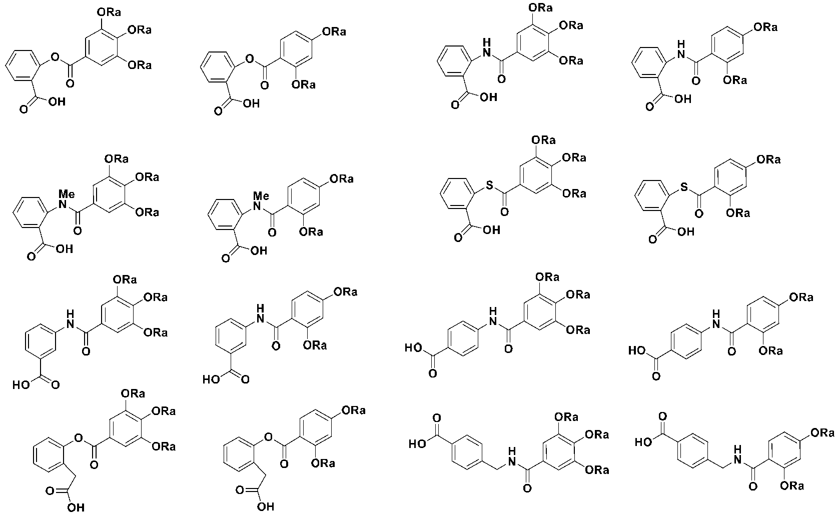 (wherein Ra each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms), and the plurality of ORa groups may be the same or different.).
[18] The compound or a salt thereof according to [17], wherein the Ra each independently represents an unsaturated hydrocarbon group derived from a compound selected from the group consisting of myristoleic acid (C14), palmitoleic acid (C16), sapienic acid (C16), oleic acid (C18), elaidic acid (C18), vaccenic acid (C18), gadoleic acid (C20), eicosenoic acid (C20), erucic acid (C22), nervonic acid (C24), linoleic acid (C18), eicosadienoic acid (C20), docosadienoic acid (C22), α- and γ-linolenic acids (C18), pinoleic acid (C18), eleostearic acid (C18), mead acid (C20), di-homo-γ-linolenic acid (C20), eicosatrienoic acid (C20), stearidonic acid (C18), arachidonic acid (C20), eicosatetraenoic acid (C20), adrenic acid (C22), bosseopentaenoic acid (C18), eicosapentaenoic acid (C20), osbond acid (C22), clupanodonic acid (C22), tetracosapentaenoic acid (C24), docosahexaenoic acid (C22), and nisinic acid (C24).
[19] A nucleotide represented by the following formula (I): 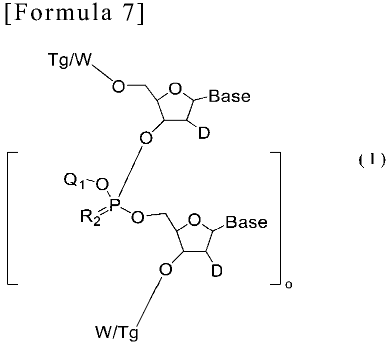 [In the formula,
   o represents any integer of 0 or more (preferably an integer of 1 to 50);
   o+1 Base each independently represents an optionally modified nucleic acid base;
   o+1 D each independently represents a hydrogen atom, a halogen atom, or a hydroxyl group which may be protected, or may form a crosslink with the 4'-position carbon (preferably a crosslink of a Locked Nucleic Acid (LNA), an amide-bridged nucleic acid, a guanidine-bridged nucleic acid, or a spirocyclopropylene-bridged nucleic acid);
   o Q₁ each independently represents a C1-4 alkyl group having an electron-withdrawing group (e.g., a cyano group, a nitro group, a 2-pyridyl group, a 4-pyridyl group, etc.), an allyl group, a C2-6 alkenyl group or a C6-10 aryl group having a cyano group, a nitro group or a halogen group, or a hydrogen atom;
   W/Tg or Tg/W represents W or Tg, and when the 5' side is W, the 3' side is Tg, whereas when the 5' side is Tg, the 3' side is W, wherein
   W represents a hydrogen atom or a temporary protecting group removable under acidic conditions or with a reagent containing a fluorine anion;
   o R₂ each independently represents O or S; and
   Tg represents a group represented by the following formula (5): 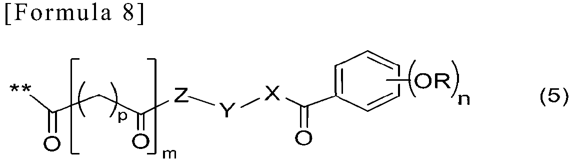 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   X is C, N, O or S, and when X is C, it represents CH₂ or may be combined with Y to form a 5- to 7-membered carbocyclic or heterocyclic ring which may have a substituent, and when X is N, it represents NR₁ or may form, together with Y, a 5- to 7-membered heterocyclic ring which may have a substituent, wherein R₁ is H or a C1-6 alkyl group;
   Y is a single bond or B(CH₂)ₜ* (wherein B is a 5- to 7-membered carbocyclic ring which may have a substituent, t is 0, 1, or 2, and * indicates a bond to X), or may form, together with X, a 5- to 7-membered carbocyclic or heterocyclic ring which may have a substituent;
   Z is a single bond or (CH₂)_{S}, wherein s is an integer of 1 to 5 (preferably an integer of 1 to 3), provided that when Y is a single bond, Z is not a single bond,
   ** indicates a bond to O.)], or
   a nucleotide represented by the following formula (II): 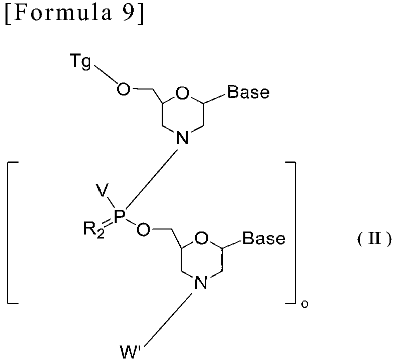 [In the formula,
   o represents any integer of 0 or more (preferably an integer of 1 to 50);
   o+1 Base each independently represents an optionally modified nucleic acid base;
   o V each independently represents a C1-6 alkoxy group, a di(C1-6 alkyl)amino group, or a piperazino group in which the 4-position nitrogen is protected with a protecting group and which may be further substituted;
   W' represents a hydrogen atom or a temporary protecting group removable under acidic conditions or with a reagent containing a fluorine anion;
   o R₂ each independently represents O or S;
   Tg represents a group represented by the following formula (5): 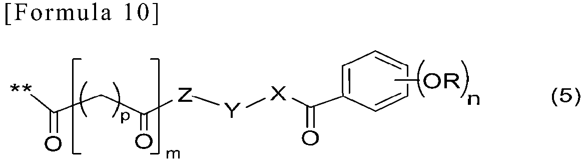 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   X is C, N, O or S, and when X is C, it represents CH₂ or may form, together with Y, a 5- to 7-membered carbocyclic or heterocyclic ring which may have a substituent, and when X is N, it represents NR₁ or may form, together with Y, a 5- to 7-membered heterocyclic ring which may have a substituent, wherein R₁ is H or a C1-6 alkyl group;
   Y is be a single bond or B(CH₂)ₜ* (wherein B is a 5- to 7-membered carbocyclic ring which may have a substituent, t is 0, 1, or 2, and * indicates a bond to X), or may form, together with X, a 5- to 7-membered carbocyclic or heterocyclic ring which may have a substituent;
   Z is a single bond or (CH₂)_{S}, wherein s is an integer of 1 to 5 (preferably an integer of 1 to 3), provided that when Y is a single bond, Z is not a single bond,
   ** indicates a bond to O.)].
[20] The nucleotide according to [19], wherein Tg in the formula (I) or formula (II) represents a group represented by the following formula (6): 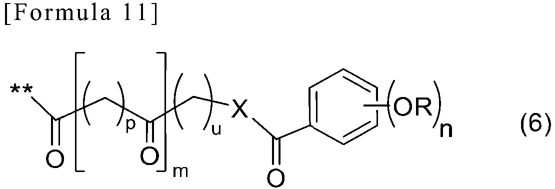 (In the formula,
   R each independently represents an optionally substituted alkenyl group having 14 to 60 carbon atoms (preferably having 14 to 50 carbon atoms, more preferably having 10 to 40 carbon atoms, and still more preferably having 14 to 30 carbon atoms);
   n represents 2 or 3, and the plurality of OR groups may be the same or different;
   m represents 0 or 1;
   p represents 1 or 2;
   u represents an integer of 1 to 5 (preferably an integer of 1 to 3);
   X represents NR₁, O or S, wherein R₁ is H or a C1-6 alkyl group;
   ** indicates a bond to O.),
   a group represented by the following formula (7): 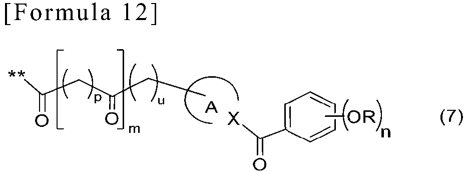 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   u represents 0 or an integer of 1 to 5 (preferably 0 or an integer of 1 to 3, more preferably 0, 1, or 2);
   ring A represents a 5- to 7-membered ring which may have a substituent;
   X represents C or N;
   ** indicates a bond to O.), or
   a group represented by the following formula (8): 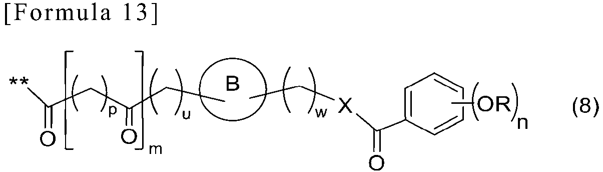 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   u represents 0 or an integer of 1 to 5 (preferably 0 or an integer of 1 to 3, more preferably 0, 1, or 2);
   w represents 0, 1, or 2;
   ring B is a carbocyclic ring having 5 to 7 carbon atoms which may have a substituent;
   X represents NR₁, O or S, wherein R₁ is H or CH₃;
   ** indicates a bond to O.).
[21] A nucleotide represented by the following formula (V) or formula (VI): 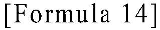 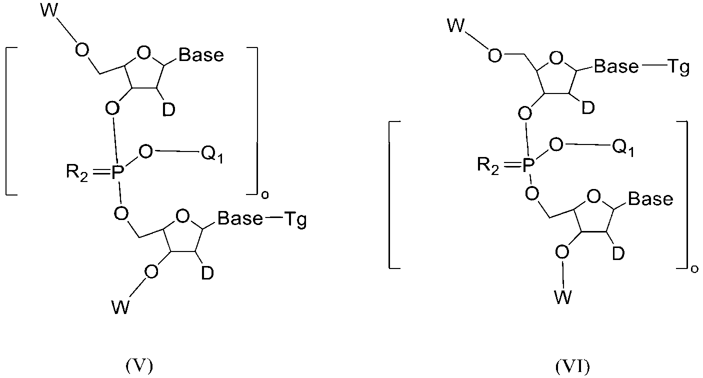 [In the formula,
   o represents any integer of 0 or more (preferably an integer of 1 to 50);
   o+1 Base each independently represent an optionally modified nucleic acid base;
   o+1 D groups each independently represent a hydrogen atom, a halogen atom, or a hydroxyl group which may be protected, or may form a crosslink with the 4'-position carbon (preferably a crosslink of a Locked Nucleic Acid (LNA), an amide-bridged nucleic acid, a guanidine-bridged nucleic acid, or a spirocyclopropylene-bridged nucleic acid);
   o Q₁ groups each independently represent a C1-4 alkyl group having an electron-withdrawing group (e.g., a cyano group, a nitro group, a 2-pyridyl group, a 4-pyridyl group, etc.), an allyl group, a C2-6 alkenyl group or a C6-10 aryl group having a cyano group, a nitro group, or a halogen group, or a hydrogen atom;
   W groups each independently represent a hydrogen atom or a temporary protecting group removable under acidic conditions or with a reagent containing a fluorine anion;
   o R₂ groups each independently represent O or S;
   Tg represents a group represented by the following formula (5): 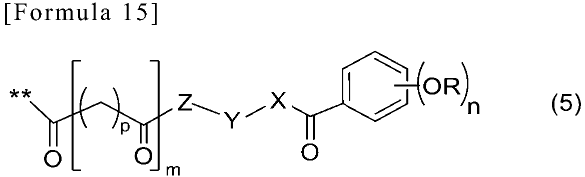 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   X is C, N, O or S, and when X is C, it represents CH₂ or may form, together with Y, a 5- to 7-membered carbocyclic or heterocyclic ring which may have a substituent, and when X is N, it represents NR₁ or may form, together with Y, a 5- to 7-membered heterocyclic ring which may have a substituent, wherein R₁ is H or a C1-6 alkyl group;
   Y is a single bond or B(CH₂)ₜ* (wherein B is a 5- to 7-membered carbocyclic ring which may have a substituent, t is 0, 1, or 2, and * indicates a bond to X), or may form, together with X, a 5- to 7-membered carbocyclic or heterocyclic ring which may have a substituent;
   Z is a single bond or (CH₂)_{S}, wherein s is an integer of 1 to 5 (preferably an integer of 1 to 3), provided that when Y is a single bond, Z is not a single bond,
   ** indicates a bond to Base.], or
   a nucleotide represented by the following formula (VII) or formula (VIII): 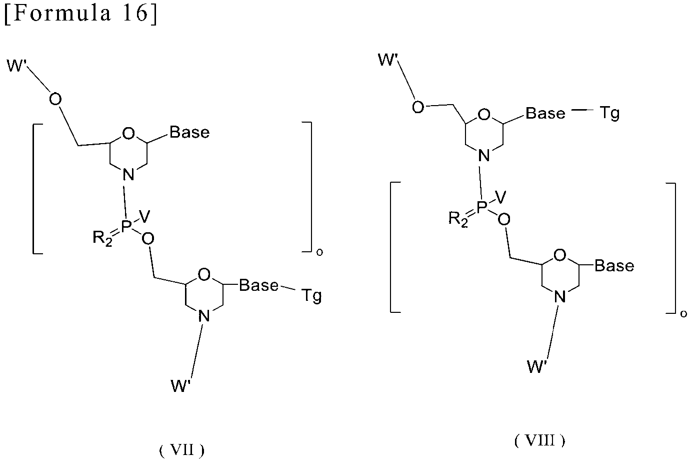 [In the formula,
   o represents any integer of 0 or more (preferably an integer of 1 to 50);
   o+1 Base each independently represents an optionally modified nucleic acid base;
   o V each independently represents a C1-6 alkoxy group, a di(C1-6 alkyl)amino group, or a piperazino group in which the 4-position nitrogen is protected with a protecting group and which may be further substituted;
   W' each independently represents a hydrogen atom or a temporary protecting group removable under acidic conditions or with a reagent containing a fluorine anion;
   o R₂ each independently represents O or S;
   Tg represents a group represented by the following formula (5): 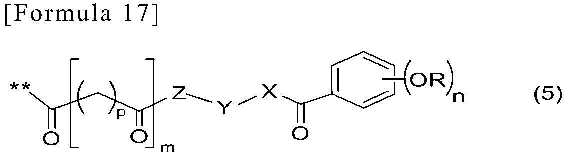
   (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   X is C, N, O or S, and when X is C, it represents CH₂ or may form, together with Y, a 5- to 7-membered carbocyclic or heterocyclic ring which may have a substituent, and when X is N, it represents NR₁ or may form, together with Y, a 5- to 7-membered heterocyclic ring which may have a substituent, wherein R₁ is H or a C1-6 alkyl group;
   Y is a single bond or B(CH₂)ₜ* (wherein B is a 5- to 7-membered carbocyclic ring which may have a substituent, t is 0, 1, or 2, and * indicates a bond to X), or may form, together with X, a 5- to 7-membered carbocyclic or heterocyclic ring which may have a substituent;
   Z is a single bond or (CH₂)_{S}, wherein s is an integer of 1 to 5 (preferably an integer of 1 to 3), provided that when Y is a single bond, Z is not a single bond,
   ** indicates a bond to Base.)].
[22] The nucleotide according to [21], wherein Tg in the formula (V), the formula (VI), the formula (VII) or the formula (VIII) represents a group represented by the following formula (6): 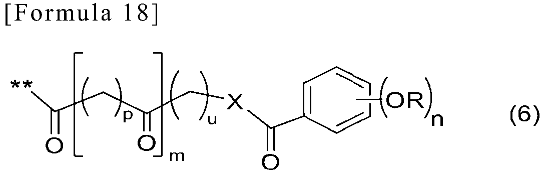 (In the formula,
   R each independently represents an optionally substituted alkenyl group having 14 to 60 carbon atoms (preferably having 14 to 50 carbon atoms, more preferably having 10 to 40 carbon atoms, and still more preferably having 14 to 30 carbon atoms);
   n represents 2 or 3, and the plurality of OR groups may be the same or different;
   m represents 0 or 1;
   p represents 1 or 2;
   u represents an integer of 1 to 5 (preferably an integer of 1 to 3);
   X represents NR₁, O or S, wherein R₁ is H or a C1-6 alkyl group;
   ** indicates a bond to Base.),
   a group represented by the following formula (7): 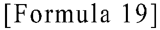 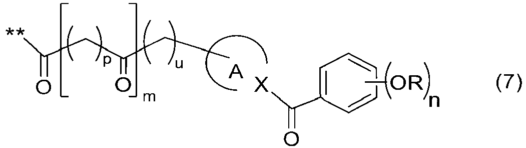 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   u represents 0 or an integer of 1 to 5 (preferably 0 or an integer of 1 to 3, more preferably 0, 1, or 2);
   ring A represents a 5- to 7-membered ring which may have a substituent;
   X represents C or N;
   ** indicates a bond to Base.), or
   a group represented by the following formula (8): 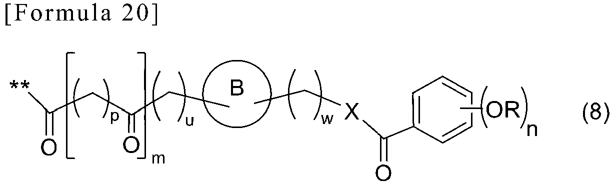 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   u represents 0 or an integer of 1 to 5 (preferably 0 or an integer of 1 to 3, more preferably 0, 1, or 2);
   w represents 0, 1, or 2;
   ring B represents a carbocyclic ring having 5 to 7 carbon atoms which may have a substituent;
   X represents NR₁, O or S, where R₁ is H or CH₃;
   ** indicates a bond to Base.).
[23] The nucleotide according to any one of [19] to [22], wherein the temporary protecting group removable under acidic conditions in the formula (I), the formula (II), the formula (V), the formula (VI), the formula (VII) or the formula (VIII) is a group selected from the group consisting of a trityl group, a 9-(9-phenyl)xanthenyl group, a 9-phenylthioxanthenyl group, a di(C1-6 alkoxy)trityl group, a mono(C1-18 alkoxy)trityl group, and a silyl group substituted with an alkyl or allyl group, and the temporary protecting group removable with a reagent containing a fluorine anion is a silyl group substituted with an alkyl or allyl group, and the temporary protecting group removable under acidic conditions in the formula (II) is a group selected from the group consisting of a trityl group, a 9-(9-phenyl)xanthenyl group, a 9-phenylthioxanthenyl group, a di(C1-6 alkoxy)trityl group, a mono(C1-18 alkoxy)trityl group, and a silyl group substituted with an alkyl or allyl group;
   wherein examples of the silyl group substituted with an alkyl or allyl group include trimethylsilyl, triethylsilyl, triisopropylsilyl (TIPS), dimethylisopropylsilyl, diethylisopropylsilyl, dimethylthexylsilyl, t-butyldimethylsilyl (TBS or TBDMS), t-butyldiphenylsilyl (TBDPS), tribenzylsilyl, tri-p-xylylsilyl, triphenylsilyl, diphenylmethylsilyl, di-t-butylmethylsilyltri(trimethylsilyl)silyl, t-butylmethoxyphenylsilyl, t-butoxydiphenylsilyl, TBoDPS, and TBDAS.
[24] The nucleotide according to [23], wherein Base in the formula (I), (II), (V), (VI), (VII) or (VIII) is a pyrimidine base or a purine base which may be substituted by 1 to 3 substituents selected from the group consisting of a halogen atom, an alkyl group, an aralkyl group, an alkoxy group, an acyl group, an alkoxyalkyl group, a hydroxyl group, an amino group, a monoalkylamino group, a dialkylamino group, a carboxy group, a cyano group and a nitro group.
[25] The nucleotide according to any one of [19] to [22], wherein D in the formula (I), (V) or (VI) is a hydroxyl group which may be protected by a methyl group, a benzyl group, a p-methoxybenzyl group, a tert-butyl group, a methoxymethyl group, a methoxyethyl group, a 2-tetrahydropyranyl group, an ethoxyethyl group, a cyanoethyl group, a cyanoethoxymethyl group, a phenylcarbamoyl group, a 1,1-dioxothiomorpholine-4-thiocarbamoyl group, an acetyl group, a pivaloyl group, a benzoyl group, a trimethylsilyl group, a triethylsilyl group, a triisopropylsilyl group, a tert-butyldimethylsilyl group (TBS), a [(triisopropylsilyl)oxy]methyl group or a 1-(4-chlorophenyl)-4-ethoxypiperidin-4-yl group.
[26] A method for producing an oligonucleotide, comprising the following steps (A1)-(A3):
   (A1) a step of reacting, in a nonpolar or polar solvent, an n-mer oligonucleotide (wherein n is any integer of 1 or more) in which the 3'- or 5'-position hydroxyl group is protected with a support protecting group and the 5'- or 3'-position hydroxyl group is protected with a temporary protecting group removable under acidic conditions or with a reagent containing a fluorine anion, with an acid and a cation scavenger, or with a reagent containing a fluorine anion (preferably pyridine hydrofluoride or tetrabutylammonium fluoride) to remove the temporary protecting group at the 5'- or 3'-position hydroxyl group, and then separating the n-mer oligonucleotide from impurities in the reaction solution;
   (A2) a step of adding a p-mer oligonucleotide (p is any integer of 1 or more, preferably 1) in which the 3'-position hydroxyl group has been phosphoramiditized and the 5'-position hydroxyl group has been protected with a temporary protecting group removable under acidic conditions or with a reagent containing a fluorine anion, or in which the 5'-position hydroxyl group has been phosphoramiditized and the 3'-position hydroxyl group has been protected with a temporary protecting group removable under acidic conditions or with a reagent containing a fluorine anion, to a solution of the n-mer oligonucleotide obtained in step (A1) from which the temporary protecting group at the 5' -position hydroxyl group or the 3'-position hydroxyl group has been removed, thereby condensing the n-mer oligonucleotide and the p-mer oligonucleotide via the 5'-position hydroxyl group or the 3'-position hydroxyl group through a phosphite triester bond; and
   (A3) a step of converting the phosphite triester bond of the n+p-mer oligonucleotide obtained in step (A2) into a phosphate triester bond or a thiophosphate triester bond using an oxidizing agent or a sulfurizing agent;
   wherein the support protecting group is a group represented by the following formula (5): 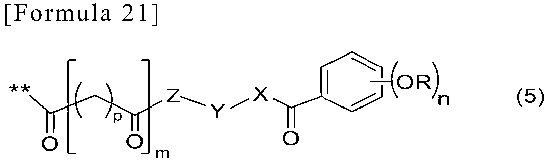 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   X is C, N, O or S, and when X is C, it represents CH₂ or may form, together with Y, a 5- to 7-membered carbocyclic or heterocyclic ring which may have a substituent, and when X is N, it represents NR₁ or may form, together with Y, a 5- to 7-membered heterocyclic ring which may have a substituent, wherein R₁ is H or a C1-6 alkyl group;
   Y is a single bond or B(CH₂)ₜ* (wherein B is a 5- to 7-membered carbocyclic ring which may have a substituent, t is 0, 1, or 2, and * indicates a bond to X), or may form, together with X, a 5- to 7-membered carbocyclic or heterocyclic ring which may have a substituent;
   Z is a single bond or (CH₂)_{S}, wherein s is an integer of 1 to 5 (preferably an integer of 1 to 3), provided that when Y is a single bond, Z is not a single bond;
   ** indicates a bond to the 3'- or 5'-position hydroxyl group.).
[27] The method for producing an oligonucleotide according to [26], wherein the support protecting group is a group represented by the following formula (6): 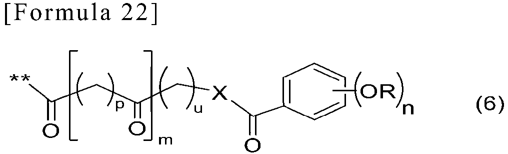 (In the formula,
   R each independently represents an optionally substituted alkenyl group having 14 to 60 carbon atoms (preferably having 14 to 50 carbon atoms, more preferably having 10 to 40 carbon atoms, and still more preferably having 14 to 30 carbon atoms);
   n represents 2 or 3, and the plurality of OR groups may be the same or different;
   m represents 0 or 1;
   p represents 1 or 2;
   u represents an integer of 1 to 5 (preferably an integer of 1 to 3);
   X represents NR₁, O or S, wherein R₁ is H or a C1-6 alkyl group;
   ** indicates a bond to the 3'- or 5'-position hydroxyl group);
   a group represented by the following formula (7): 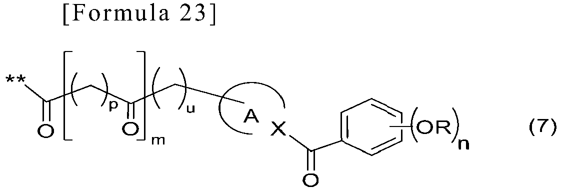 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   u represents 0 or an integer from 1 to 5 (preferably 0 or an integer from 1 to 3, more preferably 0, 1, or 2);
   ring A represents a 5- to 7-membered ring which may have a substituent;
   X represents C or N;
   ** indicates a bond to the 3'- or 5'-position hydroxyl group.); or
   a group represented by the following formula (8): 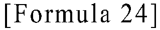 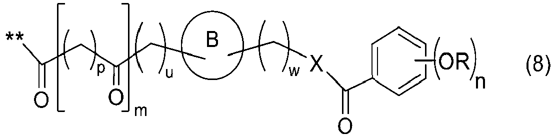 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   u represents 0 or an integer of 1 to 5 (preferably 0 or an integer of 1 to 3, more preferably 0, 1, or 2);
   w represents 0, 1, or 2);
   ring B represents a carbocyclic ring having 5 to 7 carbon atoms which may have a substituent;
   X represents NR₁, O or S, wherein R₁ is H or CH₃;
   ** indicates a bond to the 3'- or 5'-position hydroxyl group.).
[28] The method for producing an oligonucleotide according to [26] or [27], wherein in the step (A1), the step of separating the n-mer oligonucleotide from which the temporary protecting group has been removed from impurities in the reaction solution comprises neutralizing the reaction solution with a base, and then
   (i) a step of isolating the n-mer oligonucleotide from which the temporary protecting group has been removed, or (ii) a step of washing the neutralized reaction solution with water to recover an organic layer.
[29] The method according to [26] or [27], further comprising the following step (A4):
   (A4) a step of adding a polar solvent to the reaction solution obtained in the step (A1) and/or (A3), to precipitate the n+p-mer oligonucleotide, and obtaining it by solid-liquid separation.
[30] The method according to [29], further comprising the following step (A5):
   (A5) a step of removing all protecting groups from the n+p-mer oligonucleotide obtained in the step (A4).
[31] A method for producing a nucleotide, comprising a step of subjecting a nucleotide represented by the following formula (I): 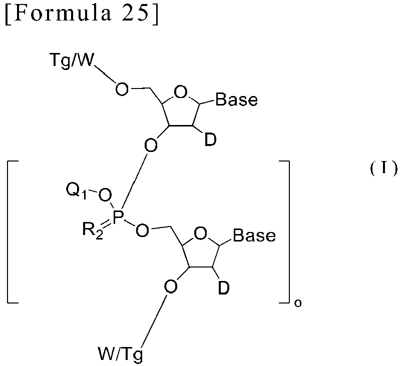 [In the formula,
   o represents any integer of 0 or more (preferably an integer of 1 to 50);
   o+1 Base each independently represents an optionally modified nucleic acid base;
   o+1 D each independently represents a hydrogen atom, a halogen atom, or a hydroxyl group which may be protected, or may form a crosslink with the 4'-position carbon (preferably a crosslink of a Locked Nucleic Acid (LNA), an amide-bridged nucleic acid, a guanidine-bridged nucleic acid, or a spirocyclopropylene-bridged nucleic acid);
   o Q₁ each independently represents a C1-4 alkyl group having an electron-withdrawing group (e.g., a cyano group, a nitro group, a 2-pyridyl group, a 4-pyridyl group, etc.), an allyl group, a C2-6 alkenyl group or a C6-10 aryl group having a cyano group, a nitro group or a halogen group, or a hydrogen atom;
   W/Tg or Tg/W represents W or Tg, and when the 5' side is W, the 3' side is Tg, whereas when the 5' side is Tg, the 3' side is W, wherein
   W represents a hydrogen atom or a temporary protecting group removable under acidic conditions or with a reagent containing a fluorine anion;
   o R₂ each independently represents O or S; and
   Tg represents a group represented by the following formula (5): 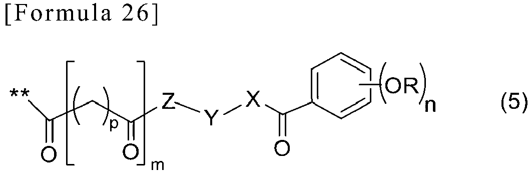 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   X is C, N, O or S, and when X is C, it represents CH₂ or may form, together with Y, a 5- to 7-membered carbocyclic or heterocyclic ring which may have a substituent, and when X is N, it represents NR₁ or may form, together with Y, a 5- to 7-membered heterocyclic ring which may have a substituent, wherein R₁ is H or a C1-6 alkyl group;
   Y is a single bond or B(CH₂)ₜ* (wherein B is a 5- to 7-membered carbocyclic ring which may have a substituent, t is 0, 1, or 2, and * indicates a bond to X), or may form, together with X, a 5- to 7-membered carbocyclic or heterocyclic ring which may have a substituent;
   Z is a single bond or (CH₂)ₛ, wherein s is an integer of 1 to 5 (preferably an integer of 1 to 3), provided that when Y is a single bond, Z is not a single bond;
   ** indicates a bond to O.)];
   or a nucleotide represented by the following formula (II): 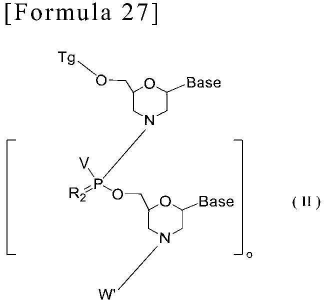 [In the formula,
   o represents any integer of 0 or more (preferably an integer of 1 to 50);
   o+1 Base each independently represents an optionally modified nucleic acid base;
   o V each independently represents an alkoxy group having 1 to 6 carbon atoms, a di(C 1-6 alkyl)amino group, or a piperazino group in which the 4-position nitrogen is protected with a protecting group and which may be further substituted;
   W' represents a hydrogen atom or a temporary protecting group removable under acidic conditions or with a reagent containing a fluorine anion;
   o R₂ each independently represents O or S;
   Tg represents a group represented by the following formula (5): 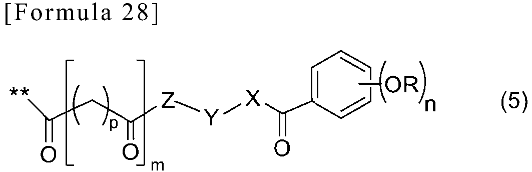 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   X is C, N, O or S, and when X is C, it represents CH₂ or may form, together with Y, a 5- to 7-membered carbocyclic or heterocyclic ring which may have a substituent, and when X is N, it represents N R₁ or may form, together with Y, a 5- to 7-membered heterocyclic ring which may have a substituent, wherein R₁ is H or a C1-6 alkyl group;
   Y is a single bond or B(CH₂)ₜ* (wherein B is a 5- to 7-membered carbocyclic ring which may have a substituent, t is 0, 1, or 2, and * indicates a bond to X), or may form, together with X, a 5- to 7-membered carbocyclic or heterocyclic ring which may have a substituent;
   Z is a single bond or (CH₂)_{S}, where s is an integer of 1 to 5 (preferably an integer of 1 to 3), provided that when Y is a single bond, Z is not a single bond;
   * indicates a bond to O.)]
   to reduction treatment, reaction with hydrazine or alkylhydrazine, or hydrolysis, to remove the protecting group represented by the formula (5), to obtain a nucleotide represented by the formula (III): 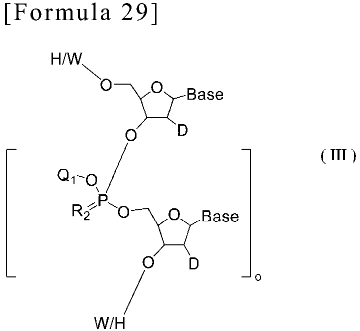 (In the formula, the definitions of Base, D, R₂, Q₁, and W are the same as those in the formula (I), and W/H represents W or hydrogen; when the 5' side is W, the 3' side is hydrogen, and when the 5' side is hydrogen, the 3' side is W.)
   or a nucleotide represented by the following formula (IV): 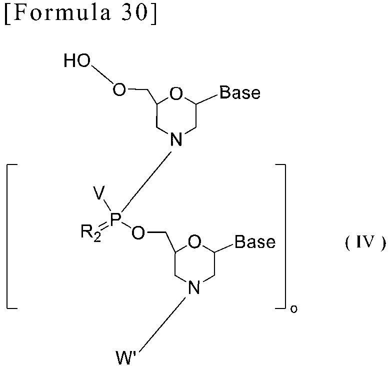 (In the formula, the definitions of Base, R₂, V, and W' are the same as those in the formula (II).).
[32] The production method according to [31], wherein the reduction treatment is performing using a boron-containing reducing agent (preferably lithium borohydride, sodium borohydride, lithium triethylborohydride, or tetrabutylammonium borohydride), or both a boron-containing reducing agent and an amine.
[33] A method for producing an oligonucleotide, comprising the following steps (B1) to (B3):
   (B1) a step of reacting, in a non-polar or polar solvent, an n-mer oligonucleotide (n is any integer of 1 or more) in which a support protecting group is bound to a nucleic acid base at the 3'-terminal or a nucleic acid base at the 5'-terminal and the 3'-position hydroxyl group and the 5'-position hydroxyl group are protected with a temporary protecting group removable under acidic conditions or with a reagent containing a fluorine anion, with an acid and a cation scavenger, or with a reagent containing a fluorine anion (preferably pyridine hydrofluoride or tetrabutylammonium fluoride) to remove the temporary protecting group at only one of the 5'-position hydroxyl group or the 3'-position hydroxyl group, and then separating the n-mer oligonucleotide from impurities in the reaction solution;
   (B2) a step of adding a p-mer oligonucleotide (p is any integer of 1 or more) in which the 3'-position hydroxyl group has been phosphoramiditized and the 5'-position hydroxyl group has been protected with a temporary protecting group removable under acidic conditions or with a reagent containing a fluorine anion, or in which the 5'-position hydroxyl group has been phosphoramiditized and the 3'-position hydroxyl group has been protected with a temporary protecting group removable under acidic conditions or with a reagent containing a fluorine anion, to a solution of the n-mer oligonucleotide obtained in step (B1) from which the temporary protecting group at the 5'-position hydroxyl group or the 3'-position hydroxyl group has been removed, thereby condensing the n-mer oligonucleotide and the p-mer oligonucleotide via the 5'-position hydroxyl group or the 3'-position hydroxyl group through a phosphite triester bond; and
   (B3) a step of converting the phosphite triester bond of the n+p-mer oligonucleotide obtained in step (B2) into a phosphate triester bond or a thiophosphate triester bond using an oxidizing agent or a sulfurizing agent;
   wherein the support protecting group is a group represented by the following formula (5): 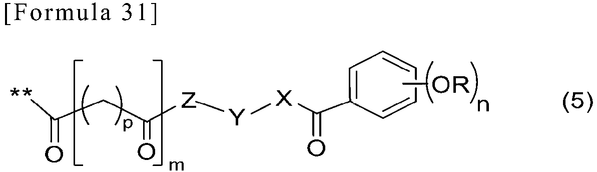 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   X is C, N, O or S, and when X is C, it represents CH₂ or may form, together with Y, a 5- to 7-membered carbocyclic or heterocyclic ring which may have a substituent, and when X is N, it represents NR₁ or may form, together with Y, a 5- to 7-membered heterocyclic ring which may have a substituent, wherein R₁ is H or a C1-6 alkyl group;
   Y is a single bond or B(CH₂)ₜ* (wherein B is a - to 7-membered carbocyclic ring which may have a substituent, t is 0, 1, or 2, and * indicates a bond to X), or may form, together with X, a 5- to 7-membered carbocyclic or heterocyclic ring which may have a substituent;
   Z is a single bond or (CH₂)_{S}, where s is an integer of 1 to 5 (preferably an integer of 1 to 3), provided that when Y is a single bond, Z is not a single bond;
   ** indicates a bond to a nucleic acid base.).
[34] The method for producing an oligonucleotide according to [33],
   wherein the support protecting group is a group represented by the following formula (6): 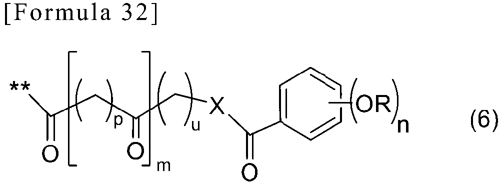 (In the formula,
   R each independently represents an optionally substituted alkenyl group having 14 to 60 carbon atoms (preferably having 14 to 50 carbon atoms, more preferably having 10 to 40 carbon atoms, and still more preferably having 14 to 30 carbon atoms);
   n represents 2 or 3, and the plurality of OR groups may be the same or different;
   m represents 0 or 1;
   p represents 1 or 2;
   u represents an integer of 1 to 5 (preferably an integer of 1 to 3);
   X represents NR₁, O or S, wherein R₁ is H or a C1-6 alkyl group;
   ** indicates a bond to a nucleic acid base.),
   a group represented by the following formula (7): 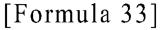 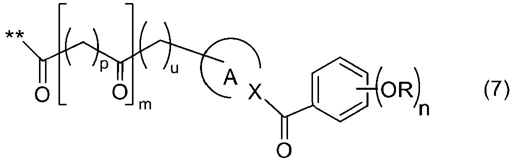 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   u represents 0 or an integer from 1 to 5 (preferably 0 or an integer from 1 to 3, more preferably 0, 1, or 2);
   ring A represents a 5- to 7-membered ring which may have a substituent;
   X represents C or N;
   ** indicates a bond to a nucleic acid base.),
   or a group represented by the following formula (8): 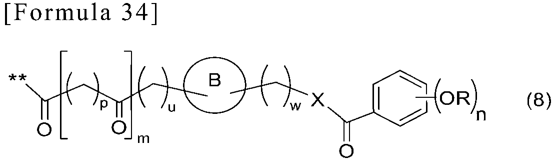 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   u represents 0 or an integer of 1 to 5 (preferably 0 or an integer of 1 to 3, more preferably 0, 1, or 2);
   w represents 0, 1, or 2);
   ring B represents a carbocyclic ring having 5 to 7 carbon atoms which may have a substituent;
   X represents NR₁, O or S, where R₁ is H or CH₃;
   ** indicates a bond to a nucleic acid base.).
[35] The method for producing an oligonucleotide according to [33] or [34], wherein in the step (B1), the step of separating the n-mer oligonucleotide from which the temporary protecting group has been removed from impurities in the reaction solution comprises neutralizing the reaction solution with a base, and then
   (i) a step of isolating the n-mer oligonucleotide from which the temporary protecting group has been removed, or (ii) a step of washing the neutralized reaction solution with water to recover an organic layer.
[36] The method according to [33] or [34], further comprising the following step (B4):
   (B4) a step of adding a polar solvent to the reaction solution obtained in the step (B1) and/or (B3) to precipitate the n+p-mer oligonucleotide, and obtaining it by solid-liquid separation.
[37] The method according to [36], further comprising the following step (B5):
   (B5) a step of removing all protecting groups from the n+p-mer oligonucleotide obtained in the step (B4).
[38] A method for producing a nucleotide, comprising a step of subjecting a nucleotide represented by the following formula (V) or the formula (VI): 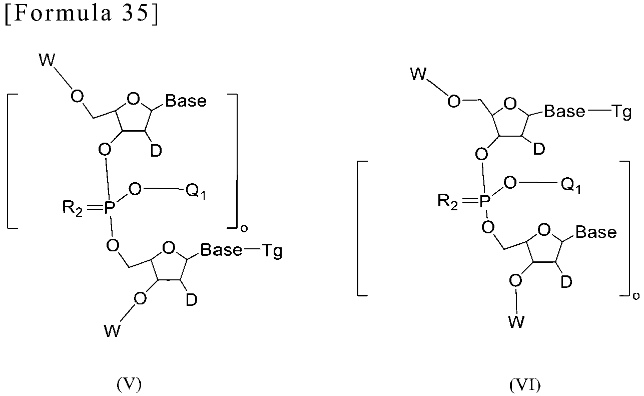 [In the formula,
   o represents any integer of 0 or more (preferably an integer of 1 to 50);
   o+1 Base each independently represents an optionally modified nucleic acid base;
   o+1 D each independently represents a hydrogen atom, a halogen atom, or a hydroxyl group which may be protected, or may form a crosslink with the 4'-position carbon (preferably a crosslink of a Locked Nucleic Acid (LNA), an amide-bridged nucleic acid, a guanidine-bridged nucleic acid, or a spirocyclopropylene-bridged nucleic acid);
   o Q₁ each independently represents a C1-4 alkyl group having an electron-withdrawing group (e.g., a cyano group, a nitro group, a 2-pyridyl group, a 4-pyridyl group, etc.), an allyl group, a C2-6 alkenyl group or a C6-10 aryl group having a cyano group, a nitro group, or a halogen group, or a hydrogen atom;
   W each independently represents a hydrogen atom or a temporary protecting group removable under acidic conditions or with a reagent containing a fluorine anion;
   o R₂ each independently represents O or S; and
   Tg represents a group represented by the following formula (5): 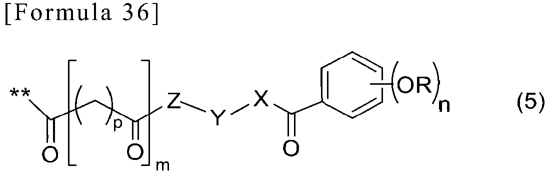 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   X is C, N, O or S, and when X is C, it represents CH₂ or may form, together with Y, a 5- to 7-membered carbocyclic or heterocyclic ring which may have a substituent, and when X is N, it represents NR₁ or may form, together with Y, a 5- to 7-membered heterocyclic ring which may have a substituent, wherein R₁ is H or a C1-6 alkyl group;
   Y is a single bond or B(CH₂)ₜ* (wherein B is a 5- to 7-membered carbocyclic ring which may have a substituent, t is 0, 1, or 2, and * indicates a bond to X), or may form, together with X, a 5- to 7-membered carbocyclic or heterocyclic ring which may have a substituent;
   Z is a single bond or (CH₂)ₛ, wherein s is an integer of 1 to 5 (preferably an integer of 1 to 3), provided that when Y is a single bond, Z is not a single bond;
   ** indicates a bond to O.)
   or a nucleotide represented by the following formula (VII) or the formula (VIII): 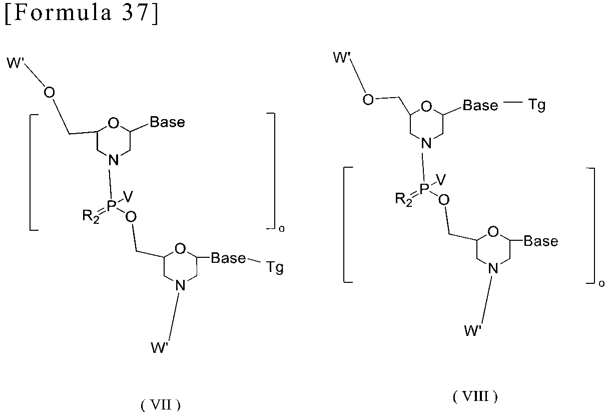 [In the formula,
   o represents any integer of 0 or more (preferably an integer of 1 to 50);
   o+1 Base each independently represents an optionally modified nucleic acid base;
   o V each independently represents an alkoxy group having 1 to 6 carbon atoms, a di(C₁₋₆ alkyl)amino group, or a piperazino group in which the 4-position nitrogen is protected with a protecting group and which may be further substituted;
   W' each independently represents a hydrogen atom or a temporary protecting group removable under acidic conditions or with a reagent containing a fluorine anion;
   o R₂ each independently represents O or S;
   Tg represents a group represented by the following formula (5): 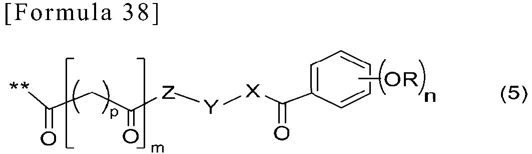 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   X is C, N, O or S, and when X is C, it represents CH₂ or may form, together with Y, a 5- to 7-membered carbocyclic or heterocyclic ring which may have a substituent, and when X is N, it represents N R₁ or may form, together with Y, a 5- to 7-membered heterocyclic ring which may have a substituent, wherein R₁ is H or a C1-6 alkyl group;
   Y is a single bond or B(CH₂)ₜ* (wherein B is a 5- to 7-membered carbocyclic ring which may have a substituent, t is 0, 1, or 2, and * indicates a bond to X), or may form, together with X, a 5- to 7-membered carbocyclic or heterocyclic ring which may have a substituent;
   Z is a single bond or (CH₂)_{S}, where s is an integer of 1 to 5 (preferably an integer of 1 to 3), provided that when Y is a single bond, Z is not a single bond;
   ** indicates a bond to Base.)
   to hydrolysis or ammolysis, to remove the protecting group represented by the formula (5), to obtain a nucleotide represented by the formula (IX): 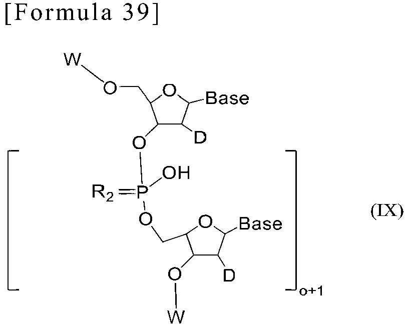 (In the formula, the definitions of Base, D, R₂, W and o are the same as those in the formula (V) or the formula (VI).)
   or the following formula (X): 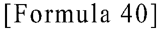 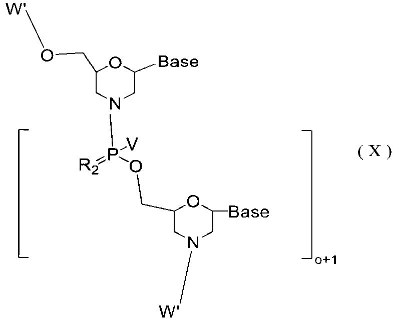 (In the formula, the definitions of Base, R₂, V, W', and o are the same as those in the formula (VII) or the formula (VIII).).
[39] A method for producing an oligonucleotide, comprising the following steps (C1) to (C4):
   (C1) a step of obtaining an n-mer oligonucleotide from which the temporary protecting group at the 5'-position hydroxyl group has been removed and a p-mer oligonucleotide from which the temporary protecting group at the 3'-position hydroxyl group has been removed by carrying out the following steps (a) and (b), respectively:
      (a) a step of reacting, in a nonpolar or polar solvent, an n-mer oligonucleotide (n is any integer of 1 or more) in which the 3'-position hydroxyl group is protected with a support protecting group and the 5'-position hydroxyl group is protected with a temporary protecting group removable under acidic conditions or with a reagent containing a fluorine anion, with an acid and a cation scavenger, or with a reagent containing a fluorine anion (preferably pyridine hydrofluoride or tetrabutylammonium fluoride) to remove the temporary protecting group at the 5'-position hydroxyl group, and then separating the n-mer oligonucleotide from impurities in the reaction solution; and
      (b) a step of reacting, in a non-polar or polar solvent, a p-mer oligonucleotide (p is any integer of 1 or more) in which a support protecting group is bound to the 3'-terminal nucleic acid base and the 3'-position hydroxyl group and the 5'-position hydroxyl group are protected with temporary protecting groups removable under acidic conditions or with a reagent containing a fluorine anion, with an acid and a cation scavenger, or with a reagent containing a fluorine anion (preferably pyridine hydrofluoride or tetrabutylammonium fluoride) to remove only the temporary protecting group at the 3'-position hydroxyl group, and then separating the p-mer oligonucleotide from impurities in the reaction solution;
      wherein the support protecting groups of (a) and (b) may be the same or different;
   (C2) a step of phosphoramiditizing the 5'-position hydroxyl group of the n-mer oligonucleotide from which the temporary protecting group at the 5'-position hydroxyl group has been removed obtained in step (C1), or a step of phosphoramiditizing the 3'-position hydroxyl group of the p-mer oligonucleotide from which the temporary protecting group at the 3'-position hydroxyl group has been removed obtained in step (C1),
   (C3) a step of adding the n-mer oligonucleotide whose 5'-position hydroxyl group has been phosphoramiditized obtained in step (C2) to a solution of the p-mer oligonucleotide from which the temporary protecting group at the 3'-position hydroxyl group has been removed obtained in step (C1), or adding the p-mer oligonucleotide whose 3'-position hydroxyl group has been phosphoramiditized obtained in step (C2) to a solution of the n-mer oligonucleotide from which the temporary protecting group at the 5'-position hydroxyl group has been removed obtained in step (C1), thereby condensing the n-mer oligonucleotide and the p-mer oligonucleotide through a phosphite triester bond; and
   (C4) a step of converting the phosphite triester bond of the n+p-mer oligonucleotide obtained in step (C3) into a phosphate triester bond or a thiophosphate triester bond using an oxidizing agent or a sulfurizing agent;
   wherein the support protecting group is a group represented by the following formula (5): 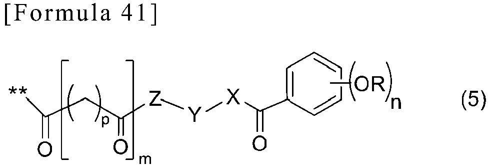 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   X is C, N, O or S, and when X is C, it represents CH₂ or may form, together with Y, a 5- to 7-membered carbocyclic or heterocyclic ring which may have a substituent, and when X is N, it represents N R₁ or may form, together with Y, a 5- to 7-membered heterocyclic ring which may have a substituent, wherein R₁ is H or a C1-6 alkyl group;
   Y is a single bond or B(CH₂)ₜ* (wherein B is a 5- to 7-membered carbocyclic ring which may have a substituent, t is 0, 1, or 2, and * indicates a bond to X), or may form, together with X, a 5- to 7-membered carbocyclic or heterocyclic ring which may have a substituent;
   Z is a single bond or (CH₂)_{S}, where s is an integer of 1 to 5 (preferably an integer of 1 to 3), provided that when Y is a single bond, Z is not a single bond;
   ** indicates a bond to the 3'-position hydroxyl group or the 3'-terminal nucleic acid base.).
[40] The method for producing an oligonucleotide according to [39], wherein the support protecting group is a group represented by the following formula (6): 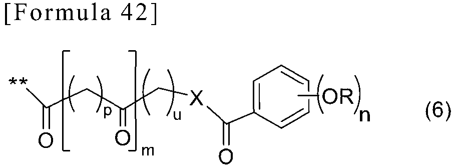 (In the formula,
   R each independently represents an optionally substituted alkenyl group having 14 to 60 carbon atoms (preferably having 14 to 50 carbon atoms, more preferably having 10 to 40 carbon atoms, and still more preferably having 14 to 30 carbon atoms);
   n represents 2 or 3, and the plurality of OR groups may be the same or different;
   m represents 0 or 1;
   p represents 1 or 2;
   u represents an integer of 1 to 5 (preferably an integer of 1 to 3);
   X represents NR₁, O or S, where R₁ is H or a C1-6 alkyl group;
   ** indicates a bond to the 3'-position hydroxyl group or the 3'-terminal nucleic acid base.),
   a group represented by the following formula (7): 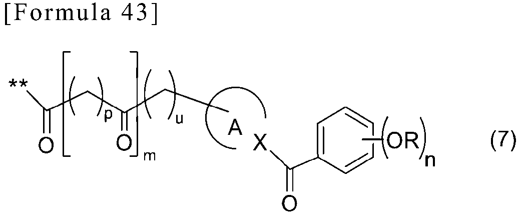 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   u represents 0 or an integer from 1 to 5 (preferably 0 or an integer from 1 to 3, more preferably 0, 1, or 2);
   ring A represents a 5- to 7-membered ring which may have a sustituent;
   X represents C or N;
   ** indicates a bond to the 3'-position hydroxyl group or the 3'-terminal nucleic acid base.),
   or a group represented by the following formula (8): 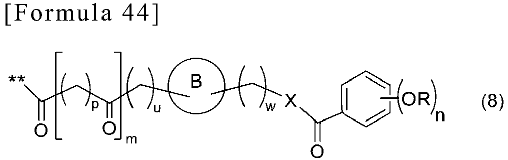 (In the formula,
   R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
   n represents 2 or 3, and the plurality of OR groups may be the same or different;
   m represents 0 or 1;
   p represents 1 or 2;
   u represents 0 or an integer of 1 to 5 (preferably 0 or an integer of 1 to 3, more preferably 0, 1, or 2);
   w represents 0, 1, or 2);
   ring B represents a carbocyclic ring having 5 to 7 carbon atoms which may have a substituent;
   X represents NR₁ , O or S, wherein R₁ is H or CH₃;
   ** indicates a bond to the 3'-position hydroxyl group or the 3'-terminal nucleic acid base.).
[41] The method for producing an oligonucleotide according to [39] or [40], wherein in the step (C1), the step of separating the n-mer oligonucleotide from which the temporary protecting group has been removed from impurities in the reaction solution or the step of separating the p-mer oligonucleotide from which the temporary protecting group has been removed from impurities in the reaction solution comprises neutralizing the reaction solution with a base, and then
   (i) a step of isolating the n-mer oligonucleotide or p-mer oligonucleotide from which the temporary protecting group has been removed, or (ii) a step of washing the neutralized reaction solution with water to recover an organic layer.
[42] The method according to [39] or [40], further comprising the following step (C5):
   (C5) a step of adding a polar solvent to the reaction solution obtained in the step (C1) and/or (C2) and/or (C4), to precipitate the n+p-mer oligonucleotide, and obtaining it by solid-liquid separation.
[43] The method according to [42], further comprising the following step (C6):
   (C6) a step of removing all protecting groups from the n+p-mer oligonucleotide obtained in the step (C5).

### [Advantageous Effect of the Invention]

The present invention provides a novel pseudo-solid-phase protecting group that can be used in the production of an oligonucleotide, and enables a novel method for producing a polyoligonucleotide by liquid-phase synthesis using the protecting group.

### [Description of Embodiments]

The invention is described below by way of exemplary embodiments, along with preferred methods and materials that can be used in the practice of the invention. The subject matter of the present invention should not be limited to the embodiments described below. It should be noted that, unless otherwise specified herein, all technical and scientific terms used herein have the same meanings as commonly understood by those skilled in the art to which this invention pertains. Also, any materials and methods equivalent or similar to those described herein can also be used in the practice of the invention. Additionally, all publications and patents cited in the present specification in connection with the invention described in the present specification constitute a part of the present specification as indicating, for example, methods, materials, etc., that can be used in the present invention.

In this specification, the description of "A - B" indicating a numerical range means a numerical range including the endpoints A and B. The same applies to "A to B". In addition, in this specification, "about" is used to mean an allowance of ±10%.

### I. Long-chain alkenyloxy-substituted benzoyl derivatives as pseudo-solid-phase protecting groups

One aspect of the invention is a long-chain alkenyloxy-substituted benzoyl derivative that serves as a pseudo-solid-phase protecting group usable in the production of oligonucleotides.

One embodiment of the compound and a salt thereof of the present invention is a compound represented by the following formula (1) (hereinafter, in this specification, it may be referred to as compound (1). The same applies to other compounds represented by the other formulas) and a salt thereof.

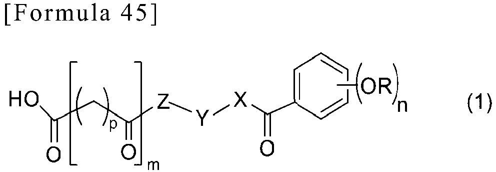

In the formula (1), R each independently represents an optionally substituted alkenyl group having 14 to 60 carbon atoms, preferably 14 to 50 carbon atoms, more preferably 14 to 40 carbon atoms, and still more preferably 14 to 30 carbon atoms.

In the present specification, the term "alkenyl group having 14 to 60 carbon atoms" refers to a linear or branched hydrocarbon group having one double bond at any position and having 14 to 60 carbon atoms. Examples thereof include, but are not limited to, myristoleyl group (C14), palmitoleyl group (C16), sapienyl group (C16), oleyl group (C18), elaidyl group (C18), vaccenyl group (C18), gadoleyl group (C20), eicosenoyl group (C20), erucyl group (C22), nervonyl group (C24), linoleyl group (C18), eicosadienoyl group (C20), docosadienoyl group (C22), α- and γ-linoleyl groups (C18), pinoleyl group (C18), eleostearyl group (C18), meadyl group (C20), dihomo-γ-linoleyl group (C20), eicosatrienoyl group (C20), stearidonyl group (C18), arachidonyl group (C20), eicosatetraenoyl group (C20), adrenyl group (C22), bosseopentaenoyl group (C18), eicosapentaenoyl group (C20), osbondoyl group (C22), clupanodonyl group (C22), tetracosapentaenoyl group (C24), docosahexaenoyl group (C22), and nisinyl group (C24). These alkenyl groups may be naturally derived or may be obtained by synthesis using known synthesis techniques. These alkenyl groups are unsaturated hydrocarbon groups that can be obtained from fatty acids selected from, but are not limited to, myristoleic acid (C14), palmitoleic acid (C16), sapienic acid (C16), oleic acid (C18), elaidic acid (C18), vaccenic acid (C18), gadoleic acid (C20), eicosenoic acid (C20), erucic acid (C22), nervonic acid (C24), linoleic acid (C18), eicosadienoic acid (C20), docosadienoic acid (C22), α- and γ-linolenic acid (C18), pinoleic acid (C18), eleostearic acid (C18), mead acid (C20), dihomo-γ-linolenic acid (C20), eicosatrienoic acid (C20), stearidonic acid (C18), arachidonic acid (C20), eicosatetraenoic acid (C20), adrenic acid (C22), bosseopentaenoic acid (C18), eicosapentaenoic acid (C20), osbond acid (C22), clupanodonic acid (C22), tetracosapentaenoic acid (C24), docosahexaenoic acid (C22), and nisinic acid (C24), and for example, include unsaturated hydrocarbon groups in which the terminal carboxy group of the compound is replaced with a methylene group, and in the present specification, these may be referred to as "unsaturated hydrocarbon groups derived from the compound".

In the formula (1), n represents 2 or 3, and the two or three ORs may be the same or different, but are preferably the same. The positions of the OR groups is not particularly limited, but is preferably 3,4,5-substituted, 2,4-substituted, or 2,3,4-substituted.

In the formula (1), m represents 0 or 1.

In the formula (1), p represents 1 or 2.

In the formula (1), X is C, N, O or S, preferably C, N or O, more preferably C or N. When X is C, it represents CH₂ or may form, together with Y, a 5- to 7-membered carbocyclic ring which may have a substituent or a 5- to 7-membered heterocyclic ring which may have a substituent

In the present specification, examples of the "5- to 7-membered carbocyclic ring which may have a substituent" include, but are not limited to, cyclopentane, cyclopentene, cyclohexane, cyclohexene, benzene, cycloheptane, cycloheptene, and naphthalene, each of which may have a substituent, and preferably cyclohexane, benzene, or naphthalene, each of which may have a substituent.

In the present specification, examples of the "5- to 7-membered heterocyclic ring which may have a substituent" include, but are not limited to, pyrrolidine, piperidine, pyrrole, pyridine, pyrazole, imidazole, pyrimidine, pyrazine, pyridazine, and triazine, each of which may have a substituent, and preferably pyrrolidine or piperidine, each of which may have a substituent.

Examples of substituents that the 5- to 7-membered carbocyclic ring or the 5- to 7-membered heterocyclic ring may have include, for example, an optionally substituted hydroxyl group, an optionally substituted amino group, an optionally substituted alkyl group, a halogen atom, a nitro group, and an optionally substituted carbonyl group, and preferably an optionally substituted hydroxyl group, an optionally substituted amino group, or an optionally substituted alkyl group.

As used herein, "optionally substituted" or "which may be substituted" means that it may be substituted with one or any number of substituents, preferably 1 to 3. When it is substituted with a plurality of substituents, the substituents may be the same or different.

In the present specification, the term "substituent" include, for example, a halogen atom, a C1-6 alkyl group, a C2-6 alkenyl group, a C2-6 alkynyl group, a C1-6 alkoxy group, a C7-16 aralkyl group, a C3-6 cycloalkyl group, a C6-14 aryl group, a 6-14 membered heteroaryl group, an aryloxy group, an acyl group, an amino group, a hydroxyl group, an oxo group, a thioxo group, and a trifluoromethyl group.

In the present specification, the term "C1-6 alkyl group" include, for example, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, and 2-ethylbutyl.

In the present specification, the term "C2-6 alkenyl group" include, for example, ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, and 5-hexenyl.

In the present specification, the term "C2-6 alkynyl group" include, for example, ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, and 4-methyl-2-pentynyl.

In the present specification, the term "C1-6 alkoxy group" include, for example, methoxy, ethoxy, propoxy, isopropoxy, butoxy, isobutoxy, sec-butoxy, tert-butoxy, pentyloxy and hexyloxy.

In the present specification, the term "C7-16 aralkyl group" include, for example, benzyl, phenethyl, naphthylmethyl, and phenylpropyl.

In the present specification, the term "C3-6 cycloalkyl group" include, for example, cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, and adamantyl.

In the present specification, the term "C6-14 aryl group" include, for example, phenyl, biphenyl, naphthyl (e.g., 1-naphthyl, 2-naphthyl), and anthracenyl (e.g., 1-anthryl, 2-anthryl, 9-anthryl).

In the present specification, the term "6- to 14-membered heteroaryl group" include, for example, pyridyl, pyrimidinyl, pyridazinyl, pyrrolyl, isoquinolyl, quinolyl, indolyl, imidazolyl, benzimidazolyl, triazolyl, furyl and thienyl.

In the present specification, the term "optionally substituted alkyl group" includes any linear alkyl group having 1 to 6 carbon atoms, more preferably 1 to 4 carbon atoms, any branched alkyl group having 3 to 6 carbon atoms with the same or different branches, and any cyclic alkyl group having 3 to 6 carbon atoms, which may be substituted with a substituent. For example, specific examples of any linear alkyl group having 1 to 6 carbon atoms include a methyl group, an ethyl group, an n-propyl group, an n-butyl group, an n-pentyl group, an n-hexyl group, and the like. Specific examples of any branched alkyl group having 3 to 6 carbon atoms with the same or different branches include an isopropyl group, an isobutyl group, a tert-butyl group, a sec-butyl group, a neopentyl group, an isopentyl group, and the like. As any cyclic alkyl group having 3 to 6 carbon atoms, a 3- to 6-membered monocyclic cycloalkyl group is preferable, and specific examples thereof include a cyclopropyl group, a cyclobutyl group, a cyclopentyl group, a cyclohexyl group, and the like. These example are not limited thereto.

In this specification, the term "halogen atom" include, for example, a fluorine atom (fluoro), a chlorine atom (chloro), a bromine atom (bromo), or an iodine atom (iodo).

In the formula (1), when X is N, it is NR₁ or, together with Y, represents a 5- to 7-membered heterocyclic ring which may have a substituent. R₁ is H or a C1-6 alkyl group, preferably H or methyl. Examples of the 5- to 7-membered heterocyclic ring which may have a substituent are the same as those described above.

In the formula (1), Y represents a single bond, B(CH₂)ₜ*, or, together with X, a 5- to 7-membered ring which may have a substituent. B represents a carbocyclic ring having 5 to 7 carbon atoms which may have a substituent, t is 0, 1, or 2, and * indicates a bond to X. Examples of the carbocyclic ring having 5 to 7 carbon atoms which may have a substituent include, but are not limited to, cyclopentane, cyclopentene, cyclohexane, cyclohexene, benzene, cycloheptane, cycloheptene, and naphthalene which may have a substituent, and preferably cyclohexane, benzene, or naphthalene which may have a substituent. Examples of the substituent of the carbocyclic ring having 5 to 7 carbon atoms include an optionally substituted hydroxyl group, an optionally substituted amino group, an optionally substituted alkyl group, a halogen atom, a nitro group, and a carbonyl group, and preferably an optionally substituted hydroxyl group, an optionally substituted amino group, or an optionally substituted alkyl group.

Z represents a single bond or (CH₂)_{S}. s is an integer of 1 to 5, preferably an integer of 1 to 3. However, both Y and Z cannot be single bonds.

In this specification, the term "salt" refers to a salt formed in the structure of the carboxylic acid moiety, and examples thereof include metal carboxylates and ammonium salts of carboxylic acids, with metal carboxylates being preferred. The metal species of the metal carboxylates is not particularly limited, and examples thereof include sodium, potassium, lithium, cesium, nickel, cobalt, cadmium, barium, calcium, magnesium, zinc, manganese, copper, cerium, zirconium, iron, lead, germanium, antimony, aluminum, titanium, and bismuth, with alkali metals or alkaline earth metals being preferred.

Another embodiment of the compound and salt thereof of the present invention is a compound represented by the formula (2)
(hereinafter, sometimes referred to as compound (2) in this specification) and a salt thereof.

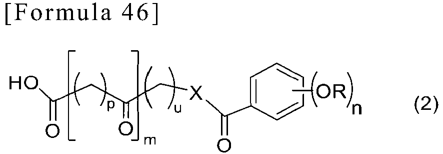

In the formula (2), R each independently represents an optionally substituted alkenyl group having 14 to 60 carbon atoms, preferably 14 to 50 carbon atoms, more preferably 14 to 40 carbon atoms, and still more preferably 14 to 30 carbon atoms. Examples of R are the same as those exemplified in compound (1).

In the formula (2), n represents 2 or 3, and the two or three ORs may be the same or different, but are preferably the same. The positions of the OR group is not particularly limited, but is preferably 3,4,5-substituted, 2,4-substituted, or 2,3,4-substituted.

In the formula (2), m represents 0 or 1.

In the formula (2), p represents 1 or 2.

In the formula (2), u represents an integer of 1 to 5, preferably an integer of 1 to 3.

In the formula (2), X represents NR₁ , O or S, and is preferably NR₁ or O. R₁ is H or a C1-6 alkyl group, and is preferably H or methyl.

Another embodiment of the compound and salt thereof of the present invention is a compound represented by the formula (3) (hereinafter, sometimes referred to as compound (3) in this specification) and a salt thereof.

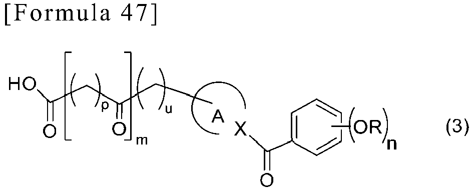

In the formula (3), R each independently represents an optionally substituted alkenyl group having 14 to 60 carbon atoms, preferably 14 to 50 carbon atoms, more preferably 14 to 40 carbon atoms, and still more preferably 14 to 30 carbon atoms. Examples of R are the same as those exemplified in compound (1).

In the formula (3), n represents 2 or 3, and the two or three ORs may be the same or different, but are preferably the same. The positions of the OR groups is not particularly limited, but is preferably 3,4,5-substituted, 2,4-substituted, or 2,3,4-substituted.

In the formula (3), m represents 0 or 1, and is preferably 0.

In the formula (3), p represents 1 or 2.

In the formula (3), u represents 0 or an integer of 1 to 5, preferably 0 or an integer of 1 to 3, and more preferably 0, 1, or 2.

In the formula (3), X represents C or N. When X is C, ring A represents a 5- to 7-membered carbocyclic or heterocyclic ring which may have a substituent, and when X is N, ring A represents a 5- to 7-membered heterocyclic ring which may have a substituent.

Another embodiment of the compound and salt thereof of the present invention is a compound represented by the formula (4) (hereinafter, sometimes referred to as compound (4) in this specification) and a salt thereof.

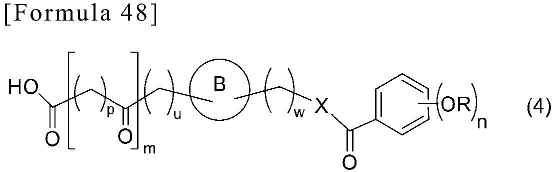

In the formula (4), R each independently represents an optionally substituted alkenyl group having 14 to 60 carbon atoms, preferably 14 to 50 carbon atoms, more preferably 14 to 40 carbon atoms, and still more preferably 14 to 30 carbon atoms. Examples of R are the same as those exemplified in compound (1).

In the formula (4), n represents 2 or 3, and the two or three ORs may be the same or different, but are preferably the same. The positions of the OR groups is not particularly limited, but is preferably 3,4,5-substituted, 2,4-substituted, or 2,3,4-substituted.

In the formula (4), m represents 0 or 1, and is preferably 0.

In the formula (4), p represents 1 or 2.

In the formula (4), u represents 0 or an integer of 1 to 5, preferably 0 or an integer of 1 to 3, and more preferably 0, 1, or 2.

In the formula (4), w represents 0, 1, or 2.

In the formula (4), ring B represents a carbocyclic ring having 5 to 7 carbon atoms which may have a substituent.

Examples of the compound (1) of the present invention include the compounds shown below.

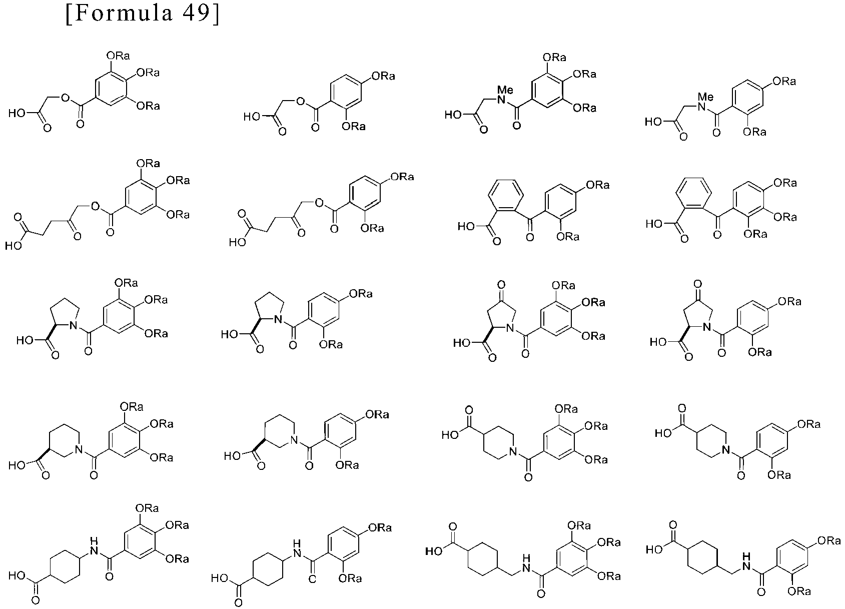

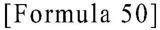

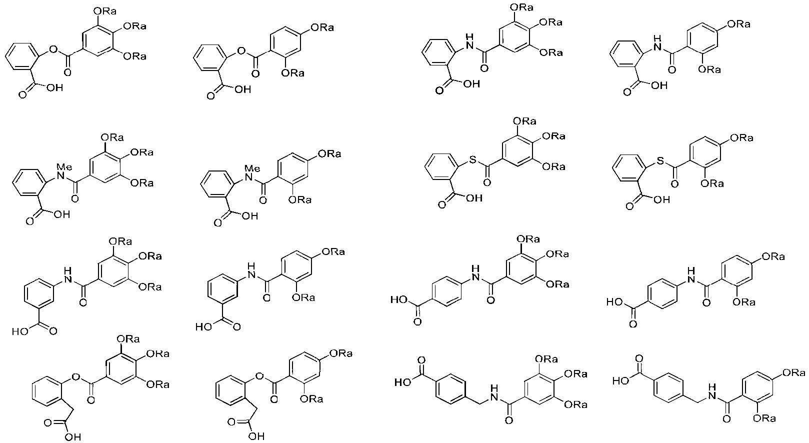

In the formulae, Ra each independently represents an optionally substituted alkenyl group having 14 to 60 carbon atoms, preferably 14 to 50 carbon atoms, more preferably 14 to 40 carbon atoms, and still more preferably 14 to 30 carbon atoms. Examples of R are the same as those exemplified in compound (1).

### II. Nucleotide having pseudo-solid-phase protecting groups bound thereto

One embodiment of the present invention is a nucleotide having a long-chain alkenyloxy-substituted benzoyl derivative, which is the pseudo-solid-phase protecting group, bound thereto.

One embodiment of the nucleotide having a pseudo-solid-phase protecting group bound thereto according to the present invention is represented by the following formula (I) (hereinafter, in this specification, this may be referred to as oligonucleotide (I)).

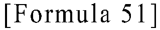

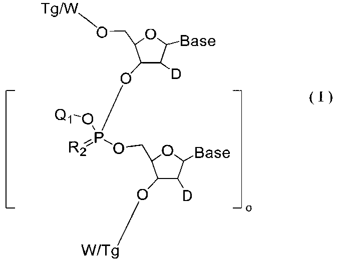

In the oligonucleotide (I), o represents any integer of 0 or more, preferably an integer of 1 to 50.

In the oligonucleotide (I), o+1 Base each independently represents an optionally modified nucleic acid base.

In the present specification, the term "nucleic acid base" includes natural nucleic acid bases and unnatural nucleic acid bases, and is not particularly limited as long as it is used in the synthesis of nucleic acids. Examples of the nucleic acid base include, but are not limited to, purine bases such as adenine and guanine, and pyrimidine bases such as uracil, cytosine, and thymine. The nucleic acid base may also be modified. Examples of the "optionally modified nucleic acid base" include nucleic acid bases modified with 1 to 3 optional substituents (e.g., halogen atoms, alkyl groups, aralkyl groups, alkoxy groups, acyl groups, alkoxyalkyl groups, hydroxyl groups, amino groups, monoalkylamino groups, dialkylamino groups, carboxy groups, cyano groups, nitro groups, alkylthio groups, etc.) at any position of the purine ring or pyrimidine ring, and examples thereof include, but are not limited to, 8-bromoadenine, 8-bromoguanine, 5-bromocytosine, 5-iodocytosine, 5-bromouracil, 5-iodouracil, 5-fluorouracil, 5-methylcytosine, 8-oxoguanine, and hypoxanthine. Preferred nucleic acid bases include, for example, adenine, guanine, 2,6-diaminopurine, thymine, 2-thiothymine, cytosine, 5-methylcytosine, uracil, 5-fluorocytosine, xanthine, 6-aminopurine, 2-aminopurine, 6-chloro-2-amino-purine, and 6-chloropurine, and particularly preferred nucleic acid bases include, for example, adenine, guanine, cytosine, 5-methylcytosine, thymine, or uracil. The plurality of nucleic acid bases present in a nucleotide may be either different or the same. In addition, the nucleic acid base may be protected.

In the present specification, the term "nucleic acid base may be protected" means that any functional group of the nucleic acid base may be protected with a protecting group, for example, the amino group in a nucleic acid base having an amino group (e.g., an adenyl group, a guanyl group, or a cytosyl group) may be protected, the hydroxyl group in a nucleic acid base having a hydroxyl group may be protected, the thiol group in a nucleic acid base having a thiol group may be protected, or the carbonyl group in a nucleic acid base having a carbonyl group may be protected in the form of a hydroxyl group conjugated with the amino group or hydroxyl group substituted on the ring, etc. The protecting groups bound to these nucleic acid bases are preferably capable of withstanding the deprotection conditions of the temporary protecting group at the 3'-position or 5'-position of the oligonucleotide.

The "protecting group of an amino group" in the nucleic acid base is not particularly limited, and examples thereof include a pivaloyl group, a pivaloyloxymethyl group, a trifluoroacetyl group, a phenoxyacetyl group, a 4-isopropylphenoxyacetyl group, a 4-tert-butylphenoxyacetyl group, an acetyl group, a benzyl (Bn) group, a benzoyl (Bz) group, a 4,4'-dimethoxytrityl (DMTr) group, a 4-methoxytrityl group, a triphenylmethyl group, a 2-naphthylmethyl group, a cyanoethoxycarbonyl group, a tetrahydropyranyl group, a trimethylsilyl group, a triethylsilyl group, a triisopropylsilyl group, a t-butyldimethylsilyl group, a t-butyldiphenylsilyl group, a 4-methoxybenzyl group, a 3,4-dimethoxybenzyl group, an isobutyryl group, a dimethylformamidinyl group, a 9-fluorenylmethyloxycarbonyl (Fmoc) group, an alkoxycarbonyl group (e.g., a t-butoxycarbonyl (Boc) group, a benzyloxycarbonyl (Cbz) group, a diphenylaminocarbonyl (DPC) group, and a cyanoethoxycarbonyl (Ceoc) group), and a sulfonyl group (e.g., a methanesulfonyl group and a p-toluenesulfonyl group). Of these, a phenoxyacetyl group, a 4-isopropylphenoxyacetyl group, an acetyl group, an isobutyryl group, and a dimethylformamidinyl group are preferred.

The "protecting group for a hydroxy group" in the nucleic acid base is not particularly limited, and examples thereof include alkyl groups (e.g., methyl and tert-butyl groups), arylmethyl groups (e.g., benzyl and p-methoxybenzyl groups), alkoxyalkyl groups (e.g., methoxymethyl, methoxyethyl, cyanoethoxymethyl and ethoxyethyl groups), 2-tetrahydropyranyl groups, cyanoethyl groups, carbamoyl groups (e.g., phenylcarbamoyl and 1,1-dioxothiomorpholine-4-thiocarbamoyl groups), acyl groups (e.g., acetyl group, pivaloyl group, isobutyryl group, benzoyl group, phenoxyacetyl group, levulinyl group, 3-benzoylpropionyl group), silyl group (for example, trimethylsilyl group, triethylsilyl group, triisopropylsilyl group, tert-butyldimethylsilyl group, tert-butyldiphenylsilyl group), [(triisopropylsilyl)oxy]methyl group (Tom group), 1-(4-chlorophenyl)-4-ethoxypiperidin-4-yl group (Cpep group), of which acetyl group, benzoyl group, benzyl group, or p-methoxybenzyl group is preferred.

The "protecting group for a thiol group" in the nucleic acid base is not particularly limited, and examples thereof include the same protecting groups as the "protecting group for the hydroxy group" and protecting groups that form disulfide bonds.

When the carbonyl group of the nucleic acid base is conjugated and protected in the form of a hydroxyl group, for example, phenol, 2,5-dichlorophenol, 3-chlorophenol, 3,5-dichlorophenol, 2-formylphenol, 2-naphthol, 4-methoxyphenol, 4-chlorophenol, 2-nitrophenol, 4-nitrophenol, 4-acetylaminophenol, pentafluorophenol, 4-pivaloyloxybenzyl alcohol, 4-nitrophenethyl alcohol, 2-(methylsulfonyl)ethanol, 2-(phenylsulfonyl)ethanol, 2-cyanoethanol, 2-(trimethylsilyl)ethanol, dimethylcarbamic acid chloride, diethylcarbamic acid chloride, ethylphenylcarbamic acid chloride, 1-pyrrolidine carboxylic acid chloride, 4-morpholine carboxylic acid chloride, diphenylcarbamic acid chloride, etc. can be reacted to protect the carbonyl group.

D in the oligonucleotide (I) represents a hydrogen atom, a halogen atom, or a hydroxyl group which may be protected, and may also form a bridge with the 4'-position carbon of ribose. The protecting group in the "hydroxyl group which may be protected" is the same as the "protecting group of a hydroxyl group".

Examples of "bridged nucleic acids (BNA)" include, but are not limited to, bridged nucleic acids such as locked nucleic acids (LNA), amide-bridged nucleic acids, guanidine-bridged nucleic acids (GuNA), or spirocyclopropylene-bridged nucleic acids. Examples of such BNA include, but are not limited to, methyleneoxy (4'-CH₂-O-2') BNA (also known as LNA, 2',4'-BNA), ethyleneoxy (4'-(CH₂)₂-O-2') BNA (also known as ENA), β-D-thio (4'-CH₂-S-2') BNA, aminooxy (4'-CH₂-ON(R₃)-2') BNA (R is H or Me), oxyamino (4'-CH₂-N(R₃)-O-2') BNA (R is H or Me), 2',4'-BNAcoc, 3'-amino-2',4'-BNA, 5'-methyl BNA, (4'-CH(CH ₃)-O-2') BNA (also known as cEt BNA), (4'-CH(CH₂OCH₃)-O-2') BNA (also known as cMOE BNA), amide BNA, (4'-C(O)-N(R)-2') BNA (R is H or Me) (also known as AmNA), guanidine-bridged BNA (GuNA), amine BNA (also known as 2'-Amino-LNA), 2'-O,4'-C-spirocyclopropylene-bridged nucleic acid (also known as scpBNA), and other BNA known to those of skill in the art.

Furthermore, examples of amide-bridged nucleic acids and their production methods can be, for example, those described in WO2014/109384, and examples of guanidine-bridged nucleic acids and their production methods can be, for example, those described in WO2014/046212, WO2017/047816, and JP2022-033869.

Q₁ in the oligonucleotide (I) represents a C1-4 alkyl grouphaving an electron-withdrawing group, allyl group, a C2-6 alkenyl group or a C6-10 aryl group having a cyano group, a nitro group or a halogen group, or a hydrogen atom. When there are two or more Q, the plurality of Q may be the same or different.

In the present specification, examples of the "C1-4 alkyl group" include methyl, ethyl, propyl, isopropyl, butyl, isobutyl, sec-butyl and tert-butyl.

In the present specification, the term "electron-withdrawing group" refers to a substituent that is generally used in organic chemistry and has the effect of reducing electron density. Examples of the electron-withdrawing group include, but are not limited to, a carboxy group, a halogen atom, a nitro group, an ester group, a cyano group, a nitro group, a 2-pyridyl group, a 4-pyridyl group, and an aryl group or an arylsulfonyl group that may have a substituent, and preferably a cyano group.

W/Tg or Tg/W in the oligonucleotide (I) represents W or Tg, and when the 5' side is W, the 3' side is Tg, whereas when the 5' side is Tg, the 3' side is W, but preferably the 3' side is Tg and the 5' side is W. Here, W represents a hydrogen atom, or a temporary protecting group removable under acidic conditions or with a reagent containing a fluorine anion.

In the present specification, the term " temporary protecting group removable under acidic conditions or with a reagent containing a fluorine anion" refers to a protecting group that is commonly used in the field of organic synthetic chemistry (particularly, nucleic acid synthesis) as a protecting group for a hydroxyl group, and is not particularly limited as long as it is a protecting group removable under acidic conditions or with a reagent containing a fluorine anion. Examples thereof include aliphatic acyl groups; aromatic acyl groups; optionally substituted aminocarbonyl groups; optionally substituted alkoxycarbonyl groups; aliphatic sulfonyl groups; aromatic sulfonyl groups; methyl groups substituted with 1 to 3 aryl groups; methyl groups substituted with 1 to 3 aryl groups which are substituted with alkyl groups having 1 to 6 carbon atoms, alkoxy groups having 1 to 6 carbon atoms, halogen atoms and/or cyano groups; or silyl groups. Specific examples include, but are not limited to, benzyl (Bn) groups, diphenylcarbonyl (DPC) groups, 4,4'-dimethoxytriphenylmethyl groups (commonly known as a 4,4'-dimethoxytrityl (DMTr) groups), 4-methoxytriphenylmethyl groups (commonly known as 4-methoxytrityl groups), triphenylmethyl groups (commonly known as trityl (Tr) groups), 9-(9-phenyl)xanthenyl groups, 9-phenylthioxanthenyl groups, di(C1-6 alkoxy)trityl groups, mono(C1-18 alkoxy)trityl groups, 2-naphthylmethyl groups, cyanoethoxycarbonyl groups, tetrahydropyranyl groups, silyl groups substituted with alkyl groups or allyl groups (e.g., trimethylsilyl, triethylsilyl, triisopropylsilyl (TIPS), dimethylisopropylsilyl, diethylisopropylsilyl, dimethylthexylsilyl, t-butyldimethylsilyl (TBS (TBDMS)), t-butyldiphenylsilyl (TBDPS), tribenzylsilyl, tri-p-xylylsilyl, triphenylsilyl, diphenylmethylsilyl, di-t-butylmethylsilyltri(trimethylsilyl)silyl, t-butylmethoxyphenylsilyl, t-butoxydiphenylsilyl, TBoDPS, TBDAS, etc.), 4-methoxybenzyl (p-methoxybenzyl) groups, 3,4-dimethoxybenzyl groups, benzoyl (Bz) groups, 2,6-dimethoxybenzyl groups, p-phenylbenzyl groups, methanesulfonyl groups, trifluoromethanesulfonyl groups, methoxymethyl groups, and acetyl groups. Preferred examples include benzyl (Bn) groups, 4,4'-dimethoxytrityl (DMTr) groups, 4-methoxytrityl groups, t-butyldimethylsilyl groups, t-butyldiphenylsilyl groups, trimethylsilyl (TMS) groups, diphenylaminocarbonyl (DPC) groups, methanesulfonyl groups, and trifluoromethanesulfonyl groups, and particularly preferred are 4,4'-dimethoxytrityl groups or 4-methoxytrityl groups.

R₂ in the oligonucleotide (I) is O or S.

One embodiment of the nucleotide having a pseudo-solid-phase protecting group bound thereto according to the present invention is represented by the following formula (II) (hereinafter, in this specification, this may be referred to as oligonucleotide (II)).

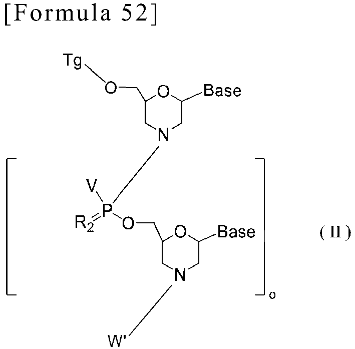

In the oligonucleotide (II), o represents any integer of 0 or more, preferably an integer of 1 to 50.

In the oligonucleotide (II), o+1 Base each independently represents an optionally modified nucleic acid base.

In the oligonucleotide (II), W' is a hydrogen atom or a temporary protecting group which is removable under acidic conditions or with a reagent containing a fluorine anion.

R₂ in the oligonucleotide (II) is O or S.

In the oligonucleotide (II), V represents a C1-6 alkoxy group, a di(C1-6 alkyl)amino group, or a piperazino group in which the 4-position nitrogen atom is protected with a protecting group and may be further substituted. When there are two or more Vs, the plurality of Vs may be the same or different.

One embodiment of the nucleotide having a pseudo-solid-phase protecting group bound thereto according to the present invention is represented by the following formula (V) (hereinafter, sometimes referred to as oligonucleotide (V) in this specification).

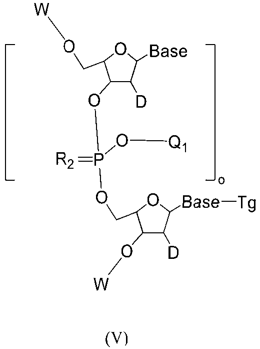

In the oligonucleotide (V), the definitions of o, Base, W, R₂, and D are the same as those in the oligonucleotide (I), provided that two Ws may be the same or different.

One embodiment of the nucleotide having a pseudo-solid-phase protecting group bound thereto according to the present invention is represented by the following formula (VI) (hereinafter, sometimes referred to as oligonucleotide (VI) in this specification).

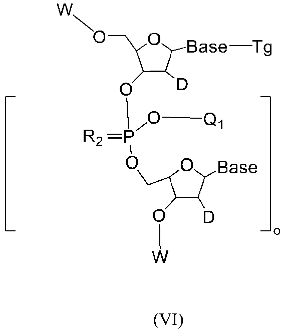

In the oligonucleotide (VI), the definitions of o, Base, W, R₂, and D are the same as those in the oligonucleotide (I), provided that two Ws may be the same or different.

One embodiment of the nucleotide having a pseudo-solid-phase protecting group bound thereto according to the present invention is represented by the following formula (VII) (hereinafter, sometimes referred to as oligonucleotide (VII) in this specification).

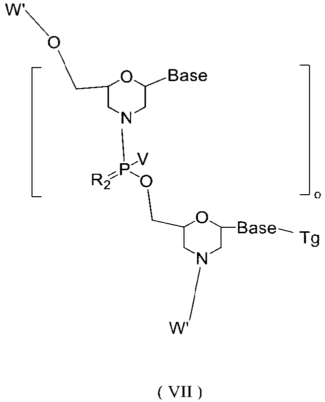

In the oligonucleotide (VII), the definitions of o, Base, W', R₂, and V are the same as those in the oligonucleotide (II), provided that the two W's may be the same or different.

One embodiment of the nucleotide having a pseudo-solid-phase protecting group bound thereto according to the present invention is represented by the following formula (VIII) (hereinafter, sometimes referred to as oligonucleotide (VIII) in this specification).

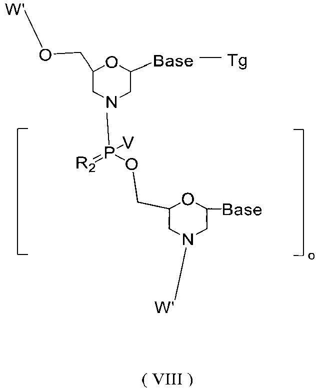

In the oligonucleotide (VIII), the definitions of o, Base, W', R₂, and V are the same as those in the oligonucleotide (II), provided that the two W's may be the same or different.

In the nucleotide having a pseudo-solid-phase protecting group bound thereto according to the present invention, Tg in the oligonucleotide (I), (II), (V), (VI), (VII) or (VIII) is the following formula (5):

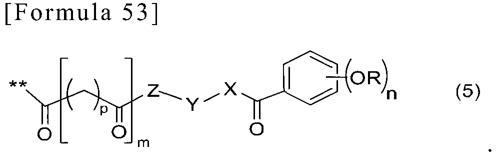

In the formula (5), ** indicates a bond to O or Base of the oligonucleotide.

In the formula (5), the definitions of the other symbols are the same as those in the formula (1).

The bond of Tg to O can be made by a bond (e.g., an ester bond) between the 3'- or 5'-position hydroxyl group of the nucleotide and the terminal carboxylic acid of Tg, but is not limited thereto. The bond of Tg to Base can be made by a bond reaction between a reactive substituent of Base and the terminal carboxylic acid of Tg, for example, by a bond (e.g., an amide bond) between the amino group of adenine, guanine, or cytosine, which is Base, and the terminal carboxylic acid of Tg.

Further, Tg of the oligonucleotide (I), (II), (V), (VI), (VII) or (VIII) is the following formula (6):

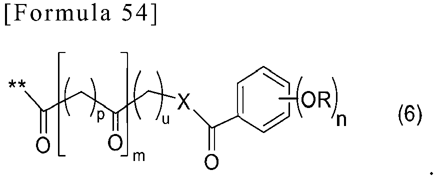

In the formula (6), ** indicates a bond to O or Base of the oligonucleotide.

In the formula (6), the definitions of the other symbols are the same as those in the formula (2).

Further, Tg in the oligonucleotide (I), (II), (V), (VI), (VII) or (VIII) is the following formula (7):

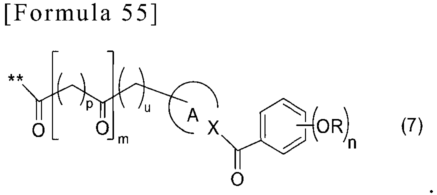

In the formula (7), ** indicates a bond to O or Base of the oligonucleotide.

In the formula (7), the definitions of the other symbols are the same as those in the formula (3).

Further, Tg in the oligonucleotide (I), (II), (V), (VI), (VII) or (VIII) or oligonucleotide (II) is the following formula (8):

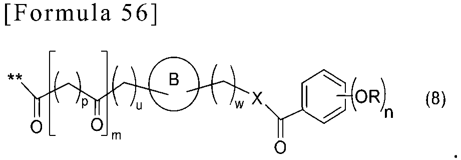

In the formula (8), ** indicates a bond to O or Base of the oligonucleotide.

In the formula (8), the definitions of the other symbols are the same as those in the formula (4).

The pseudo-solid-phase protecting group of the present invention, bound to a nucleotide represented by the formula (I) or the formula (II), can be deprotected by subjecting the nucleotide to reduction treatment, reaction with hydrazine or an alkylhydrazine, or hydrolysis. The reduction treatment can be carried out using, but is not limited to, a boron-containing reducing agent, preferably lithium borohydride, sodium borohydride, lithium triethylborohydride, or tetrabutylammonium borohydride, or both a boron-containing reducing agent and an amine.

The pseudo-solid-phase protecting group of the present invention, bound to a nucleotide represented by the formula (V), (VI), (VII) or (VIII), can be deprotected by subjecting the nucleotide to hydrolysis or ammolysis. Ammolysis can be performed using, but is not limited to, an alcoholic solution of ammonia, for example, a methanolic solution of ammonia.

### III. Methods for Producing Nucleotides

One embodiment of the present invention is a method for producing an oligonucleotide, comprising the following steps (A1) to (A3) (hereinafter, in this specification, it may be referred to as a method for producing an oligonucleotide or simply as a production method of the present invention):
Step (A1): a step of reacting, in a nonpolar or polar solvent, an n-mer (oligo)nucleotide (n is any integer of 1 or more) in which the 3'-position hydroxyl group or the 5'-position hydroxyl group is protected with a support protecting group and the 5'-position hydroxyl group or the 3'-position hydroxyl group is protected with a temporary protecting group removable under acidic conditions or with a reagent containing a fluorine anion, with an acid and a cation scavenger, or with a reagent containing a fluorine anion to remove the temporary protecting group at the 5'-position hydroxyl group or the 3'-position hydroxyl group, and then separating the n-mer (oligo)nucleotide from impurities in the reaction solution (sometimes referred to as a "deprotection step and a separation step" in the present specification);
Step (A2): a step of adding a p-mer (oligo)nucleotide (p is any integer of 1 or more) in which the 3'-position hydroxyl group has been phosphoramiditized and the 5'-position hydroxyl group has been protected with a temporary protecting group removable under acidic conditions or with a reagent containing a fluorine anion, or in which the 5'-position hydroxyl group has been phosphoramiditized and the 3'-position hydroxyl group has been protected with a temporary protecting group removable under acidic conditions or with a reagent containing a fluorine anion, to a solution of the n-mer (oligo)nucleotide obtained in step (A1) from which the temporary protecting group at the 5'-position hydroxyl group or the 3'-position hydroxyl group has been removed, thereby condensing the n-mer (oligo)nucleotide and the p-mer (oligo)nucleotide via the 5'-position hydroxyl group or the 3'-position hydroxyl group through a phosphite triester bond (sometimes referred to as a "condensation step" in this specification); and
Step (A3): a step of converting the phosphite triester bond of the n+p-mer oligonucleotide obtained in step (A2) into a phosphate triester bond or a thiophosphate triester bond using an oxidizing agent or a sulfurizing agent (sometimes referred to as an "oxidation or sulfurization reaction step" in this specification).

One aspect of the present invention is a method for producing an oligonucleotide, comprising the following steps (B1) to (B3) (hereinafter, in this specification, may be referred to as a method for producing an oligonucleotide or simply as a production method of the present invention):
(B1) a step of reacting, in a nonpolar or polar solvent, an n-mer oligonucleotide (n is any integer of 1 or more) in which a support protecting group is bound to a nucleic acid base at the 3'terminal or a nucleic acid base at the 5'terminal and the 3'-position hydroxyl group and the 5'-position hydroxyl group are protected with a temporary protecting group removable under acidic conditions or with a reagent containing a fluorine anion, with an acid and a cation scavenger, or with a reagent containing a fluorine anion (preferably pyridine hydrofluoride or tetrabutylammonium fluoride) to remove the temporary protecting group at only one of the 5'-position hydroxyl group or the 3'-position hydroxyl group, and then separating the n-mer oligonucleotide from impurities in the reaction solution;
(B2) a step of adding a p-mer oligonucleotide (p is any integer of 1 or more) in which the 3'-position hydroxyl group has been phosphoramiditized and the 5'-position hydroxyl group has been protected with a temporary protecting group removable under acidic conditions or with a reagent containing a fluorine anion, or in which the 5'-position hydroxyl group has been phosphoramiditized and the 3'-position hydroxyl group has been protected with a temporary protecting group removable under acidic conditions or with a reagent containing a fluorine anion, to a solution of the n-mer oligonucleotide obtained in step (B1) from which the temporary protecting group at the 5' -position hydroxyl group or the 3'-position hydroxyl group has been removed, thereby condensing the n-mer oligonucleotide and the p-mer oligonucleotide via the 5'-position hydroxyl group or the 3'-position hydroxyl group through a phosphite triester bond; and
(B3) a step of converting the phosphite triester bond of the n+p-mer oligonucleotide obtained in the step (B2) into a phosphate triester bond or a thiophosphate triester bond using an oxidizing agent or a sulfurizing agent.

### Step (1)

Step (A1) includes a step of removing a temporary protecting group from an n-mer (oligo)nucleotide in which one of the 3'- or 5'-position hydroxyl group is protected with a support protecting group and the other is protected with a removable temporary protecting group (sometimes referred to as a "deprotection step" in this specification), and a step of separating the n-mer (oligo)nucleotide from which the temporary protecting group has been removed from impurities in the reaction solution (sometimes referred to as a "separation step" in this specification).

Step (B1) includes a step of removing a temporary protecting group at only one of the 5'-position hydroxyl group or the 3'-position hydroxyl group from an n-mer (oligo)nucleotide in which a support protecting group is bound to a nucleic acid base at the 3'-terminal or a nucleic acid base at the 5'-terminal, and in which the 3'-position hydroxyl group and the 5'-position hydroxyl group are protected with temporary protecting groups that can be removed under acidic conditions or with a reagent containing a fluorine anion (sometimes referred to as a "deprotection step" in this specification), and a step of separating the n-mer (oligo)nucleotide from which the temporary protecting group has been removed from impurities in the reaction solution (sometimes referred to as a "separation step" in this specification).

n represents any integer of 1 or more. The upper limit of n is not particularly limited, but is preferably 50 or less, more preferably 40 or less, and further preferably 30 or less.

The reaction for removing the temporary protecting group in step (1) (A1 or B1, hereinafter sometimes simply referred to as step (1)) can be carried out with reference to a method known per se. As a method known per se, for example, the reaction can be carried out using reaction conditions described in WO2012/157723 or WO2019/131719. Although not limited thereto, for example, the reaction can be carried out as follows.

The solvent (non-polar or polar solvent) in step (1) of the production method of the present invention is not particularly limited as long as it does not affect the reaction, but since higher solubility in the solvent is expected to result in better reactivity, it is preferable to select a solvent that has a high solubility for the nucleotide to which the pseudo-solid-phase protecting group is bound. Examples of the solvent used in step (1) include aromatic solvents, ester solvents, aliphatic solvents, ether solvents, amide solvents, sulfoxide solvents, nitrile solvents, halogen solvents, and combinations thereof. Preferred examples of the solvent include benzene, toluene, xylene, mesitylene, hexane, pentane, heptane, nonane, cyclohexane, ethyl acetate, isopropyl acetate, tetrahydrofuran, 2-methyltetrahydrofuran, tert-butyl methyl ether, cyclopentyl methyl ether, N,N-dimethylformamide, N,N-dimethylacetamide, N-methylpyrrolidone, dimethyl sulfoxide, acetonitrile, propionitrile, dichloromethane, chloroform, 1,2-dichloroethane, and combinations thereof. More preferred are toluene, xylene, hexane, heptane, cyclohexane, ethyl acetate, isopropyl acetate, tetrahydrofuran, 2-methyltetrahydrofuran, tert-butyl methyl ether, cyclopentyl methyl ether, N,N-dimethylformamide, N,N-dimethylacetamide, N-methylpyrrolidone, dimethyl sulfoxide, and combinations thereof.

The concentration of the n-mer (oligo)nucleotide in the solvent in the deprotection step of step (1) is not particularly limited as long as it is dissolved, but is preferably 1 to 30% by weight.

The acid used in step (1) is not particularly limited, and examples thereof include trifluoroacetic acid, dichloroacetic acid, trifluoromethanesulfonic acid, trichloroacetic acid, methanesulfonic acid, hydrochloric acid, acetic acid, and p-toluenesulfonic acid. Preferred are trifluoroacetic acid, dichloroacetic acid, and trichloroacetic acid. The amount of the acid used may be 1 to 100 moles, and preferably 1 to 40 moles, per 1 mole of the n-mer nucleotide.

The acid may be used alone or in the presence of a cation scavenger. Examples of the cation scavenger are not particularly limited as long as they do not substantially cause reprotection of the removed temporary protecting group or side reactions with the deprotected functional group.

The cation scavenger used in step (1) is not particularly limited as long as it can be used for the deprotection of a protecting group removable under acidic conditions, and examples thereof include optionally substituted alkylthiol derivatives, optionally substituted pyrrole derivatives, optionally substituted indole derivatives, and optionally substituted furan derivatives. Examples of the substituent include, but are not limited to, halogen atoms, C1-6 alkyl groups, C2-6 alkenyl groups, C2-6 alkynyl groups, C1-6 alkoxy groups, hydroxyl groups, aryloxy groups, amino groups, nitro groups, trifluoromethyl groups, phenyl groups, carboxy groups, and the like. Substitution with two or more of these groups may be used. Preferred examples include thiomalic acid, acetylcysteine, pyrrole, 3-methylpyrrole, 2,4-dimethylpyrrole, indole, 4-methylindole, 5-methylindole, 6-methylindole, 7-methylindole, 5,6-dimethylindole, and 6,7-dimethylindole, furan, 2-methylfuran, 3-methylfuran, 2-pyrrolecarboxylic acid, 3-pyrrolecarboxylic acid, indole-2-carboxylic acid, indole-3-carboxylic acid, indole-5-carboxylic acid, 2-furancarboxylic acid, and 3-furancarboxylic acid. The amount of the cation scavenger used is 1 to 50 moles, preferably 5 to 20 moles, per 1 mole of n-mer (oligo)nucleotide.

The reagent containing a fluorine anion used in step (1) is not particularly limited, and examples thereof include pyridine hydrofluoride and tetrabutylammonium fluoride. The amount of the reagent containing a fluorine anion to be used is 1 to 10 moles, preferably 1 to 5 moles, per 1 mole of n-mer (oligo)nucleotide.

The support protecting group bonded to the 3'- or 5'-position hydroxyl group of the n-mer (oligo)nucleotide used in step (A1), or the support protecting group bonded to the 3'-terminal nucleic acid base or the 5'-terminal nucleic acid base of the n-mer (oligo)nucleotide used in step (B1) is a pseudo-solid-phase protecting group of the present invention, and specifically, is a long-chain alkenyloxy-substituted benzoyl derivative represented by any of compounds (1)-(4) in this specification.

The reaction temperature in the deprotection step of step (1) is not particularly limited as long as the reaction proceeds, but is preferably, for example, 10 to 50°C, and more preferably 0 to 40°C. The reaction time may vary depending on reaction conditions, such as the type of n-mer (oligo)nucleotide as a reaction substrate, the type of acid, the type of solvent, the reaction temperature, etc., but may be, for example, 5 minutes to 5 hours.

In step (B1), the temporary protecting group at only one of the 5'-position hydroxyl group or the 3'-position hydroxyl group is removed. Removal of only one of the temporary protecting groups can be performed by appropriately selecting the type of temporary protecting group bonded to the 5'-position hydroxyl group or the 3'-position hydroxyl group. For example, when the temporary protecting group at the 5'-position hydroxyl group is removed but the temporary protecting group at the 3'-position hydroxyl group is not removed, a combination of temporary protecting groups that cleaves the bond of the temporary protecting group at the 5'-position hydroxyl group under appropriate reaction conditions but does not cleave the bond of the temporary protecting group at the 3'-position hydroxyl group may be used. Such a combination of temporary protecting groups and reaction conditions for the cleavage reaction can be appropriately selected by those skilled in the art. For example, but not limited to, a silyl-type protecting group with high chemical stability that is not deprotected by an acid can be used for one, and a trityl-type protecting group that is not deprotected by a reagent containing a fluorine anion can be used for the other, and for example, a combination of a TBDPS group and a DMTr group can be mentioned.

Step (1) includes a step of separating the compound containing the pseudo-solid-phase protecting group after removing the temporary protecting group (sometimes referred to as a "separation step"). The separation can be performed by (i) a step of isolating the n-mer oligonucleotide from which the temporary protecting group has been removed from the reaction solution (sometimes referred to as an "isolation step"), or (ii) a step of washing the reaction solution with water and recovering the organic layer (sometimes referred to as a "water washing step").

In the separation step of step (1), it is preferable to neutralize the reaction solution with a base before the isolation step or recovery step. Neutralizing the reaction solution with a base can, for example, suppress a decrease in purity, and may also allow the deprotected protecting group to be removed into the aqueous layer. There are no particular limitations on the base used as long as it can neutralize the reaction solution, and examples of the base include pyridine, 2,4,6-trimethylpyridine, benzimidazole, 1,2,4-triazole, N-phenylimidazole, 2-amino-4,6-dimethylpyrimidine, 1,10-phenanthroline, imidazole, N-methylimidazole, 2-chlorobenzimidazole, 2-bromobenzimidazole, 2-methylimidazole, 2-phenylbenzimidazole, N-phenylbenzimidazole, and 5-nitrobenzimidazole. Two or more of these may be used in combination.

The isolation step in step (1) can be carried out by crystallizing and recovering the (oligo)nucleotide to which the pseudo-solid-phase protecting group is bound from the reaction solution as is or after neutralization using a polar solvent. This allows the (oligo)nucleotide containing the pseudo-solid-phase protecting group to be isolated from the reaction solution containing impurities. Examples of polar solvents include alcohol-based solvents such as methanol and ethanol, nitrile-based solvents such as acetonitrile, ketone-based solvents such as acetone, ether-based solvents such as tetrahydrofuran, amide-based solvents such as dimethylformamide, sulfoxide-based solvents such as dimethylsulfoxide, water, and mixtures of two or more of these. Alcohol-based solvents and nitrile-based solvents are preferred, and specifically, methanol or acetonitrile are preferred. The polar solvent may contain water to minimize the loss of the product into the polar solvent. The content of water in the polar solvent is, for example, 0 to 10% (v/v), preferably 0 to 8% (v/v). Solid-liquid separation can be carried out with reference to a method known per se.

The water washing step in step (1) can be carried out by washing the reaction solution as it is or after neutralization with water and recovering the organic layer, thereby removing impurities.

### Step (2)

Step (A2) or (B2) is a step of condensing an n-mer (oligo)nucleotide prepared in the step (A1) or (B1), in which the 3'- or 5'-position hydroxyl group is protected with a support protecting group or a temporary protecting group and the temporary protecting group of the other hydroxyl group has been removed, with a p-mer (oligo)nucleotide in which the 3'- or 5'-position hydroxyl group is protected with a temporary protecting group and the other hydroxyl group has been phosphoramiditized, to prepare an n+p-mer oligonucleotide bound by a phosphite triester bond.

p represents any integer of 1 or more. When performing an elongation reaction of a nucleotide, p is preferably 1, and when performing a coupling reaction of an oligonucleotide, p is any integer, and the upper limit is not particularly limited, but is preferably 20 or less, more preferably 10 or less, and still more preferably 5 or less.

The condensation reaction in step (2) (A2 or B2, hereinafter sometimes simply referred to as step (2)) can be carried out with reference to a method known per se. As a method known per se, for example, the reaction conditions described in WO2012/157723 or WO2019/131719 can be used. Although not limited thereto, for example, the reaction can be carried out as follows.

When the n-mer (oligo)nucleotide deprotected in step (1) is recovered by crystallization, it can be dissolved in the solvent used in step (2) to carry out the condensation reaction. When the n-mer (oligo)nucleotide deprotected in step (1) is separated and recovered in the solvent, the condensation reaction can be carried out in the same solvent or, if necessary, by changing the solvent.

In step (2), a weak acid or an acidic neutral salt can be added as an activator to improve the reaction efficiency. Examples of the weak acid or the acidic neutral salt include pyridine trifluoroacetate, 1H-tetrazole, 5-benzylthio-1H-tetrazole, 4,5-dicyanoimidazole, etc. The amount of the weak acid or the acidic neutral salt used can be in the range of 0.1 to 50 molar equivalents, for example 1 to 5 molar equivalents, per 1 mole of the n-mer (oligo)nucleotide from which the temporary protecting group at the hydroxyl group has been removed, obtained in step (1) as the reaction substrate.

In the condensation step of step (2), since a side reaction of elimination of the temporary protecting group may occur if the acidity of the reaction solution becomes high, it is preferable to add N-methylimidazole in order to suppress the acidification of the reaction solution. The amount of N-methylimidazole used can be in the range of 0.1 to 10 molar equivalents, for example 0.1 to 1 molar equivalent, per 1 mole of the n-mer (oligo)nucleotide from which the temporary protecting group at the hydroxyl group has been removed obtained in step (1).

The mixing ratio of the p-mer (oligo)nucleotide in which the 5'- or 3'-position hydroxyl group is protected with a temporary protecting group and the other hydroxyl group has been phosphoramiditized to the n-mer (oligo)nucleotide obtained in step (A1) in which the 3'- or 5'-position hydroxyl group is protected with a support protecting group and the temporary protecting group of the other hydroxyl group has been removed, can be 1 to 10 molar equivalents, preferably 1 to 5 molar equivalents, of the p-mer (oligo)nucleotide per 1 mole of the n-mer (oligo)nucleotide.

The mixing ratio of a p-mer (oligo)nucleotide in which the 5'- or 3'-position hydroxyl group is protected with a temporary protecting group and the other hydroxyl group is phosphoramiditized, to the n-mer (oligo)nucleotide obtained in step (B1) in which only one of the 3'- or 5'-position hydroxyl group is protected with a temporary protecting group and the temporary protecting group of the other hydroxyl group is removed, can be 1 to 10 molar equivalents, preferably 1 to 5 molar equivalents, of the p-mer (oligo)nucleotide per 1 mole of the n-mer (oligo)nucleotide.

Step (2) can be carried out in a solvent that does not affect the condensation reaction. Examples of the solvent include, but are not limited to, the same non-polar and polar solvents as those in step (1), such as aromatic solvents, ester solvents, aliphatic solvents, ether solvents, amide solvents, sulfoxide solvents, halogen solvents, nitrile solvents, and combinations thereof.

The reaction temperature in step (2) is not particularly limited as long as the reaction proceeds, but is preferably, for example, -20 to 100°C, more preferably 20 to 50°C. The reaction time may vary depending on the type of n-mer (oligo)nucleotide to be condensed, the reaction temperature, etc., but may be, for example, 5 minutes to 24 hours.

### Step (3)

Step (A3) or (B3) is a step (oxidation or sulfurization reaction step) of converting the phosphite triester bond of the n+p-mer oligonucleotide obtained by the step (A2) or (B2) into a phosphate triester bond or a thiophosphate triester bond, respectively, using an oxidizing agent or a sulfurizing agent.

The oxidation or sulfurization reaction step in step (3) (A3 or B3, hereinafter sometimes simply referred to as step (3)) can be carried out with reference to a method known per se. As a method known per se, for example, the reaction can be carried out using reaction conditions described in WO2012/157723 or WO2019/131719. Although not limited thereto, for example, the reaction can be carried out as follows.

The reaction in step (3) can also be carried out by simply adding an oxidizing agent or a sulfurizing agent directly to the reaction solution in step (2) without isolating the n+p-mer oligonucleotide obtained in step (2).

The oxidation reaction using an oxidizing agent in step (3) can be carried out according to a method generally known in oligonucleotide synthesis. Examples of the oxidizing agent include, but are not limited to, iodine, (1S)-(+)-(10-camphorsulfonyl)oxaziridine, tert-butyl hydroperoxide (TBHP), 2-butanone peroxide, 1,1-dihydroperoxycyclododecane, bis(trimethylsilyl)peroxide, cumene hydroperoxide, hydrogen peroxide, and m-chloroperbenzoic acid, with iodine, (1S)-(+)-(10-camphorsulfonyl)oxazolidine, tert-butyl hydroperoxide (TBHP), 2-butanone peroxide, and 1,1-dihydroperoxycyclododecane being preferred.

The sulfurization reaction using a sulfurizing agent in step (3) can be carried out according to a method generally known in oligonucleotide synthesis. Examples of sulfurizing agents include, but are not limited to, N,N-dimethyl-N'-(3-thioxo-3H-1,2,4-dithiazol-5-yl)imidoformamide (DDTT), 3H-1,2-benzodithiol-3-one-1,1-dioxide (Beaucage reagent), 3H-1,2-benzodithiol-3-one, phenylacetyl disulfide (PADS), tetraethylthiuram disulfide (TETD), 3-amino-1,2,4-dithiazol-5-thione (ADTT), or sulfur, with N,N-dimethyl-N'-(3-thioxo-3H-1,2,4-dithiazol-5-yl)imidoformamide being preferred.

The phosphite triester bond of an oligonucleotide can be converted to a thiophosphate triester bond by a sulfurization reaction. Thiophosphate has an asymmetric center at the phosphorus atom, so it gives an optically active substance. Even if a deprotection reaction is performed in the manufacturing process of an oligonucleotide thiophosphate, the asymmetric center at the phosphorus atom remains, giving an optically active substance.

The reaction in step (3) is usually carried out in a solvent. The solvent used in the reaction is preferably anhydrous solvent, and examples thereof include halogen-based solvents (e.g., chloroform), aliphatic solvents (e.g., cyclohexane), aromatic solvents (e.g., toluene), ester-based solvents (e.g., ethyl acetate), ether-based solvents (e.g., tert-butyl methyl ether), nitrile-based solvents (e.g., acetonitrile), and mixed solvents of two or more selected from these.

The amount of the oxidizing agent or sulfurizing agent added may be about 1.0 to about 5.0 molar equivalents, preferably about 1.0 to about 1.5 molar equivalents, per 1 mole of the phosphoramidite obtained in step (2). Alternatively, the amount of the oxidizing agent or sulfurizing agent added may be, for example, 1 to 50 molar equivalents, preferably 1 to 5 molar equivalents, per 1 mole of the (oligo)nucleotide obtained in step (2).

The reaction temperature is not particularly limited as long as the reaction proceeds, but may be 0 to 100°C, preferably 20 to 50°C. The reaction time may vary depending on the type of n+p-mer oligonucleotide, the type of oxidizing agent or sulfurizing agent used, the reaction temperature, and the like, but may be, for example, 1 minute to 3 hours.

### Step (4) (recovery step)

The production method of the present invention may further include a step of recovering the n+p-mer oligonucleotide obtained in step (3) (sometimes referred to as a "recovery step"). The recovery step can be carried out in the same manner as the separation step (isolation step or water washing step) described in step (1). Preferably, it is an isolation step in which the (oligo)nucleotide bound to the pseudo-solid-phase protecting group is crystallized and recovered using a polar solvent.

Therefore, one embodiment of the production method of the present invention further includes a step of adding a polar solvent to the reaction solution obtained in step (3) to precipitate the n+p-mer oligonucleotide, and obtaining it by solid-liquid separation.

Alternatively, step (4) (recovery step) can be carried out after step (1).

### Step (5) (Deprotection of all protecting groups)

The production method of the present invention may further include a step of removing all protecting groups from the n+p-mer oligonucleotides recovered in step (4) (sometimes referred to as a "all protecting groups removing step"). The step of removing all protecting groups can be carried out with reference to a method known per se. Examples of the method known per se include the deprotection method described in Protective Group In Organic Synthesis, 3rd Edition, John Willy & Sons (1999), etc., and the conditions described in WO2012/157723 and WO2019/131719.

Therefore, one embodiment of the production method of the present invention further comprises a step of removing all protecting groups from the n+p-mer oligonucleotide obtained in the step (4).

### (Confirmation of reaction progress)

The progress of the reaction in each step of the oligonucleotide production method can be monitored and confirmed by the same techniques commonly used in liquid-phase organic synthesis reactions, for example, thin layer silica gel chromatography (TLC), high performance liquid chromatography (HPLC), or the like.

Another aspect of the present invention is a method for producing (oligo)nucleotides without using a halogenated solvent. In conventional (oligo)nucleotide production methods, halogenated solvents are often used in the elongation reaction. On the other hand, in the (oligo)nucleotide production method of the present invention, the solubility of the pseudo-solid-phase protecting group is excellent, so that the elongation reaction can be carried out without using a halogenated solvent.

### IV. Oligonucleotide-Oligonucleotide Coupling

One embodiment of the present invention is a method for producing an oligonucleotide by coupling oligonucleotides to each other, comprising the following steps (C1)-(C4):
(C1) a step of obtaining an n-mer oligonucleotide from which the temporary protecting group at the 5'-position hydroxyl group has been removed and a p-mer oligonucleotide from which the temporary protecting group at the 3'-position hydroxyl group has been removed by carrying out the following steps (a) and (b), respectively:
   (a) a step of reacting, in a non-polar or polar solvent, an n-mer oligonucleotide (n is any integer of 1 or more) in which the 3' -position hydroxyl group is protected with a support protecting group and the 5'-position hydroxyl group is protected with a temporary protecting group removable under acidic conditions or with a reagent containing a fluorine anion, with an acid and a cation scavenger, or with a reagent containing a fluorine anion (preferably pyridine hydrofluoride or tetrabutylammonium fluoride) to remove the temporary protecting group at the 5'-position hydroxyl group, and then separating the n-mer oligonucleotide from impurities in the reaction solution;
   (b) a step of reacting, in a non-polar or polar solvent, a p-mer oligonucleotide (p is any integer of 1 or more) in which a support protecting group is bound to a nucleic acid base at the 3' -terminal and the 3'-position hydroxyl group and the 5'-position hydroxyl group are protected with temporary protecting groups removable under acidic conditions or with a reagent containing a fluorine anion, with an acid and a cation scavenger, or with a reagent containing a fluorine anion (preferably pyridine hydrofluoride or tetrabutylammonium fluoride) to remove only the temporary protecting group at the 3'-position hydroxyl group, and then separating the p-mer oligonucleotide from impurities in the reaction solution;
   wherein the support protecting groups of (a) and (b) may be the same or different;
(C2) a step of phosphoramiditizing the 5'-position hydroxyl group of the n-mer oligonucleotide from which the temporary protecting group at the 5'-position hydroxyl group has been removed obtained in step (C1), or a step of phosphoramiditizing the 3'-position hydroxyl group of the p-mer oligonucleotide from which the temporary protecting group at the 3'-position hydroxyl group has been removed obtained in step (C1),
(C3) a step of adding the n-mer oligonucleotide whose 5'-position hydroxyl group has been phosphoramiditized obtained in step (C2) to a solution of the p-mer oligonucleotide from which the temporary protecting group at the 3'-position hydroxyl group has been removed obtained in step (C1), or adding the p-mer oligonucleotide whose 3'-position hydroxyl group has been phosphoramiditized obtained in step (C2) to a solution of the n-mer oligonucleotide from which the temporary protecting group at the 5'-position hydroxyl group has been removed obtained in step (C1), thereby condensing the n-mer oligonucleotide and the p-mer oligonucleotide through a phosphite triester bond;
(C4) a step of converting the phosphite triester bond of the n+p-mer oligonucleotide obtained in the step (C3) into a phosphate triester bond or a thiophosphate triester bond using an oxidizing agent or a sulfurizing agent.

Step (C1) is a step of obtaining an n-mer oligonucleotide from which the temporary protecting group at the 5'-position hydroxyl group has been removed, and a p-mer oligonucleotide from which the temporary protecting group at the 3'-position hydroxyl group has been removed. n in the n-mer oligonucleotide represents any integer of 1 or more. The lower limit of n is not particularly limited, but is preferably 2 or more, more preferably 5 or more, and still more preferably 10 or more, and the upper limit of n is not particularly limited, but is preferably 40 or less, more preferably 30 or less, and still more preferably 20 or less. p in the p-mer oligonucleotide represents any integer of 1 or more. The lower limit of p is not particularly limited, but is preferably 2 or more, more preferably 5 or more, and still more preferably 10 or more, and the upper limit of p is not particularly limited, but is preferably 40 or less, more preferably 30 or less, and still more preferably 20 or less.

The deprotection step and the separation step in step C1 can be carried out with reference to a method known per se. As a method known per se, for example, the reaction conditions described in WO2012/157723 and WO2019/131719 can be used. Although not limited thereto, for example, the deprotection step and the separation step can be carried out in the same manner as described in the step (1).

Step (C2) is a step of phosphoramiditizing the 5'-position hydroxyl group of the n-mer oligonucleotide from which the temporary protecting group at the 5'-position hydroxyl group has been removed obtained in step (C1), or a step of phosphoramiditizing the 3'-position hydroxyl group of the p-mer oligonucleotide from which the temporary protecting group at the 3'-position hydroxyl group has been removed obtained in step (C1). The step of phosphoramiditizing the hydroxyl group can be carried out with reference to a method known per se. As a method known per se, for example, it can be carried out using the reaction conditions described in Patent Document 10.

Step (C3) is a step of condensing an n-mer oligonucleotide with a p-mer oligonucleotide, one of which is phosphoramidite, through a phosphite triester bond. The condensation step can be carried out by referring to a method known per se. Although not limited thereto, it can be carried out, for example, in the same manner as described in the step (2).

Step (C4) is a step of converting the phosphite triester bond of the n+p-mer oligonucleotide obtained in step (C3) into a phosphate triester bond or a thiophosphate triester bond. The oxidation or sulfurization reaction step can be carried out with reference to a method known per se. As a method known per se, for example, it can be carried out using the reaction conditions described in WO2012/157723 or WO2019/131719. Although not limited thereto, it can be carried out, for example, in the same manner as described in the step (3).

After step (C4), a recovery step and a step of removing all protecting groups from the recovered n+p-mer oligonucleotide can be performed. Although not limited thereto, for example, the steps can be performed in the same manner as described in the steps (4) and (5).

In addition, the recovery step can be carried out after step (C1) or after step (C2), or after any two of steps (C1), (C2), and (C4), or even after all of steps (C1), (C2), and (C4).

### [Examples]

The present invention will be specifically described below with reference to Examples; however, the present invention is not limited to the following Examples.

### (Example 1) Synthesis of Compound 4 (2-((3,4,5-tri-((Z)-octadec-9-enyloxy)benzoyl)oxy)acetic acid)

Compound 4, which is a pseudo-solid-phase protecting group, was synthesized as follows.

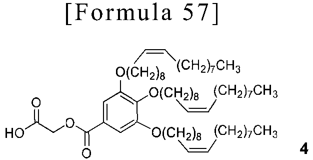

### Synthesis of Compound 1 (Methyl 3,4,5-tri-((Z)-octadec-9-enyloxy)benzoate)

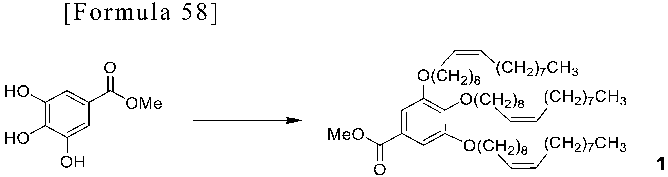

Under a nitrogen atmosphere, methyl gallate (0.52 g, 2.82 mmol), K₂CO₃ (2.34 g, 16.9 mmol), DMF (10.4 mL), and oleyl bromide (2.99 g, 9.04 mmol) were mixed at room temperature and stirred for 10 minutes, then followed by heated to 70 °C and stirring for 3.5 hours. After completion of the reaction, the mixture was cooled to room temperature, and toluene (20 mL), water (20 mL, 38 v) and ethyl acetate (10 mL) were added, followed by separation of the aqueous layer. The separated aqueous layer was extracted with a mixed solution of n-heptane (10 mL) and ethyl acetate (10 mL). The combined organic layers were washed with water (20 mL) and concentrated under reduced pressure to obtain the crude product of Compound 1. The obtained crude product was purified by silica gel column chromatography (n-hexane/ethyl acetate) to give Compound 1 as an oily material (2.48 g, 2.65 mmol, yield: 94%). ¹H NMR (600 MHz, CDCl₃): δ 7.27 - 7.24 (m, 2H), 5.37 - 5.32 (m, 6H), 4.03 - 3.98 (m, 6H), 3.89 (s, 3H), 2.05 - 1.98 (m, 12H), 1.81 (quin, J = 7.1 Hz, 4H), 1.77 - 1.71 (m, 2H), 1.51 - 1.38 (m, 6H), 1.37 - 1.23 (m, 60H), 0.90 - 0.85 (m, 9H)

### Synthesis of Compound 2 (3,4,5-tri-((Z)-octadec-9-enyloxy)benzoic acid)

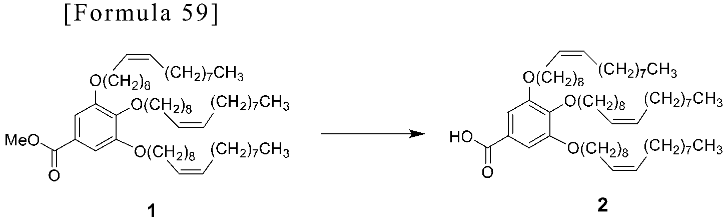

Compound 1 (2.33 g, 2.49 mmol), ethanol (23.3 mL) and 1 M NaOH aqueous solution (5.60 mL, 3.74 mmol) were mixed at room temperature, followed by heated to 80 °C and stirring for 5 hours. Tetrahydrofuran (THF) (4.66 mL) was added, followed by stirring at 70 °C for 3 hours. After completion of the reaction, the reaction mixture was cooled to 0 °C, and 2 M HCl aqueous solution (2.5 mL, 5.0 mmol) was added dropwise to adjust the pH to 3 - 4. Ethyl acetate (20 mL), n-heptane (40 mL) and water (20 mL) were added to the reaction solution, and the aqueous layer was separated. The organic layer was concentrated under reduced pressure, and the obtained crude product was purified by silica gel column chromatography (n-hexane/ethyl acetate) to give Compound 2 as a colorless oily substance (2.19 g, yield: 95%).
¹H NMR (600 MHz, CDCl₃): δ 7.33 - 7.28 (m, 2H), 5.38 - 5.32 (m, 6H), 4.06 - 3.99 (m, 6H), 2.05 - 1.98 (m, 12H), 1.85 - 1.77 (m, 4H), 1.77 - 1.71 (m, 2H), 1.52 - 1.42 (m, 6H), 1.37 - 1.23 (m, 60H), 0.90 - 0.85 (m, 9H)

### Synthesis of Compound 3

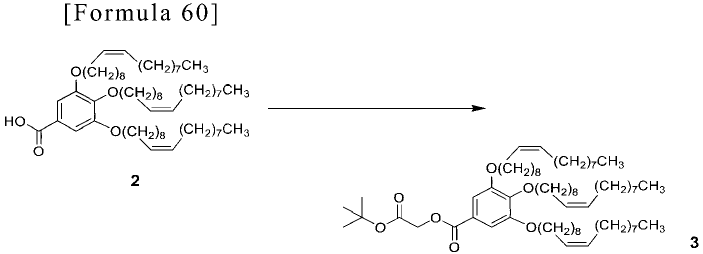

Under a nitrogen atmosphere, Compound 2 (2.00 g, 2.17 mmol), THF (12 mL), triethylamine (439 mg, 4.34 mmol) and tert-butyl bromoacetate (847 mg, 4.34 mmol) were mixed, followed by heated to 60 °C and stirring for 2 hours. Triethylamine (219 mg, 2.17 mmol) and tert-butyl bromoacetate (423 mg, 2.17 mmol) were added to the reaction solution, followed by stirring at 60 °C for 1 hour. Triethylamine (219 mg, 2.17 mmol) and tert-butyl bromoacetate (423 mg, 2.17 mmol) were again added, followed by stirring at 60 °C for 2 hours. After confirming completion of the reaction, the mixture was cooled to room temperature, and ethyl acetate (20 mL), n-hexane (20 mL) and water (20 mL) were added, followed by separation of the aqueous layer. The organic layer was washed with water (20 mL) and concentrated under reduced pressure to obtain the crude product. The obtained crude product was purified by silica gel column chromatography (n-hexane/ethyl acetate). Azeotropic distillation with toluene (100 mL) was carried out three times to give Compound 3 as a colorless oily substance (2.21 g, yield: 95%).
¹H NMR (600 MHz, CDCl₃): δ 7.30 (s, 2H), 5.38 - 5.31 (m, 6H), 4.74 - 4.66 (m, 2H), 4.01 (q, J = 6.7 Hz, 6H), 2.05 - 1.97 (m, 12H), 1.83 - 1.76 (m, 4H), 1.76 - 1.69 (m, 2H), 1.50 - 1.43 (m, 15H), 1.36 - 1.23 (m, 60H), 0.91 - 0.85 (m, 9H)

### Synthesis of Compound 4

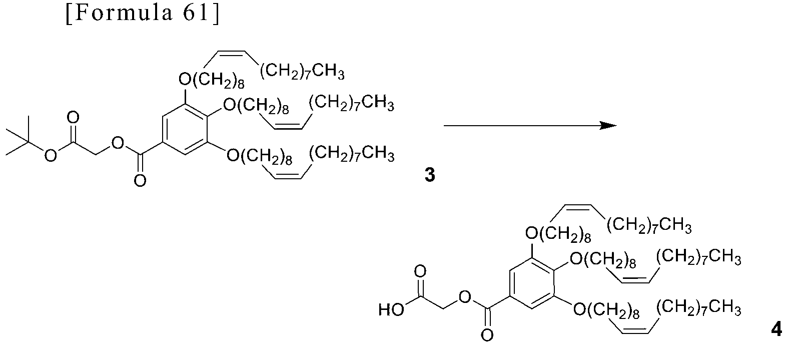

Under a nitrogen atmosphere, Compound 3 (2.00 g, 1.93 mmol), chloroform (8 mL) and trifluoroacetic acid (2.96 mL, 38.6 mmol) were mixed, followed by stirring at 50 °C for 2 hours. After confirming completion of the reaction, 5% NaHCO₃ aqueous solution (40 mL) was slowly added dropwise to the reaction solution cooled to 0 °C to adjust the pH to 4 - 5. Ethyl acetate (30 mL) was added, and the layers were separated. The aqueous layer was extracted with ethyl acetate (30 mL), and the organic layers were combined, washed with 20% NaCl aqueous solution (20 mL), and concentrated under reduced pressure to obtain the crude product. The obtained crude product was purified by silica gel column chromatography (n-hexane/ethyl acetate) to give Compound 4 as a viscous yellow oil (1.81 g, yield: 96%).
¹H NMR (600 MHz, CDCl₃): δ 7.30 (s, 2H), 5.38 - 5.32 (m, 6H), 4.87 (s, 2H), 4.05 - 3.97 (m, 6H), 2.04 - 1.99 (m, 12H), 1.81 (quin, J = 7.1 Hz, 4H), 1.77 - 1.71 (m, 2H), 1.47 (quin, J = 7.4 Hz, 6H), 1.36 - 1.24 (m, 60H), 0.88 (t, J = 7.0 Hz, 9H)

### (Example 2) Production of polyoligonucleotide bound to Compound 4

A 3-mer polyoligonucleotide was produced using Compound 4 as a pseudo-solid-phase protecting group as follows.

### Synthesis of Compound 5

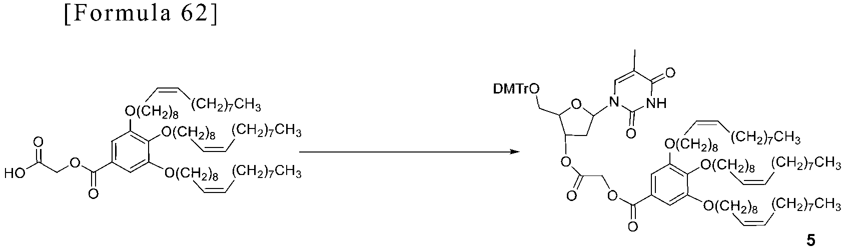

Under a nitrogen atmosphere, to a solution of Compound 4 (1.10 g, 1.13 mmol), 5'-O-(4,4'-dimethoxytrityl)thymidine (DMT-dT) (1.23 g, 2.25 mmol) and 4-dimethylaminopyridine (DMAP) (27.5 mg, 0.23 mmol) in THF (14 mL), 1-ethyl-3-(3-dimethylaminopropyl)carbodiimide hydrochloride (EDCI) (432 mg, 2.25 mmol) was added at room temperature, followed by stirring at the same temperature for 21 hours. After confirming completion of the reaction, ethyl acetate (30 mL) and 5% NaHCO₃ aqueous solution (20 mL) were added to the reaction solution, and the organic layer was separated from the aqueous layer. The aqueous layer was extracted with ethyl acetate (15 mL), and the combined organic layers were washed with 20% NaCl aqueous solution (20 mL) and water (20 mL). The organic layer was concentrated under reduced pressure, and the obtained crude product was purified by silica gel column chromatography (n-hexane/ethyl acetate) to give Compound 5 as a viscous, colorless oil (1.43 g, yield: 84%).
¹H NMR (600 MHz, CDCl₃): δ 7.96 (s, 1H), 7.60 (s, 1H), 7.39 - 7.34 (m, 2H), 7.31 - 7.22 (m, 9H), 6.83 (d, J = 8.7 Hz, 4H), 6.46 - 6.41 (m, 1H), 5.58 - 5.53 (m, 1H), 5.37 - 5.32 (m, 6H), 4.84 - 4.79 (m, 2H), 4.18 (s, 1H), 4.03 - 3.98 (m, 6H), 3.78 (d, J = 1.1 Hz, 6H), 3.53 - 3.43 (m, 2H), 2.56 - 2.43 (m, 2H), 2.05 - 1.99 (m, 11H), 1.83 - 1.77 (m, 4H), 1.77 - 1.71 (m, 2H), 1.50 - 1.43 (m, 6H), 1.36 - 1.23 (m, 63H), 0.87 (t, J = 7.0 Hz, 9H)

### Synthesis of Compound 6

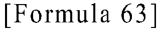

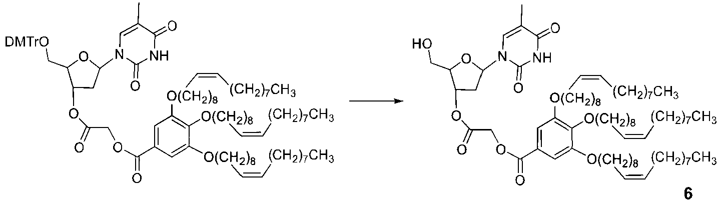

Under a nitrogen atmosphere, to a solution of Compound 5 (2.46 g, 1.63 mmol) in ethyl acetate (24.6 mL), mercaptosuccinic acid (1.23 g, 8.17 mmol) and trifluoroacetic acid (1.25 mL, 16.4 mmol) were added, followed by stirring under ice cooling for 1 hour. Then the solution was warmed to room temperature and stirred for an additional 1.5 hours. After confirming completion of the reaction, the reaction solution was washed four times with 5% NaHCO₃ aqueous solution (25 mL) and once with 10% brine (25 mL), followed by concentration under reduced pressure to give Compound 6 as a colorless amorphous solid (1.97 g, yield: 100%).
¹H NMR (600 MHz, CDCl₃): δ 8.08 - 7.97 (m, 1H), 7.47 - 7.42 (m, 1H), 7.29 (s, 2H), 6.23 - 6.15 (m, 1H), 5.51 - 5.44 (m, 1H), 5.38 - 5.32 (m, 6H), 4.83 (s, 2H), 4.18 - 4.13 (m, 1H), 4.06 - 3.98 (m, 6H), 3.98 - 3.89 (m, 2H), 2.55 - 2.47 (m, 1H), 2.46 - 2.38 (m, 1H), 2.36 - 2.28 (m, 1H), 2.04 - 1.98 (m, 11H), 1.95 - 1.91 (m, 3H), 1.84 - 1.77 (m, 4H), 1.77 - 1.71 (m, 2H), 1.51 - 1.38 (m, 6H), 1.37 - 1.23 (m, 60H), 0.88 (t, J = 7.0 Hz, 9H)

### Synthesis of Compound 7

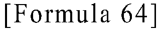

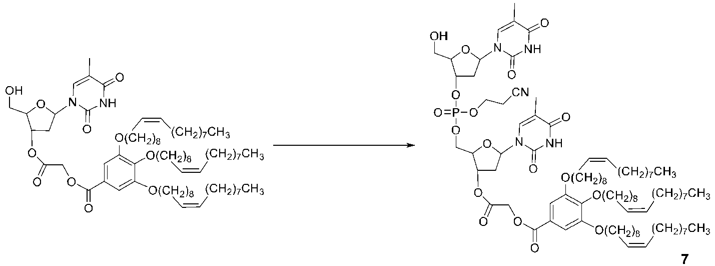

Compound 6 (500 mg, 0.42 mmol) was subjected to azeotropic distillation with ethyl acetate (10 mL) three times. To the obtained Compound 6 were added molecular sieves 3A (500 mg), DMT-dT-CE-phosphoramidite (464 mg, 0.62 mmol) and ethyl acetate (5.0 mL), followed by stirring at room temperature for 1 hour. 4,5-Dicyanoimidazole (DCI) (121 mg, 1.03 mmol) was then added to the reaction mixture, followed by stirring at room temperature for 2 hours. After confirming completion of the reaction, iodine (237 mg, 0.93 mmol) in an acetonitrile:pyridine:water = 95:4:1 mixture (4.67 mL) was added dropwise to the reaction mixture at room temperature, followed by stirring for 1 hour. After confirming completion of the reaction, 1 M Na₂S₂O₃ aqueous solution (7.5 mL) was added, followed by stirring. The insoluble matter was removed by filtration, and the layers were separated. The organic layer was washed with 5% brine (7.5 mL), and dried over sodium sulfate (10 g). After concentrating the organic layer under reduced pressure, azeotropic distillation with ethyl acetate (5 mL) was performed twice. The residue was dissolved in ethyl acetate (5 mL), mercaptosuccinic acid (312 mg, 2.08 mmol) was added, and the mixture was cooled to 0 °C. Trifluoroacetic acid (318 µL, 4.15 mmol) was added, followed by stirring at the same temperature for 1 hour, then warming to room temperature and stirring for an additional 1 hour. After confirming completion of the reaction, water (7.5 mL) was added and the organic layer was separated. The obtained organic layer was washed six times with 5% NaHCO₃ aqueous solution (15 mL) and six times with 5% NaCl aqueous solution (15 mL). The obtained organic layer was concentrated to give Compound 7 as a white amorphous solid (564 mg, yield: 87%).

### Synthesis of Compound 8

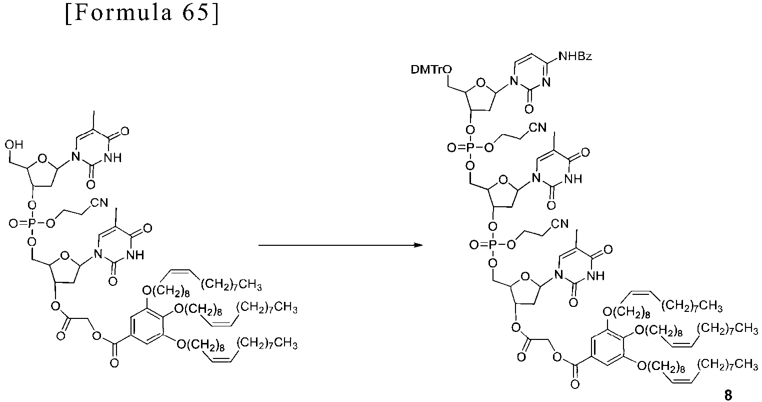

Compound 7 (200 mg, 0.13 mmol) was subjected to azeotropic distillation with ethyl acetate (2 mL) four times. To the obtained Compound 7 were added molecular sieves 3A (200 mg), DMT-dC-CE-phosphoramidite (214 mg, 0.26 mmol) and ethyl acetate (2.0 mL), followed by stirring at room temperature for 1 hour. DCI (49.9 mg, 0.42 mmol) was then added to the reaction solution, followed by stirring at room temperature for 2 hours. Subsequently, DMT-dC-CE-phosphoramidite (106 mg, 0.13 mmol) and DCI (25.0 mg, 0.21 mmol) were added, followed by stirring at room temperature for 2 hours. After confirming completion of the reaction, iodine (146 mg, 0.58 mmol) in acetonitrile:pyridine:water = 95:4:1 (2.7 mL) was added dropwise to the reaction solution at room temperature, followed by stirring for 1 hour. After confirming completion of the reaction, 1 M Na₂S₂O₃ aqueous solution (3.0 mL) and ethyl acetate (3.0 mL) were added. The insoluble matter was removed by filtration, and the aqueous layer was separated from the organic layer. The obtained organic layer was washed with 5% NaHCO₃ aqueous solution (3.0 mL) and 5% brine (3.0 mL), and dried over sodium sulfate (4.0 g). After concentration under reduced pressure, the obtained crude product was purified by silica gel column chromatography (ethyl acetate/methanol) to give Compound 8 as a pale yellow amorphous solid (256 mg, yield: 86%).

### (Example 3) Deprotection of pseudo-solid-phase protecting group

Compound 4, as the pseudo-solid-phase protecting group, was removed from the oligonucleotide as follows.

### Synthesis of Compound 9

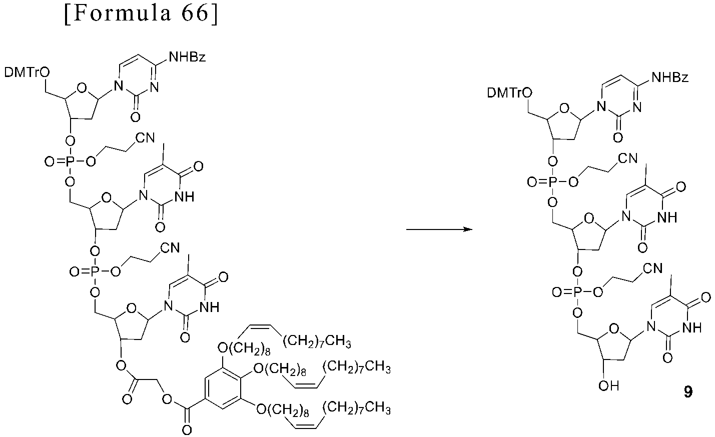

Under a nitrogen atmosphere, to a solution of Compound 8 (100 mg, 0.04 mmol) in anhydrous THF (2.0 mL) and isopropanol (0.5 mL), a 4 M lithium borohydride solution in THF(54.1 µL, 0.22 mmol) was added dropwise at 0 °C, followed by stirring at 0 °C for 1 hour. Then 20% ammonium chloride aqueous solution (2.0 mL), 10% brine (1.0 mL) and ethyl acetate (4.0 mL) were added, followed by stirring, and the layers were separated. The aqueous layer was extracted with ethyl acetate (4.0 mL), and the combined organic layers were washed with 10% brine (2.0 mL). The obtained organic layer was concentrated and purified by silica gel column chromatography (ethyl acetate/methanol) to give Compound 9 as a white amorphous solid (26.9 mg, 46%).

### (Example 4) Synthesis of Compound 11 (2-((3,4,5-tri-((Z)-octadec-9-enyloxy)benzoyl)-N-methylamino)acetic acid)

Compound 11, as the pseudo-solid-phase protecting group, was synthesized as follows.

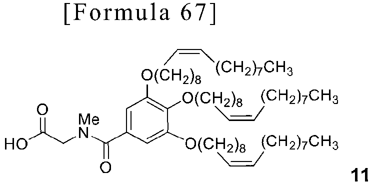

### Synthesis of Compound 10

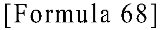

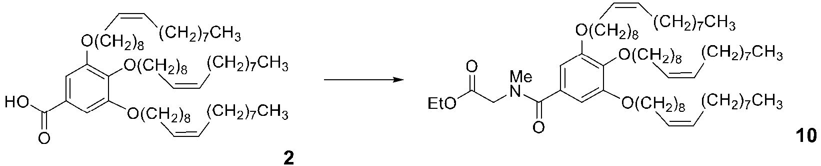

Under a nitrogen atmosphere, to a solution of Compound 2 (1.57 g, 1.7 mmol), EDCI (0.42 g, 2.2 mmol) and 1-hydroxybenzotriazole (0.29 g, 2.2 mmol) in N,N-dimethylformamide (13.9 mL) and toluene (3.3 mL) were added sarcosine ethyl hydrochloride (0.54 g, 3.5 mmol) and triethylamine (375 mg, 3.7 mmol) at room temperature, followed by stirring for 14 hours. EDCI (85 mg, 0.44 mmol) was added, followed by stirring at room temperature for 1 hour. EDCI (85 mg, 0.44 mmol) was further added, followed by stirring at room temperature for 1 hour. n-Heptane (16.3 mL) and 1 M HCl (14.7 mL) were added to the reaction solution, followed by stirring, and the organic layer and aqueous layer were separated. The obtained organic layer was washed with 1 M HCl (8.1 mL) and then with 5% brine (8.1 mL). The aqueous layers from the washing were combined and extracted with a solution of heptane (8.1 mL) and a small amount of ethyl acetate. The combined organic layers were dried over magnesium sulfate, concentrated, and purified by silica gel chromatography (spherical neutral silica gel, developing solvent: n-hexane/ethyl acetate) to obtain oily Compound 10 (1.61 g, yield: 93%). ¹H NMR (600 MHz, CDCl₃): δ 0.83-0.93 (m, 12H), 1.19-1.39 (m, 60H), 1.40-1.50 (m, 6H), 1.68-1.84 (m, 6H), 1.96-2.06 (m, 12H), 2.99-3.16 (m, 3H), 3.84-4.32 (m, 10H), 5.30-5.40 (m, 6H), 6.56-6.70 (m, 2H)

### Synthesis of Compound 11

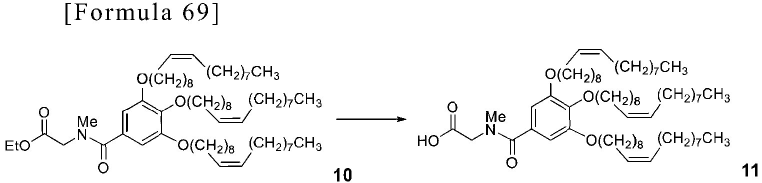

Under a nitrogen atmosphere, to a solution of Compound 10 (1.47 g, 1.44 mmol) in THF (12.0 mL) was added 1 M NaOH aqueous solution (1.76 mL), followed by stirring at room temperature for 1 hour. Ethanol (6.0 mL) and 2 M NaOH aqueous solution (0.6 mL) were added, followed by stirring at room temperature for 40 minutes. Ethanol (6.0 mL) was added, followed by stirring at room temperature for 1 hour. 2 M aqueous HCl (1.8 mL) was added to acidify the mixture, and the reaction mixture was concentrated under reduced pressure. Ethyl acetate (15 mL) and water (12 mL) were added to the concentrate, followed by stirring, and the organic and aqueous layers were separated. The aqueous layer was extracted with ethyl acetate (12 mL). The combined organic layers were washed with 5% brine (12 mL) and concentrated. Ethyl acetate was added, followed by concentration to remove water, and purification by silica gel chromatography (spherical neutral silica gel, developing solvent: n-hexane/ethyl acetate → ethyl acetate/methanol) to give oily Compound 11 (1.39 g, yield: 97.0%).
¹H NMR (600 MHz, CDCl₃): δ 0.85-0.90 (m, 9H), 1.19-1.39 (m, 60H), 1.40-1.51 (m, 6H), 1.68-1.84 (m, 6H), 1.96-2.06 (m, 12H), 3.10 (s, 3H), 3.85-4.30 (m, 8H), 5.30-5.40 (m, 6H), 6.56-6.70 (m, 2H)

### (Example 5) Production of polyoligonucleotide bound to Compound 11

A 3-mer polyoligonucleotide was produced using Compound 11 as a pseudo-solid-phase protecting group as follows.

### Synthesis of Compound 12

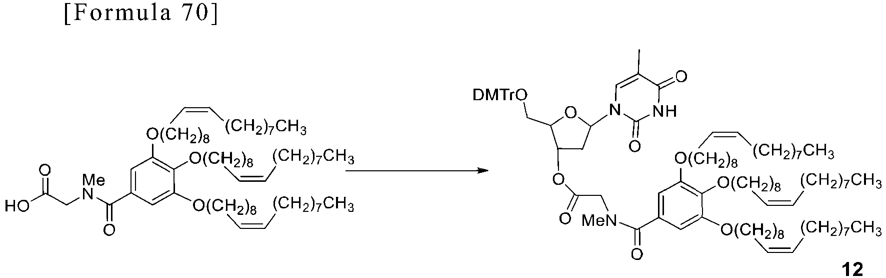

Under a nitrogen atmosphere, to a solution of Compound 11 (380 mg, 0.38 mmol) and DMT-dT (336 mg, 0.62 mmol) in THF (4.1 mL) were added DMAP (10.0 mg) and EDCI (118 mg, 0.62 mmol), followed by stirring at room temperature for 2 hours. EDCI (39 mg) was added, followed by stirring at room temperature for 3 hours. Water (4.1 mL) and ethyl acetate (4.1 mL) were added, followed by stirring, and the organic and aqueous layers were separated. The organic layer was concentrated, and azeotropic distillation with acetonitrile and ethyl acetate was performed to remove water. Then, purification by silica gel chromatography (spherical neutral silica gel, developing solvent: n-hexane/ethyl acetate) gave Compound 12 as a white amorphous solid (518 mg, yield: 89.0%).
¹H NMR (600 MHz, CDCl₃): δ 0.83-0.93 (m, 9H), 1.17-1.39 (m, 60H), 1.40-1.52 (m, 6H), 1.60 (s, 3H), 1.68-1.84 (m, 6H), 1.94-2.08 (m, 12H), 2.38-2.59 (m, 2H), 3.08 (s, 3H), 3.45-3.55 (m, 2H), 3.79 (s, 6H), 3.85-4.00 (m, 6H), 4.05-4.35 (m, 3H), 5.30-5.40 (m, 6H), 5.49-5.61 (m, 1H), 6.34-6.50 (m, 1H), 6.65 (s, 2H), 6.84 (d, 4H, J = 12.0 Hz), 7.20-7.40 (m, 9H), 7.62 (s, 1H), 8.12 (s, 1H)

### Synthesis of Compound 13

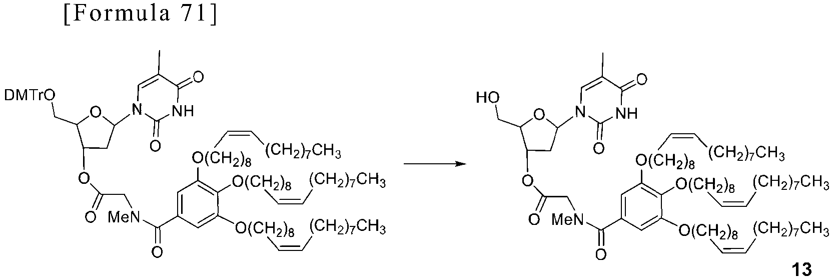

Under a nitrogen atmosphere, to a solution of Compound 12 (468 mg, 0.308 mmol) in dichloromethane (4.7 mL) were added pyrrole (0.107 mL, 1.54 mmol) and trifluoroacetic acid (0.295 mL, 0.385 mmol) at room temperature, followed by stirring for 1 hour. After methanol (4.7 mL) was added, followed by concentration and purification by silica gel chromatography (spherical neutral silica gel, developing solvent: n-hexane/ethyl acetate → ethyl acetate/methanol) to give Compound 13 as a white amorphous solid (353 mg, yield: 94%).
¹H NMR (600 MHz, CDCl₃): δ 0.81-0.96 (m, 9H), 1.17-1.39 (m, 60H), 1.40-1.53 (m, 6H), 1.68-1.86 (m, 6H), 1.93 (s, 3H), 1.98-2.10 (m, 12H), 2.35-2.59 (m, 2H), 3.10 (s, 3H), 3.86-4.05 (m, 8H), 4.05-4.35 (m, 3H), 5.40-5.26 (m, 6H), 5.48 (brs, 1H), 6.00-6.28 (brs, 1H), 6.53-6.75 (brs, 2H), 7.48 (brs, 1H), 8.16 (brs, 1H)

### Synthesis of Compound 14

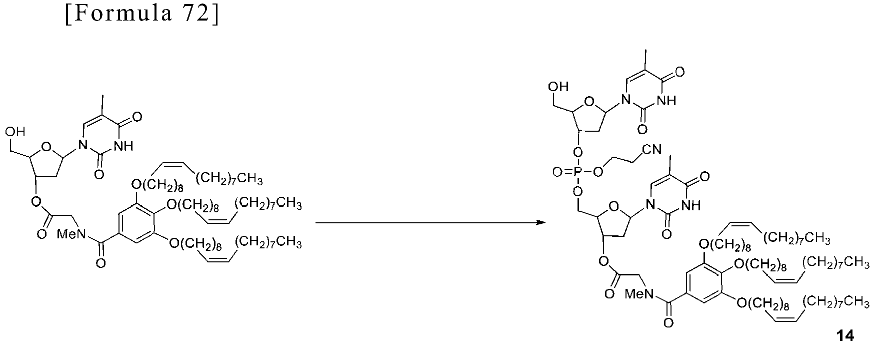

Under a nitrogen atmosphere, to a solution of Compound 13 (174.5 mg, 0.143 mmol) and DMT-dT-CE phosphoramidite (213 mg, 0.286 mmol) in dichloromethane (2.6 mL) was added 5-benzylthio-1H-tetrazole (55 mg, 0.286 mmol), followed by stirring at room temperature for 2 hours. DMT-dT-CE phosphoramidite (53 mg, 0.071 mmol) and 5-benzylthio-1H-tetrazole (13.8 mg, 0.072 mmol) were added to the reaction solution, followed by stirring at room temperature for 30 minutes. 0.1 M iodine (THF/pyridine/water solution, 3.93 mL) was added to the reaction solution, followed by stirring at room temperature for 30 minutes. 0.1 M iodine (THF/pyridine/water solution, 0.393 mL) was added, followed by stirring for 10 minutes. 1 M sodium thiosulfate aqueous solution (4.32 mL) and ethyl acetate (about 3 mL) were added, followed by stirring, and the organic and aqueous layers were separated. The organic layer was washed with saturated sodium hydrogencarbonate aqueous solution (3 mL) and 5% brine (3 mL), and concentrated. Ethyl acetate was added, followed by concentration to remove water, and crude purification was performed by silica gel chromatography (spherical neutral silica gel, developing solvent: hexane/ethyl acetate → ethyl acetate/methanol). From the crude purified product (325 mg), 311 mg was taken and dissolved in ethyl acetate (4.6 mL). Trifluoroacetic acid (0.081 mL, 1.06 mmol) and pyrrole (0.092 mL, 1.33 mmol) were added, followed by stirring at room temperature for 45 minutes. Trifluoroacetic acid (0.082 mL, 1.07 mmol) was added to the reaction solution, followed by stirring for 15 minutes. Trifluoroacetic acid (0.082 mL, 1.07 mmol) and pyrrole (0.173 mL, 2.49 mmol) were added to the reaction solution, followed by stirring for 30 minutes. Saturated sodium hydrogencarbonate aqueous solution (4.6 mL) was added to the reaction solution, followed by stirring, and the organic and aqueous layers were separated. The organic layer was washed with 5% brine (4.6 mL) and concentrated. After ethyl acetate was added, followed by concentration to remove water and purification by silica gel chromatography (spherical neutral silica gel, developing solvent: ethyl acetate/methanol) to give Compound 14 as a white amorphous solid (189 mg, yield: 88.1%).
¹H NMR (600 MHz, CDCl₃): δ 0.82-0.92 (m, 9H), 1.20-1.39 (m, 60H), 1.40-1.52 (m, 6H), 1.70-1.83 (m, 6H), 1.91 (s, 3H), 2.00 (s, 3H), 1.98-2.06 (m, 12H), 2.37-2.63 (m, 4H), 2.77-2.86 (m, 2H), 3.11 (s, 3H), 3.79-4.04 (m, 8H), 4.08-4.50 (m, 8H), 5.20 (brs, 1H), 5.30-5.50 (m, 7H), 6.08-6.20 (m, 2H), 6.28 (brs, 1H), 6.64 (brs, 2H), 7.29-7.50 (m, 2H), 8.38-9.07 (m, 2H)

### Synthesis of Compound 15

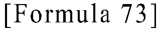

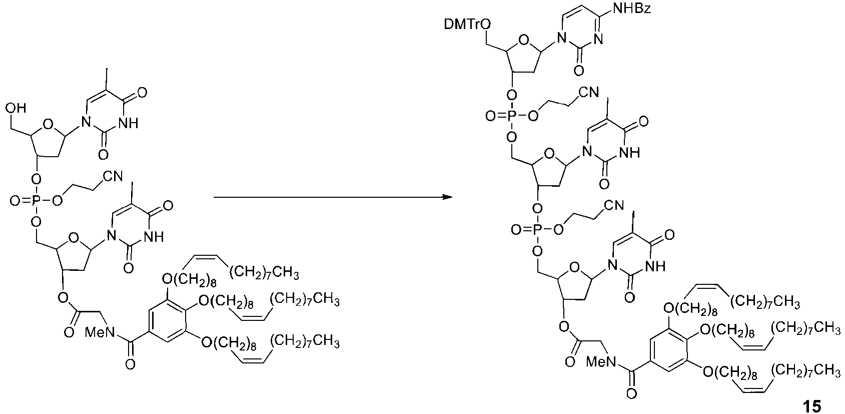

Compound 14 (147 mg, 0.0934 mmol) was subjected to azeotropic distillation with ethyl acetate three times. Under a nitrogen atmosphere, to Compound 14 was added ethyl acetate (1.5 mL) to prepare a solution, followed by the addition of DMT-dC-CE phosphoramidite (159 mg, 0.191 mmol) and molecular sieves 3A (150 mg), and stirring at room temperature for 1 hour. DCI (37 mg, 0.317 mmol) was added, followed by stirring at room temperature for 3 hours. 0.2 M iodine (ethyl acetate/pyridine/water solution, 1.43 mL) was added to the reaction solution, followed by stirring for 30 minutes. 0.2 M iodine (ethyl acetate/pyridine/water solution, 0.48 mL) was added to the reaction solution, followed by stirring for 70 minutes. 1 M sodium thiosulfate aqueous solution (2.25 mL) was added, followed by stirring and filtrating the molecular sieves while washing with ethyl acetate. The layers of the filtrate were separated, and the obtained organic layer was washed with 5% brine (1.5 mL) and concentrated. After ethyl acetate was added, followed by concentration to remove water, and purification by silica gel chromatography (spherical neutral silica gel, developing solvent: ethyl acetate/methanol) to give Compound 15 as a white solid (210 mg, yield: 97%).

### (Example 6) Deprotection of pseudo-solid-phase protecting group

Compound 11, as the pseudo-solid-phase protecting group, was removed from the oligonucleotide as follows.

### Synthesis of Compound 16

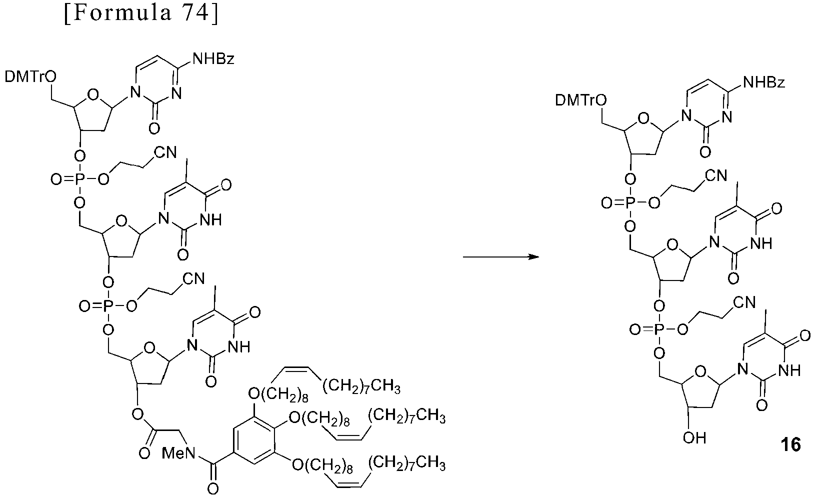

Under a nitrogen atmosphere, at 0 °C, to a solution of Compound 15 (98 mg, 0.042 mmol) in anhydrous THF (4.7 mL) and isopropanol (0.52 mL) was added dropwise a 0.5 M lithium borohydride in THF (0.42 mL, 0.21 mmol). After stirring at 0 °C for 1 hour and 20 minutes, 10% ammonium chloride aqueous solution (1.5 mL) and ethyl acetate were added, followed by stirring, and the organic and aqueous layers were separated. The aqueous layer was extracted with ethyl acetate, and the combined organic layers were concentrated to remove water, and then, purification by silica gel chromatography (spherical neutral silica gel, developing solvent: ethyl acetate/methanol) gave Compound 16 as a white solid (36 mg, 63%).

### (Example 7) Synthesis of Compound 20 (2-(2,4-di-((Z)-octadec-9-enyloxy)benzoyl)benzoic acid)

Compound 20, as the pseudo-solid-phase protecting group, was synthesized as follows.

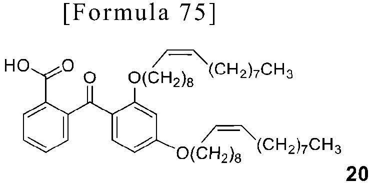

### Synthesis of Compound 18 (Ethyl 2-(2,4-dihydroxybenzoyl)benzoate)

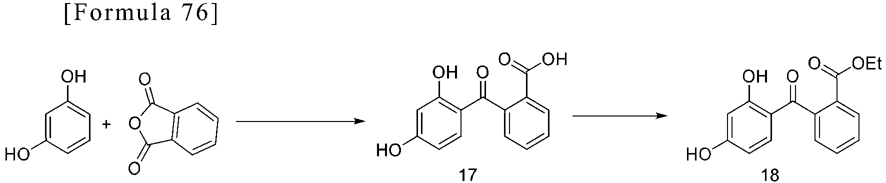

Under a nitrogen atmosphere, to a solution of resorcinol (3.00 g, 27.2 mmol) and phthalic anhydride (4.03 g, 27.2 mmol) in nitrobenzene (45 mL) was added aluminum chloride (8.34 g, 62.56 mmol), followed by stirring at room temperature for 13.5 hours. The reaction solution was added dropwise at room temperature to a vigorously stirred mixture of hexane (90 mL) and 0.5 M HCl (90 mL). After stirring at room temperature for 5 hours, the precipitated solid was collected by filtration. The obtained yellow solid was left standing at room temperature to give Compound 17 (2-(2,4-dihydroxybenzoyl)benzoic acid) as a crude product (7.75 g). The obtained crude product (Compound 17) was subjected to azeotropic distillation with ethanol three times, then dissolved in ethanol (12 mL). After cooling to 0 °C, thionyl chloride (2.96 mL, 40.8 mmol) was added dropwise. The reaction solution was heated to 80 °C and stirred for 2 hours. After cooling to 0 °C, thionyl chloride (1 mL) was added, the reaction solution was heated to 80 °C, followed by stirring for 1 hour. After cooling the reaction solution to room temperature, it was concentrated, and ethyl acetate (77 mL) and water (62 mL) were added, followed by stirring and separating the layers. The organic layer was concentrated, then ethyl acetate (6 mL) and hexane (30 mL) were added, and the mixture was stirred at room temperature for 1 hour. The precipitated brown solid was collected to give Compound 18 (4.00 g, yield: 51%).
¹H NMR (600 MHz, CDCl₃): δ 1.15 (t, 3H, J = 7.2 Hz), 4.17 (q, 2H, J = 7.2 Hz), 6.21 (dd, 1H, J = 9.0 Hz, 2.4 Hz), 6.40 (d, 1H, J = 2.4 Hz), 6.97 (d, 1H, J = 9.0 Hz), 7.38 (d, 1H, J = 7.2 Hz), 7.58 (t, 1H, J = 7.2 Hz), 7.65 (t, 1H, J = 7.2 Hz), 8.09 (d, 1H, J = 7.2 Hz), 12.35 (brs, 1H)
¹³C NMR (150 MHz, CDCl₃): δ 13.56, 61.79, 103.48, 107.81, 114.76, 127.47, 128.79, 129.69, 130.43, 132.44, 134.91, 139.98, 162.82, 165.25, 165.97, 200.98

### Synthesis of Compound 19

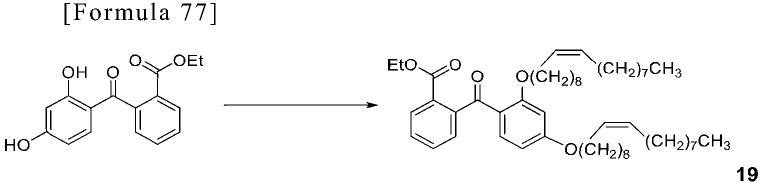

Under a nitrogen atmosphere, at 80 °C, to a suspension of Compound 18 (0.80 g, 2.79 mmol) and potassium carbonate (1.55 g, 11.2 mmol) in DMF (16 mL) was added dropwise a DMF (4 mL) solution of oleyl tosylate (2.95 g, 6.99 mmol), followed by stirring at 80 °C for 2 hours and 15 minutes and the reaction mixture was heated to 90 °C. After stirring at 90 °C for 2 hours and 15 minutes, oleyl tosylate (0.24 g) was added dropwise. The mixture was stirred at 90 °C for 1 hour and 15 minutes, then cooled to room temperature. Ethyl acetate (40 mL) and water (20 mL) were added to the reaction mixture, followed by stirring, and the layers were separated. The organic layer was washed three times with 5% brine (10 mL), dried over magnesium sulfate, and concentrated. Purification by silica gel chromatography (spherical neutral silica gel, developing solvent: hexane/ethyl acetate) gave Compound 19 as an oily material (2.13 g, yield: 97%).
¹H NMR (600 MHz, CDCl₃): δ 0.85-0.95 (m, 9H), 1.06-1.39 (m, 44H), 1.41-1.50 (m, 2H), 1.74-1.85 (m, 2H), 1.95-2.11 (m, 8H), 3.65 (t, 2H, J = 6.6 Hz), 3.99 (t, 2H, J = 6.0 Hz), 4.11 (q, 2H, J = 7.2 Hz), 5.35 (m, 4H), 6.31 (d, 1H, J = 2.4 Hz), 6.55 (dd, 1H, J = 2.4, 8.4 Hz), 7.26 (d, 1H, J = 7.8 Hz), 7.43 (t, 1H, J = 7.8 Hz), 7.51 (t, 1H, J = 7.8 Hz), 7.93 (d, 1H, J = 7.8 Hz), 8.00 (d, 1H, J = 8.4 Hz)

### Synthesis of Compound 20

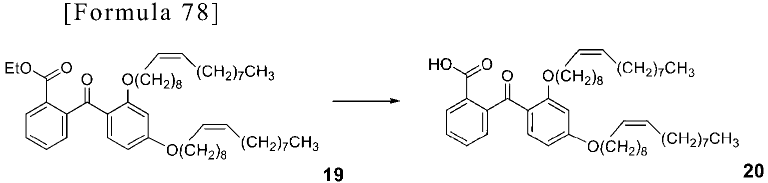

Under a nitrogen atmosphere, to a solution of Compound 19 (2.05 g, 2.60 mmol) in THF (2.9 mL) were added ethanol (19.3 mL) and 2 M NaOH (1.84 mL), followed by heating to 85 °C and stirring for 2 hours. After cooling the reaction mixture to room temperature, it was concentrated, and 1 M HCl (19 mL) and ethyl acetate (19 mL) were added, followed by stirring, and the layers were separated. The obtained organic layer was washed with 5% brine (19 mL) and concentrated. Ethyl acetate was added, followed by concentration to remove water, and then purification by silica gel chromatography (spherical neutral silica gel, developing solvent: hexane/ethyl acetate) gave Compound 20 as a colorless oily material (1.94 g, yield: 99%).
¹H NMR (600 MHz, CDCl₃): δ 0.78-1.40 (m, 50H), 1.40-1.49 (m, 2H), 1.71-1.84 (m, 2H), 1.93-2.11 (m, 8H), 3.65 (brs, 2H), 3.91-4.04 (m, 2H), 5.29-5.41 (m, 4H), 6.32 (brs, 1H), 6.54 (brs, 1H), 7.25-7.67 (m, 3H), 7.80-8.08 (m, 2H)

### (Example 8) Production of polyoligonucleotide bound to Compound 20

A 3-mer polyoligonucleotide was produced using Compound 20 as a pseudo-solid-phase protecting group as follows.

### Synthesis of Compound 21

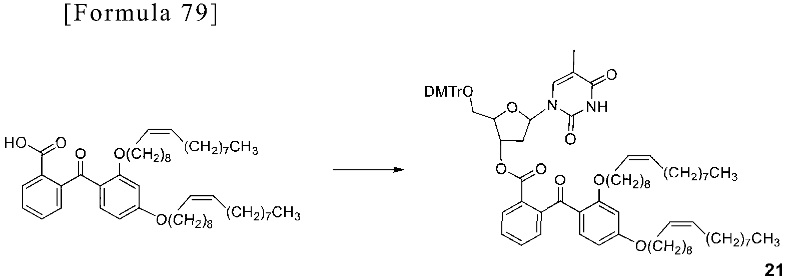

Under a nitrogen atmosphere, at 0 °C, to a suspension of DMT-dT (594 mg, 1.09 mmol), EDCI (226 mg, 1.18 mmol) and DMAP (22.2 mg, 0.182 mmol) in toluene (10 mL) was added dropwise a solution of Compound 20 (689 mg, 0.91 mmol) in toluene (3.5 mL), followed by stirring at 0 °C for 16 hours. EDCI (35 mg) was added, followed by stirring at 0 °C for 2 hours. Furthermore, EDCI (87 mg) was added, followed by stirring at 0 °C for 3 hours. Ethyl acetate (5.5 mL) and saturated sodium hydrogencarbonate aqueous solution (10 mL) were added, followed by stirring, and then the layers were separated. The obtained organic layer was washed with 5% brine (3 mL), and concentrated. After addition of ethyl acetate and concentration to remove water, purification by silica gel chromatography (spherical neutral silica gel, developing solvent: hexane/ethyl acetate) gave viscous colorless oily Compound 21 (997 mg, yield: 86%).

### Synthesis of Compound 22

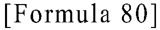

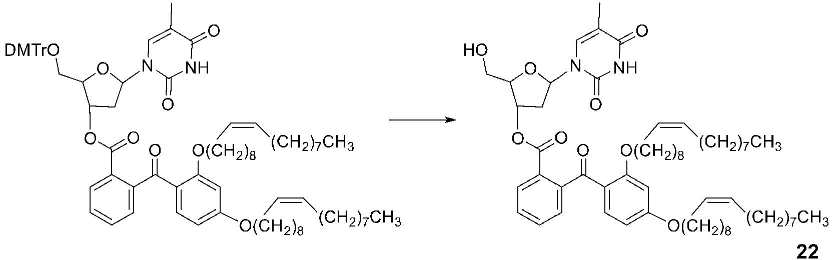

Under a nitrogen atmosphere, to a solution of Compound 21 (292 mg, 0.227 mmol) in ethyl acetate (2.9 mL) were added mercaptosuccinic acid (170 mg, 1.14 mmol) and trifluoroacetic acid (0.104 mL, 1.36 mmol) at 0°C, followed by stirring for 1.5 hours while allowing the temperature to rise to room temperature. Saturated sodium hydrogencarbonate aqueous solution (3 mL) was added to the reaction solution, followed by stirring and the layers were separated. The obtained organic layer was further washed twice with saturated sodium hydrogencarbonate aqueous solution (3 mL) and then washed with 5% brine (3 mL). The obtained organic layer was dried over magnesium sulfate, concentrated, and Compound 22 was obtained as a highly viscous colorless oil (223 mg, yield: 100%). The obtained Compound 22 was used in the next reaction without further purification. ¹H-NMR (600 MHz, CDCl₃) δ: 0.85-0.93 (m, 6H), 1.05-1.39 (m, 44H), 1.41-1.49 (m, 2H), 1.75-1.83 (m, 2H), 1.90 (s, 3H), 1.98-2.06 (m, 8H), 2.14 (dd, 1H, J = 13.8, 1.8 Hz), 2.43-2.57 (m, 1H), 2.75 (brs, 1H), 3.70 (t, 2H, J = 6.3 Hz), 3.76-3.87 (m, 2H), 3.90-3.96 (m, 1H), 4.00 (t, 2H, J = 6.3 Hz), 5.31-5.40 (m, 4H), 5.43 (m, 1H), 5.65 (dd, 1H, J = 8.4, 6.0 Hz), 6.37 (d, 1H, J = 1.8 Hz), 6.60 (dd, 1H, J = 8.4, 2.4 Hz), 7.20 (brs, 1H), 7.25 (d, 1H, J = 7.8 Hz), 7.47 (t, 1H, J = 7.2 Hz), 7.54 (t, 1H, J = 7.2 Hz), 7.97 (d, 1H, J = 7.8 Hz), 8.01 (d, 1H, J = 8.4 Hz), 8.10 (brs, 1H)

### Synthesis of Compound 23

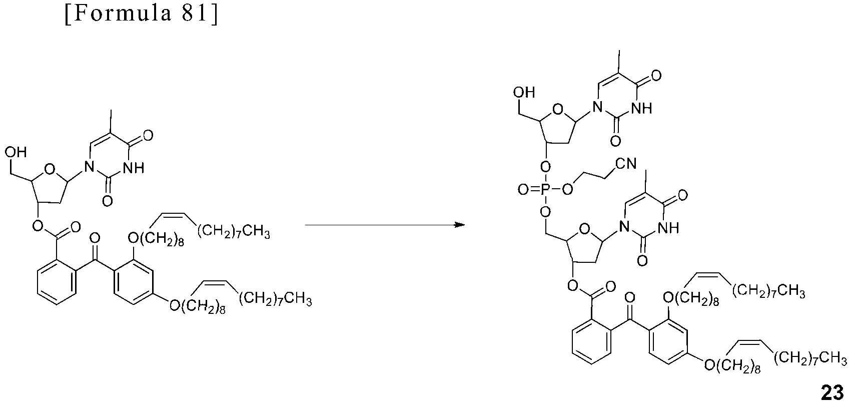

Compound 22 (136.5 mg, 0.139 mmol) was subjected to azeotropic distillation with ethyl acetate (1.4 mL) three times. Under a nitrogen atmosphere, ethyl acetate (1.4 mL) was added to compound 22 to prepare a solution, followed by the addition of molecular sieves 3A (137 mg) and DMTr-dT-CE phosphoramidite (207 mg, 0.278 mmol), and the mixture was stirred at room temperature for 1 hour. DCI (55 mg, 0.463 mmol) was then added, and stirring was continued at room temperature for 3 hours. To the reaction solution was added 0.1 M iodine (ethyl acetate/pyridine/water solution, 2.1 mL), and the mixture was stirred for 10 minutes. A 1 M sodium thiosulfate aqueous solution (2.05 mL) was added, followed by stirring, and the molecular sieves were removed by filtration while washing with ethyl acetate. The filtrate was subjected to phase separation, and the obtained organic layer was washed with 5% brine (2.05 mL) and then concentrated. The obtained residue was treated with ethyl acetate and concentrated to remove water, followed by addition of ethyl acetate (1.4 mL) and mercaptosuccinic acid (104 mg, 0.695 mmol). The resulting mixture was cooled to 0°C, and trifluoroacetic acid (0.101 mL, 1.32 mmol) was added, then the mixture was stirred for 1 hour 30 minutes while warming to room temperature. Water (2 mL) was added to the reaction solution, followed by stirring and the layers were separated. The obtained organic layer was washed three times with saturated sodium hydrogencarbonate aqueous solution (2 mL) and then with 5% brine (2 mL). The organic layer was dried over magnesium sulfate, concentrated to dryness, and compound 23 was obtained as a white solid (179 mg, yield 96%). The obtained compound 23 was used in the next reaction without further purification.
1H-NMR (600 MHz, CDCl₃): δ 0.81-0.93 (m, 6H), 1.04-1.39 (m, 44H), 1.40-1.50 (m, 2H), 1.76-1.83 (m, 2H), 1.87-1.91 (m, 3H), 1.93 (s, 3H), 1.97-2.06 (m, 8H), 2.20-2.29 (m, 1H), 2.32-2.58 (m, 3H), 2.72-2.85 (m, 2H), 3.03-3.34 (brm, 1H), 3.67-3.75 (m, 2H), 3.81-3.90 (m, 2H), 4.01 (t, 2H, J = 6.6 Hz), 4.04-4.08 (brm, 1H), 4.21-4.24 (m, 1H), 4.24-4.37 (m, 4H), 5.13-5.21 (m, 1H), 5.31-5.40 (m, 4H), 5.41-5.49 (m, 1H), 5.86-6.01 (m, 1H), 6.17 (q, 1H, J = 7.2 Hz), 6.38 (d, 1H, J = 2.4 Hz), 6.60 (d, 1H, J = 9.0 Hz), 7.17-7.20 (m, 1H), 7.24-7.27 (m, 1H), 7.45 (d, 1H, J = 7.2 Hz), 7.46-7.51 (m, 1H), 7.55 (t, 1H, J = 7.2 Hz), 7.92-7.98 (m, 2H), 8.31-8.61 (brm, 2H).

### Synthesis of Compound 24

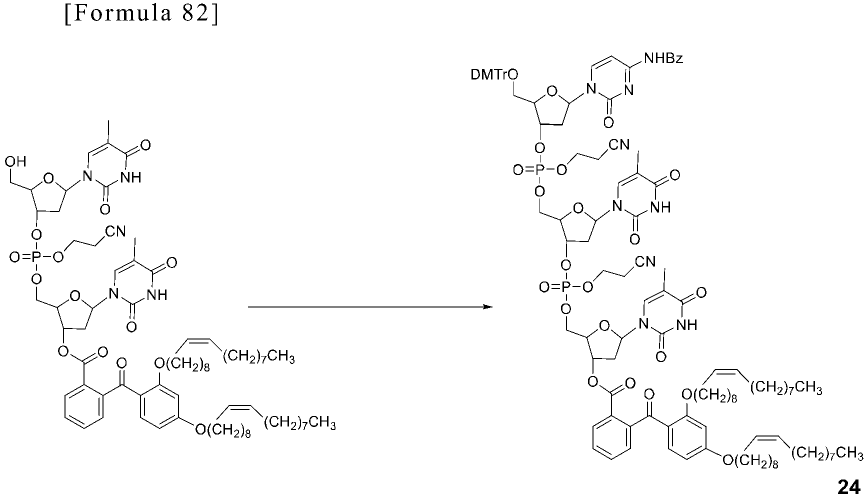

Azeotropic distillation was performed three times on Compound 23 (215 mg, 0.160 mmol) with ethyl acetate (2.2 mL). Under a nitrogen atmosphere, ethyl acetate (2.15 mL) was added to Compound 23 to prepare a solution, followed by the addition of molecular sieves 3A (215 mg) and DMTTr-dC-CE phosphoramidite (267 mg, 0.320 mmol), and the mixture was stirred at room temperature for 1 h. DCI (63 mg, 0.533 mmol) was added, and the mixture was stirred at room temperature for 2 h 30 min. To the reaction solution was added 0.2 M iodine (ethyl acetate/pyridine/water solution, 2.4 mL), and the mixture was stirred at room temperature for 30 min. A 1 M sodium thiosulfate aqueous solution (3.2 mL) was added and stirred, followed by filtration of the molecular sieves while washing with ethyl acetate. The filtrate was subjected to phase separation, and the obtained organic layer was washed three times with saturated sodium thiosulfate aqueous solution (3.2 mL) and then with 5% brine (3.2 mL). The organic layer was divided into two portions, one of which was concentrated, and after removal of water, purified by silica gel chromatography (spherical neutral silica gel, developing solvent: ethyl acetate/ethanol) to give Compound 24 as a white solid (158 mg, yield: 95%).
MS (ESI⁺): [M+H]⁺ 2088.9655.

### (Example 9) Deprotection of the pseudo-solid phase protecting group

The pseudo-solid phase protecting group, Compound 20, was removed from the oligonucleotide as follows.

### Synthesis of Compound 25

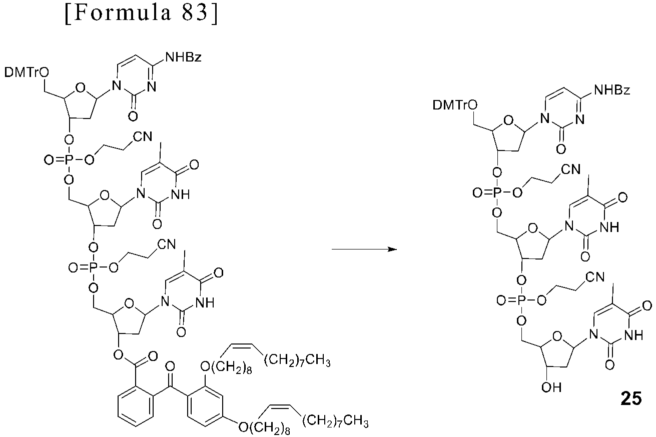

Under a nitrogen atmosphere, at 0°C, a 0.5 M THF solution of lithium borohydride (0.4 mL, 0.20 mmol) was added dropwise to a solution of Compound 24 (84 mg, 0.040 mmol) in anhydrous THF (2.0 mL) and isopropanol (0.52 mL). After stirring at 0°C for 50 minutes, a 10% ammonium chloride aqueous solution (1.25 mL) and ethyl acetate were added, followed by stirring and the layers were separated. The aqueous layer was extracted with ethyl acetate, and the combined organic layers were concentrated to remove water. Then, purification by silica gel chromatography (spherical neutral silica gel, developing solvent: ethyl acetate/methanol) gave Compound 25 as a white solid (42 mg, yield: 79%).

### (Example 9) Production of polyoligonucleotide

An 8-mer polyoligonucleotide was produced using Compound 20 as a pseudo-solid-phase protecting group as described below.

### Synthesis of Compound 26

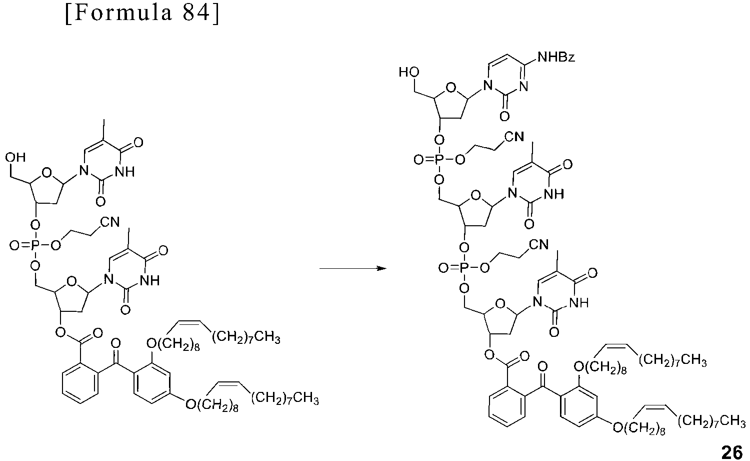

Compound 23 (338 mg, 0.252 mmol) was subjected to azeotropic distillation three times with ethyl acetate (3.4 mL). Under a nitrogen atmosphere, to Compound 23 were added molecular sieves 3A (340 mg), DMTTr-dC-CE phosphoramidite (315 mg, 0.378 mmol), and ethyl acetate (3.4 mL), and the mixture was stirred at room temperature for 50 minutes. DCI (77.4 mg, 0.655 mmol) was added, and the mixture was stirred at room temperature for 80 minutes. After confirming completion of the reaction, 0.2 M iodine (ethyl acetate/pyridine/water solution, 3.5 mL) was added to the reaction mixture, and the mixture was stirred for 20 minutes. A 1 M aqueous sodium thiosulfate solution (3.4 mL) was added, and after stirring, the molecular sieves were removed by filtration while washing with ethyl acetate. The filtrate was subjected to phase separation, and the obtained organic layer was washed with 5% brine (1.7 mL), followed by concentration. Ethyl acetate was added to the obtained residue, and the mixture was concentrated to remove water, then ethyl acetate (3.4 mL) and mercaptosuccinic acid (189.2 mg, 1.26 mmol) were added. The resulting mixture was cooled to 0°C, and trifluoroacetic acid (0.289 mL, 3.78 mmol) was added. The mixture was stirred for 37 minutes while allowing the temperature to rise to room temperature. After confirming completion of the reaction, saturated aqueous sodium hydrogencarbonate (5.1 mL) was added, the mixture was stirred, and layers were separated. The obtained organic layer was washed with saturated aqueous sodium hydrogencarbonate, 10% aqueous ammonium chloride, and saturated aqueous sodium chloride, respectively. The obtained organic layer was dried over magnesium sulfate, concentrated to dryness, and Compound 26 as a pale yellow solid (419 mg, 93%) was obtained. The obtained Compound 26 was used in the next reaction without further purification.
MS (ESI⁺): [M]⁺ 1785.8345.

### Synthesis of Compound 27

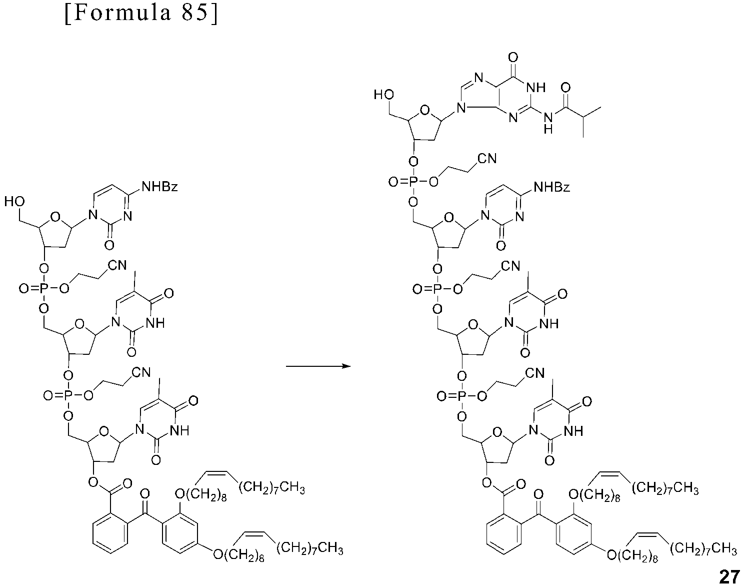

Compound 26 (177 mg, 0.099 mmol) was subjected to azeotropic distillation three times with ethyl acetate (1.8 mL). Under a nitrogen atmosphere, ethyl acetate (1.8 mL) was added to Compound 26 to dissolve it, followed by the addition of molecular sieves 3A (177 mg), and the mixture was stirred at room temperature for 30 minutes. DMTTr-dG-CE phosphoramidite (125 mg, 0.149 mmol) was added, and the mixture was stirred at room temperature for 10 minutes. DCI (30.4 mg, 0.257 mmol) was added, and the mixture was stirred for about 1 hour. Additional DMTTr-dG-CE phosphoramidite (total 41.7 mg, 0.0497 mmol) and DCI (total 60.8 mg, 0.515 mmol) were added to complete the reaction. To the reaction mixture was added 0.2 M iodine (ethyl acetate/pyridine/water solution, 1.98 mL), and the mixture was stirred for 20 minutes. A 1 M aqueous sodium thiosulfate solution (2.7 mL) was added, and after stirring, the molecular sieves were removed by filtration while washing with ethyl acetate. The filtrate was subjected to phase separation, and the obtained organic layer was washed with saturated aqueous sodium chloride (1.8 mL), dried over magnesium sulfate, and concentrated. To the obtained residue were added ethyl acetate (2.7 mL) and mercaptosuccinic acid (74.3 mg, 0.495 mmol). The resulting mixture was cooled to 0°C, and trifluoroacetic acid (0.0826 mL, 1.08 mmol) was added. The mixture was stirred for about 1 hour while allowing the temperature to rise to room temperature. When starting material was detected, additional trifluoroacetic acid was added. After confirming completion of the reaction, saturated aqueous sodium hydrogencarbonate (2.7 mL) was added, the mixture was stirred, and the layers were separated. When insoluble material was observed, a small amount of THF was added to dissolve it. The obtained organic layer was washed with saturated aqueous sodium chloride (1.8 mL). The obtained organic layer was dried over magnesium sulfate, concentrated to dryness, and Compound 27 as a pale yellow solid (246 mg) was obtained. The obtained Compound 27 was used in the next reaction without further purification.
MS (ESI⁺): [M+1]⁺ 2238.9575.

### Synthesis of Compound 28

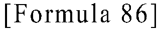

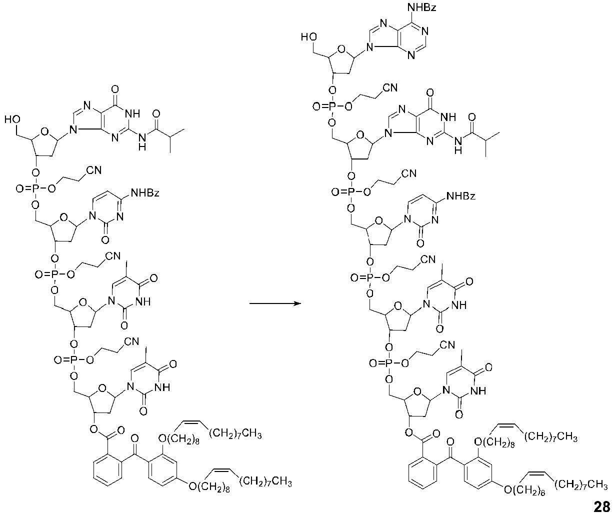

Compound 27 (246 mg) was subjected to the same procedure as in the synthesis of Compound 27, except that the corresponding phosphoramidite was used, to give Compound 28 as a pale yellow solid (272 mg). The resulting Compound 28 was used in the next reaction without further purification.
MS (ESI⁺): [M + 1]⁺ 2709.6452.

### Synthesis of Compound 29

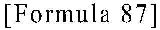

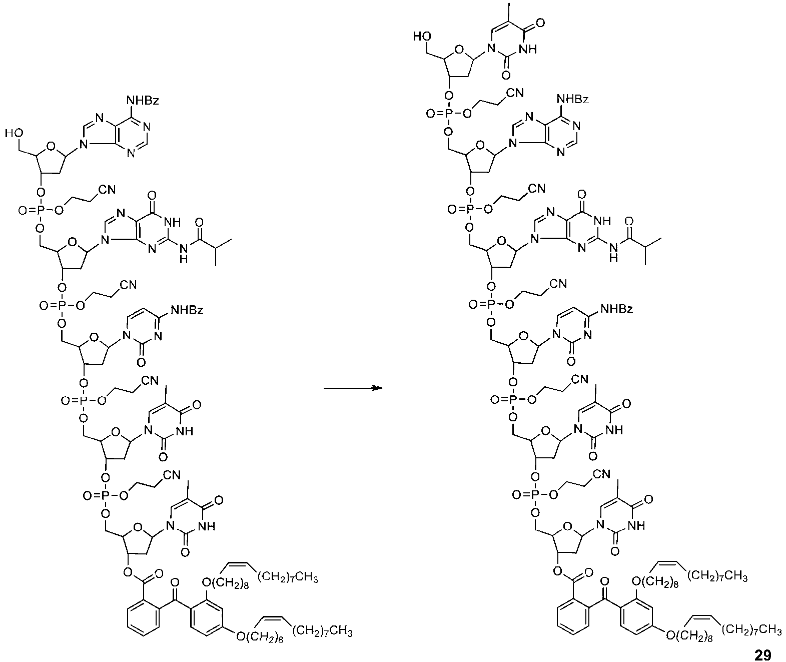

Compound 28 (272 mg, 0.100 mmol) was subjected to azeotropic distillation with ethyl acetate (2 mL) three times. Under a nitrogen atmosphere, ethyl acetate (1 mL) and THF (1 mL) were added to Compound 28 to dissolve it, followed by the addition of molecular sieves 3A (266 mg), and the mixture was stirred at room temperature for about 30 minutes. DMT-dT-CE phosphoramidite (111 mg, 0.149 mmol) was added, and the mixture was stirred at room temperature for 15 minutes. DCI (52.7 mg, 0.446 mmol) was added, and the mixture was stirred for about 1 hour. DMT-dT-CE phosphoramidite (37 mg, 0.0497 mmol) and DCI (17.6 mg, 0.149 mmol) were further added to complete the reaction. A 0.2 M iodine (ethyl acetate/pyridine/water solution, 1.98 mL) was added to the reaction mixture, and the mixture was stirred for 20 minutes. A 1 M aqueous sodium thiosulfate solution (2.7 mL) was added and stirred, followed by filtration of the molecular sieves while washing with ethyl acetate and THF. The filtrate was subjected to phase separation, and the obtained organic layer was washed with saturated brine (1.8 mL). After drying over magnesium sulfate, the solution was concentrated. Ethyl acetate (2.7 mL) and mercaptosuccinic acid (74.3 mg, 0.495 mmol) were added to the obtained residue. The resulting mixture was cooled to 0 °C, followed by the addition of trifluoroacetic acid (0.114 mL, 1.49 mmol). The mixture was stirred for about 1 hour while allowing the temperature to rise to room temperature. When starting material was detected, trifluoroacetic acid was added. After confirming completion of the reaction, a saturated aqueous sodium bicarbonate solution (3.5 mL) was added, and the mixture was stirred and the layers were separated. When insoluble material was observed, THF was added to dissolve it. The obtained organic layer was washed with a saturated brine (1.8 mL). The obtained organic layer was dried over magnesium sulfate, concentrated to dryness, and Compound 29 was obtained as a pale yellow solid (303 mg). The resulting Compound 29 was used in the next reaction without further purification.

### Synthesis of Compound 30

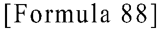

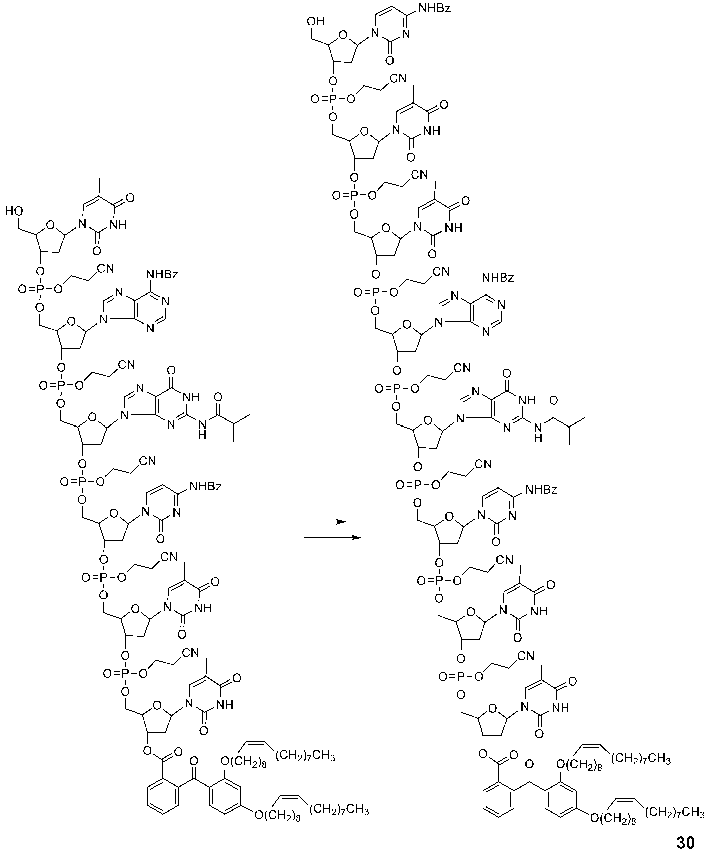

Compound 29 (303 mg) was subjected to the same procedure as for the synthesis of Compound 29 using the corresponding phosphoramidite, repeated twice, to obtain Compound 30 as a pale yellow solid (280 mg). MS(ESI+): [M+2H]²⁺ 1935.1655.

### (Example 10) Synthesis of Compound 34

Compound 34, which is a pseudo-solid-phase protecting group, was synthesized as follows.

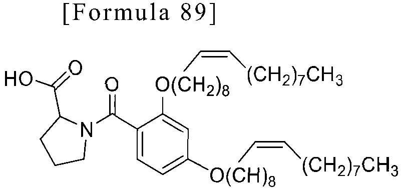

### Synthesis of Compound 31

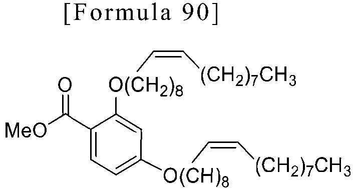

Under a nitrogen atmosphere, methyl 2,4-dihydroxybenzoate (1.20 g, 7.14 mmol), K₂CO₃ (3.95 g, 28.6 mmol), DMAc (24 mL), and oleyl bromide (5.20 g, 15.7 mmol) were mixed at room temperature, then the temperature was raised to 80°C and the mixture was stirred for 3 hours. Oleyl bromide (2.36 g, 7.14 mmol) was mixed at 80°C and the mixture was stirred for 2 hours. After completion of the reaction, the mixture was cooled with ice to 0°C, and n-hexane (24 mL), water (24 mL), and ethyl acetate (24 mL) were added, and the aqueous layer was separated. The separated organic layer was washed three times with water (24 mL), and concentrated under reduced pressure to obtain the crude product of Compound 31. The obtained crude product was purified by silica gel column chromatography (n-hexane/ethyl acetate) to give Compound 31 as an oily material in 98% yield (4.66 g, 6.96 mmol).
¹H-NMR (600 MHz, CDCl₃): δ 7.88-7.77 (m, 1H), 6.45 (s, 2H), 5.38-5.32 (m, 4H), 3.98 (q, J = 6.9 Hz, 4H), 3.84 (s, 3H), 2.05-1.97 (m, 8H), 1.87-1.75 (m, 4H), 1.53-1.41 (m, 4H), 1.33-1.24 (m, 40H), 0.91-0.84 (m, 6H)
¹³C-NMR (150 MHz, CDCl₃): δ 166.4, 163.7, 160.9, 133.8, 129.9, 112.4, 105.1, 100.3, 68.9, 68.2, 51.5, 31.9, 29.8, 29.8, 29.5, 29.5, 29.5, 29.3, 29.2, 29.3, 27.2, 27.2, 26.0, 26.0, 22.7, 14.1

### Synthesis of Compound 32

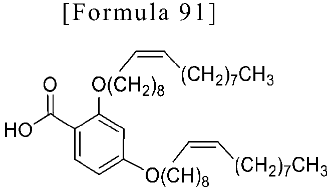

Under a nitrogen atmosphere, to a solution of Compound 31 (4.52 g, 6.75 mmol) in THF (22.6 mL) were added ethanol (22.6 mL) and 4 M sodium hydroxide (6.75 mL). The mixture was then heated to 70°C and stirred for 2 hours. After completion of the reaction, the mixture was cooled with ice to 0°C, followed by the addition of 2 M HCl (15 mL), ethyl acetate (90 mL), water (90 mL), and n-hexane (90 mL), and the aqueous layer was separated. The separated organic layer was washed with water (90 mL), and concentrated under reduced pressure to give the crude product of Compound 32. The obtained crude product was purified by silica gel column chromatography (n-hexane/ethyl acetate) to give Compound 32 as an oily material in 98% yield (4.35 g, 6.64 mmol).
¹H-NMR (600 MHz, CDCl₃): δ 10.75 (br s, 1H), 8.11 (br d, J = 8.7 Hz, 1H), 6.62 (br d, J = 8.7 Hz, 1H), 6.50 (s, 1H), 5.38-5.32 (m, 4H), 4.19 (br t, J = 6.4 Hz, 2H), 4.01 (br t, J = 6.4 Hz, 2H), 2.02 (br s, 8H), 1.90 (quin, J = 6.9 Hz, 2H), 1.80 (quin, J = 6.8 Hz, 2H), 1.51-1.38 (m, 4H), 1.34-1.24 (m, 40H), 0.88 (br t, J = 6.6 Hz, 6H)
¹³C-NMR (150 MHz, CDCl₃): δ 165.3, 164.6, 159.0, 135.5, 130.0, 130.0, 129.8, 129.7, 110.3, 107.1, 99.8, 70.2, 68.6, 31.9, 29.8, 29.5, 29.4, 29.3, 29.2, 29.5, 29.1, 28.9, 27.2, 27.2, 27.2, 26.0, 25.9, 22.7, 14.1

### Synthesis of Compound 33

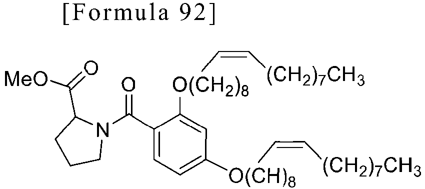

Under a nitrogen atmosphere, at 0°C, EDCI (2.52 g, 13.1 mmol) was added to a solution of Compound 32 (4.30 g, 6.56 mmol), L-proline methyl hydrochloride (2.17 g, 13.1 mmol), DMAP (160 mg, 1.31 mmol), and DIPEA (4.57 mL, 26.3 mmol) in DMF (43 mL), and the mixture was stirred at room temperature for 2 hours. Ethyl acetate (43 mL), n-hexane (43 mL), and water (43 mL) were added, and after stirring, the aqueous layer was separated. The obtained organic layer was washed three times with water (43 mL) and concentrated. The obtained crude product was purified by silica gel column chromatography (n-hexane/ethyl acetate) to give Compound 33 as an oily material in 86% yield (4.35 g, 5.67 mmol). ¹H-NMR (600 MHz, CDCl₃): δ 7.24 (d, J = 8.3 Hz, 0.7H), 7.11 (br d, J = 8.3 Hz, 0.3H), 6.47 (br d, J = 8.3 Hz, 0.7H), 6.45-6.36 (m, 1.3H), 5.35 (br s, 4H), 4.64 (br s, 0.7H), 4.36-4.27 (m, 0.3H), 3.97-3.90 (m, 4H), 3.76 (s, 2.7H), 3.49 (s, 1H), 3.48-3.34 (m, 1.3H), 2.29-2.17 (m, 1H), 2.07-1.93 (m, 10.3H), 1.88-1.81 (m, 0.7H), 1.79-1.68 (m, 4H), 1.44 (s, 4H), 1.37-1.24 (m, 40H), 0.88 (br t, J = 6.8 Hz, 6H) with rotamer. ¹³C-NMR (150 MHz, CDCl₃): δ 172.8, 168.1, 161.3, 156.4, 130.0, 129.8, 129.8, 129.4, 105.4, 100.0, 68.6, 68.2, 58.7, 52.1, 52.0, 47.9, 46.2, 31.9, 31.2, 29.8, 29.8, 29.7, 29.5, 29.5, 29.4, 29.4, 29.3, 29.3, 29.3, 29.2, 27.2, 27.2, 27.2, 26.1, 26.0, 26.0, 24.8, 23.2, 22.7, 14.1.

### Synthesis of Compound 34

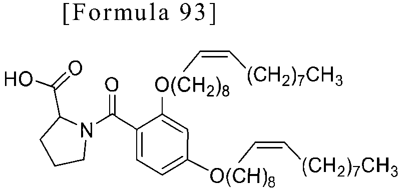

Under a nitrogen atmosphere, 4 M sodium hydroxide (5.5 mL) was added to a solution of Compound 33 (4.25 g, 5.54 mmol) in THF (21 mL), and the mixture was stirred at room temperature for 1 hour. Then, ethanol (21 mL) was added, the temperature was raised to 70°C, and stirring was continued for 2 hours. After completion of the reaction, the mixture was cooled with ice to 0°C, and 2 M HCl (12 mL), ethyl acetate (21 mL), water (21 mL), and n-hexane (21 mL) were added, followed by separation of the aqueous layer. The separated organic layer was washed with water (21 mL) and concentrated under reduced pressure to obtain the crude product of Compound 34. The obtained crude product was purified by silica gel column chromatography (n-hexane/ethyl acetate) to give Compound 34 as an oily material in 98% yield (4.09 g, 5.43 mmol). ¹H-NMR (600 MHz, CDCl₃): δ 8.92 (s, 1H), 7.24 (br d, J = 8.3 Hz, 0.9H), 7.17-7.12 (m, 0.1H), 6.50 (br d, J = 8.3 Hz, 0.9H), 6.45 (s, 1H), 6.39 (s, 0.1H), 5.35 (s, 4H), 4.75 (d, J = 7.6 Hz, 0.9H), 4.35-4.27 (m, 0.1H), 3.99-3.91 (m, 4H), 3.80-3.68 (m, 0.2H), 3.45 (q, J = 8.1 Hz, 0.9H), 3.34 (br s, 0.9H), 2.56-2.50 (m, 0.9H), 2.26-1.93 (m, 10.1H), 1.90-1.83 (m, 1H), 1.81-1.71 (m, 4H), 1.47-1.37 (m, 4H), 1.34-1.25 (m, 40H), 0.88 (br t, J = 6.6 Hz, 6H) with rotamer.
¹³C-NMR (150 MHz, CDCl₃): δ 172.0, 171.5, 162.1, 156.3, 130.0, 129.8, 129.8, 129.3, 117.7, 105.6, 100.0, 68.6, 68.3, 60.3, 48.8, 31.9, 29.8, 29.8, 29.5, 29.5, 29.5, 29.4, 29.3, 29.3, 29.2, 29.2, 29.1, 27.7, 27.2, 27.2, 27.2, 26.0, 26.0, 24.6, 22.7, 14.1.

### (Example 11) Production of an oligonucleotide bound to Compound 34

An oligonucleotide was produced using Compound 34 as a pseudo-solid-phase protecting group as follows.

### Synthesis of Compound 35

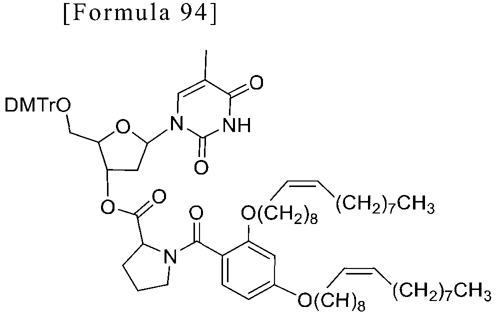

Under a nitrogen atmosphere, at 0°C, DMT-dT (2.88 g, 5.29 mmol) was added to a solution of Compound 34 (1.99 g, 2.64 mmol), EDCI (2.03 g, 5.29 mmol), 1-hydroxybenzotriazole monohydrate (1.62 g, 5.29 mmol), and DIPEA (3.68 mL, 10.6 mmol) in DMF (20 mL), and the mixture was raised to room temperature and the mixture was stirred for 15 hours. To the reaction mixture were added EDCI (253 mg, 1.32 mmol), DIPEA (691 µL, 3.97 mmol), and DMT-dT (720 mg, 1.32 mmol), followed by stirring at room temperature for 20 hours. Then, the mixture was raised to 40°C, and stirring was continued for 2 hours. To the reaction mixture were added EDCI (253 mg, 1.32 mmol), 1-hydroxybenzotriazole monohydrate (203 mg, 1.32 mmol), and DMT-dT (720 mg, 1.32 mmol), followed by stirring at room temperature for 24 hours. Ethyl acetate (40 mL), n-hexane (20 mL), and water (20 mL) were added, and after stirring, the aqueous layer was separated. The obtained organic layer was washed three times with saturated aqueous sodium hydrogencarbonate (20 mL), washed with water (20 mL), and concentrated. The obtained crude product was purified by silica gel column chromatography (n-hexane/ethyl acetate) to give Compound 35 as an oily material in 78% yield (2.66 g, 2.08 mmol).
¹H-NMR (600 MHz, CDCl₃): δ 8.61 (s, 0.2H), 8.55 (s, 0.8H), 7.61 (s, 0.8H), 7.51 (m, 0.2H), 7.41-7.33 (m, 2H), 7.31-7.09 (m, 6H), 7.14-7.07 (m, 1H), 6.84 (br d, J = 8.3 Hz, 4H), 6.50-6.20 (m, 3H), 5.63 (br d, J = 5.3 Hz, 0.8H), 5.37-5.33 (m, 4H), 5.20-5.11 (m, 0.2H), 4.59-4.56 (m, 0.8H), 4.43-4.33 (m, 0.2H), 4.16 (br s, 0.7H), 3.96-3.64 (m, 10.8H), 3.53-3.36 (m, 3.5H), 2.63-2.46 (m, 1.5H), 2.33-2.21 (m, 1.2H), 2.08-1.92 (m, 10.5H), 1.91-1.82 (m, 0.8H), 1.81-1.67 (m, 5H), 1.51-1.13 (m, 47H), 0.88 (br t, J = 6.6 Hz, 6H) with rotamer.
¹³C-NMR (150 MHz, CDCl₃): δ 172.0, 171.5, 162.1, 156.3, 130.0, 129.8, 129.8, 129.3, 117.7, 105.6, 100.0, 68.6, 68.3, 60.3, 48.8, 31.9, 29.8, 29.8, 29.5, 29.5, 29.5, 29.4, 29.3, 29.3, 29.2, 29.2, 29.1, 27.7, 27.2, 27.2, 27.2, 26.0, 26.0, 24.6, 22.7, 14.0.

### Synthesis of Compound 36

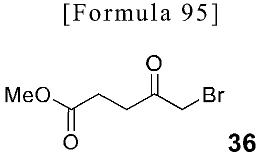

Under a nitrogen atmosphere, levulinic acid (6.16 mL, 60 mmol) and methanol (80 mL) were mixed, heated to 70°C, and stirred for 10 minutes. A mixed solution of bromine (3.08 mL, 60 mmol) and methanol (20 mL) was slowly added dropwise to the reaction solution over 30 minutes, and the mixture was stirred at 70°C for 2 hours. After confirming completion of the reaction, the mixture was cooled to room temperature, dichloromethane (40 mL) and water (20 mL) were added, and the aqueous layer was separated. The organic layer was washed with water (20 mL) and concentrated under reduced pressure to obtain the crude product. The obtained crude product was purified by silica gel column chromatography (n-hexane/ethyl acetate) to obtain compound 36 as a pale yellow oil (4.51 g, yield: 36%).
¹H-NMR (500 MHz, CDCl₃) δ 3.96 (s, 2H), 3.69 (s, 3H), 2.97 (t, J = 7.5 Hz, 3H), 2.67 (t, J = 7.5 Hz, 3H)

### Synthesis of compound 37

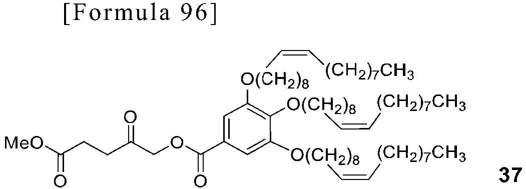

Under a nitrogen atmosphere, 3,4,5-tri-((Z)-octadec-9-enyloxy)benzoic acid (10.05 g, 11.0 mmol), DMF (110 mL), sodium hydrogen carbonate (1.85 g, 22.0 mmol) and compound 36 (3.76 g, 17.6 mmol) were mixed, heated to 60°C, and stirred overnight. After confirming completion of the reaction, the mixture was cooled to room temperature, ethyl acetate (20 mL), n-hexane (20 mL), and water (20 mL) were added, and the aqueous layer was separated. The organic layer was washed with water (20 mL) and concentrated under reduced pressure to obtain a crude product. The obtained crude product was purified by silica gel column chromatography (n-hexane/ethyl acetate) to give compound 37 as a colorless oil (9.86 g, yield: 85%).
¹H-NMR (500 MHz, CDCl₃) δ 7.30 (s, 2H), 5.38-5.31 (m, 6H), 4.90 (s, 2H), 4.02 (q, J = 6.7 Hz, 6H), 3.69 (s, 3H), 2.81 (t, J = 6.3 Hz, 2H), 2.68 (t, J = 6.3 Hz, 2H), 2.04-1.99 (m, 12H), 1.84-1.70 (m, 6H), 1.49-1.43 (m, 6H), 1.40-1.20 (m, 60H), 0.88 (t, J = 7.0 Hz, 9H)

### Synthesis of compound 38

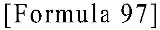

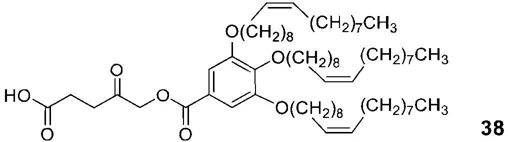

Under a nitrogen atmosphere, a mixture of compound 37 (9.86 g, 9.40 mmol), chloroform (350 mL), and 0.5 M aqueous sodium hydroxide solution (37.6 mL) was stirred at room temperature overnight. After confirming completion of the reaction, 6 M HCl (6 mL), water (20 mL), and ethyl acetate (20 mL) were added, and the aqueous layer was separated. The organic layer was washed with water (20 mL) and concentrated under reduced pressure to obtain a crude product. The obtained crude product was purified by silica gel column chromatography (n-hexane/ethyl acetate) to give compound 38 as a white amorphous solid (1.21 g, yield: 18%).
¹H-NMR (500 MHz, CDCl₃) δ 7.30 (s, 2H), 5.38-5.31 (m, 6H), 4.90 (s, 2H), 4.02 (q, J = 6.7 Hz, 6H), 2.81 (t, J = 6.3 Hz, 2H), 2.68 (t, J = 6.3 Hz, 2H), 2.04-1.99 (m, 12H), 1.84-1.70 (m, 6H), 1.49-1.43 (m, 6H), 1.40-1.20 (m, 60H), 0.88 (t, J = 7.0 Hz, 9H).

### Synthesis of Compound 39

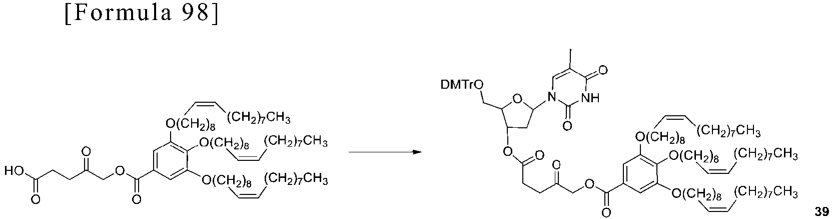

Under a nitrogen atmosphere, to a solution of compound 38 (439 mg, 0.42 mmol), DMT-dT (392 mg, 0.72 mmol), and DMAP (88 mg, 0.72 mmol)in dichloromethane (11 mL), EDCI (138 mg, 0.72 mmol) was added at room temperature, and the mixture was stirred at the same temperature for 30 hours. After confirming completion of the reaction, the solvent was concentrated under reduced pressure, and the obtained crude product was purified by silica gel column chromatography (n-hexane/ethyl acetate) to obtain compound 39 as a white amorphous solid (543 mg, 83%).
¹H-NMR (500 MHz, CDCl₃): δ 8.74 (s, 1H), 7.60 (s, 1H), 7.39-7.35 (m, 2H), 7.32-7.20 (m, 9H), 6.83 (d, J = 9.5 Hz, 4H), 6.46-6.42 (m, 1H), 5.48-5.46 (m, 1H), 5.38-5.30 (m, 6H), 4.90 (s, 2H), 4.15-4.12 (m, 1H), 4.04-3.97 (m, 6H), 3.78 (s, 6H), 3.50-3.42 (m, 2H), 2.86-2.75 (m, 2H), 2.68 (t, J = 6.3 Hz, 2H), 2.50-2.40 (m, 2H), 2.09-1.90 (m, 12H), 1.84-1.70 (m, 6H), 1.50-1.40 (m, 6H), 1.40-1.20 (m, 67H), 0.88 (t, J = 7.0 Hz, 3H).

### Synthesis of Compound 40

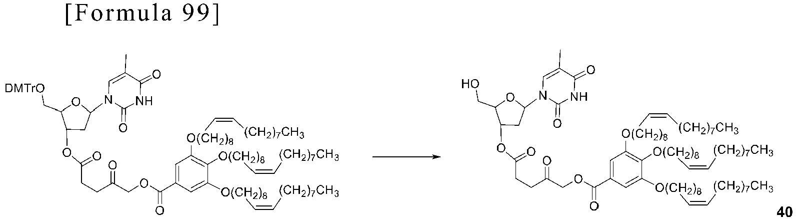

Under a nitrogen atmosphere, to an ethyl acetate (5.4 mL) solution of compound 39 (543 mg, 0.35 mmol) were added mercaptosuccinic acid (263 mg, 1.75 mmol) and trifluoroacetic acid (0.40 mL, 5.25 mmol), and the mixture was stirred under ice cooling for 1.5 hours. After confirming completion of the reaction, the reaction solution was washed three times with 5% NaHCO₃ aqueous solution (8 mL) and once with 10% NaCl aqueous solution (8 mL), followed by concentration under reduced pressure to obtain compound 40 as a white amorphous solid (401 mg, 91%). The obtained compound 40 was used in the next reaction without further purification.

### Synthesis of Compound 41

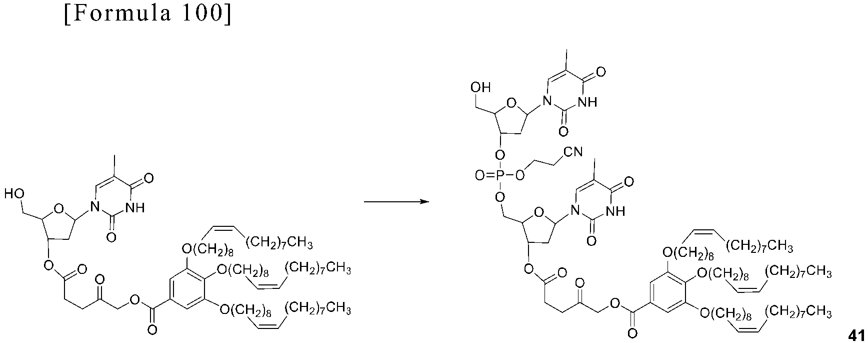

Compound 40 (401 mg, 0.32 mmol) was subjected to azeotropic distillation three times with ethyl acetate (4.0 mL). To the obtained compound 40 were added molecular sieves 3A (400 mg), DMT-dT-CE phosphoramidite (715 mg, 0.96 mmol), and ethyl acetate (4.0 mL), and the mixture was stirred at room temperature for 1 hour. To the reaction solution was added DCI (113 mg, 0.96 mmol), and the mixture was stirred at room temperature for 2 hours. After confirming completion of the reaction, iodine (244 mg, 0.96 mmol) dissolved in acetonitrile/pyridine/water solution (4.8 mL) was added dropwise to the reaction solution at room temperature, and the mixture was stirred for 1 hour. After confirming completion of the reaction, 1 M Na₂S₂O₃ aqueous solution (6.0 mL) was added and stirred. The insoluble matter was removed by filtration, and the layers were separated. The organic layer was washed with 5% brine (6.0 mL), then dried over sodium sulfate (5 g). After concentration of the organic layer under reduced pressure, azeotropic distillation was performed twice with ethyl acetate (5 mL). The residue was dissolved in ethyl acetate (4.0 mL), to which mercaptosuccinic acid (240 mg, 1.60 mmol) was added, and the mixture was cooled to 0°C. Trifluoroacetic acid (0.37 mL, 4.80 mmol) was added, and the mixture was stirred at the same temperature for 1 hour, then warmed to room temperature and stirred for an additional 1 hour. After confirming completion of the reaction, water (6 mL) was added, and the organic layer was separated. The obtained organic layer was washed three times with 5% NaHCO₃ aqueous solution (6 mL) and once with 5% NaCl aqueous solution (6 mL). The obtained organic layer was concentrated to give compound 41 as a white amorphous solid (311 mg, 59%). The obtained compound 41 was used in the next reaction without further purification.

### Synthesis of Compound 42

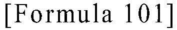

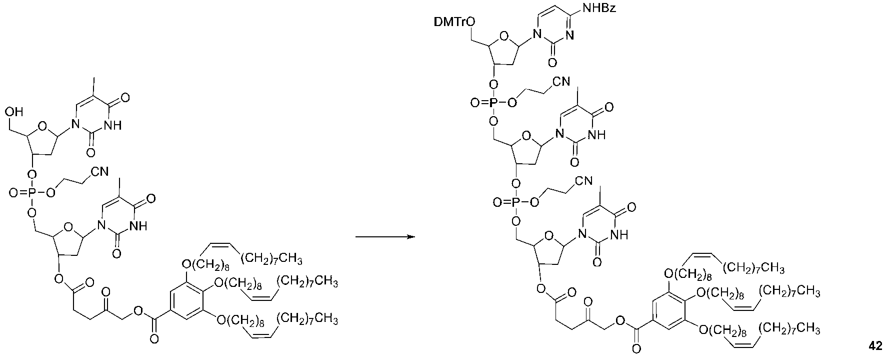

Using compound 41 (311 mg, 0.19 mmol), compound 42 was obtained as a pale yellow amorphous solid (301 mg, yield: 68%) in the same manner as the synthesis of compound 8, except that the amounts of reagents were appropriately changed.

### Synthesis of Compound 43

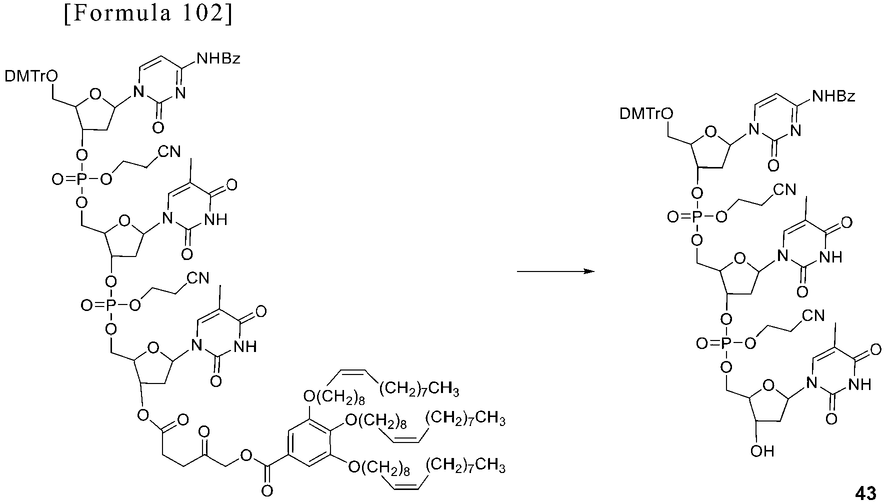

Under a nitrogen atmosphere, hydrazine monohydrate (12.1 µL, 0.39 mmol) was added dropwise to a solution of compound 42 (301 mg, 0.13 mmol) in anhydrous THF (6.4 mL), pyridine (3.2 mL), and acetic acid (1.6 mL). After stirring for 2.5 hours, THF (1 mL) was added and stirring was continued for an additional 4 hours. After confirming completion of the reaction, the solution was concentrated and purified by silica gel column chromatography (ethyl acetate/methanol) to obtain compound 43 as a white amorphous solid.

### Synthesis of Compound 44

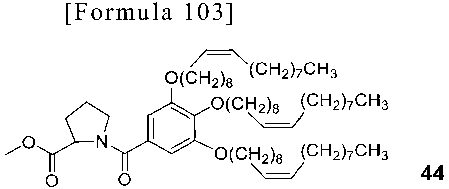

Under a nitrogen atmosphere, 3,4,5-tri-((Z)-octadec-9-enyloxy)benzoic acid (compound 2, 5.68 g, 6.16 mmol), DMF (270 mL), THF (30 mL), L-proline methyl ester hydrochloride (1.34 g, 8.09 mmol), COMU (3.47 g, 8.09 mmol), and DIPEA (3.45 mL, 20.5 mmol) were mixed and stirred overnight at room temperature after warming. After confirming completion of the reaction, the mixture was cooled to room temperature, and ethyl acetate (20 mL), n-hexane (20 mL), and water (20 mL) were added, and the aqueous layer was separated. The organic layer was washed with water (20 mL), and was concentrated under reduced pressure to give a crude product. The obtained crude product was purified by silica gel column chromatography (n-hexane/ethyl acetate) to obtain compound 44 as a colorless oily material (9.86 g, yield: 85%). ¹H-NMR (500 MHz, CDCl₃) δ 6.77 (s, 2H), 5.39-5.30 (m, 6H), 4.65-4.60 (m, 1H), 3.97 (q, J = 6.5 Hz, 6H), 3.96-3.88 (m, 1H), 3.78 (s, 3H), 3.72-3.64 (m, 1H), 3.63-3.55 (m, 1H), 2.36-2.26 (m, 1H), 2.01-2.95 (m, 13H), 1.93-1.84 (m, 1H), 1.83-1.68 (m, 6H), 1.52-1.40 (m, 6H), 1.38-1.20 (m, 60H), 0.87 (t, J = 7.0 MHz, 9H).

### Synthesis of Compound 45

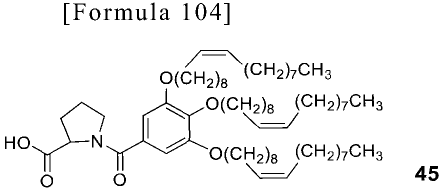

Under a nitrogen atmosphere, compound 44 (4.95 g, 4.79 mmol), potassium hydroxide (0.47 g, 8.4 mmol), THF (25 mL), methanol (5 mL), and deionized water (1 mL) were mixed and stirred overnight at 60 °C. After confirming completion of the reaction, 6 M HCl (2 mL), water (20 mL), and ethyl acetate (20 mL) were added, and the aqueous layer was separated. The organic layer was washed with water (20 mL) and concentrated under reduced pressure to obtain a crude product. The obtained crude product was purified by silica gel column chromatography (n-hexane/ethyl acetate) to obtain compound 45 as a white amorphous solid (2.69 g, yield: 56%).
¹H-NMR (500 MHz, CDCl₃) δ 6.73 (s, 2H), 5.39-5.30 (m, 6H), 4.81-4.72 (1H), 3.98 (q, J = 6.3 Hz, 6H), 3.70-3.62 (m, 1H), 3.59-3.48 (m, 1H), 2.60-2.50 (m, 1H), 2.19-2.07 (m, 1H), 2.06-1.95 (m, 13H), 1.95-1.85 (m, 1H), 1.84-1.69 (m, 6H), 1.50-1.40 (m, 6H), 1.38-1.20 (m, 60H), 0.87 (t, J = 7.0 MHz, 9H).

### Synthesis of Compound 46

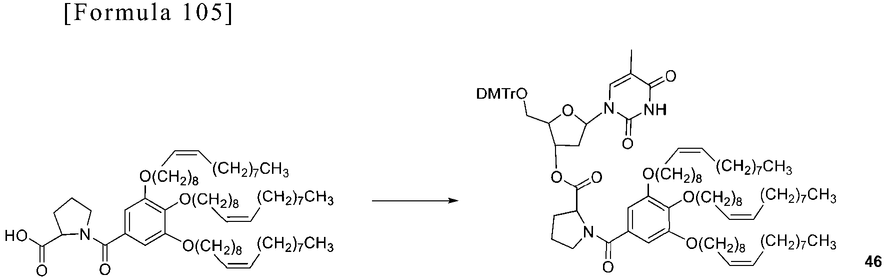

Using compound 45 (501 mg, 0.49 mmol), compound 46 was obtained as a white amorphous solid (648 mg, 86%) in the same manner as the synthesis of compound 39, except that the amounts of reagents and solvents were appropriately adjusted. The obtained compound 46 was used in the next reaction without further purification.
¹H-NMR (500 MHz, CDCl₃): δ 8.94 (s, 1H), 7.60 (s, 1H), 7.40-7.35 (m, 2H), 7.32-7.20 (m, 9H), 6.83 (d, J = 9.5 Hz, 4H), 6.78 (s, 2H), 6.50 (m, 1H), 5.62 (m, 1H), 5.39-5.30 (m, 6H), 4.60-4.55 (m, 1H), 4.12 (s, 1H), 4.00-3.90 (m, 6H), 3.79 (s, 6H), 3.71-3.55 (m, 2H), 3.54-3.44 (m, 2H), 2.63-2.46 (m, 1H), 2.54-2.44 (m, 1H), 2.40-2.30 (m, 1H), 2.20-1.85 (m, 15H), 1.84-1.60 (m, 6H), 1.65-1.20 (m, 69H), 0.87 (t, J = 7.0 MHz, 9H).

### Synthesis of Compound 47

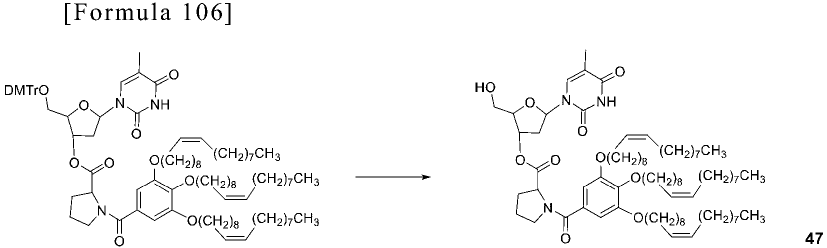

Using compound 46 (604 mg, 0.39 mmol), in the same manner as the synthesis of compound 40, except that the amounts of reagents and solvents were appropriately changed, compound 47 was obtained as a white amorphous solid (357 mg, yield 74%). The obtained compound 47 was used in the next reaction without further purification.

### Synthesis of compound 48

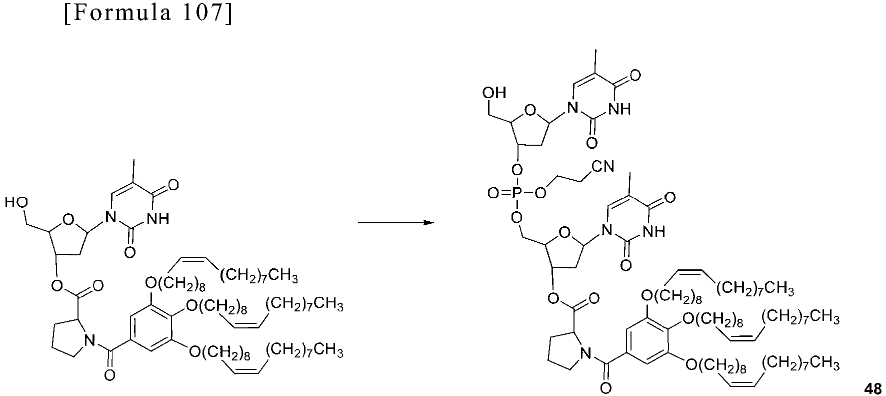

Using compound 47 (357 mg, 0.29 mmol), in the same manner as the synthesis of compound 41, except that the amounts of reagents and solvents were appropriately changed, compound 48 was obtained as a white amorphous solid (250 mg, yield 86%). The obtained compound 48 was used in the next reaction without further purification.

### Synthesis of compound 49

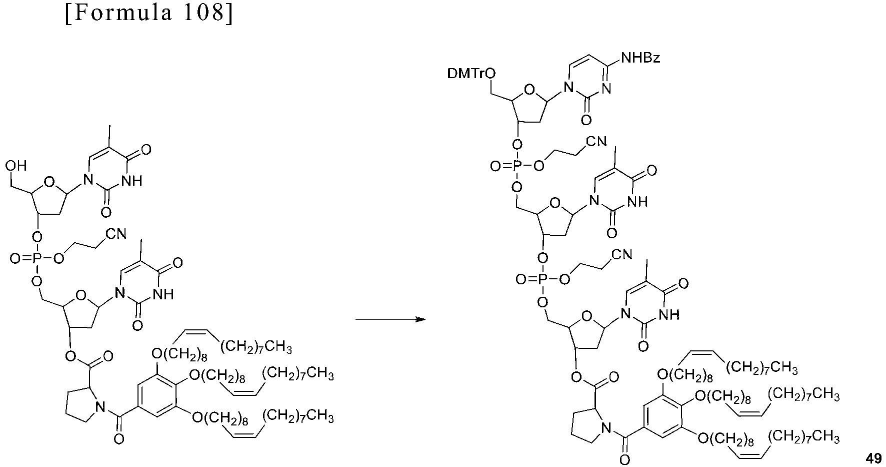

Using compound 48 (397 mg, 0.25 mmol), in the same manner as the synthesis of compound 42, except that the amounts of reagents and solvents were appropriately changed, compound 49 was obtained as a pale yellow amorphous solid (423 mg, yield 72%).

### Synthesis of compound 50

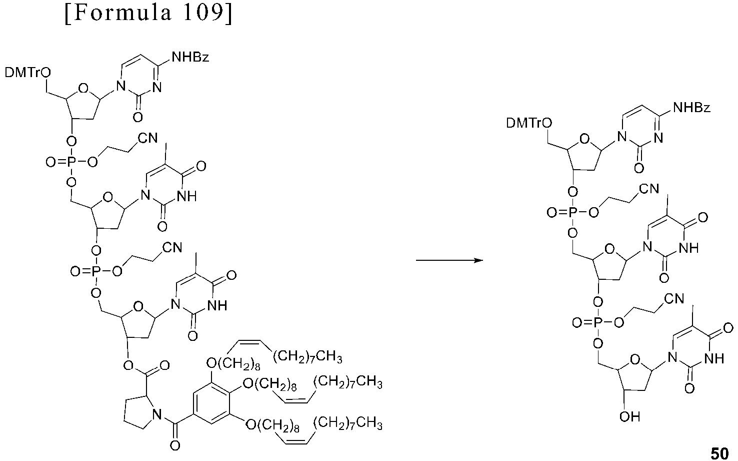

Under a nitrogen atmosphere, to a solution of compound 49 (423 mg, 0.18 mmol) in anhydrous THF (8.0 mL) and isopropanol (2.0 mL), a 4 M THF solution of lithium borohydride (0.23 mL, 0.90 mmol) was added dropwise at 0°C. After stirring at 0°C for 1 hour, 10% ammonium chloride aqueous solution (7.0 mL) and ethyl acetate were added, stirred, and the layers were separated to obtain the organic layer. The aqueous layer was extracted with ethyl acetate, then the combined organic layers were concentrated and water was removed, and the obtained crude product was purified by silica gel column chromatography (ethyl acetate/methanol) to give compound 50 as a white solid.

### Synthesis of compound 51

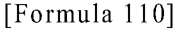

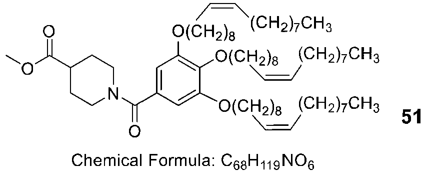

Under a nitrogen atmosphere, 3,4,5-tri-((Z)-octadec-9-enyloxy)benzoic acid (compound 2, 1.88 g, 2.04 mmol), DMF (90 mL), THF (10 mL), methyl 4-piperidinecarboxylate (351 µL, 2.65 mmol), COMU (1.11 g, 2.65 mmol), and DIPEA (1.12 mL, 2.65 mmol) were mixed and stirred overnight while heating from room temperature. After confirming completion of the reaction, the mixture was cooled to room temperature, and ethyl acetate (20 mL), n-hexane (20 mL), and water (20 mL) were added, and the aqueous layer was separated. The organic layer was washed with water (20 mL) and concentrated under reduced pressure to obtain a crude product. The obtained crude product was purified by silica gel column chromatography (n-hexane/ethyl acetate) to give compound 51 as a colorless oil (1.50 g, yield: 71%).
¹H-NMR (500 MHz, CDCl₃) δ 6.56 (s, 2H), 5.41-5.29 (m, 6H), 4.15-4.09 (m, 1H), 3.95 (q, J = 6.8 Hz, 6H), 3.71 (s, 3H), 3.11-2.91 (m, 2H), 2.64-2.54 (m, 1H), 2.07-1.88 (m, 13H), 1.82-1.66 (m, 7H), 1.51-1.41 (m, 6H), 1.38-1.20 (m, 62H), 0.88 (t, J = 6.8 MHz, 9H).

### Synthesis of compound 52

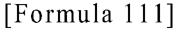

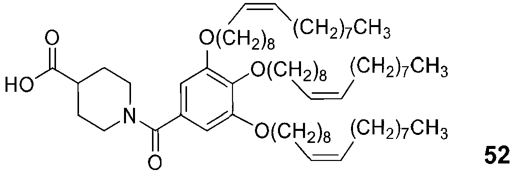

Under a nitrogen atmosphere, compound 51 (1.50 g, 1.45 mmol), potassium hydroxide (143 mg, 25.4 mmol), THF (10 mL), methanol (2.5 mL), and ion-exchanged water (500 µL) were mixed and stirred overnight at 60 °C. After confirming the completion of the reaction, 6 M HCl (2 mL), water (20 mL), and ethyl acetate (20 mL) were added, and the aqueous layer was separated. The organic layer was washed with water (20 mL) and concentrated under reduced pressure to obtain a crude product. The obtained crude product was purified by silica gel column chromatography (n-hexane/ethyl acetate) to give compound 52 as a white amorphous solid (1.45 g, yield: 97%).
¹H-NMR (500 MHz, CDCl₃) δ 6.56 (s, 2H), 5.41-5.29 (m, 6H), 4.15-4.09 (m, 1H), 3.95 (q, J = 6.5 MHz, 6H), 3.71 (s, 3H), 3.11-2.95 (m, 2H), 2.69-2.60 (m, 1H), 2.06-1.90 (m, 13H), 1.82-1.70 (m, 8H), 1.50-1.40 (m, 6H), 1.39-1.20 (m, 60H), 0.88 (t, J = 7.0 MHz, 9H).

### Synthesis of Compound 53

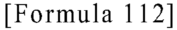

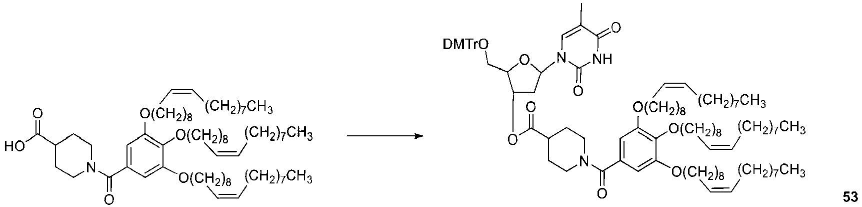

Under a nitrogen atmosphere, to a solution of Compound 52 (450 mg, 0.43 mmol), DMT-dT (396 mg, 0.73 mmol), and DMAP (89 mg, 0.73 mmol) in dichloromethane (10 mL), EDCI (140 mg, 0.73 mmol) was added at room temperature, and the mixture was stirred at the same temperature for 30 hours. After confirming completion of the reaction, the solvent was concentrated under reduced pressure to obtain a crude product, which was purified by silica gel column chromatography (n-hexane/ethyl acetate) to obtain Compound 53 as a white amorphous solid (571 mg, yield 85%).
¹H-NMR (500 MHz, CDCl₃) δ 7.99 (s, 1H), 7.61 (s, 1H), 7.39-7.36 (m, 2H), 7.33-7.21 (m, 9H), 6.84 (d, J = 8.5 Hz, 4H), 6.56 (s, 2H), 6.44-6.39 (m, 1H), 5.50-5.45 (m, 1H), 5.40-5.30 (m, 6H), 4.06-4.10 (m, 1H), 4.00-3.90 (m, 6H), 3.79 (m, 6H), 3.54-3.43 (m, 2H), 3.10-2.90 (m, 2H), 2.65-2.55 (m, 1H), 2.50-2.40 (m, 2H), 2.10-1.90 (m, 13H), 1.84-1.69 (m, 6H), 1.50-1.40 (m, 6H), 1.40-1.20 (m, 66H), 0.88 (t, J = 7.0 MHz, 9H).

### Synthesis of Compound 54

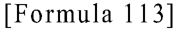

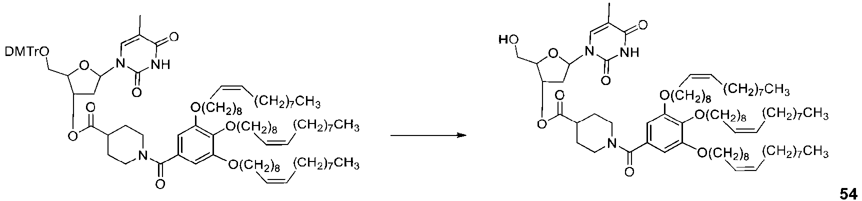

Using compound 53 (571 mg, 0.37 mmol), in the same manner as the synthesis of compound 40, except that the amounts of reagents and solvents were appropriately changed, compound 54 was obtained as a white amorphous solid (520 mg, yield 99%). Compound 54 was used in the next reaction without further purification.

### Synthesis of Compound 55

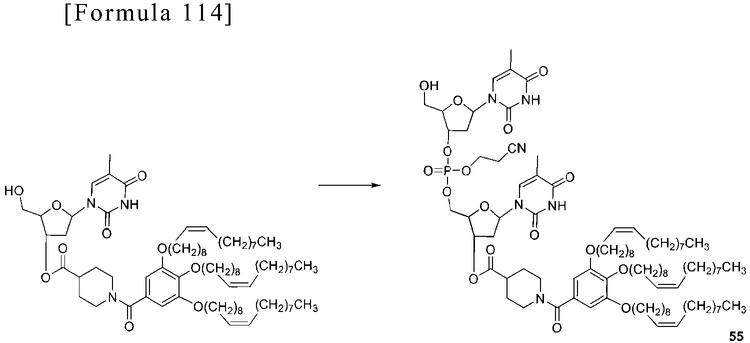

Using compound 54 (469 mg, 0.37 mmol), in the same manner as the synthesis of compound 41, except that the amounts of reagents and solvents were appropriately changed, compound 55 was obtained as a white amorphous solid (501 mg, yield 76%). Compound 55 was used in the next reaction without further purification.

### Synthesis of Compound 56

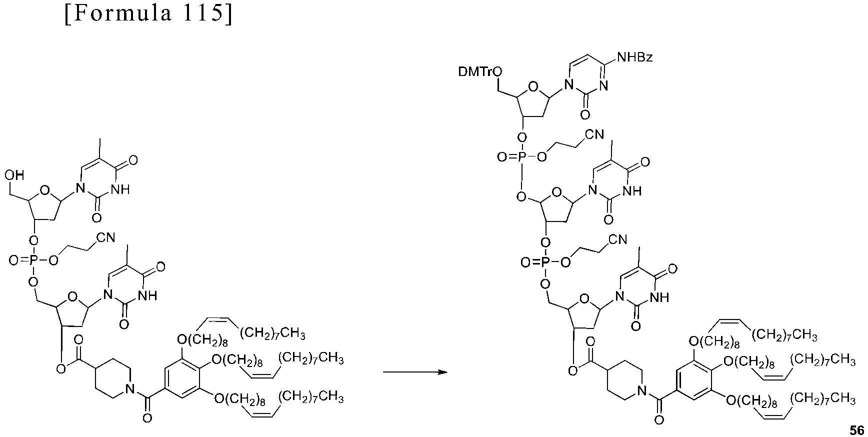

Using compound 55 (501 mg, 0.31 mmol), in the same manner as the synthesis of compound 42, except that the amounts of reagents and solvents were appropriately changed, compound 56 was obtained as a pale yellow amorphous solid (147 mg, yield 20%).

### Synthesis of Compound 9

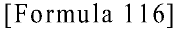

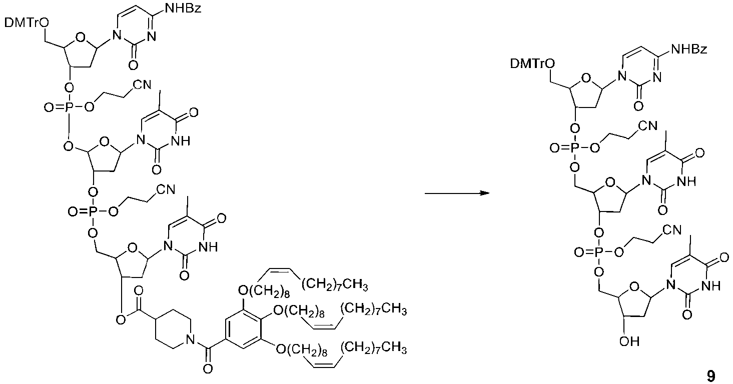

Using compound 56 (147 mg, 0.06 mmol), in the same manner as the synthesis of compound 50, except that the amounts of reagents and solvents were appropriately changed, compound 9 was obtained.

### Synthesis of Compound 58

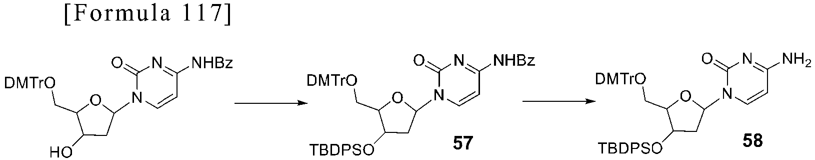

Under a nitrogen atmosphere, TBDPSCl (0.529 mL) was added dropwise at room temperature to a mixture of compound 1 (N⁴-Benzoyl-5'-O-(4,4'-dimethoxytrityl)-2'-deoxycytidine) (0.868 g, 1.37 mmol), imidazole (0.466 g, 6.85 mmol), and acetonitrile (7 mL). After stirring at room temperature for 3.5 hours, methanol (0.277 mL) was added. MTBE (7 mL) and saturated aqueous sodium bicarbonate (7 mL) were added, stirred, and the precipitated solid was removed by filtration using MTBE as the washing solvent. The filtrate was subjected to phase separation, and the obtained organic layer was dried over magnesium sulfate and concentrated. Purification by silica gel chromatography (spherical neutral silica gel, eluent: hexane/ethyl acetate) gave compound 57 as a white solid. To the THF solution (9.7 mL) of compound 57 was added a 7 M ammonia in methanol (9.7 mL) at room temperature. The mixture was stirred at room temperature for 88 hours. After concentration of the reaction mixture, purification by silica gel chromatography (spherical neutral silica gel, eluent: ethyl acetate/methanol) gave compound 58 as a white solid (1.067 g, yield 101%).
MS (ESI⁺): [M+H]⁺ 768.3470.

### Synthesis of Compound 59

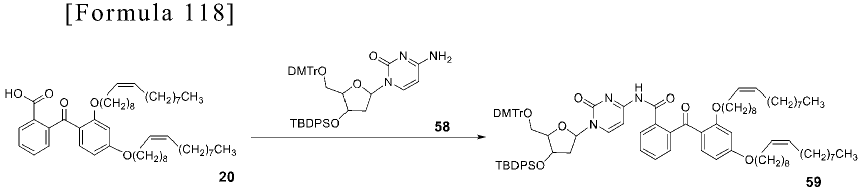

Under a nitrogen atmosphere, to a toluene (1.23 mL) solution of Compound 20 (123 mg, 0.162 mmol) were added pyridine (0.0196 mL, 0.243 mmol) and thionyl chloride (0.0584 mL, 0.81 mmol) at room temperature, and the mixture was stirred for 45 minutes. After concentration of the reaction solution, azeotropic distillation with toluene was carried out to prepare an acid chloride of Compound 20.

Under a nitrogen atmosphere, in a separate reaction vessel, Compound 58 (136.7 mg, 0.178 mmol), toluene (0.86 mL), and pyridine (0.86 mL) were added to prepare a solution. Furthermore, DIPEA (0.14 mL, 0.81 mmol) and molecular sieves 4A (200 mg) were added, and the mixture was stirred for 30 minutes. The solution containing the acid chloride of Compound 20 prepared above was added, and the mixture was stirred at room temperature for 45 minutes. After addition of DMAP (2 mg, 0.0162 mmol), the mixture was further stirred at room temperature for 20 minutes, followed by heating to 45°C and stirring for 1 hour 30 minutes. After cooling the reaction solution to around room temperature, saturated aqueous sodium bicarbonate and ethyl acetate were added, and the mixture was stirred and the layers were separated. The obtained organic layer was washed with saturated aqueous sodium chloride, dried over magnesium sulfate, and concentrated. Purification by silica gel chromatography (spherical neutral silica gel, developing solvent: ethyl acetate/methanol) afforded Compound 59 (124 mg, yield: 51%) as a viscous colorless oil.
MS (ESI⁺): [M+H]⁺ 1508.9227.

### Synthesis of Compound 61

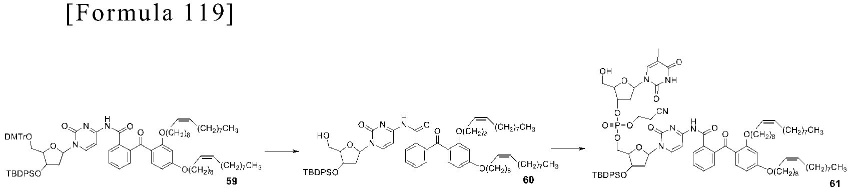

Under a nitrogen atmosphere, to an ethyl acetate (2.54 mL) solution of Compound 59 (212 mg, 0.140 mmol) were added mercaptosuccinic acid (105 mg, 0.70 mmol) and trifluoroacetic acid (0.078 mL, 1.02 mmol) at 0°C, and the mixture was stirred for 1 hour 15 minutes while warming to room temperature. After cooling the reaction solution to 0°C, trifluoroacetic acid (0.020 mL, 0.261 mmol) was added, and the mixture was stirred for 37 minutes while warming to room temperature. After cooling the reaction solution to 0°C, trifluoroacetic acid (0.020 mL, 0.261 mmol) was added, and the mixture was stirred for 30 minutes while warming to room temperature. After cooling to 0°C, saturated aqueous sodium bicarbonate (3.1 mL) was added to the reaction solution, and the mixture was stirred while warming to room temperature, followed by phase separation. The obtained organic layer was washed with saturated aqueous sodium chloride (2 mL). The obtained organic layer was dried over magnesium sulfate and concentrated to give Compound 60 (180.6 mg) as a viscous colorless oil. The obtained Compound 60 was subjected to azeotropic distillation with ethyl acetate (1.8 mL) twice. Under a nitrogen atmosphere, Compound 60 was dissolved in ethyl acetate (2.5 mL), followed by addition of molecular sieves 3A (180 mg), and the mixture was stirred for 30 minutes. To the obtained solution were added DMT-dT-CE phosphoramidite (130 mg, 0.175 mmol) and DCI (33.9 mg, 0.288 mmol), and the mixture was stirred at room temperature for 45 minutes. DMT-dT-CE phosphoramidite (26.1 mg, 0.035 mmol) and DCI (16.5 mg, 0.14 mmol) were added, and the mixture was stirred at room temperature for 40 minutes. After cooling the reaction solution to 0°C, 0.2 M iodine solution (ethyl acetate/pyridine/water, 1.89 mL) was added, and the mixture was stirred for 40 minutes while warming to room temperature. After cooling the reaction solution to 0°C, 1 M aqueous sodium thiosulfate solution (2 mL) was added, and the mixture was stirred while warming to room temperature, followed by filtration, washing the molecular sieves with ethyl acetate. The layers of filtrate were separated, and the obtained organic layer was washed with saturated aqueous sodium bicarbonate (2 mL) and saturated aqueous sodium chloride (2 mL), dried over magnesium sulfate, and concentrated. To the obtained residue were added ethyl acetate (2.5 mL) and mercaptosuccinic acid (105 mg, 0.70 mmol). After cooling the obtained mixture to 0°C, trifluoroacetic acid (0.096 mL, 1.26 mmol) was added, and the mixture was stirred for 1 hour while warming to room temperature. The reaction solution was cooled to 0°C, followed by addition of saturated aqueous sodium bicarbonate (2.8 mL), and the mixture was stirred while warming to room temperature, then the payers were separated. The obtained organic layer was washed with saturated aqueous sodium chloride (1 mL). The obtained organic layer was dried over magnesium sulfate, concentrated to dryness, and Compound 61 (207 mg, yield: 93.6%) was obtained as a white solid. The obtained Compound 61 was used in the next reaction without further purification.
MS (ESI⁺): [M+H]⁺ 1563.8636.

### Synthesis of Compound 62

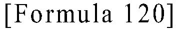

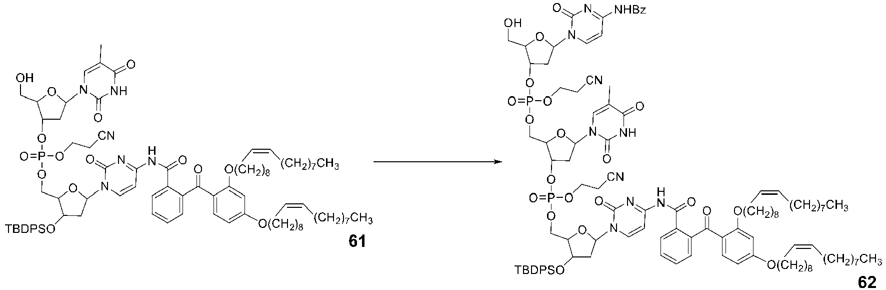

Compound 61 (198 mg, 0.125 mmol) was subjected to azeotropic distillation with ethyl acetate (2 mL) twice. Under a nitrogen atmosphere, Compound 61 was dissolved in ethyl acetate (2.4 mL), followed by addition of molecular sieves 3A (200 mg), and the mixture was stirred for 30 minutes. To the obtained solution were added DMT-dC-CE phosphoramidite (157 mg, 0.188 mmol) and DCI (34 mg, 0.288 mmol), and the mixture was stirred at room temperature for 1 hour 30 minutes. After cooling the reaction solution to 0°C, 0.2 M iodine solution (ethyl acetate/pyridine/water, 1.69 mL) was added, and the mixture was stirred for 23 minutes while warming to room temperature. After cooling the reaction solution to 0°C, 1 M aqueous sodium thiosulfate solution (2.5 mL) was added, and the mixture was stirred, followed by filtration of the molecular sieves while washing with ethyl acetate. The layers of filtrate were separated, and the obtained organic layer was washed with saturated aqueous sodium bicarbonate (2 mL), followed by washing with saturated aqueous sodium chloride (1 mL). The obtained organic layer was dried over magnesium sulfate and concentrated. To the obtained residue were added ethyl acetate (2 mL) and mercaptosuccinic acid (98 mg, 0.655 mmol). After cooling the obtained mixture to 0°C, trifluoroacetic acid (0.0765 mL, 1 mmol) was added, and the mixture was stirred for 1 hour while warming to room temperature. The reaction solution was cooled to 0°C, followed by addition of saturated aqueous sodium bicarbonate (2.5 mL), and the mixture was stirred while warming to room temperature, then the layers were separated. The obtained organic layer was washed with saturated aqueous sodium chloride (1 mL). The obtained organic layer was dried over magnesium sulfate, concentrated to dryness, and Compound 62 (248.7 mg, yield: 94.7%) was obtained as a white solid. The obtained Compound 62 was used in the next reaction without further purification.

### Synthesis of Compound 63

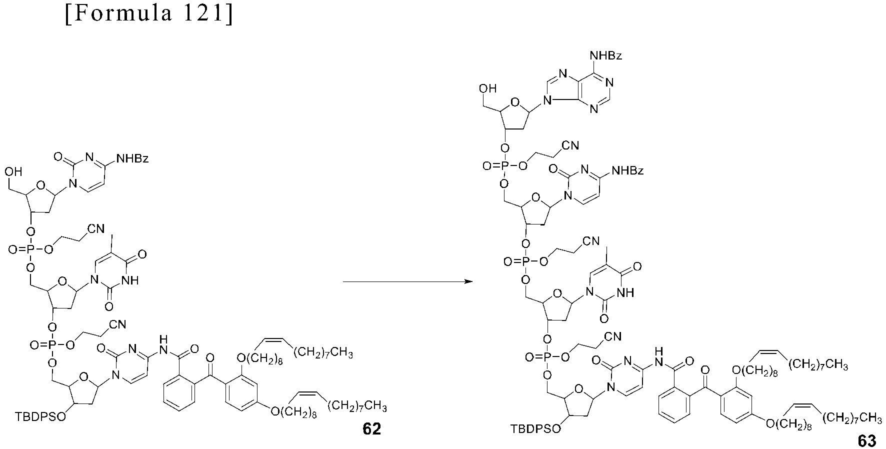

Compound 62 (238.7 mg, 0.119 mmol) was subjected to azeotropic distillation with ethyl acetate (1.7 mL) twice. Under a nitrogen atmosphere, Compound 7 was dissolved in ethyl acetate (2.4 mL), followed by addition of molecular sieves 3A (240 mg), and the mixture was stirred for 40 minutes. To the obtained reaction solution were added DMT-dA-CE phosphoramidite (153.6 mg, 0.179 mmol) and DCI (35.2 mg, 0.298 mmol), and the mixture was stirred at room temperature for 2 hours. Furthermore, DCI (14.1 mg, 0.119 mmol) was added, and the mixture was stirred at room temperature for 30 minutes. After confirming completion of the reaction, Boc-Ser-OH (17.1 mg, 0.0833 mmol) was added, and the mixture was stirred for 18 minutes. After cooling the reaction solution to 0°C, 0.2 M iodine solution (ethyl acetate/pyridine/water, 1.61 mL) was added, and the mixture was stirred for 19 minutes while warming to room temperature. After cooling the reaction solution to 0°C, 1 M aqueous sodium thiosulfate solution (2.4 mL) was added, and the mixture was stirred, followed by filtration of the molecular sieves, while washing with ethyl acetate. The layers of filtrate were separated, and the obtained organic layer was washed with saturated aqueous sodium bicarbonate (2 mL), followed by washing with saturated aqueous sodium chloride (1 mL). The obtained organic layer was dried over magnesium sulfate and concentrated. To the obtained residue were added ethyl acetate (2.9 mL) and mercaptosuccinic acid (89.3 mg, 0.595 mmol). After cooling the obtained mixture to 0°C, trifluoroacetic acid (0.111 mL, 1.45 mmol) was added, and the mixture was stirred for 1 hour while warming to room temperature. The reaction solution was cooled to 0°C, followed by addition of saturated aqueous sodium bicarbonate (2.8 mL), and the mixture was stirred while warming to room temperature, then the layers were separated. The obtained organic layer was washed with saturated aqueous sodium chloride (2 mL). The obtained organic layer was dried over magnesium sulfate, concentrated to dryness, and Compound 63 (279.3 mg, yield: 94.6%) was obtained as a pale yellow solid. The obtained Compound 63 was used in the next reaction without further purification.

### Synthesis of Compound 64

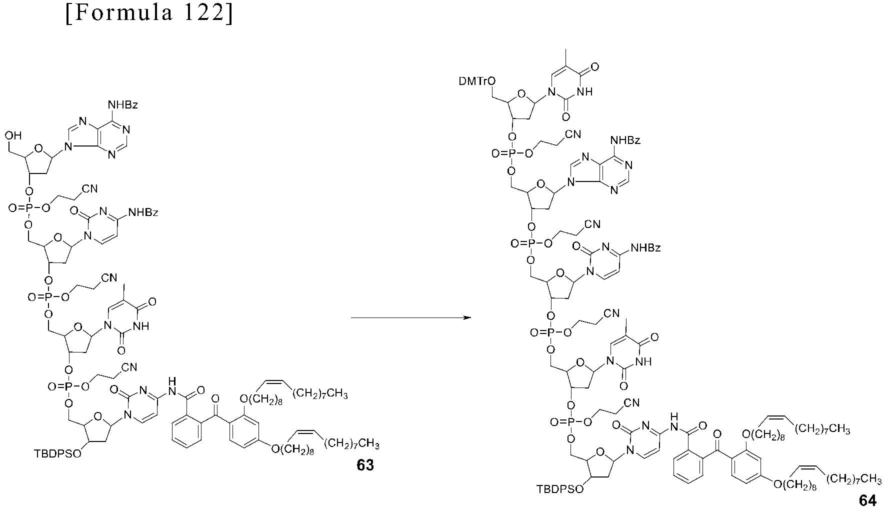

Compound 63 (269.3 mg, 0.109 mmol) was subjected to azeotropic distillation twice with ethyl acetate (1.5 mL). Under a nitrogen atmosphere, ethyl acetate (2.4 mL) was added to compound 63 to prepare a solution, followed by the addition of molecular sieves 3A (240 mg), DMTTr-dT-CE phosphoramidite (124.4 mg, 0.167 mmol), and DCI (39.3 mg, 0.333 mmol), and the mixture was stirred at room temperature for 52 minutes. After confirming completion of the reaction, Boc-Ser-OH (17.1 mg, 0.0833 mmol) was added, and the mixture was stirred for 24 minutes. After cooling the reaction solution to 0°C, 0.2 M iodine (ethyl acetate/pyridine/water solution, 1.55 mL) was added, and the mixture was stirred for 10 minutes while warming to room temperature. After cooling the reaction solution to 0°C, 1 M sodium thiosulfate aqueous solution (2.5 mL) was added, and the mixture was stirred, followed by filtration of the molecular sieves, while washing with ethyl acetate. The layers of filtrate were separated, and the obtained organic layer was washed twice with saturated sodium bicarbonate solution (2.4 mL) and washed with saturated sodium chloride solution (1.2 mL). The obtained organic layer was dried over magnesium sulfate and concentrated. Purification by silica gel chromatography (spherical neutral silica gel, developing solvent: ethyl acetate/methanol) afforded compound 64 (325.4 mg, yield 95.4%) as a pale yellow solid.
MS(ESI⁺): [M+H]⁺ 1570.1425.

### Synthesis of compound 64

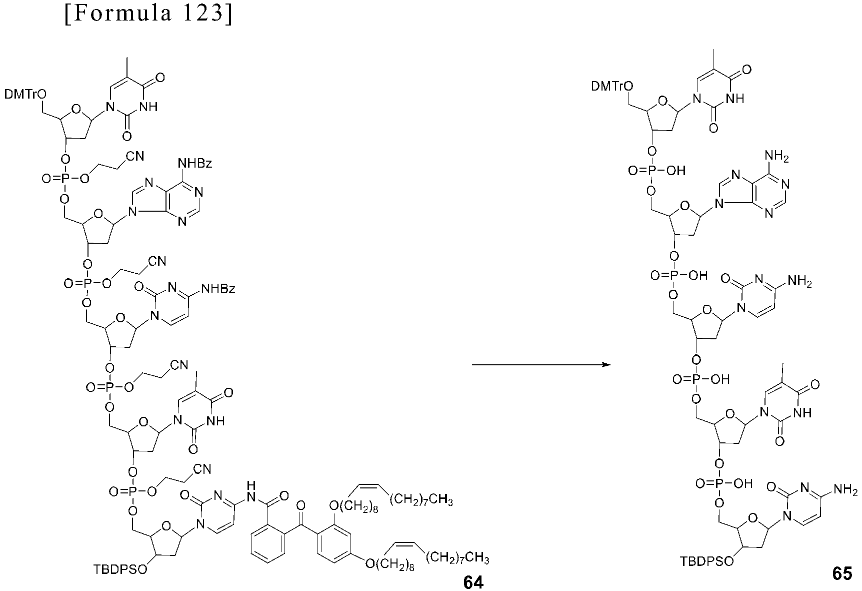

To compound 64 (10.2 mg, 0.00325 mmol) were added 7 M ammonia in methanol (0.3 mL) and THF (0.3 mL), and the mixture was stirred at room temperature for 40 hours. LC-Mass analysis of the reaction solution confirmed that compound 65 was the main product. MS(ESI⁺): [M+H]⁺ 1978.5303.

### Synthesis of compound 66

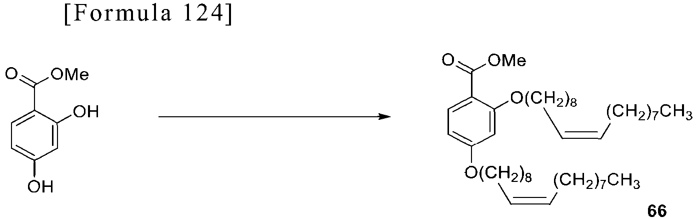

Under a nitrogen atmosphere, methyl 2,4-dihydroxybenzoate (1.20 g, 7.14 mmol), K₂CO₃ (3.95 g, 28.6 mmol), DMAc (24 mL), and oleyl bromide (5.20 g, 15.7 mmol) were mixed at room temperature, and the mixture was heated to 80°C and stirred at 80 °C for 3 hours. Oleyl bromide (2.36 g, 7.14 mmol) was then added at 80 °C, and the mixture was stirred for 2 hours. After completion of the reaction, the reaction mixture was cooled with ice to 0°C, followed by addition of n-hexane (24 mL), water (24 mL), and ethyl acetate (24 mL), and the aqueous layer was separated. The separated organic layer was washed three times with water (24 mL) and concentrated under reduced pressure to obtain a crude product of Compound 66. The obtained crude product was purified by silica gel column chromatography (n-hexane/ethyl acetate) to give Compound 66 as an oily material in 96% yield (4.59 g, 6.86 mmol).
¹H-NMR (500 MHz, CDCl₃) δ 7.88-7.77 (m, 1H), 6.45 (s, 2H), 5.38-5.32 (m, 4H), 3.98 (q, J = 6.9 Hz, 4H), 3.84 (s, 3H), 2.05-1.97 (m, 8H), 1.87-1.75 (m, 4H), 1.53-1.41 (m, 4H), 1.33-1.24 (m, 40H), 0.91-0.84 (m, 6H).
¹³C-NMR (500 MHz, CDCl₃) δ 166.4, 163.7, 160.9, 133.8, 129.9, 112.4, 105.1, 100.3, 68.9, 68.2, 51.5, 31.9, 29.8, 29.8, 29.5, 29.5, 29.5, 29.3, 29.2, 29.3, 27.2, 27.2, 26.0, 26.0, 22.7, 14.1.

### Synthesis of Compound 67

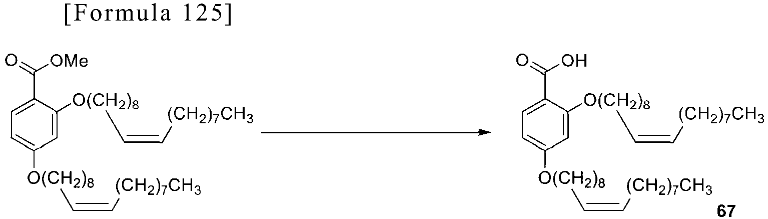

Under a nitrogen atmosphere, to a solution of Compound 66 (4.52 g, 6.75 mmol) in THF (22.6 mL) were added ethanol (22.6 mL) and 4 M sodium hydroxide (6.75 mL). The mixture was then heated to 70°C and stirred for 2 hours. After completion of the reaction, the mixture was cooled with ice to 0°C, and 2 M HCl (15 mL), ethyl acetate (90 mL), water (90 mL), and n-hexane (90 mL) were added, followed by separation of the aqueous layer. The separated organic layer was washed with water (90 mL), and concentrated under reduced pressure to give the crude product of Compound 67. The crude product was purified by silica gel column chromatography (n-hexane/ethyl acetate) to give Compound 67 as an oily material in 98% yield (4.35 g, 6.64 mmol).
¹H-NMR (500 MHz, CDCl₃) δ 10.75 (br s, 1H), 8.11 (br d, J = 8.7 Hz, 1H), 6.62 (br d, J = 8.7 Hz, 1H), 6.50 (s, 1H), 5.38-5.32 (m, 4H), 4.19 (br t, J = 6.4 Hz, 2H), 4.01 (br t, J = 6.4 Hz, 2H), 2.02 (br s, 8H), 1.90 (quin, J = 6.9 Hz, 2H), 1.80 (quin, J = 6.8 Hz, 2H), 1.51-1.38 (m, 4H), 1.34-1.24 (m, 40H), 0.88 (br t, J = 6.6 Hz, 6H).
¹³C-NMR (500 MHz, CDCl₃) δ 165.3, 164.6, 159.0, 135.5, 130.0, 130.0, 129.8, 129.7, 110.3, 107.1, 99.8, 70.2, 68.6, 31.9, 29.8, 29.5, 29.4, 29.3, 29.2, 29.5, 29.1, 28.9, 27.2, 27.2, 27.2, 26.0, 25.9, 22.7, 14.1.

### Synthesis of Compound 68

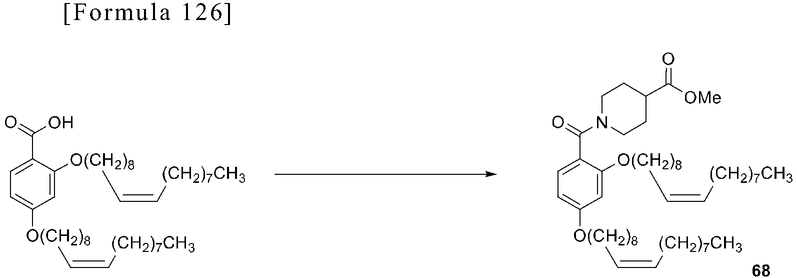

Under a nitrogen atmosphere, at 0°C, to a solution of Compound 67 (4.30 g, 6.56 mmol), 4-piperidinecarboxylic acid methyl ester (1.88 g, 13.1 mmol), 1-hydroxybenzotriazole monohydrate (1.77 g, 13.1 mmol), and DIPEA (4.57 mL, 26.3 mmol) in DMF (43 mL) were added 1-(3-dimethylaminopropyl)-3-ethylcarbodiimide hydrochloride [EDCI] (2.52 g, 13.1 mmol), and the mixture was stirred at room temperature for 4 hours. EDCI (1.26 g, 6.56 mmol) and 1-hydroxybenzotriazole monohydrate (0.86 g, 6.56 mmol) were added, and the mixture was further stirred for 2 hours. After the addition of ethyl acetate (43 mL), n-hexane (43 mL), and water (43 mL) and stirring, the aqueous layer was separated. The obtained organic layer was washed three times with water (43 mL) and concentrated. The obtained crude product is purified by silica gel column chromatography (n-hexane/ethyl acetate) to give Compound 68 as an oily material in 92% yield (4.69 g, 6.01 mmol).
¹H-NMR (500 MHz, CDCl₃) δ ppm 0.84-0.91 (m, 6H), 1.24-1.33 (m, 35H), 1.43 (s, 3H), 1.72-1.81 (m, 5H), 1.96-2.05 (m, 9H), 2.50-2.55 (m, 1H), 2.93 (br t, J = 12.14 Hz, 1H), 3.04 (br s, 1H), 3.49-3.57 (m, 1H), 3.69 (s, 3H), 3.90-3.99 (m, 3H), 4.59 (br t, J = 14.66 Hz, 1H), 5.31-5.38 (m, 4H), 6.40-6.48 (m, 2H), 7.11 (br d, J = 8.51 Hz, 0.3H), 7.16 (br d, J = 8.20 Hz, 0.7H).
¹³C-NMR (126 MHz, CDCl₃) δ ppm 14.13, 22.69, 26.00, 26.05, 27.21, 27.24, 28.04, 29.17, 29.24, 29.34, 29.38, 29.48, 29.54, 29.78, 29.78, 30.33, 31.92, 41.04, 4.18, 51.83, 68.19, 68.34, 68.53, 99.82, 9.86, 105.44, 05.44, 125.53, 129.81, 130.00, 168.00, 174.79.

### Synthesis of Compound 69

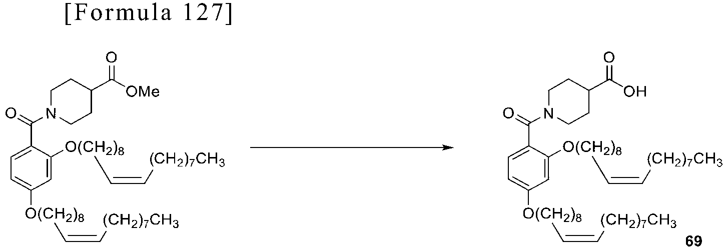

Under a nitrogen atmosphere, to a solution of Compound 68 (4.60 g, 5.90 mmol) in THF (21 mL) was added 4 M sodium hydroxide (5.9 mL, 23.58 mmol), and the mixture was stirred at room temperature for 1 hour. Ethanol (21 mL) was then added, and the mixture was heated to 70°C and stirred for 2 hours. After completion of the reaction, the mixture was cooled with ice to 0°C, and 2 M HCl (18.4 mL), ethyl acetate (21 mL), water (21 mL), and n-hexane (21 mL) were added, followed by separation of the aqueous layer. The separated organic layer was washed with water (21 mL) and concentrated under reduced pressure to give the crude product of Compound 69. Purification of the obtained crude product by silica gel column chromatography (n-hexane/ethyl acetate) gave Compound 69 as an amorphous solid in 99% yield (4.60 g, 5.90 mmol). ¹H-NMR (500 MHz, CDCl₃) δ ppm 0.87 (td, J = 7.01, 1.73 Hz, 6H), 1.22-1.35 (m, 42H), 1.39-1.45 (m, 2H), 1.49-1.60 (m, 1H), 1.83-1.93 (m, 1H), 1.97-2.04 (m, 8H), 2.23-2.33 (m, 1H), 2.65-2.76 (m, 1H), 2.87-2.98 (m, 1H), 3.42 (br d, J = 10.09 Hz, 1H), 3.85 (br s, 4H), 4.48 (br d, J = 8.83 Hz, 1H), 5.31-5.38 (m, 4H), 7.14 (br d, J = 7.88 Hz, 1H). ¹³C-NMR (126 MHz, CDCl₃) δ ppm 14.12, 22.69, 25.79, 26.19, 27.25, 29.04, 29.12, 29.33, 29.39, 29.59, 29.67, 29.70, 29.82, 29.93, 30.32, 31.92, 42.23, 44.19, 47.39, 68.14, 68.38, 99.61, 105.54, 118.16, 129.56, 129.75, 129.90, 129.95, 155.92, 161.28, 168.20, 182.53.

### Synthesis of Compound 70

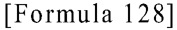

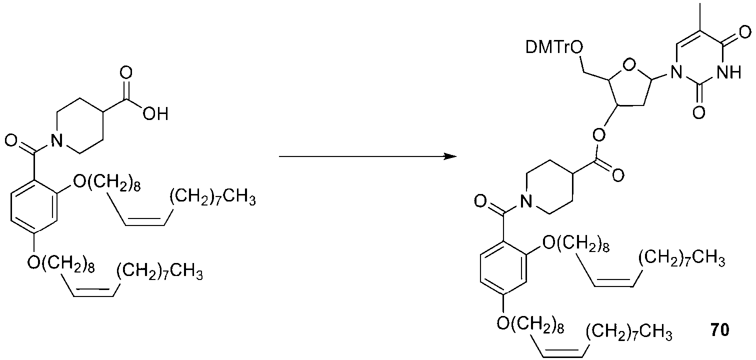

Under a nitrogen atmosphere, at 0°C, to a solution of Compound 69 (4.50 g, 5.87 mmol), 1-(3-dimethylaminopropyl)-3-ethylcarbodiimide hydrochloride [EDCI] (2.25 g, 11.75 mmol), 1-hydroxybenzotriazole monohydrate (1.80 g, 11.75 mmol), and DIPEA (3.04 g, 23.49 mmol) in DMF (45 mL), 5'-O-(4,4'-dimethoxytrityl)thymidine (2.88 g, 5.29 mmol) was added, and the mixture was stirred for 24 hours while allowing the temperature to rise to room temperature. EDCI (253 mg, 1.32 mmol), DIPEA (691 µL, 3.97 mmol), and 5'-O-(4,4'-dimethoxytrityl)thymidine (6.40 g, 11.75 mmol) were then added to the reaction mixture, followed by stirring at room temperature for 24 hours. After completion of the reaction, ethyl acetate (90 mL), n-hexane (45 mL), and water (45 mL) were added, stirred, and the aqueous layer was separated. The obtained organic layer was washed three times with saturated aqueous sodium bicarbonate solution (20 mL) and then washed with water (20 mL), followed by concentration. The obtained crude product was purified by silica gel column chromatography (n-hexane/ethyl acetate) to give Compound 70 as an oily material in 99% yield (8.10 g, 5.86 mmol). ¹H-NMR (500 MHz, CDCl₃) δ ppm 0.83-0.91 (m, 6H), 1.23-1.34 (m, 38H), 1.36 (br s, 7H), 1.37-1.52 (m, 5H), 1.65-1.81 (m, 7H), 1.97-2.05 (m, 9H), 2.39-2.48 (m, 2H), 2.50-2.58 (m, 1H), 2.85-2.94 (m, 1H), 3.43-3.52 (m, 2H), 3.79 (s, 6H), 3.88-4.00 (m, 4H), 4.04-4.09 (m, 1H), 4.65 (br s, 1H), 5.31-5.38 (m, 4H), 5.47 (br s, 1H), 6.42 (br s, 2H), 6.47 (br d, J = 8.20 Hz, 1H), 6.83 (d, J = 8.51 Hz, 4H), 7.23-7.31 (m, 8H), 7.37 (br d, J = 7.57 Hz, 2H), 7.59-7.62 (m, 1H), 8.21-8.32 (m, 1H). ¹³C-NMR (126 MHz, CDCl₃) δ ppm 11.59, 14.12, 22.68, 26.04, 27.20, 27.22, 29.24, 29.28, 29.32, 29.38, 29.47, 29.53, 29.77, 31.90, 55.26, 68.20, 76.90, 77.12, 84.30, 87.26, 105.50, 113.34, 127.28, 128.06, 128.13, 129.79, 129.80, 130.00, 130.09, 135.33, 158.82.

### Synthesis of Compound 71

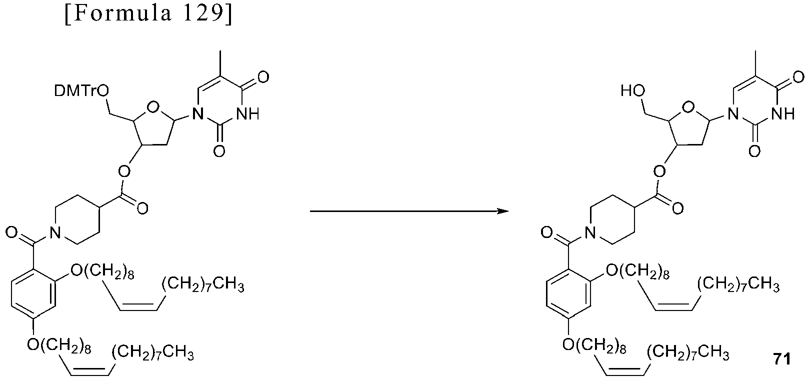

Under a nitrogen atmosphere, to a solution of Compound 70 (4.00 g, 3.09 mmol) in ethyl acetate (40 mL) were added mercaptosuccinic acid (2.32 g, 15.47 mmol) and trifluoroacetic acid (2.37 mL, 30.9 mmol), and the mixture was stirred under ice cooling for 4 hours. After confirming completion of the reaction, the reaction mixture was washed four times with saturated NaHCO₃ aqueous solution (40 mL) and once with 10% NaCl aqueous solution (40 mL), followed by concentration under reduced pressure to give Compound 71 as an amorphous solid (3.09 g, yield: 100%). The obtained Compound 71 was used in the next step without further purification.
¹H-NMR (500 MHz, CDCl₃) δ ppm 0.85-0.91 (m, 6H), 1.24-1.46 (m, 48H), 1.73-1.80 (m, 4H), 2.02 (br s, 10H), 2.31-2.46 (m, 2H), 2.57 (br s, J = 10.40 Hz, 1H), 2.89-2.98 (m, 1H), 3.57 (br s, 1H), 3.88-4.06 (br s, 4H), 4.60-4.69 (m, 1H), 5.32-5.39 (m, 5H), 6.22-6.26 (m, 1H), 6.42 (br s, 1H), 6.48 (br d, J = 8.20 Hz, 1H), 7.18 (br d, J = 8.20 Hz, 1H), 7.53-7.56 (m, 1H), 8.54-8.65 (m, 1H).
¹³C-NMR (126 MHz, CDCl₃) δ ppm 12.58, 14.12, 22.68, 25.95, 26.04, 27.20, 27.22, 29.16, 29.24, 29.32, 29.38, 29.47, 29.52, 29.77, 31.90, 62.59, 68.20, 75.09, 85.20, 85.93, 105.51, 111.44, 129.79, 130.00, 150.31.

### Synthesis of Compound 72

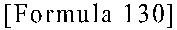

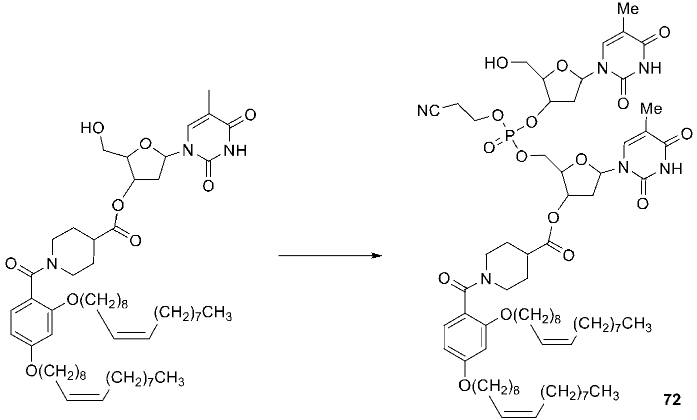

Compound 71 (500 mg, 0.51 mmol) was subjected to azeotropic distillation with ethyl acetate (10 mL) three times. To the obtained Compound 71 were added molecular sieves 3A (500 mg), DMT-dT-CE phosphoramidite (752 mg, 1.01 mmol), and ethyl acetate (5 mL), and the mixture was stirred at room temperature for 1 hour. To the reaction solution was added DCI (197 mg, 1.67 mmol), and stirring was continued at room temperature for 2 hours. After confirming completion of the reaction, 0.2 M iodine solution (ethyl acetate/pyridine/water, 7.6 mL) was added dropwise to the reaction solution at room temperature, and the mixture was stirred for 1 hour. After confirming completion of the reaction, 1 M Na₂S₂O₃ aqueous solution (7.5 mL) was added, and the mixture was stirred. The insoluble material was removed by filtration while washing with ethyl acetate, and the layers were separated. The organic layer was washed with 10% brine (7.5 mL), followed by dried over sodium sulfate. The organic layer was concentrated under reduced pressure, and azeotropic distillation with ethyl acetate (5 mL) was performed three times. To the residue was added ethyl acetate (5 mL) to dissolve, and mercaptosuccinic acid (379 mg, 2.52 mmol) was added, and the mixture was cooled to 0°C. Trifluoroacetic acid (0.39 mL, 5.05 mmol) was added, and the mixture was stirred at the same temperature for 3 hours. After confirming completion of the reaction, water (7.5 mL) was added, and the organic layer was separated. The obtained organic layer was washed three times with saturated aqueous sodium hydrogencarbonate solution (7.5 mL) and once with 10% brine (7.5 mL). The obtained organic layer was concentrated to give Compound 72 as an amorphous solid (470 mg, yield: 69%). The obtained Compound 72 was used in the next step without further purification.
¹H-NMR (500 MHz, CDCl₃) δ ppm 0.88 (t, J = 6.78 Hz, 6H), 1.25-1.48 (m, 44H), 1.75-1.82 (m, 4H), 1.86-1.93 (m, 3H), 2.01 (br s, 8H), 2.38-2.43 (m, 2H), 2.43-2.62 (m, 3H), 2.82 (br t, J = 5.36 Hz, 2H), 2.87-2.97 (m, 1H), 3.50-3.64 (m, 1H), 3.84-4.01 (m, 6H), 4.14 (br s, 1H), 4.23 (br dd, J = 7.41, 2.36 Hz, 1H), 4.29-4.36 (m, 2H), 4.39 (br s, 2H), 4.62-4.70 (m, 1H), 5.18-5.24 (m, 1H), 5.30-5.40 (m, 5H), 6.12-6.21 (m, 1H), 6.25 (br d, J = 6.62 Hz, 1H), 6.42 (br s, 1H), 6.48 (br d, J = 8.20 Hz, 1H), 7.15-7.21 (m, 1H), 7.35 (br dd, J = 3.15, 1.89 Hz, 1H), 7.46 (d, J = 6.18 Hz, 1H), 9.03 (br s, 1H).
¹³C-NMR (126 MHz, CDCl₃) δ ppm 12.52, 14.12, 22.68, 26.05, 27.20, 27.22, 29.25, 29.28, 29.32, 29.38, 29.48, 29.52, 29.77, 31.91, 41.02, 68.23, 76.89, 76.90, 105.57, 129.89.
HRMS (ESI): m/z calculated for C₇₂H₁₁₁N₆O₁₆P [M + H]⁺: 1347.7794; found: 1347.7795.

### Synthesis of Compound 73

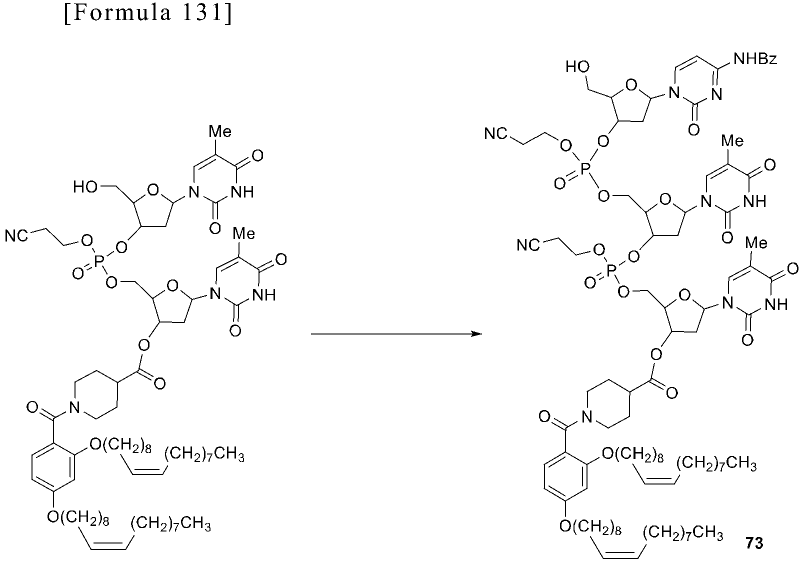

Compound 72 (450 mg, 0.33 mmol) was subjected to azeotropic distillation with ethyl acetate (9.0 mL) three times. Under a nitrogen atmosphere, to Compound 72 were added molecular sieves 3A (450 mg), DMT-dC-CE phosphoramidite (418 mg, 0.501 mmol), and ethyl acetate (4.5 mL), and the mixture was stirred at room temperature for 1 hour. DCI (130 mg, 1.102 mmol) was added, and stirring was continued at room temperature for 1 hour. After confirming completion of the reaction, 0.2 M iodine (ethyl acetate/pyridine/water solution, 5.0 mL) was added to the reaction solution, and the mixture was stirred for 1 hour. A 1 M Na₂S₂O₃ aqueous solution (7.0 mL) was added, and the mixture was stirred. The insoluble matter was removed by filtration while washing with ethyl acetate, and the layers of filtrate were separated. The organic layer was washed with 10% brine (7.0 mL), and then dried over sodium sulfate, and concentrated. The organic layer was concentrated under reduced pressure, and azeotropic distillation with ethyl acetate (9.0 mL) was carried out three times. To the residue were added ethyl acetate (4.5 mL) and mercaptosuccinic acid (251 mg, 1.67 mmol). The obtained mixture was cooled to 0 °C, and trifluoroacetic acid (0.383 mL, 5.01 mmol) was added. The mixture was stirred at the same temperature for 3 hours. After confirming completion of the reaction, saturated sodium hydrogencarbonate aqueous solution (7.5 mL) was added, and the mixture was stirred and the layers were separated. The obtained organic layer was further washed twice with saturated sodium hydrogencarbonate aqueous solution (7.5 mL) and once with 10% brine (7.5 mL). The obtained organic layer was dried over sodium sulfate, concentrated to dryness, and Compound 73 was obtained as a pale yellow solid (560 mg, 94%). The obtained Compound 73 was used in the next step without further purification.
¹H-NMR (500 MHz, CDCl₃) δ ppm 0.85-0.90 (m, 6H), 1.11-1.15 (m, 1H), 1.22-1.35 (m, 18H), 1.36-1.48 (m, 2H), 1.70-1.92 (m, 5H), 1.98-2.04 (m, 4H), 2.40 (bd dd, J = 6.94, 4.41 Hz, 1H), 2.52-2.60 (m, 1H), 2.83 (br s, 2H), 2.86-2.94 (m, 1H), 3.51-3.60 (m, 1H), 3.84-4.00 (m, 3H), 4.06-4.22 (m, 1H), 4.32 (br s, 4H), 4.59-4.67 (m, 1H), 5.22 (br s, 1H), 6.14-6.26 (m, 1H), 6.48 (br d, J = 8.20 Hz, 1H), 7.40-7.61 (m, 2H), 7.93 (br d, J = 7.57 Hz, 1H), 7.93 (br d, J = 7.57 Hz, 1H), 8.34 (br dd, J = 17.18, 8.04 Hz, 1H).
¹³C-NMR (126 MHz, CDCl₃) δ ppm 12.41, 14.12, 19.25, 19.77, 19.77, 19.81, 22.68, 25.92, 26.06, 27.22, 27.22, 29.14, 29.26, 29.31, 29.40, 29.49, 29.52, 29.66, 29.77, 31.91, 40.99, 62.84, 68.25, 105.57, 127.38, 128.02, 128.02, 128.63, 128.83, 129.90, 168.18.
HRMS (ESI): m/z calculated for C₉₁H₁₃₀N₁₀O₂₃P₂: [M + H]⁺ 1793.8786; found, 1793.8833.

### Synthesis of Compound 74

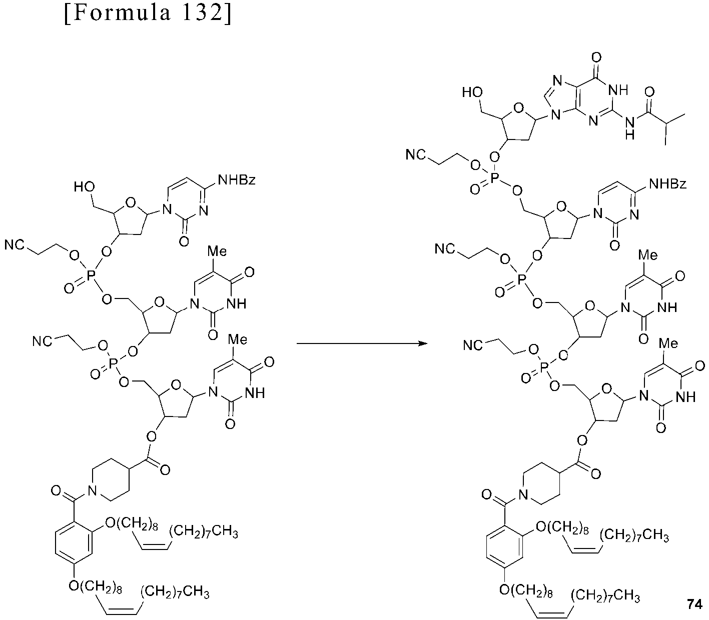

Compound 73 (550 mg, 0.31 mmol) was subjected to azeotropic distillation with ethyl acetate (11.0 mL) three times. Under a nitrogen atmosphere, to Compound 73 were added molecular sieves 3A (550 mg), DMT-dG-CE phosphoramidite (515 mg, 0.613 mmol), and ethyl acetate (6.0 mL), and the mixture was stirred at room temperature for 1 hour. DCI (123 mg, 0.687 mmol) was added, and the reaction mixture was stirred at room temperature for 2 hours. After confirming the completion of the reaction, 0.2 M iodine (ethyl acetate/pyridine/water solution, 4.0 mL) was added to the reaction mixture, and the mixture was stirred for 1 hour. A 1 M Na₂S₂O₃ aqueous solution (11.0 mL) was added, and the mixture was stirred. The insoluble matter was removed by filtration while washing with ethyl acetate, and the layers of filtrate were separated. The organic layer was washed with a 10% brine (11.0 mL) and dried over sodium sulfate, and concentrated. The organic layer was concentrated under reduced pressure and subjected to azeotropic distillation with ethyl acetate (11.0 mL) three times. To the residue were added ethyl acetate (6.0 mL) and mercaptosuccinic acid (230 mg, 1.53 mmol). The resulting mixture was cooled to 0 °C, followed by the addition of trifluoroacetic acid (0.235 mL, 3.07 mmol). The mixture was then warmed to room temperature and stirred for 2 hours. After confirming the completion of the reaction, saturated sodium hydrogencarbonate aqueous solution (9.0 mL) was added, and the mixture was stirred and the layers were separated. The obtained organic layer was further washed twice with saturated sodium hydrogencarbonate aqueous solution (9.0 mL) and once with saturated brine (9.0 mL). The organic layer was dried over sodium sulfate, concentrated to dryness, and Compound 74 was obtained as a pale yellow solid (500 mg, 73%). The obtained Compound 74 was used in the next step without further purification.
¹H-NMR (500 MHz, CDCl₃) δ ppm 0.84-0.90 (m, 6H), 1.16-1.24 (m, 5H), 1.24-1.33 (m, 21H), 1.34-1.47 (m, 6H), 1.98-2.04 (m, 5H), 2.08-2.26 (m, 3H), 2.33-2.41 (m, 1H), 2.49-2.61 (m, 1H), 2.73-2.96 (m, 5H), 3.50-3.62 (m, 1H), 3.74-3.84 (m, 1H), 3.88-3.95 (m, 2H), 3.98 (br s, 1H), 4.09-4.19 (m, 1H), 4.24 (br d, J = 5.36 Hz, 1H), 4.32 (br s, 3H), 4.41-4.51 (m, 1H), 4.62 (br dd, J = 3.31, 2.05 Hz, 1H), 5.15-5.30 (m, 1H), 5.30-5.39 (m, 3H), 6.12-6.26 (m, 1H), 6.42 (s, 1H), 6.47 (br d, J = 8.20 Hz, 1H), 7.40-7.58 (m, 2H), 7.89-7.94 (m, 1H).
¹³C-NMR (126 MHz, CDCl₃) δ ppm 14.12, 22.67, 26.06, 27.22, 29.25, 29.31, 29.41, 29.48, 29.51, 29.76, 30.33, 31.90, 129.76, 129.79, 130.00, 130.00.

### Synthesis of Compound 75

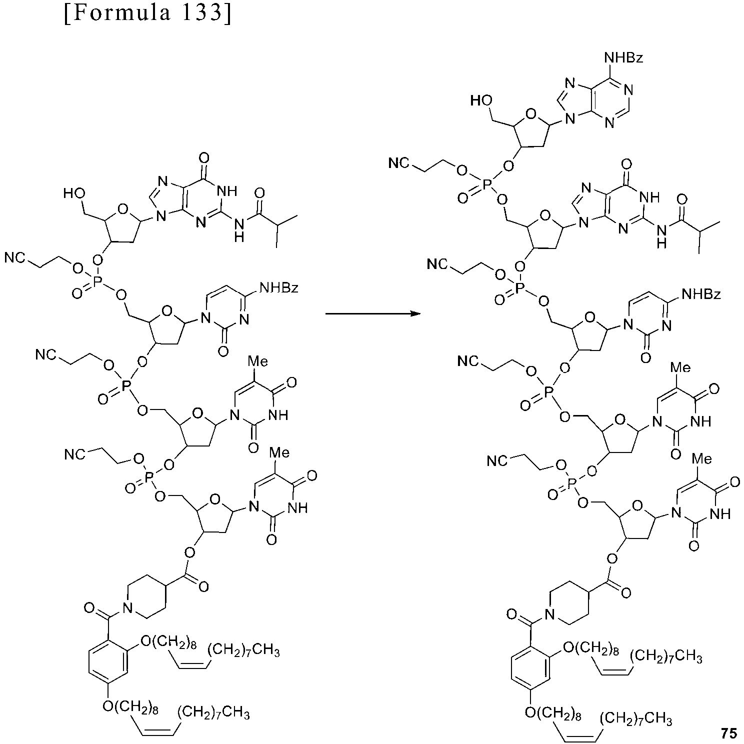

Compound 74 (480 mg, 0.33 mmol) was subjected to azeotropic distillation with ethyl acetate (10.0 mL) three times. Under a nitrogen atmosphere, to Compound 74 were added molecular sieves 3A (480 mg), DMT-dA-CE phosphoramidite (413 mg, 0.481 mmol), ethyl acetate (5.0 mL), and THF (5.0 mL), and the mixture was stirred at room temperature for 1 hour. DCI (94.6 mg, 0.804 mmol) was added, and the mixture was stirred at room temperature for 2 hours. DMT-dA-CE phosphoramidite (138 mg, 0.160 mmol) and DCI (31.5 mg, 0.270 mmol) were added, and the mixture was further stirred at room temperature for 30 hours. DMT-dA-CE phosphoramidite (275 mg, 0.321 mmol) and DCI (31.5 mg, 0.270 mmol) were added, and the mixture was further stirred at room temperature for 60 hours. After the reaction, 0.2 M iodine (ethyl acetate/pyridine/water solution, 4.2 mL) was added to the reaction solution, and the mixture was stirred for 14 hours. A 1 M Na₂S₂O₃ aqueous solution (7.5 mL) and THF (4.5 mL) were added, and the mixture was stirred. After insoluble matter was removed by filtration while washing with ethyl acetate, the layers of the filtrate were separated. The organic layer was washed with a 10% brine (7.5 mL), dried over sodium sulfate, and concentrated. After the organic layer was concentrated under reduced pressure, azeotropic distillation with ethyl acetate (10.0 mL) was performed three times. To the residue were added ethyl acetate (10.0 mL) and mercaptosuccinic acid (321 mg, 2.14 mmol). The obtained mixture was cooled to 0°C, and trifluoroacetic acid (0.654 mL, 8.54 mmol) was added. The mixture was stirred at the same temperature for 2 hours. After confirming completion of the reaction, saturated sodium hydrogencarbonate aqueous solution (7.5 mL) and THF (4.5 mL) were added, the mixture was stirred, and the layers were separated. The obtained organic layer was further washed twice with saturated sodium hydrogencarbonate aqueous solution (7.5 mL) and once with a 10% brine (7.5 mL). The obtained organic layer was dried over sodium sulfate, concentrated to dryness, and Compound 75 was obtained as a pale yellow solid (720 mg, 99%). The obtained Compound 75 was used in the next step without further purification.
¹H-NMR (500 MHz, CDCl₃) δ ppm 0.86 (br d, J = 6.62 Hz, 12H), 1.18-1.40 (m, 78H), 1.43 (s, 14H), 1.78-1.93 (m, 6H), 2.01 (br s, 12H), 2.27 (s, 2H), 2.56-2.63 (m, 3H), 2.70-2.93 (m, 12H), 3.69-3.87 (m, 6H), 3.87-3.93 (m, 5H), 4.30-4.45 (m, 8H), 4.46-4.68 (m, 4H), 5.28 (br s, 1H), 5.35 (br s, 6H), 6.98 (s, 1H), 7.12-7.25 (m, 2H), 7.39-7.54 (m, 6H), 7.56 (br s, 4H), 7.64-7.68 (m, 2H), 7.74-7.89 (m, 2H), 7.91-7.97 (m, 1H), 8.02 (br d, J = 6.94 Hz, 5H), 8.09 (br d, J = 8.83 Hz, 1H), 8.40 (br s, 2H), 8.78 (br s, 2H).
¹³C-NMR (126 MHz, CDCl₃) δ ppm 14.12, 19.19, 21.19, 22.67, 26.08, 27.22, 27.22, 29.26, 29.31, 29.43, 29.51, 29.77, 30.33, 31.90, 34.24, 55.26, 67.98, 113.18, 113.57, 125.52, 127.78, 127.85, 128.26, 129.15, 129.75, 129.79, 129.99, 132.58, 135.80, 151.52.
HRMS (ESI): m/z calculated for C₁₂₈H₁₇₀N₂₂O₃₆P₄: [M + 2H]²⁺ 1358.5628; found, 1358.5629.

### Synthesis of Compound 76

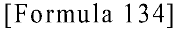

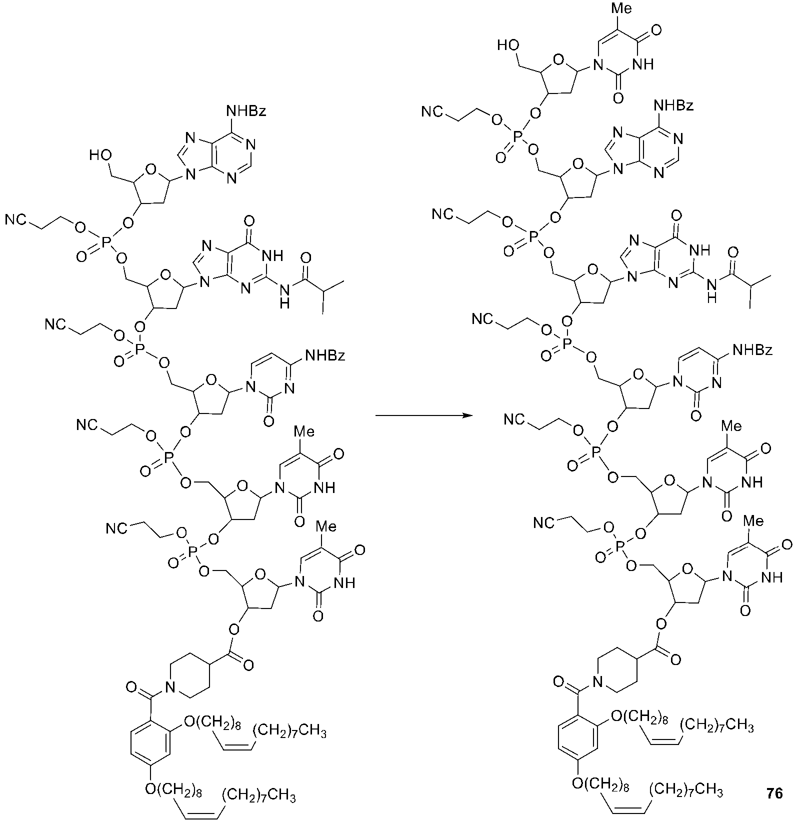

Compound 75 (560 mg, 0.206 mmol) was subjected to azeotropic distillation with ethyl acetate (11.0 mL) three times. Under a nitrogen atmosphere, to Compound 75 were added molecular sieves 3A (560 mg), DMT-dT-CE phosphoramidite (230 mg, 0.309 mmol), ethyl acetate (11.0 mL), and THF (9.0 mL), and the mixture was stirred at room temperature for 1 hour. DCI (73.0 mg, 0.618 mmol) was added, and the mixture was stirred at room temperature for 6 hours. DMT-dT-CE phosphoramidite (115 mg, 0.155 mmol) and DCI (73.0 mg, 0.618 mmol) were added, and the mixture was further stirred at room temperature for 18 hours. DMT-dT-CE phosphoramidite (115 mg, 0.155 mmol) and DCI (73.0 mg, 0.618 mmol) were added, and the mixture was further stirred at room temperature for 7 hours. DMT-dT-CE phosphoramidite (76.7 mg, 0.103 mmol) and DCI (110 mg, 0.927 mmol) were added, and the mixture was further stirred at room temperature for 90 hours. After the reaction, 0.2 M iodine (ethyl acetate/pyridine/water solution, 4.1 mL) was added to the reaction mixture, followed by stirring for 2 hours. 1 M Na₂S₂O₃ aqueous solution (7.5 mL) and THF (5.6 mL) were added, and the mixture was stirred. After insoluble materials were removed by filtration while washing with ethyl acetate, the layers of filtrate were separated. The organic layer was washed with saturated brine (7.5 mL), dried over sodium sulfate. The organic layer was concentrated under reduced pressure, and subjected to subjected to azeotropic distillation with ethyl acetate (11.0 mL) three times. To the residue were added ethyl acetate (11.0 mL), THF (5.5 mL), and mercaptosuccinic acid (309 mg, 2.06 mmol). The resulting mixture was cooled to 0°C, and trifluoroacetic acid (0.315 mL, 4.12 mmol) was added. The mixture was stirred at the same temperature for 3 hours. After confirming the completion of the reaction, saturated sodium hydrogencarbonate aqueous solution (7.5 mL) was added, and the mixture was stirred and the layers were separated. The obtained organic layer was further washed twice with saturated sodium hydrogencarbonate aqueous solution (8.0 mL) and once with 10% brine (8.0 mL). The organic layer was dried over sodium sulfate, concentrated and dried to give Compound 76 as a pale yellow solid (500 mg, 79%). ¹H-NMR (500 MHz, CDCl₃) δ ppm 0.86 (br d, J = 6.94 Hz, 6H), 1.19-1.38 (m, 40H), 1.41 (dt, J = 4.10, 2.05 Hz, 4H), 1.43 (s, 8H), 2.01 (br s, 4H), 2.27 (s, 4H), 2.48-2.70 (m, 12H), 2.75-2.83 (m, 4H), 3.80 (s, 4H), 3.83-3.99 (m, 1H), 4.09-4.14 (m, 1H), 5.32-5.36 (m, 1H), 6.36-6.44 (m, 1H), 6.68-6.85 (m, 1H), 6.93-6.98 (m, 1H), 7.09-7.32 (m, 4H), 7.34-7.57 (m, 1H).
¹³C-NMR (126 MHz, CDCl₃) δ ppm 14.12, 21.19, 22.68, 23.86, 26.08, 27.22, 27.22, 29.26, 29.31, 29.43, 29.52, 29.70, 29.77, 30.34, 31.90, 34.07, 34.24, 55.26, 70.65, 81.45, 113.18, 113.57, 125.52, 127.08, 127.78, 127.85, 128.19, 128.26, 129.15, 129.77, 129.80, 130.00, 135.80, 139.49, 147.35, 158.66.
HRMS (ESI): m/z calculated for C₁₄₁H₁₈₆N₂₅O₄₃P₅: [M + 2H]²⁺: 1537.0990; found, 1537.0990.

### Synthesis of Compound 77

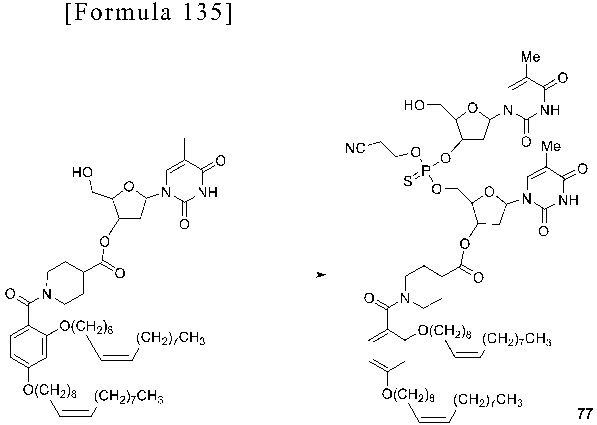

Compound 76 (500 mg, 0.51 mmol) was subjected to azeotropic distillation with ethyl acetate (5 mL) three times. To the obtained Compound 76 were added molecular sieves 3A (500 mg), DMT-dT-CE phosphoramidite (564 mg, 0.757 mmol) and ethyl acetate (5 mL), followed by stirring at room temperature for 1 hour. To the reaction solution was added DCI (197 mg, 1.67 mmol), followed by stirring at room temperature for 18 hours. After confirming the completion of the reaction, ((dimethylamino-methylidene)amino-3H-1,2,4-dithiazaoline)-3-thione (135 mg, 0.656 mmol) was added, followed by stirring at room temperature for 24 hours. After confirming the completion of the reaction, 2-propanol (18.2 mg, 0.303 mmol) was added, followed by stirring at the same temperature for 30 minutes. Saturated sodium hydrogencarbonate aqueous solution (5 mL) was added, and after insoluble materials were removed by filtration while washing with ethyl acetate, the layers were separated. The organic layer was washed with saturated brine (5.0 mL), dried over sodium sulfate, concentrated under reduced pressure, and subjected to azeotropic distillation with ethyl acetate (5 mL) three times. The residue was dissolved in ethyl acetate (5 mL), and mercaptosuccinic acid (379 mg, 2.52 mmol) was added, followed by cooling to 0°C. Trifluoroacetic acid (0.39 mL, 5.05 mmol) was added, and after warming to room temperature, the mixture was stirred for 7 hours. After confirming the completion of the reaction, saturated sodium hydrogencarbonate aqueous solution (7.5 mL) was added, and the aqueous layer was separated. The organic layer was further washed three times with saturated sodium hydrogencarbonate aqueous solution (7.5 mL) and once with 10% NaCl aqueous solution (7.5 mL). The organic layer was dried over sodium sulfate, concentrated to dryness under reduced pressure to give Compound 77 as a pale yellow solid (590 mg, yield: 86%). The obtained Compound 77 was used in the next step without further purification.
¹H-NMR (500 MHz, CDCl₃) δ ppm 0.88 (t, J = 6.94 Hz, 6H), 1.24-1.47 (m, 45H), 1.50-1.57 (m, 1H), 1.71-1.84 (m, 9H), 1.88-1.95 (m, 4H), 2.01 (br s, 8H), 2.30-2.54 (m, 4H), 2.55-2.63 (m, 1H), 2.75-2.86 (m, 3H), 2.88-2.98 (m, 1H), 3.01-3.12 (m, 1H), 3.54-3.60 (m, 1H), 3.84-3.97 (m, 6H), 4.15 (br s, H), 4.24 (br s, 1H), 4.26-4.37 (m, 3H), 4.41 (br s, 1H), 4.65 (br s, H), 5.20-5.27 (m, H), 5.29-5.39 (m, 5H), 6.14-6.20 (m, 1H), 6.29 (br s, 1H), 6.42 (br s, 1H), 6.48 (br d, J = 8.20 Hz, 1H), 7.19 (br d, J = 8.20 Hz, 1H), 7.35 (br d, J = 7.57 Hz, 1H), 8.99 (br s, 1H).
¹³C-NMR (126 MHz, CDCl₃) δ ppm 12.54, 14.11, 22.67, 26.05, 27.21, 27.21, 9.24, 29.28, 29.31, 29.38, 29.47, 29.52, 29.77, 31.90, 40.93, 41.00, 62.14, 62.79, 62.96, 68.23, 86.66, 105.56, 111.44, 111.83, 129.89, 136.59, 150.41, 156.12, 163.52, 63.60, 168.13.
HRMS (ESI): m/z calculated for C₇₂H₁₁₁N₆O₁₅PS: [M + H]⁺: 1363.7566; found, 1363.7658.

### Synthesis of Compound 78

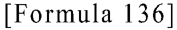

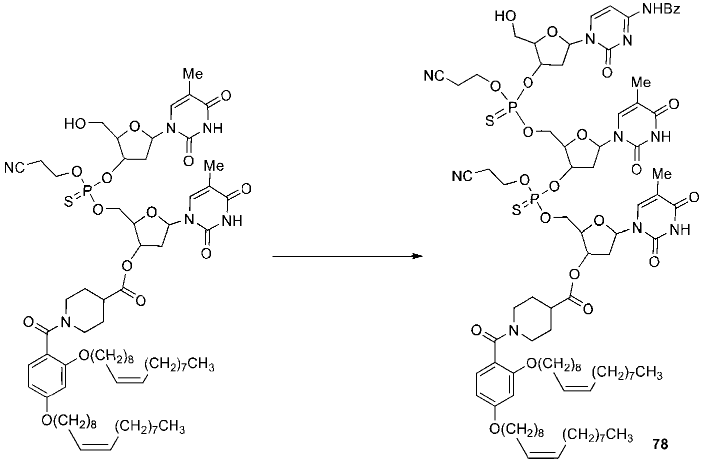

Compound 77 (580 mg, 0.430 mmol) was subjected to azeotropic distillation with ethyl acetate (5.8 mL) three times. To the obtained compound 77 were added molecular sieves 3A (580 mg), DMT-dC-CE phosphoramidite (709 mg, 0.851 mmol) and ethyl acetate (5.8 mL), followed by stirring at room temperature for 1 hour. DCI (200 mg, 1.70 mmol) was added to the reaction solution, followed by stirring at room temperature for 18 hours. ((Dimethylamino-methylidene)amino-3H-1,2,4-dithiazaoline)-3-thione (114 mg, 0.553 mmol) was added, followed by stirring at room temperature for 1 hour. After confirming the completion of the reaction, 2-propanol (18 mg, 0.255 mmol) was added, followed by stirring at the same temperature for 30 minutes. Saturated sodium hydrogencarbonate aqueous solution (5.8 mL) was added and stirred. The insoluble matter was removed by filtration while washing with ethyl acetate, and the layers were separated. The organic layer was washed with saturated brine (5.8 mL) and then dried over sodium sulfate. After concentration of the organic layer under reduced pressure, it was subjected to azeotropic distillation with ethyl acetate (5.8 mL) three times. To the obtained residue were added ethyl acetate (5.8 mL) to dissolve it and mercaptosuccinic acid (319 mg, 2.13 mmol), and cooled to 0°C. Trifluoroacetic acid (0.33 mL, 4.25 mmol) was added, the mixture was warmed to room temperature, and stirred for 2 hours. After confirming the completion of the reaction, saturated sodium hydrogencarbonate aqueous solution (5.8 mL) was added, and the organic layer was separated. The obtained organic layer was further washed three times with saturated sodium hydrogencarbonate aqueous solution (5.8 mL) and once with saturated brine (5.8 mL). The organic layer was dried over sodium sulfate, concentrated to dryness under reduced pressure, and compound 78 was obtained as a pale yellow solid (700 mg, yield 90%). The obtained compound 78 was used in the next step without further purification.
¹H-NMR (500 MHz, CDCl₃) δ ppm 0.88 (br t, J = 6.46 Hz, 6H), 1.23-1.44 (m, 50H), 1.72-1.81 (m, 8H), 1.83-1.97 (m, 12H), 1.98-2.04 (m, 8H), 2.32-2.54 (m, 4H), 2.54-2.62 (m, 2H), 2.68-2.84 (m, 6H), 2.84-2.97 (m, 3H), 3.47-3.64 (m, 1H), 3.73-3.82 (m, 4H), 3.85-4.01 (m, 6H), 4.13-4.19 (m, 1H), 4.19-4.26 (m, 1H), 4.29-4.48 (m, 8H), 4.63 (br d, J = 3.78 Hz, 1H), 5.17 (br s, 1H), 5.19-5.24 (m, 1H), 5.31-5.38 (m, 4H), 6.15-6.18 (m, 1H), 6.19-6.28 (m, 2H), 6.42 (br s, 1H), 6.48 (br d, J = 8.20 Hz, 1H), 6.79-6.87 (m, 2H), 7.15-7.25 (m, 2H), 7.27-7.45 (m, 4H), 7.45-7.62 (m, 5H), 7.89-7.97 (m, 2H), 8.32 (br dd, J = 7.25, 3.78 Hz, 1H).
¹³C-NMR (126 MHz, CDCl₃) δ ppm 12.48, 14.12, 19.56, 22.68, 26.06, 27.23, 29.40, 29.26, 29.26, 29.31, 29.49, 29.49, 29.52, 29.78, 31.91, 32.61, 55.26, 55.33, 68.25, 113.19, 113.53, 127.78, 127.85, 127.96, 127.98, 128.63, 128.70, 128.86, 129.00, 129.30, 129.90.
HRMS (ESI): m/z calculated for C₉₁H₁₃₀N₁₀O₂₁P₂S₂: [M + H]⁺ : 1825.8329; found, 1825.8406

### Synthesis of compound 79

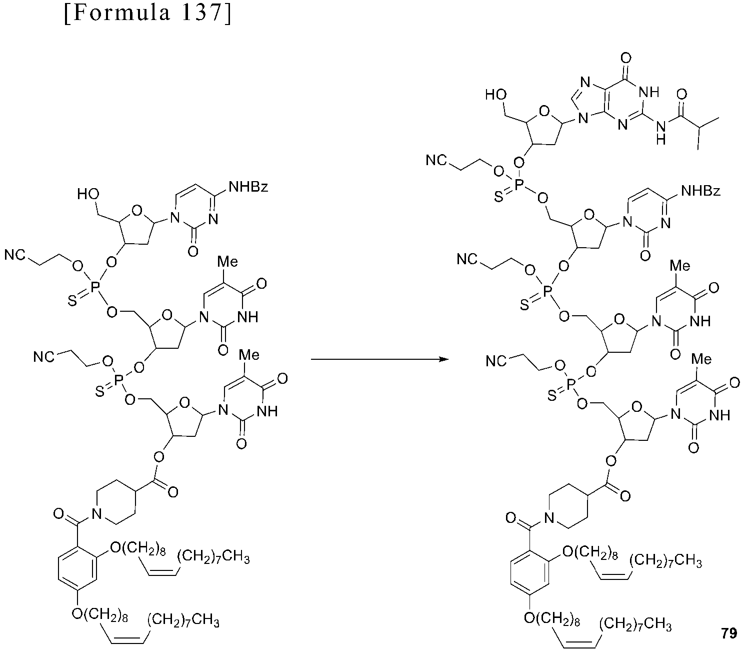

Compound 78 (680 mg, 0.37 mmol) was subjected to azeotropic distillation with ethyl acetate (7 mL) three times. To the obtained compound 78 were added molecular sieves 3A (680 mg), DMT-dG-CE phosphoramidite (626 mg, 0.750 mmol) and ethyl acetate (7 mL), followed by stirring at room temperature for 1 hour. To the reaction solution was added DCI (176 mg, 1.41 mmol), followed by stirring at room temperature for 2 hours. After confirming completion of the reaction, ((dimethylamino-methylidene)amino-3H-1,2,4-dithiazaoline)-3-thione (99.4 mg, 0.48 mmol) was added, followed by stirring at room temperature for 1 hour. After confirming completion of the reaction, 2-propanol (20 mg, 0.22 mmol) was added and the mixture was stirred at the same temperature for 30 minutes. Mercaptosuccinic acid (280 mg, 1.86 mmol) was added, and the mixture was cooled to 0°C. Trifluoroacetic acid (0.29 mL, 3.72 mmol) was added, then the mixture was warmed to room temperature and stirred for 15 hours. After confirming completion of the reaction, saturated sodium hydrogencarbonate aqueous solution (6.8 mL) was added, and the organic layer was separated. The organic layer was further washed three times with saturated sodium hydrogencarbonate aqueous solution (7.5 mL) and once with saturated NaCl aqueous solution (6.8 mL). The obtained organic layer was concentrated to dryness, and compound 79 was obtained as an amorphous solid (830 mg, yield: 97%). The obtained compound 79 was used in the next step without further purification. ¹H-NMR (500 MHz, CDCl₃) δ ppm 0.84-0.91 (m, 6H), 1.17-1.35 (m, 40H), 1.37-1.46 (m, 2H), 1.71-1.82 (m, 5H), 1.88-1.98 (m, 8H), 1.98-2.03 (m, 6H), 2.06-2.14 (m, 1H), 2.32-2.53 (m, 3H), 2.55-2.62 (m, 1H), 2.70-2.80 (m, 2H), 2.81-2.90 (m, 5H), 2.95-3.01 (m, 1H), 3.25 (d, J = 14.50 Hz, 1H), 3.75-3.96 (m, 7H), 4.14-4.26 (m, 1H), 4.34 (br s, 5H), 4.39-4.44 (m, 1H), 4.46-4.54 (m, 1H), 4.62 (br dd, J = 7.09, 5.20 Hz, 1H), 5.12-5.31 (m, 2H), 5.31-5.39 (m, 3H), 6.12-6.28 (m, 2H), 6.82-6.87 (m, 1H), 7.15-7.20 (m, 1H), 7.28-7.49 (m, 4H), 7.51-7.58 (m, 1H), 7.70-7.89 (m, 2H), 8.02 (br s, 1H).
¹³C-NMR (126 MHz, CDCl₃) δ ppm 14.12, 19.62, 22.68, 26.06, 27.23, 29.18, 29.26, 29.31, 29.41, 29.49, 29.52, 29.67, 29.77, 31.91, 32.61, 55.33, 76.58, 113.53, 127.38, 127.96, 127.98, 128.26, 128.64, 129.30, 129.90.
HRMS (ESI): m/z calculated for C₁₀₈H₁₅1N₁₆O₂₇P₃S₃: [M + H]⁺ : 2293.9310; found, 2293.9310.

### Synthesis of Compound 80

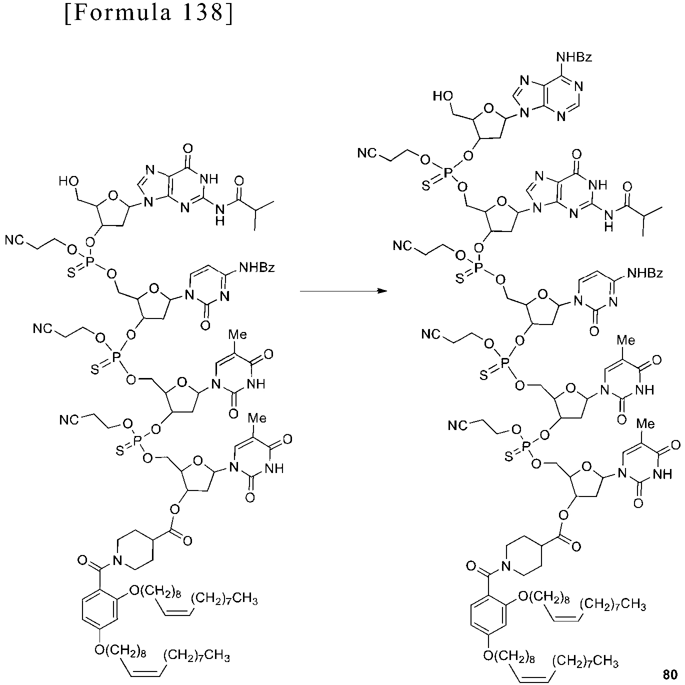

Compound 79 (800 mg, 0.35 mmol) was subjected to azeotropic distillation with ethyl acetate (8 mL) three times. To the obtained compound 79 were added molecular sieves 3A (800 mg), DMT-dA-CE phosphoramidite (598 mg, 0.70 mmol) and ethyl acetate (8 mL), followed by stirring at room temperature for 1 hour. To the reaction solution was added DCI (165 mg, 1.40 mmol), followed by stirring at room temperature for 2 hours. DMT-dA-CE phosphoramidite (150 mg, 0.18 mmol) and DCI (41 mg, 0.35 mmol) were added, followed by stirring for an additional 2 hours. After confirming completion of the reaction, ((dimethylamino-methylidene)amino-3H-1,2,4-dithiazaoline)-3-thione (93.1 mg, 0.45 mmol) was added, followed by stirring at room temperature for 1 hour. After confirming completion of the reaction, 2-propanol (18 mg, 0.21 mmol) was added and the mixture was stirred at the same temperature for 30 minutes. Saturated sodium hydrogencarbonate aqueous solution (8.0 mL) was added and stirred. After removing the insoluble matter by filtration while washing with ethyl acetate, the layers of filtrate were separated. The organic layer was further washed three times with saturated sodium hydrogencarbonate aqueous solution (8.0 mL) and once with saturated brine (8.0 mL), then dried over sodium sulfate. The organic layer was concentrated under reduced pressure. The residue was subjected to azeotropic distillation with ethyl acetate (8 mL) three times. The residue was dissolved in ethyl acetate (7.0 mL), and mercaptosuccinic acid (170 mg, 1.14 mmol) was added, then cooled to 0°C. Trifluoroacetic acid (0.17 mL, 2.27 mmol) was added, followed by stirring at the same temperature for 2 hours. After confirming completion of the reaction, saturated sodium hydrogencarbonate aqueous solution (7.0 mL) and THF (3.5 mL) were added, and the organic layer was separated. The obtained organic layer was further washed twice with saturated sodium hydrogencarbonate aqueous solution (5.8 mL) and once with saturated NaCl aqueous solution (7.0 mL). The organic layer was dried over sodium sulfate, then concentrated to dryness under reduced pressure. The obtained crude product was purified by silica gel column chromatography (eluent: ethyl acetate/methanol) to give compound 80 as a pale yellow solid (480 mg, yield: 50%).
¹H-NMR (500 MHz, CDCl₃) δ ppm 0.87 (br t, J = 6.46 Hz, 6H), 1.10-1.33 (m, 54H), 1.34-1.46 (m, 6H), 1.70-1.80 (m, 2H), 1.81-1.99 (m, 6H), 1.99-2.04 (m, 3H), 2.06 (s, 1H), 2.07-2.15 (m, 1H), 2.22-2.36 (m, 1H), 2.36-2.42 (m, 1H), 2.51-2.66 (m, 1H), 2.73-2.96 (m, 4H), 3.75-3.84 (m, 1H), 3.86-3.96 (m, 2H), 4.11-4.20 (m, 1H), 4.23 (br s, 1H), 4.26-4.47 (m, 6H), 4.49-4.60 (m, 1H), 4.60-4.67 (m, 1H), 5.11-5.30 (m, 1H), 5.31-5.39 (m, 2H), 6.05-6.26 (m, 1H), 6.42 (br s, 1H), 6.47 (br d, J = 8.51 Hz, 1H), 7.27-7.45 (m, 2H), 7.46-7.64 (m, 2H), 7.86 (br d, J = 5.99 Hz, 1H), 8.04 (br d, J = 7.25 Hz, 1H), 9.62-9.74 (m, 1H).
¹³C-NMR (126 MHz, CDCl₃) δ ppm 14.12, 19.63, 21.19, 22.68, 26.06, 27.22, 27.22, 29.13, 29.13, 29.26, 29.26, 29.31, 29.40, 29.48, 29.52, 29.66, 29.77, 30.33, 31.91, 34.23, 68.25, 113.19, 125.52, 127.85, 128.27, 128.72, 129.15, 129.90, 135.81.
HRMS (ESI): m/z calculated for C₁₂₈H₁₇₀N₂₂O₃₂P₄S₄: [M + 2H]²⁺ : 1390.5171; found, 1390.5170.

### Synthesis of Compound 81

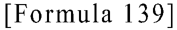

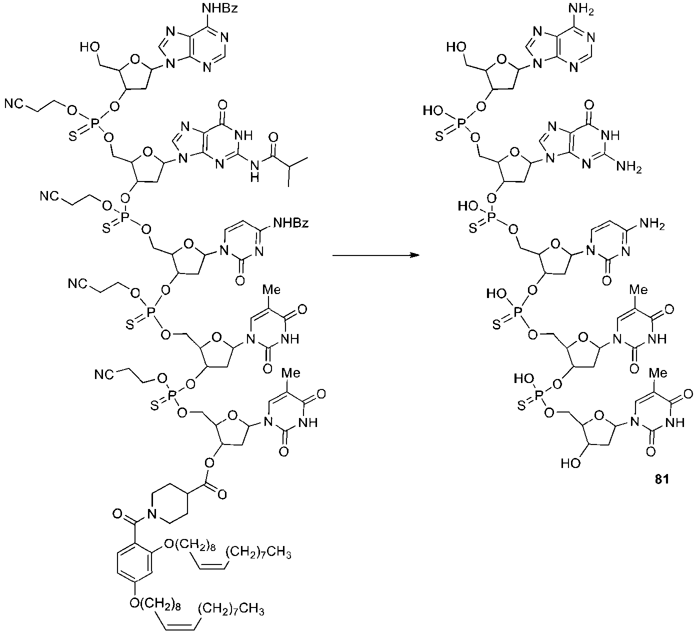

Under a nitrogen atmosphere, to a solution of compound 80 (50 mg, 0.02 mmol) in anhydrous THF (0.2 mL) was added 7 M ammonia in methanol (0.2 mL) at room temperature. The mixture was stirred at 50°C for 48 hours. After the reaction, the reaction solution was concentrated to dryness under reduced pressure. MTBE was added to the residue to form a suspension. The suspension was filtered, washed with MTBE, and compound 81 (26 mg) was obtained as a pale yellow solid.

### Synthesis of Compound 82

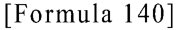

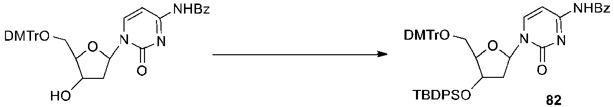

Under a nitrogen atmosphere, deoxycytidine (1.50 g, 2.37 mmol) and MeCN (12 mL) were charged. Imidazole (0.81 g, 11.84 mmol) and TBDPSCl (1.30 g, 4.73 mmol) were mixed at room temperature and stirred for 2 hours. After completion of the reaction, the mixture was cooled with ice to 0°C, followed by addition of saturated sodium hydrogencarbonate aqueous solution (15 mL) and filtration while washing with MTBE (30 mL). After the layers of the filtrate were separated, the obtained organic layer was dried over sodium sulfate. The organic layer was concentrated under reduced pressure to give a crude product, which was purified by silica gel column chromatography (n-hexane/ethyl acetate) to give compound 82 as an oily material (2.04 g, yield: 100%).
¹H-NMR (500 MHz, CDCl₃) δ ppm 1.02 (s, 9H), 1.99-2.05 (m, 1H), 2.67-2.74 (m, 1H), 3.08 (dd, J = 10.56, 3.63 Hz, 1H), 3.29 (dd, J = 10.56, 2.68 Hz, 1H), 3.79 (dd, J = 2.21 Hz, 6H), 4.10-4.18 (m, 1H), 4.47-4.51 (m, 1H), 6.36 (t, J = 5.83 Hz, 1H), 6.75-6.79 (m, 4H), 7.12 (dd, J = 8.83, 1.26 Hz, 5H), 7.21 (m, 1H), 7.30 (t, J = 7.43 Hz, 2H), 7.34-7.38 (m, 2H), 7.40-7.45 (m, 2H), 7.51 (t, J = 7.49 Hz, 2H), 7.55-7.62 (m, 5H), 7.82-7.90 (m, 2H), 8.14 (br d, J = 6.94 Hz, 1H), 8.59 (br s, 1H).
¹³C-NMR (126 MHz, CDCl₃) δ ppm 19.03, 26.83, 42.24, 55.23, 62.50, 72.64, 86.82, 86.95, 113.21, 127.04, 127.87, 127.92, 128.16, 129.03, 129.97, 130.03, 130.06, 132.93, 133.02, 133.10, 135.18, 135.32, 135.70, 144.03, 158.61.

### Synthesis of Compound 83

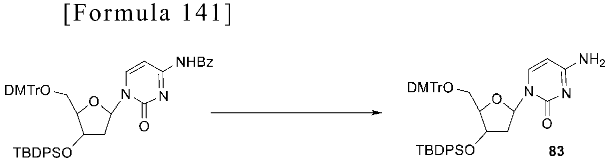

Under a nitrogen atmosphere, 7 M ammonia in methanol (18 mL) was added to a solution of compound 82 (1.80 g, 2.06 mmol) in anhydrous THF (18 mL) at room temperature, followed by stirring at 50°C for 15 hours. After confirming completion of the reaction, the mixture was concentrated under reduced pressure to give a crude product, which was purified by silica gel column chromatography (n-hexane/ethyl acetate) to give compound 83 as a white solid (1.60 g, yield: 100%). ¹H-NMR (500 MHz, CDCl₃) δ ppm 1.00 (s, 9H), 1.26 (t, J = 7.09 Hz, 1H), 1.85 (s, 1H), 1.96 (dt, J = 13.24, 5.99 Hz, 1H), 2.04 (s, 1H), 2.53-2.59 (m, 1H), 3.04 (dd, J = 10.56, 3.31 Hz, 1H), 3.28 (dd, J = 10.40, 2.84 Hz, 1H), 3.78 (s, 6H), 4.06-4.14 (m, 1H), 4.45-4.49 (m, 1H), 5.19 (d, J = 7.25 Hz, 1H), 6.36 (t, J = 5.99 Hz, 1H), 6.71-6.76 (m, 4H), 7.09-7.13 (m, 34H), 7.18-7.22 (m, 5H), 7.25-7.30 (m, 3H), 7.32-7.36 (m, 2H), 7.37-7.45 (m, 2H), 7.53-7.59 (m, 4H), 7.81 (d, J = 7.57 Hz, 1H). ¹³C-NMR (126 MHz, CDCl₃) δ ppm 19.03, 26.82, 42.08, 55.24, 62.48, 72.51, 86.23, 86.29, 86.62, 93.27, 113.12, 126.90, 127.79, 127.82, 128.19, 129.87, 129.94, 130.06, 130.11, 133.05, 133.12, 135.34, 135.41, 135.70, 141.73, 144.32, 155.70, 158.52, 165.30.

### Synthesis of Compound 84

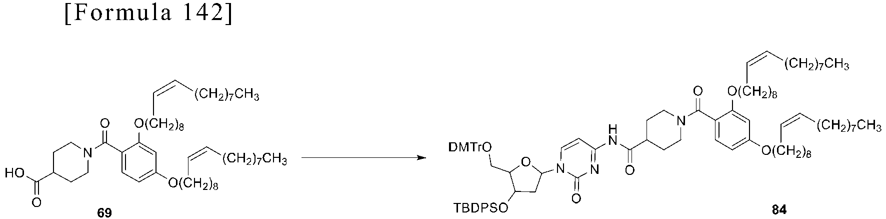

Under a nitrogen atmosphere, to a solution of compound 69 (551 mg, 0.72 mmol), compound 83 (500 mg, 1.31 mmol), 1-hydroxybenzotriazole monohydrate (100 mg, 0.65 mmol) and DIPEA (337 mg, 2.61 mmol) in DMF (5 mL), 1-(3-dimethylaminopropyl)-3-ethylcarbodiimide hydrochloride [EDCI] (250 mg, 1.31 mmol) was added at room temperature, followed by stirring at room temperature for 24 hours. After confirming completion of the reaction, the mixture was cooled to 0°C, and ethyl acetate (10 mL), n-hexane (10 mL) and water (10 mL) were added and stirred, after which the aqueous layer was separated. The obtained organic layer was successively washed with saturated sodium hydrogencarbonate solution (5 mL) and water (43 mL), followed by dried over sodium sulfate. The organic layer was concentrated under reduced pressure to give a crude product, which was purified by silica gel column chromatography (n-hexane/ethyl acetate) to give compound 84 as a solid (940 mg, yield: 95%).
¹H-NMR (500 MHz, CDCl₃) δ ppm 0.88 (bd t, J = 6.94 Hz, 6H), 1.01 (s, 9H), 1.24-1.34 (m, 37H), 1.42-1.46 (m, 2H), 1.70-1.82 (m, 7H), 1.92-2.05 (m, 10H), 2.49-2.58 (m, 1H), 2.63-2.70 (m, 1H), 2.83 (br d, J = 11.03 Hz, 1H), 3.06 (br d, J = 10.40 Hz, 1H), 3.28 (br d, J = 10.40 Hz, 1H), 3.57-3.65 (m, 1H), 3.78 (s, 6H), 3.87-4.00 (m, 4H), 4.16 (br s, 1H), 4.45 (br s, 1H), 4.71-4.80 (m, 1H), 5.31-5.39 (m, 4H), 6.30 (t, J = 5.83 Hz, 1H), 6.42 (br s, 1H), 6.46-6.50 (m, 1H), 6.75 (dd, J = 8.99, 3.00 Hz, 4H), 7.10 (br d, J = 7.88 Hz, 5H), 7.28-7.36 (m, 4H), 7.42 (q, J = 7.46 Hz, 2H), 7.54-7.60 (m, 4H), 8.09 (br d, J = 7.25 Hz, 1H).
¹³C-NMR (126 MHz, CDCl₃) δ ppm 14.12, 19.03, 22.68, 26.05, 26.81, 27.22, 29.24, 29.27, 29.32, 29.38, 29.47, 29.52, 29.77, 31.90, 42.20, 55.21, 113.19, 127.86, 127.86, 127.90, 128.15, 129.80, 130.01, 135.67, 158.59.

### Synthesis of Compound 85

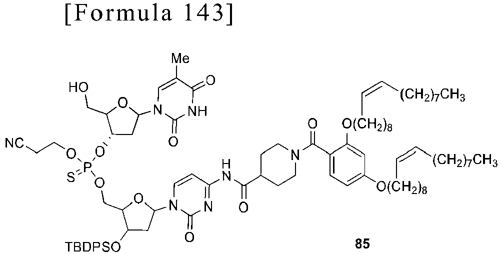

Compound 84 (900 mg, 0.59 mmol) was subjected to azeotropic distillation with ethyl acetate (9 mL) three times. To compound 84, ethyl acetate (9 mL) and mercaptosuccinic acid (446 mg, 2.97 mmol) were added, and cooled to 0°C. Trifluoroacetic acid (0.46 mL, 2.97 mmol) was added, warmed to room temperature, and stirred for 2 hours. After confirming completion of the reaction, saturated brine (9 mL) was added and the aqueous layer was separated. The organic layer was further washed twice with saturated sodium hydrogencarbonate solution (9 mL) and once with saturated brine (9 mL). The obtained organic layer was dried over sodium sulfate. The organic layer was concentrated under reduced pressure, followed by azeotropic distillation with ethyl acetate (9 mL) three times. To the residue were added molecular sieves 3A (900 mg), DMT-dT-CE phosphoramidite (663 mg, 0.890 mmol) and ethyl acetate (9 mL), followed by stirring at room temperature for 1 hour. To the reaction solution, DCI (210 mg, 1.78 mmol) was added and stirred at room temperature for 1 hour. After confirming completion of the reaction, ((dimethylamino-methylidene)amino-3H-1,2,4-dithiazaoline)-3-thione (158 mg, 0.772 mmol) was added and stirred at room temperature for 1 hour. After confirming completion of the reaction, 2-propanol (20 mg, 0.36 mmol) was added and stirred at the same temperature for 30 minutes. Mercaptosuccinic acid (535 mg, 3.56 mmol) was added and cooled to 0°C. Trifluoroacetic acid (0.68 mL, 5.05 mmol) was added, warmed to room temperature, and stirred for 18 hours. After confirming completion of the reaction, saturated sodium hydrogencarbonate aqueous solution (9.0 mL) was added and the aqueous layer was separated. The organic layer was further washed three times with saturated sodium hydrogencarbonate aqueous solution (9.0 mL) and once with saturated NaCl aqueous solution (9.0 mL). The organic layer was dried over sodium sulfate, then concentrated to dryness under reduced pressure to obtain compound 85 as a pale yellow solid (900 mg, yield: 95%). The obtained compound 85 was used in the next step without further purification.
¹H-NMR (500 MHz, CDCl₃) δ ppm 0.87 (t, J = 6.62 Hz, 6H), 1.08 (s, 9H), 1.24-1.34 (m, 40H), 1.35-1.47 (m, 6H), 1.72-1.80 (m, 6H), 2.01 (br s, 8H), 2.19-2.34 (m, 2H), 2.40-2.46 (m, 1H), 2.57-2.78 (m, 2H), 2.79-2.95 (m, 1H), 2.96 (s, 1H), 3.60 (br d, J = 12.61 Hz, 1H), 3.64-3.82 (m, 3H), 3.84-3.95 (m, 3H), 3.98-4.20 (m, 2H), 4.30-4.37 (m, 1H), 5.06-5.15 (m, 1H), 5.31-5.39 (m, 2H), 6.04-6.14 (m, 1H), 6.31 (br s, 1H), 6.46-6.49 (m, 1H), 6.83 (d, J = 8.75 Hz, 1H), 7.12-7.22 (m, 1H), 7.23-7.38 (m, 2H), 7.38-7.50 (m, 4H), 7.60-7.66 (m, 2H), 8.88-9.05 (m, 1H). ¹³C-NMR (126 MHz, CDCl₃) δ ppm 14.12, 19.00, 22.68, 26.05, 26.83, 27.20, 27.23, 29.25, 29.32, 29.39, 29.48, 29.52, 29.77, 31.90, 5.25, 68.20, 113.17, 127.07, 127.95, 128.12, 129.14, 129.80, 129.99, 130.27, 135.72.
HRMS (ESI): m/z calculated for C₈₇H₁₂₈N₇O₁₄PSSi: [M + H]⁺ : 1586.8747; found, 1586.8832.

### Synthesis of Compound 86

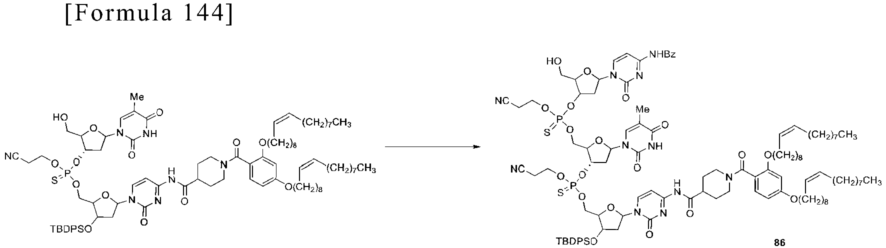

Compound 85 (700 mg, 0.59 mmol) was subjected to azeotropic distillation with ethyl acetate (9 mL) three times. To compound 85 were added molecular sieves 3A (700 mg), DMT-dC-CE phosphoramidite (552 mg, 0.66 mmol) and ethyl acetate (7 mL), followed by stirring at room temperature for 1 hour. To the reaction solution was added DCI (156 mg, 1.32 mmol), followed by stirring at room temperature for 1 hour. After confirming completion of the reaction, ((dimethylamino-methylidene)amino-3H-1,2,4-dithiazaoline)-3-thione (118 mg, 0.57 mmol) was added, followed by stirring at room temperature for 2 hours. After confirming completion of the reaction, 2-propanol (16 mg, 0.26 mmol) was added, followed by stirring at the same temperature for 30 minutes. Mercaptosuccinic acid (331 mg, 2.21 mmol) was added and the mixture was cooled to 0°C. Trifluoroacetic acid (0.34 mL, 4.41 mmol) was added, the mixture was warmed to room temperature, and stirred for 2 hours. After confirming completion of the reaction, saturated sodium hydrogencarbonate solution (7.0 mL) was added, followed by filtration while washing with ethyl acetate, and the aqueous layer was separated. The organic layer was further washed four times with saturated sodium hydrogencarbonate solution (7.0 mL) and once with saturated NaCl aqueous solution (7.0 mL). The organic layer was dried over sodium sulfate, then concentrated to dryness under reduced pressure to obtain compound 86 as a pale yellow solid (930 mg, yield: 99%). The obtained compound 86 was used in the next step without further purification. ¹H-NMR (500 MHz, CDCl₃) δ ppm 0.87 (t, J = 6.46 Hz, 6H), 1.04-1.11 (m, 9H), 1.25-1.34 (m, 40H), 1.38 (br d, J = 6.62 Hz, 1H), 1.43 (s, 2H), 1.76 (br s, 6H), 1.83-1.84 (m, 1H), 1.86-1.93 (m, 4H), 1.95-2.03 (m, 9H), 2.27 (s, 1H), 2.35-2.55 (m, 2H), 2.63-2.86 (m, 8H), 2.96 (s, 1H), 2.96-3.03 (m, 1H), 3.21-3.32 (m, 1H), 3.53-3.66 (m, 1H), 3.72-3.81 (m, 6H), 3.84-4.04 (m, 8H), 4.14-4.36 (m, 7H), 5.10 (br d, J = 5.99 Hz, 1H), 5.32-5.39 (m, 4H), 6.02-6.14 (m, 1H), 6.16-6.33 (m, 2H), 6.79-6.86 (m, 3H), 7.13-7.21 (m, 3H), 7.25-7.34 (m, 7H), 7.35-7.52 (m, 10H), 7.57-7.66 (m, 5H), 7.87-7.97 (m, 3H).
¹³C-NMR (126 MHz, CDCl₃) δ ppm 14.12, 19.00, 22.67, 26.05, 26.83, 27.20, 27.23, 29.25, 29.31, 29.40, 29.48, 29.52, 29.77, 31.90, 55.25, 55.32, 113.18, 113.52, 127.07, 130.27, 135.71, 139.47, 158.64. HRMS (ESI): m/z calculated for C₁₀₆H₁₄₇N₁₁O₂₀P₂S₂Si: [M + H]⁺ : 2048.9510; found, 2048.9567.

### Synthesis of Compound 87

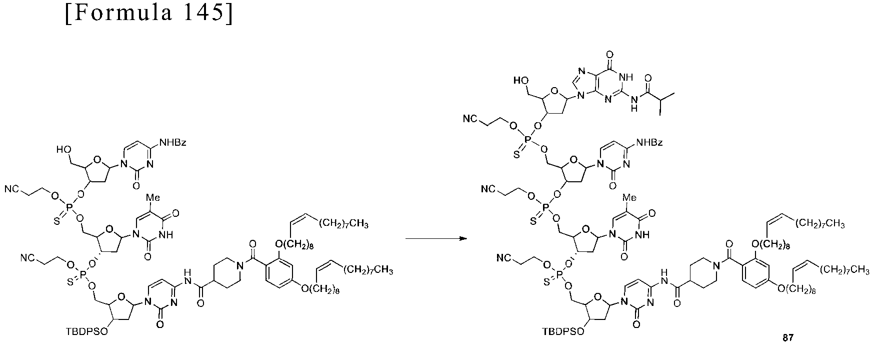

Compound 86 (800 mg, 0.39 mmol) was subjected to azeotropic distillation three times with ethyl acetate (8 mL). To compound 86 were added molecular sieves 3A (800 mg), DMT-dG-CE phosphoramidite (492 mg, 0.59 mmol) and ethyl acetate (8 mL), followed by stirring at room temperature for 1 hour. To the reaction solution was added DCI (138 mg, 1.17 mmol), followed by stirring at room temperature for 2 hours. After confirming completion of the reaction, ((dimethylamino-methylidene)amino-3H-1,2,4-dithiazaoline)-3-thione (104 mg, 0.51 mmol) was added, followed by stirring at room temperature for 1 hour. After confirming completion of the reaction, 2-propanol (16 mg, 0.26 mmol) was added and the mixture was stirred at the same temperature for 30 minutes. To the mixture was added mercaptosuccinic acid (293 mg, 1.95 mmol), and cooled to 0°C. Trifluoroacetic acid (0.30 mL, 3.90 mmol) was added, and the mixture was warmed to room temperature and stirred for 2 hours. After confirming completion of the reaction, saturated sodium hydrogencarbonate aqueous solution (8.0 mL) was added, followed by filtration while washing with ethyl acetate, and the aqueous layer was separated. The obtained organic layer was further washed three times with saturated sodium hydrogencarbonate aqueous solution (8.0 mL) and once with saturated NaCl aqueous solution (8.0 mL). The organic layer was dried over sodium sulfate, concentrated to dryness under reduced pressure, and compound 87 was obtained as a pale yellow solid (790 mg, yield: 72%).
¹H-NMR (500 MHz, CDCl₃) δ ppm 0.85-0.90 (m, 6H), 1.05-1.10 (m, 9H), 1.19-1.38 (m, 57H), 1.73-1.93 (m, 2H), 1.95-2.03 (m, 12H), 2.05 (s, 6H), 2.08 (br d, J = 6.94 Hz, 1H), 2.30-2.52 (m, 6H), 2.67-2.90 (m, 12H), 2.91-3.04 (m, 6H), 3.22-3.32 (m, 1H), 3.73-3.96 (m, 2H), 4.17-4.26 (m, 1H), 4.32 (br s, 1H), 4.37 (br s, 1H), 4.46-4.54 (m, 1H), 5.01-5.18 (m, 1H), 5.32-5.39 (m, 1H), 6.14-6.33 (m, 1H), 6.79-6.87 (m, 1H), 7.15-7.25 (m, 1H), 7.27-7.45 (m, 1H), 7.45-7.50 (m, 1H), 7.55 (br dd, J = 16.39, 7.25 Hz, 1H), 7.63 (br d, J = 6.62 Hz, 1H), 7.75-7.91 (m, 1H). ¹³C-NMR (126 MHz, CDCl₃) δ ppm 14.12, 18.96, 19.01, 21.76, 22.67, 26.05, 26.84, 27.21, 27.22, 27.23, 29.25, 29.31, 29.40, 29.48, 29.51, 29.77, 31.90, 55.32, 77.41, 113.18, 113.52, 127.94, 127.98, 128.06, 128.25, 129.14, 129.28, 129.80, 129.98, 130.27, 135.62, 135.71.

### Synthesis of Compound 88

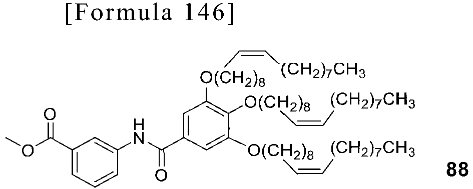

Under a nitrogen atmosphere, 3,4,5-tri-((Z)-octadec-9-enyloxy)benzoic acid (compound 2, 4.26 g, 4.62 mmol), DMF (180 mL), THF (20 mL), methyl 2-aminobenzoate (0.91 g, 6.02 mmol), COMU (2.57 g, 6.00 mmol), and DIPEA (2.58 mL, 15.2 mmol) were mixed and stirred overnight while warming to room temperature. After confirming completion of the reaction, the mixture was cooled to room temperature, and ethyl acetate (20 mL), n-hexane (20 mL) and water (20 mL) were added, followed by separation of the aqueous layer. The obtained organic layer was washed with water (20 mL) and concentrated under reduced pressure to give a crude product. The obtained crude product was purified by silica gel column chromatography (n-hexane/ethyl acetate) to give compound 88 as a colorless oily material (2.55 g, yield: 52%). ¹H-NMR (500 MHz, CDCl₃) δ 8.12-8.04 (m, 2H), 7.85-7.76 (m, 2H), 7.47 (t, J = 8.0 MHz, 1H), 7.05 (s, 2H), 5.39-5.31 (m, 6H), 4.07-3.99 (m, 6H), 3.93 (s, 3H), 2.05-1.98 (m, 12H), 1.88-1.69 (m, 6H), 1.58-1.20 (m, 66H), 0.88 (t, J = 7.0 MHz, 9H).

### Synthesis of Compound 89

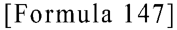

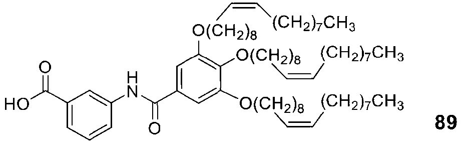

Under a nitrogen atmosphere, compound 88 (2.55 g, 2.45 mmol), potassium hydroxide (0.24 g, 4.28 mmol), THF (12.5 mL), methanol (3.2 mL) and ion-exchanged water (0.6 mL) were mixed and stirred at 60 °C overnight. After confirming completion of the reaction, 6 M HCl (2 mL), water (20 mL) and ethyl acetate (20 mL) were added, and the aqueous layer was separated. The organic layer was washed with water (20 mL) and concentrated under reduced pressure to obtain a crude product. The obtained crude product was purified by silica gel column chromatography (n-hexane/ethyl acetate) to give compound 89 as a white amorphous solid (1.54 g, yield 60%).
¹H-NMR (500 MHz, CDCl₃) δ ppm 8.21-8.13 (m, 2H), 7.93-7.86 (m, 2H), 7.50 (t, J = 8.0 MHz, 1H), 7.09 (s, 2H), 5.39-5.31 (m, 6H), 4.08-4.00 (m, 6H), 2.07-1.96 (m, 12H), 1.88-1.71 (m, 6H), 1.58-1.20 (m, 63H), 0.87 (t, J = 6.8 MHz, 9H).

### Synthesis of compound 90

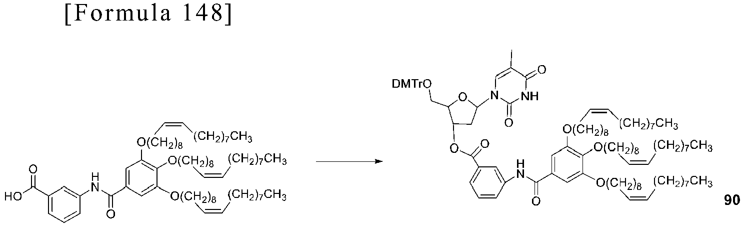

Under a nitrogen atmosphere, to a solution of compound 89 (624 mg, 0.60 mmol), DMT-dT (555 mg, 1.02 mmol) and DMAP (124 mg, 1.02 mmol) in dichloromethane (15 mL) was added EDCI (194 mg, 1.02 mmol) at room temperature, followed by stirring at the same temperature for 30 hours. After confirming completion of the reaction, the solvent was concentrated under reduced pressure to obtain a crude product, which was purified by silica gel column chromatography (n-hexane/ethyl acetate) to give compound 90 as a white amorphous solid (847 mg, 90%). ¹H-NMR (500 MHz, CDCl₃) δ ppm 8.18-8.13 (m, 1H), 8.06-8.00 (m, 2H), 7.91 (s, 1H), 7.85-7.80 (m, 1H), 7.67 (s, 1H), 7.49 (t, J = 8.0 MHz, 1H), 7.44-7.40 (m, 2H), 7.35-7.20 (m, 9H), 7.01 (s, 2H), 6.85 (d, J = 8.0 MHz, 4H), 6.55-6.50 (m, 1H), 5.73-5.70 (m, 1H), 5.40-5.31 (m, 6H), 4.32-4.28 (m, 1H), 4.08-3.99 (m, 6H), 3.80 (s, 6H), 3.60-3.52 (m, 2H), 2.66-2.52 (m, 2H), 2.08-1.95 (m, 12H), 1.88-1.72 (m, 6H), 1.57-1.20 (m, 67H), 0.88 (t, J = 6.8 MHz, 9H).

### Synthesis of compound 91

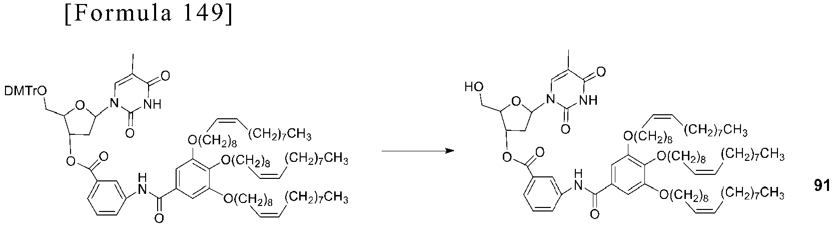

Under a nitrogen atmosphere, to an ethyl acetate (6.0 mL) solution of compound 90 (647 mg, 0.41 mmol) were added mercaptosuccinic acid (308 mg, 2.05 mmol) and trifluoroacetic acid (0.47 mL, 6.15 mmol), followed by stirring under ice cooling for 1.5 hours. After confirming completion of the reaction, the reaction solution was washed three times with a 5% NaHCO₃ aqueous solution (9 mL) and once with a 10% NaCl aqueous solution (9 mL), and concentrated under reduced pressure to obtain compound 91 as a white amorphous solid (415 mg, yield: 80%). The obtained compound 91 was used in the next reaction without further purification.

### Synthesis of compound 92

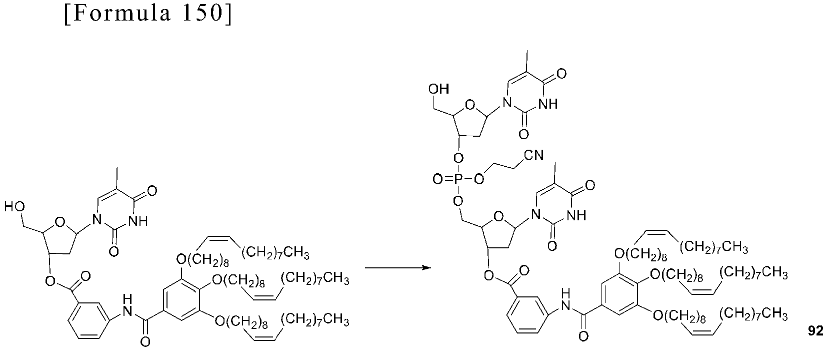

Compound 91 (415 mg, 0.33 mmol) was subjected to azeotropic distillation three times with ethyl acetate (4.0 mL). To the obtained compound 91 were added molecular sieves 3A (400 mg), DMT-dT-CE phosphoramidite (492 mg, 0.66 mmol) and ethyl acetate (4.0 mL), followed by stirring at room temperature for 1 hour. To the reaction solution was added DCI (117 mg, 0.99 mmol), followed by stirring at room temperature for 1 hour. After confirming completion of the reaction, iodine (251 mg, 0.99 mmol) dissolved in acetonitrile/pyridine/water (5.0 mL) was dropwise added to the reaction solution at room temperature, followed by stirring for 1 hour. After confirming completion of the reaction, a 1 M Na₂S₂O₃ aqueous solution (6.0 mL) was added and stirred. The insoluble material was removed by filtration, and the layers were separated. The organic layer was washed with 5% brine (6.0 mL), then dried over sodium sulfate (5 g). After concentration of the organic layer under reduced pressure, azeotropic distillation was performed twice with ethyl acetate (4.0 mL). The residue was dissolved in ethyl acetate (4.0 mL), mercaptosuccinic acid (311 mg, 1.65 mmol) was added, and the mixture was cooled to 0 °C. Trifluoroacetic acid (0.63 mL, 8.25 mmol) was added, followed by stirring at the same temperature for 1 hour, then the mixture was warmed to room temperature and was stirred for 1 hour. After confirming completion of the reaction, water (6 mL) was added and the organic layer was separated. The obtained organic layer was washed three times with a 5% NaHCO₃ aqueous solution (6 mL) and once with a 5% NaCl aqueous solution (6 mL). The obtained organic layer was concentrated to obtain compound 92 as a white amorphous solid (467 mg, yield: 89%). The obtained compound 92 was used in the next reaction without further purification.

### Synthesis of compound 93

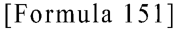

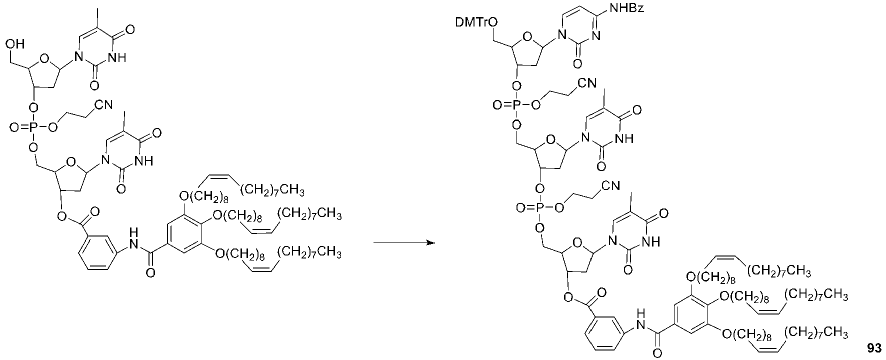

Compound 92 (470 mg, 0.29 mmol) was subjected to azeotropic distillation four times with ethyl acetate (4 mL). Molecular sieves 3A (400 mg), DMT-dC-CE phosphoramidite (484 mg, 0.58 mmol) and ethyl acetate (4 mL) were added and stirred at room temperature for 1 hour. To the reaction solution was added DCI (103 mg, 0.87 mmol), followed by stirring at room temperature for 2 hours. After confirming completion of the reaction, iodine (221 mg, 0.87 mmol) dissolved in acetonitrile/pyridine/water (4.4 mL) was dropwise added to the reaction solution at room temperature, followed by stirring for 1 hour. After confirming completion of the reaction, a 1 M Na₂S₂O₃ aqueous solution (6.0 mL) and ethyl acetate (6.0 mL) were added. The insoluble material was removed by filtration, and the layers were separated. The obtained organic layer was washed with a 5% NaHCO₃ aqueous solution (6.0 mL) and a 5% NaCl aqueous solution (6.0 mL), and then dried over sodium sulfate (4.0 g). After concentration, the obtained crude product was purified by silica gel column chromatography (ethyl acetate/methanol) to give compound 93 as a pale yellow amorphous solid (658 mg, yield: 97%).

### Synthesis of compound 50

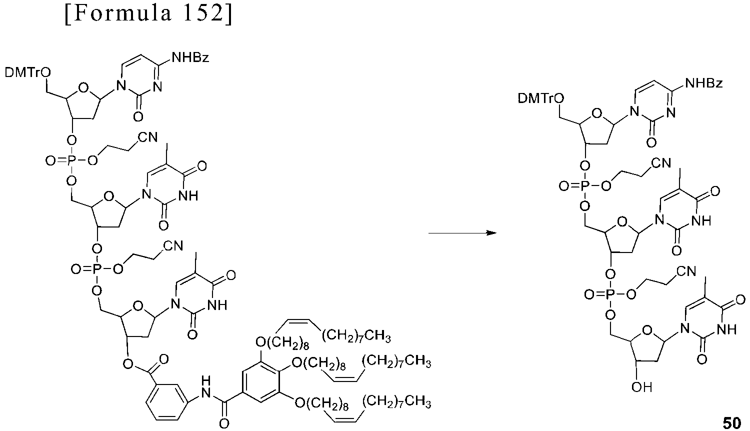

Under a nitrogen atmosphere, at 0 °C, to a solution of compound 93 (658 mg, 0.28 mmol) in anhydrous THF (12 mL) and isopropanol (3.0 mL) was dropwise added a 4 M THF solution of lithium borohydride (0.35 mL, 1.4 mmol). After stirring at 0 °C for 1 hour, a 10% ammonium chloride aqueous solution (5 mL) and ethyl acetate were added, followed by stirring, and the layers were separated to obtain the organic layer. The aqueous layer was extracted with ethyl acetate, and the combined organic layers were concentrated to remove water. The obtained crude product was purified by silica gel column chromatography (ethyl acetate/methanol) to give compound 50 as a white solid.

### Synthesis of compound 94

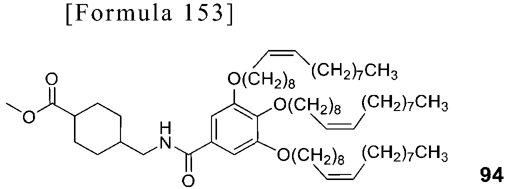

Under a nitrogen atmosphere, 3,4,5-tri-((Z)-octadec-9-enyloxy)benzoic acid (2.73 g, 2.30 mmol), DMF (90 mL), THF (10 mL), methyl 4-(aminomethyl)cyclohexanecarboxylate hydrochloride (0.63 g, 3.00 mmol), COMU (1.28 g, 3.00 mmol), and DIPEA (1.29 mL, 7.60 mmol) were mixed and stirred overnight while heating from room temperature. After confirming completion of the reaction, the mixture was cooled to room temperature, and ethyl acetate (20 mL), n-hexane (20 mL) and water (20 mL) were added, and the aqueous layer was separated. The organic layer was washed with water (20 mL) and concentrated under reduced pressure to obtain a crude product. The obtained crude product was purified by silica gel column chromatography (n-hexane/ ethyl acetate) to give compound 94 as a colorless oily material (2.13 g, yield: 87%).
¹H-NMR (400 MHz, CDCl₃) δ 6.94 (s, 2H), 6.05 (t, J = 6 MHz, 1H), 5.41-5.29 (m, 6H), 3.66 (s, 3H), 3.30 (t, J = 6.4 MHz, 2H), 2.32-2.20 (m, 1H), 2.08-1.94 (m, 14H), 1.93-1.85 (m, 2H), 1.84-1.67 (m, 6H), 1.67-1.52 (m, 1H), 1.53-1.17 (m, 74H), 1.15-0.97 (m, 2H), 0.88 (t, J = 6.8 MHz, 9H).

### Synthesis of Compound 95

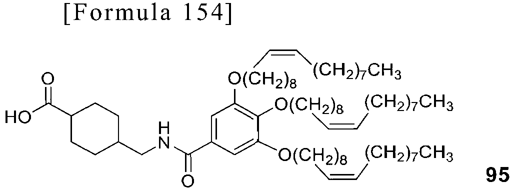

Under a nitrogen atmosphere, compound 94 (2.12 g, 2.00 mmol), potassium hydroxide (0.22 g, 4.00 mmol), THF (15.4 mL), methanol (3.8 mL) and ion-exchanged water (0.8 mL) were mixed and stirred overnight at 60 °C. After confirming completion of the reaction, 6 M HCl (2 mL), water (20 mL) and ethyl acetate (20 mL) were added, and the aqueous layer was separated. The organic layer was washed with water (20 mL) and concentrated under reduced pressure to obtain a crude product. The obtained crude product was purified by silica gel column chromatography (n-hexane/ethyl acetate) to give compound 95 as a white amorphous solid (1.01 g, yield: 48%).
¹H-NMR (400 MHz, CDCl₃) δ 6.94 (s, 2H), 6.06 (t, J = 6 MHz, 1H), 5.40-5.28 (m, 6H), 4.05-3.94 (m, 6H), 3.31 (t, J = 6.4 MHz, 2H), 2.35-2.24 (m, 1H), 2.11-1.94 (m, 14H), 1.93-1.84 (m, 2H), 1.84-1.66 (m, 6H), 1.65-1.61 (m, 1H), 1.51-1.15 (m, 69H), 1.94-0.94 (m, 2H), 0.88 (t, J = 6.8 MHz, 3H).

### Synthesis of Compound 96

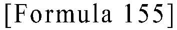

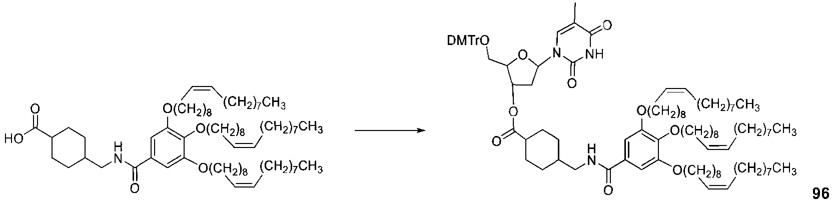

Under a nitrogen atmosphere, compound 95 (106 mg, 0.10 mmol), DMT-dT (93 mg, 0.17 mmol) and DMAP (21 mg, 0.17 mmol) were dissolved in dichloromethane (3 mL), and EDCI (32 mg, 1.02 mmol) was added at room temperature, followed by stirring at the same temperature for 30 hours. After confirming completion of the reaction, the solvent was concentrated under reduced pressure, and the obtained crude product was purified by silica gel column chromatography (dichloromethane/methanol) to give compound 96 as a white amorphous solid mixture, which was used for the next reaction.

### Synthesis of Compound 97

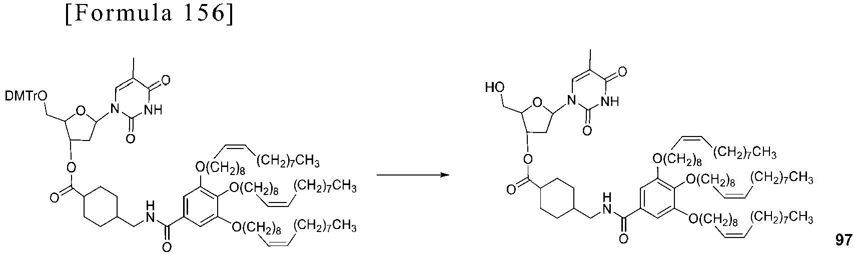

Under a nitrogen atmosphere, to a solution of the mixture containing compound 96 (192 mg) in ethyl acetate (1.5 mL) was added mercaptosuccinic acid (75 mg, 0.50 mmol) and trifluoroacetic acid (0.11 mL, 1.50 mmol), followed by stirring under ice-cooling for 1.5 hours. After confirming completion of the reaction, the reaction mixture was washed three times with a 5% NaHCO₃ aqueous solution (2 mL) and once with a 10% NaCl aqueous solution (2 mL), and then concentrated under reduced pressure to obtain a crude product. The obtained crude product was purified by silica gel column chromatography (dichloromethane/methanol) to give compound 97 as a white amorphous solid (126 mg, yield quant).
¹H-NMR (400 MHz, CDCl₃) δ 8.33-8.15 (m, 1H), 7.49 (s, 1H), 6.93 (s, 2H), 6.25-6.20 (m, 1H), 6.01 (t, J = 3.8 MHz, 1H), 5.39-5.29 (m, 9H), 4.05-3.86 (m, 9H), 3.30 (t, J = 4.4 MHz, 2H), 2.49-2.24 (m, 4H), 2.13-1.95 (m, 12H), 1.95-1.87 (m, 4H), 1.86-1.68 (m, 6H), 1.64-1.55 (m, 3H), 1.53-1.17 (m, 73H), 1.16-1.10 (m, 2H), 0.87 (t, J = 4.6 MHz, 3H).

### Synthesis of Compound 98

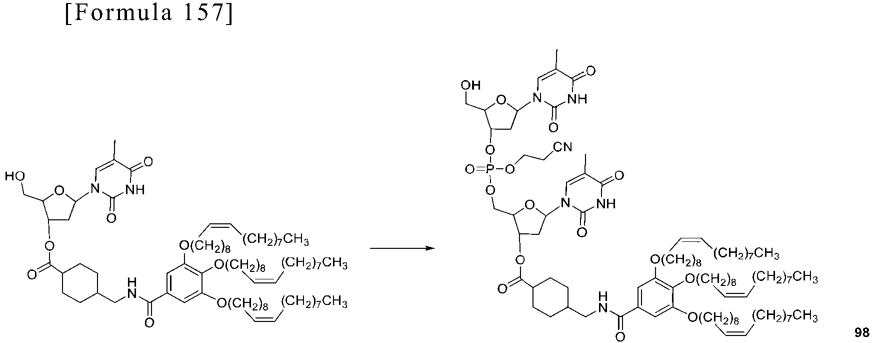

Compound 97 (126 mg, 0.10 mmol) was subjected to azeotropic distillation three times using ethyl acetate (1.5 mL). To the obtained compound 97 were added molecular sieves 3A (100 mg), DMT-dT-CE phosphoramidite (149 mg, 0.20 mmol) and ethyl acetate (1.5 mL), followed by stirring at room temperature for 1 hour. To the reaction solution was added DCI (35 mg, 0.30 mmol), and stirred at room temperature for 1 hour. After confirming completion of the reaction, iodine (76 mg, 0.30 mmol) dissolved in acetonitrile/pyridine/water solution (1.5 mL) was added dropwise to the reaction solution at room temperature, followed by stirring for 1 hour. After confirming completion of the reaction, 1 M Na₂S₂O₃ aqueous solution (2.0 mL) was added, followed by stirring. After insoluble material was removed by filtration, the layers were separated. The organic layer was washed with 5% NaCl aqueous solution (2.0 mL), and then dried over sodium sulfate (5 g). After concentration of the organic layer under reduced pressure, azeotropic distillation was performed twice using ethyl acetate (2.0 mL). The residue was dissolved in ethyl acetate (2.0 mL), and mercaptosuccinic acid (94 mg, 0.50 mmol) was added, followed by cooling to 0 °C. Trifluoroacetic acid (0.19 mL, 2.50 mmol) was added, followed by stirring at the same temperature for 1 hour and then further stirring at room temperature for 1 hour. After confirming completion of the reaction, water (2 mL) was added, and the organic layer was separated. The obtained organic layer was washed three times with 5% NaHCO₃ aqueous solution (2 mL) and once with 5% NaCl aqueous solution (2 mL). The obtained organic layer was concentrated to give compound 98 as a white amorphous solid (161 mg, yield: 99%). The obtained compound 98 was used in the next step without further purification.

### Synthesis of Compound 99

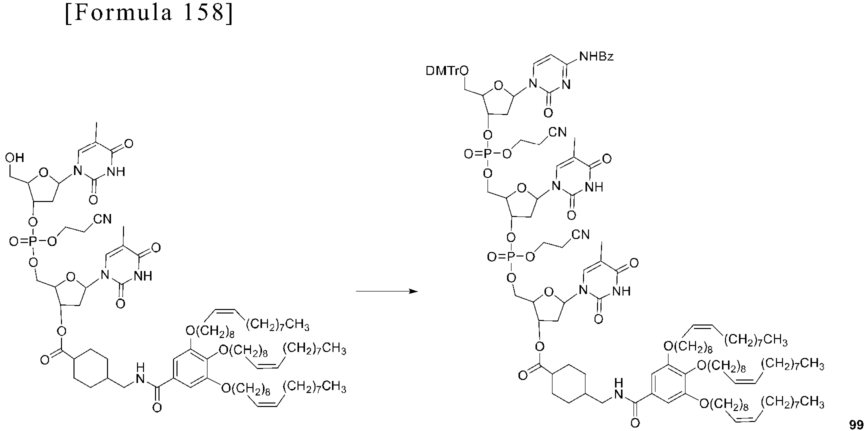

Compound 98 (161 mg, 0.10 mmol) was subjected to azeotropic distillation four times using ethyl acetate (1.5 mL). Molecular sieves 3A (150 mg), DMT-dC-CE phosphoramidite (167 mg, 0.20 mmol) and ethyl acetate (1.5 mL) were added, followed by stirring at room temperature for 1 hour. DCI (36 mg, 0.30 mmol) was added to the reaction solution, followed by stirring at room temperature for 2 hours. After confirming completion of the reaction, iodine (76 mg, 0.30 mmol) dissolved in acetonitrile/pyridine/water solution (1.5 mL) was added dropwise to the reaction solution at room temperature, followed by stirring for 1 hour. After confirming completion of the reaction, 1 M Na₂S₂O₃ aqueous solution (1.5 mL) and ethyl acetate (1.5 mL) were added. After insoluble material was removed by filtration, the layers were separated. The obtained organic layer was washed with 5% NaHCO₃ aqueous solution (1.5 mL) and 5% NaCl aqueous solution (1.5 mL), and then dried over sodium sulfate (1.0 g). After concentration, the obtained crude product was purified by silica gel column chromatography (dichloromethane/methanol) to give compound 99 as a pale yellow amorphous solid (394 mg, quantitative yield).

### Synthesis of Compound 50

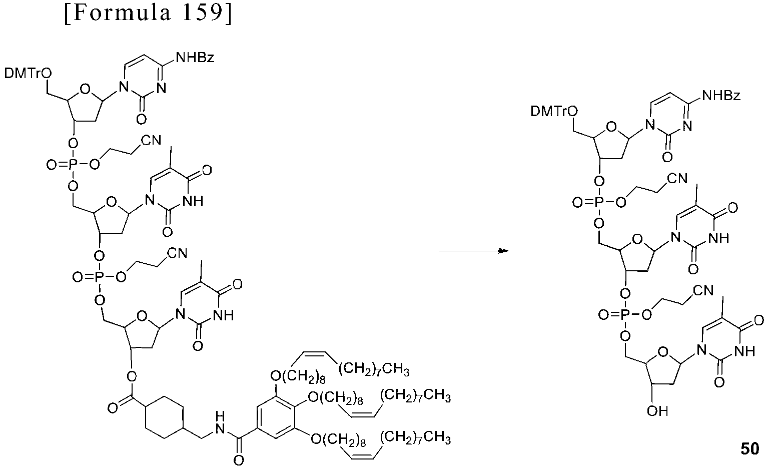

Under a nitrogen atmosphere, at 0 °C, 4 M lithium borohydride solution in THF (0.12 mL, 0.48 mmol) was added dropwise to a solution of compound 99 (394 mg, 0.10 mmol) in anhydrous THF (4 mL) and isopropanol (1.0 mL). After stirring at 0 °C for 1 hour, 10% ammonium chloride aqueous solution (2 mL) and ethyl acetate were added, followed by stirring, and the layers were separated to obtain the organic layer. The aqueous layer was extracted with ethyl acetate, and the combined organic layers were concentrated and dried to remove water. The obtained crude product was purified by silica gel column chromatography (ethyl acetate/methanol) to give compound 50 as a white solid.

### Synthesis of Compound 100

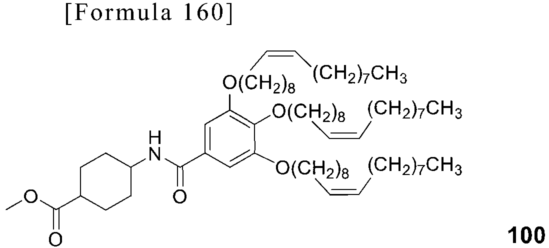

Under a nitrogen atmosphere, 3,4,5-tri-((Z)-octadec-9-enyloxy)benzoic acid (compound 2, 2.73 g, 2.30 mmol), DMF (90 mL), THF (10 mL), Methyl 4-Aminocyclohexanecarboxylate Hydrochloride (0.63 g, 3.00 mmol), COMU (1.28 g, 3.00 mmol) and DIPEA (1.29 mL, 7.60 mmol) were mixed, and stirred overnight while heating from room temperature. After confirming completion of the reaction, the mixture was cooled to room temperature, and ethyl acetate (20 mL), n-hexane (20 mL) and water (20 mL) were added, followed by separation of the aqueous layer. The organic layer was washed with water (20 mL) and concentrated under reduced pressure to obtain the crude product. The crude product was purified by silica gel column chromatography (n-hexane/ ethyl acetate) to give compound 100 as a colorless oil (1.30 g, yield: 52%).
¹H-NMR (400 MHz, CDCl₃) δ 6.91 (s, 2H), 6.77 (d, J = 8 MHz, 1H), 5.40-5.29 (m, 6H), 4.06-3.87 (m, 7H), 3.69 (s, 3H), 2.34-2.22 (m, 1H), 2.22-2.12 (m, 2H), 2.12-1.93 (m, 14H), 1.92-1.59 (m, 6H), 1.53-1.40 (m, 6H), 1.40-1.16 (m, 64H), 0.88 (t, J = 7 MHz, 9H).

### Synthesis of compound 101

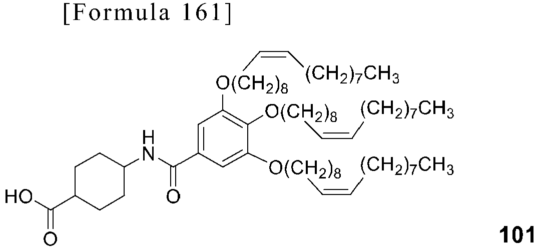

Under a nitrogen atmosphere, compound 100 (1.30 g, 1.24 mmol), potassium hydroxide (0.22 g, 4.00 mmol), THF (15.4 mL), methanol (3.8 mL) and ion-exchanged water (0.8 mL) were mixed, and stirred overnight at 60 °C. After confirming completion of the reaction, 6 M HCl (2 mL), water (20 mL) and ethyl acetate (20 mL) were added, followed by separation of the aqueous layer. The organic layer was washed with water (20 mL) and concentrated under reduced pressure to obtain the crude product. The crude product was purified by silica gel column chromatography (n-hexane/ ethyl acetate) to give compound 101 as a white amorphous solid (0.29 g, yield: 23%).

¹H-NMR (400 MHz, CDCl₃) δ 6.90 (s, 2H), 5.78 (d, J = 8 MHz, 1H), 5.40-5.26 (m, 6H), 4.04-3.88 (m, 7H), 2.38-2.24 (m, 1H), 2.23-2.06 (m, 4H), 2.06-1.90 (m, 12H), 1.88-1.55 (m, 7H), 1.54-1.39 (m, 6H), 1.39-1.16 (m, 64H), 0.87 (t, J = 7 MHz, 9H).

### Synthesis of compound 102

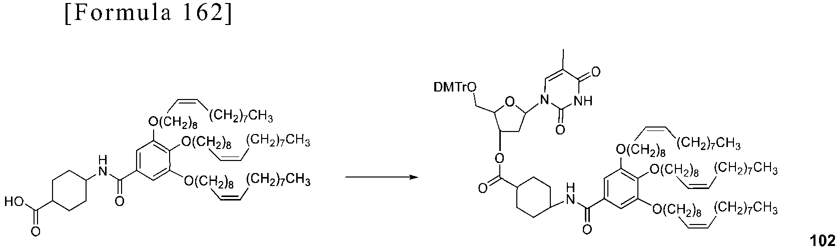

Under a nitrogen atmosphere, to a solution of compound 101 (104 mg, 0.10 mmol), DMT-dT (93 mg, 0.17 mmol) and DMAP (21 mg, 0.17 mmol) in dichloromethane (3 mL), EDCI (32 mg, 1.02 mmol) was added at room temperature, and followed by stirring at the same temperature for 30 hours. After confirming completion of the reaction, the solvent was concentrated under reduced pressure, and the obtained crude product was purified by silica gel column chromatography (dichloromethane/methanol) to give compound 102 as a mixture of a white amorphous solid, which was used for the next reaction.

### Synthesis of Compound 103

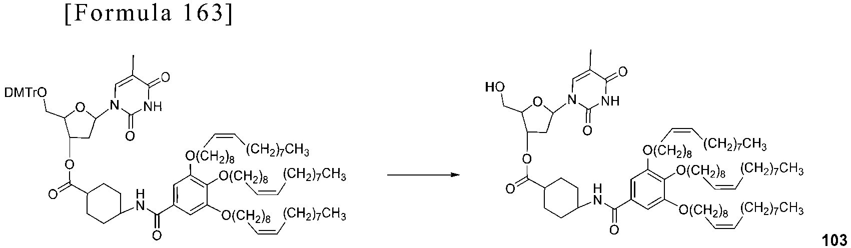

Under a nitrogen atmosphere, to an solution of the mixture containing compound 102 (184 mg) in ethyl acetate (1.5 mL) were added mercaptosuccinic acid (75 mg, 0.50 mmol) and trifluoroacetic acid (0.11 mL, 1.50 mmol), followed by stirring under ice cooling for 1.5 hours. After confirming completion of the reaction, the reaction solution was washed three times with 5% NaHCO₃ aqueous solution (2 mL) and once with 10% NaCl aqueous solution (2 mL), and concentrated under reduced pressure to obtain the crude product. The obtained crude product was purified by silica gel column chromatography (dichloromethane/methanol) to obtain compound 103 as a white amorphous solid (125 mg, yield: 99%).
¹H-NMR (400 MHz, CDCl₃) δ 7.59 (m, 1H), 6.91 (s, 2H), 6.30-6.25 (m, 1H), 6.03-5.84 (m, 1H), 5.42 (m, 7H), 4.06-4.01 (m, 1H), 4.01-3.85 (m, 9H), 2.45-2.24 (m, 3H), 2.21-2.11 (m, 2H), 2.10-2.02 (m, 2H), 2.02-1.93 (m, 12H), 1.92-1.85 (m, 3H), 1.83-1.67 (m, 6H), 1.64-1.52 (m, 1H), 1.50-1.38 (m, 4H), 1.38-1.04 (m, 67H), 0.86 (t, J = 4.8 MHz, 9H).

### Synthesis of compound 104

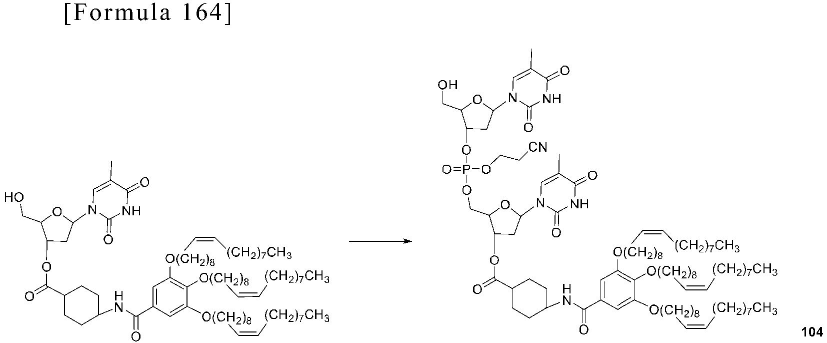

Compound 103 (126 mg, 0.10 mmol) was subjected to azeotropic distillation three times with ethyl acetate (1.5 mL). To the obtained compound 103 were added molecular sieves 3A (100 mg), DMT-dT-CE phosphoramidite (149 mg, 0.20 mmol) and ethyl acetate (1.5 mL), followed by stirring at room temperature for 1 hour. To the reaction solution was added DCI (35 mg, 0.30 mmol), followed by stirring at room temperature for 1 hour. After confirming completion of the reaction, iodine (76 mg, 0.30 mmol) dissolved in an acetonitrile/pyridine/water solution (1.5 mL) was added dropwise to the reaction solution at room temperature, followed by stirring for 1 hour. After confirming completion of the reaction, 1 M Na₂S₂O₃ aqueous solution (2.0 mL) was added, followed by stirring. After the insoluble matter was removed by filtration, and the layers were separated. The organic layer was washed with 5% brine (2.0 mL), and dried over sodium sulfate (5 g). After the organic layer was concentrated under reduced pressure, azeotropic distillation was performed twice with ethyl acetate (2.0 mL). The residue was dissolved in ethyl acetate (2.0 mL), mercaptosuccinic acid (94 mg, 0.50 mmol) was added, and the mixture was cooled to 0°C. Trifluoroacetic acid (0.19 mL, 2.50 mmol) was added, followed by stirring at the same temperature for 1 hour, then warming to room temperature and stirring for an additional 1 hour. After confirming completion of the reaction, water (2 mL) was added, and the organic layer was separated. The obtained organic layer was washed three times with 5% NaHCO₃ aqueous solution (2 mL) and once with 5% NaCl aqueous solution (2 mL). The obtained organic layer was concentrated to obtain compound 104 as a white amorphous solid (160 mg, quant. yield). The obtained compound 104 was used in the next step without further purification.

### Synthesis of compound 105

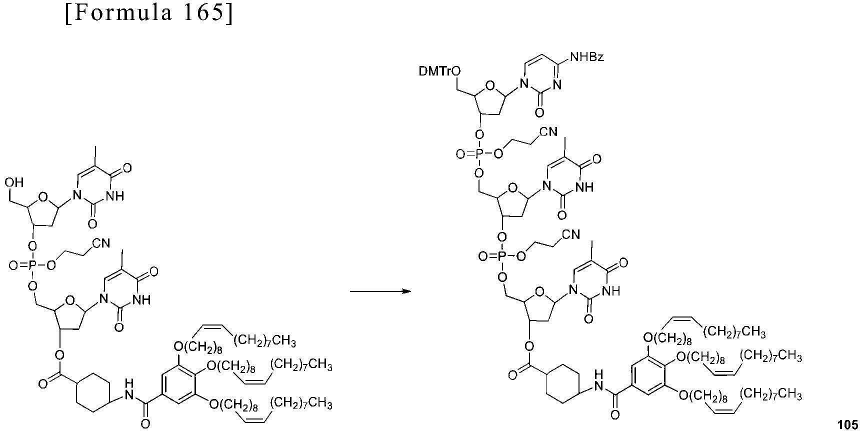

Compound 104 (160 mg, 0.10 mmol) was subjected to azeotropic distillation four times with ethyl acetate (1.5 mL). Molecular sieves 3A (150 mg), DMT-dC-CE phosphoramidite (167 mg, 0.20 mmol) and ethyl acetate (1.5 mL) were added, followed by stirring at room temperature for 1 hour. To the reaction solution was added DCI (36 mg, 0.30 mmol), followed by stirring at room temperature for 2 hours. After confirming completion of the reaction, iodine (76 mg, 0.30 mmol) dissolved in an acetonitrile/pyridine/water solution (1.5 mL) was added dropwise to the reaction solution at room temperature, followed by stirring for 1 hour. After confirming completion of the reaction, 1 M Na₂S₂O₃ aqueous solution (1.5 mL) and ethyl acetate (1.5 mL) were added. After the insoluble matter was removed by filtration, the layers were separated. The organic layer was washed with 5% NaHCO₃ aqueous solution (1.5 mL) and 5% NaCl aqueous solution (1.5 mL), and dried over sodium sulfate (1.0 g). After concentration, the obtained crude product was purified by silica gel column chromatography (dichloromethane/methanol) to obtain compound 105 as a pale yellow amorphous solid (353 mg, quant. yield).

### Synthesis of compound 50

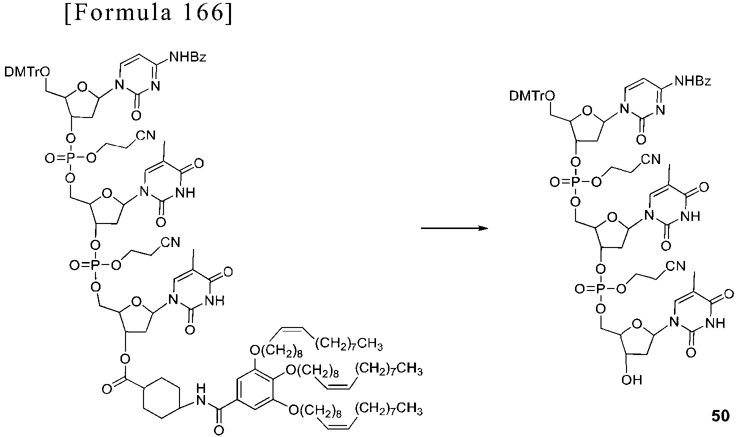

Under a nitrogen atmosphere, at 0 °C, to a solution of compound 105 (353 mg, 0.10 mmol) in anhydrous THF (4 mL) and isopropanol (1.0 mL) was added dropwise a 4 M lithium borohydride solution in THF (0.12 mL, 0.48 mmol). After stirring at 0 °C for 1 hour, 10% ammonium chloride aqueous solution (2 mL) and ethyl acetate were added, followed by stirring, and the layers were separated to obtain the organic layer. The aqueous layer was extracted with ethyl acetate, and the organic layers were combined and concentrated to remove water. The obtained crude product was purified by silica gel column chromatography (ethyl acetate/methanol) to obtain compound 50 as a white solid.

### Synthesis of compound 106

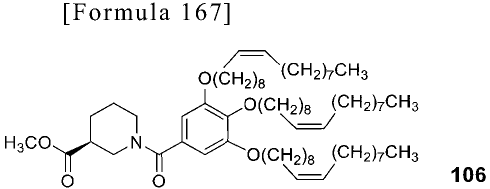

Under a nitrogen atmosphere, 3,4,5-tri-((Z)-octadec-9-enyloxy)benzoic acid (compound 2, 1.99 g, 2.16 mmol), DMF (10 mL), THF (90 mL), ethyl (R)-(-)-3-piperidinecarboxylate (0.462 mL, 3.00 mmol), COMU (1.29 g, 3.00 mmol) and DIPEA (1.28 mL, 7.60 mmol) were mixed and stirred overnight while heating from room temperature. After confirming completion of the reaction, the mixture was cooled to room temperature, and ethyl acetate (20 mL), n-hexane (20 mL) and water (20 mL) were added, and the aqueous layer was separated. The organic layer was washed with water (20 mL) and concentrated under reduced pressure to obtain the crude product. The obtained crude product was purified by silica gel column chromatography (n-hexane/ethyl acetate) to obtain compound 106 as a colorless oily material (1.99 g, yield: 88%).
¹H NMR (300 MHz, CDCl₃) δ 6.58 (2H, s), 5.40-5.30 (6H, m), 4.19-4.05 (2H, m), 3.96 (6H, t, J = 6.5 Hz), 3.21-2.92 (2H, m), 2.59-2.44 (1H, m), 2.14-1.98 (12H, m), 1.84-1.66 (8H, m), 1.61-1.08 (73H, m), 0.88 (9H, t, J = 6.9 Hz);
¹³C{¹H} NMR (125 MHz, CDCl₃) δ 176.4, 170.8, 153.3, 140.9, 139.5, 130.1, 130.0, 105.7, 73.6, 69.4, 60.9, 32.1, 30.0, 29.7, 29.6, 29.5, 27.4, 26.3, 22.9, 14.4, 14.3.

### Synthesis of compound 107

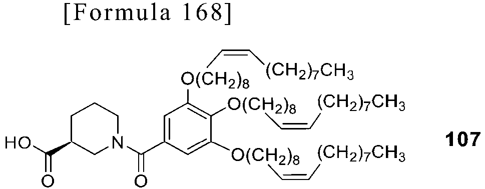

Under a nitrogen atmosphere, compound 106 (1.99 g, 1.89 mmol), potassium hydroxide (0.22 g, 3.92 mmol), THF (15.4 mL), ethanol (3.84 mL) and ion-exchange water (0.77 mL) were mixed and stirred overnight at 70 °C. After confirming completion of the reaction, 6 M HCl (2 mL), water (20 mL) and ethyl acetate (20 mL) were added, and the aqueous layer was separated. The organic layer was washed with water (20 mL) and concentrated under reduced pressure to obtain the crude product. The obtained crude product was purified by silica gel column chromatography (n-hexane/ethyl acetate) to obtain compound 107 as a white amorphous solid (1.46 g, yield: 76%).
¹H NMR (600 MHz, CDCl₃) δ 6.58 (2H, s), 5.37-5.32 (6H, m), 3.97-3.94 (6H, m), 3.17-3.02 (2H, m), 2.62-2.49 (1H, m), 2.16-2.14 (1H, m), 2.03-2.00 (12H, m), 1.81-1.71 (8H, m), 1.50-1.43 (6H, m), 1.33-1.26 (64H, m), 0.88 (9H, t, J = 6.9 Hz);
¹³C{¹H} NMR (100 MHz, CDCl₃) δ 177.8, 171.0, 153.3, 139.6, 130.5, 130.1, 129.9, 105.8, 73.6, 69.4, 32.1, 30.5, 30.0, 29.9, 29.8, 29.7, 29.6, 29.5, 27.4, 26.3, 22.8, 14.3.

### Synthesis of compound 108

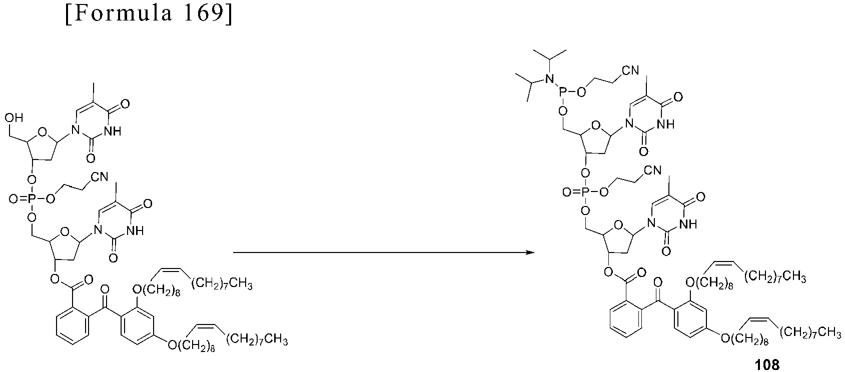

Under a nitrogen atmosphere, to a dichloromethane (2.8 mL) solution of compound 23 (98.3 mg, 0.0733 mmol) and 2-cyanoethyl N,N,N',N'-tetraisopropylphosphorodiamidite (72.4 mg, 0.24 mmol), 5-(benzylthio)-1H-tetrazole (49.4 mg, 0.257 mmol) was added, folowed by stirring at room temperature for 40 minutes. The reaction solution was purified by silica gel column chromatography (spherical neutral silica gel, eluent: ethyl acetate) , thereby obtaining compound 108 as a white solid in 83.6% yield (94.4 mg, 0.0613 mmol).

### Synthesis of compound 109

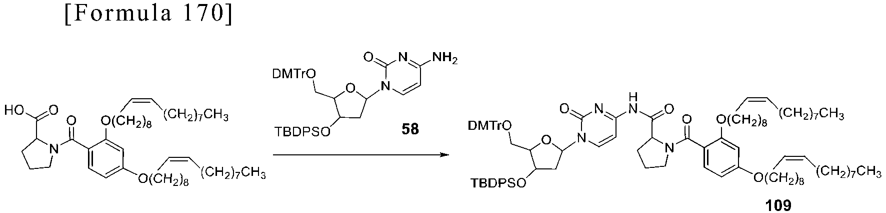

Under a nitrogen atmosphere, to a DMF (4.2 mL) solution of compound 34 (416 mg, 0.553 mmol) and compound 58 (467 mg, 0.608 mmol), EDCI (212 mg, 1.106 mmol) and HOBT hydrate (84.7 mg, 0.553 mmol) were added, followed by the addition of DIPEA (0.385 mL, 2.21 mmol), and stirring at room temperature for 2 hours. EDCI (106 mg, 0.553 mmol) and DIPEA (0.193 mL, 1.11 mmol) were further added thereto, followed by stirring at room temperature for 2 hours. To the reaction solution were added water (5 mL) and ethyl acetate (10 mL), followed by stirring and separating the layers. The obtained organic layer was washed twice with water (5 mL), dried over magnesium sulfate, and concentrated under reduced pressure to obtain the crude product of compound 109. The obtained crude product was purified by silica gel column chromatography (spherical neutral silica gel, eluent: ethyl acetate/hexane), thereby obtaining compound 109 as a viscous colorless oily material in 69.2% yield (575 mg, 0.383 mmol).
MS(ESI⁺): [M+1]⁺ 1501.9947.

### Synthesis of compound 110

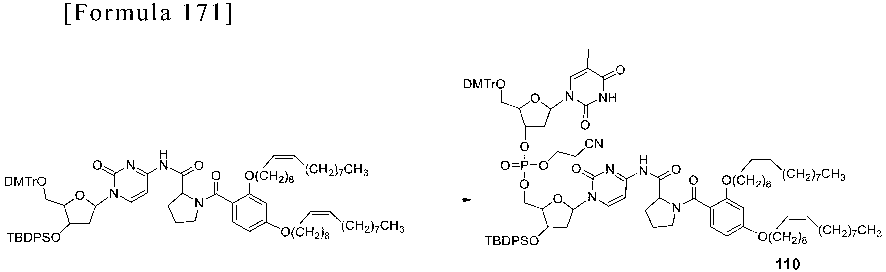

Under a nitrogen atmosphere, to an ethyl acetate (4.9 mL) solution of compound 109 (484 mg, 0.322 mmol), mercaptosuccinic acid (242 mg, 1.611 mmol) and trifluoroacetic acid (0.225 mL, 2.94 mmol) were added at 0°C, followed by stirring for 1 hour while allowing the temperature to rise to room temperature. After cooling the reaction solution to 0°C, saturated aqueous sodium bicarbonate (6.5 mL) was added thereto, following by stirring while allowing the temperature to rise to room temperature, and separating the layers. The obtained organic layer was washed with saturated brine (3.4 mL). The obtained organic layer was dried over magnesium sulfate and concentrated. The obtained residue was subjected to azeotropic distillation twice with ethyl acetate (3.4 mL). Under a nitrogen atmosphere, to the obtained residue was added ethyl acetate (3.4 mL) to prepare a solution, followed by the addition of molecular sieves 3A (484 mg), and stirring for 30 minutes. To the obtained solution were added DMT-r-dT-CE phosphoramidite (300 mg, 0.403 mmol) and DCI (99.0 mg, 0.838 mmol), followed by stirring at room temperature for 1 hour. Furthermore, DCI (38.1 mg, 0.323 mmol) was added, followed by stirring at room temperature for 45 minutes. To the reaction solution was added 0.2 M iodine (ethyl acetate/pyridine/water solution, 4.67 mL), followed by stirring at room temperature for 30 minutes. To the reaction solution was added 1 M sodium thiosulfate aqueous solution(3.8 mL), followed by stirring, and filtrating the molecular sieves while washing with ethyl acetate. The layers of the filtrate were separated, and the obtained organic layer was washed three times with saturated aqueous sodium bicarbonate (3.4 mL). Furthermore, it was washed with saturated brine (3.4 mL), dried over magnesium sulfate, and concentrated under reduced pressure to obtain the crude product of compound 110. The obtained crude product was purified by silica gel column chromatography (spherical neutral silica gel, eluent: ethyl acetate), thereby obtaining compound 110 as a white solid in 92.5% yield (553 mg, 0.298 mmol).

### Synthesis of compound 112

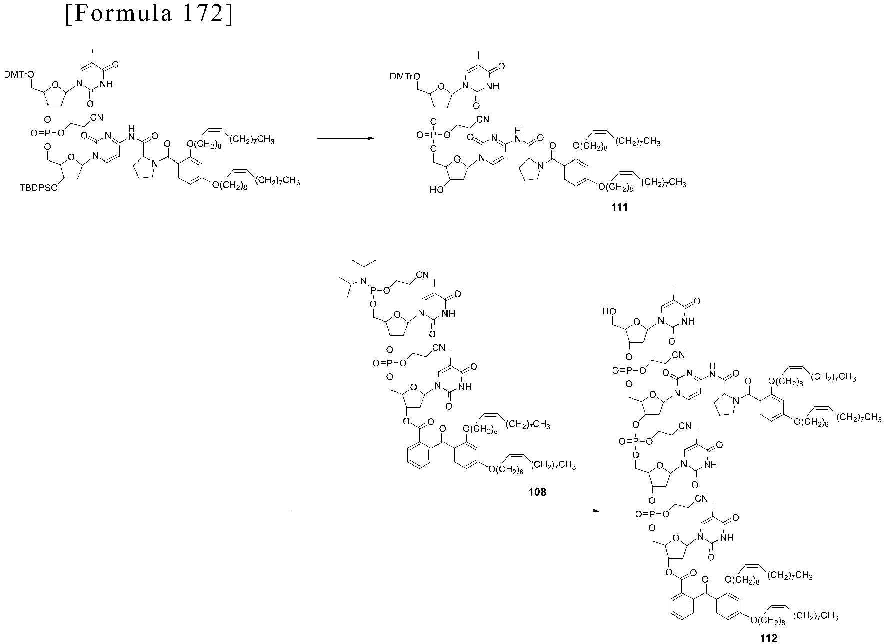

Under a nitrogen atmosphere, to a THF (3.1 mL) solution of compound 110 (307 mg, 0.165 mmol) and imidazole (449 mg, 6.6 mmol), a pre-prepared ethyl acetate (3 mL) solution of hydrogen fluoride pyridine (0.0924 mL, corresponding to 3.3 mmol hydrogen fluoride) was added dropwise at 0°C. After the reaction solution was stirred at 0°C for 1 hour 45 minutes, saturated aqueous sodium bicarbonate (3 mL) and ethyl acetate (3 mL) were added, followed by stirring while allowing the temperature to rise to room temperature, and the layers were separated. The obtained organic layer was washed with saturated aqueous sodium bicarbonate (3 mL) and saturated brine (3 mL), and dried over magnesium sulfate. The obtained solution was concentrated under reduced pressure and further subjected to crude purification with silica gel, thereby obtaining the crude product of compound 111 as a viscous colorless oily material (356 mg). From the obtained crude product of compound 111, 87.2 mg was taken, and compound 108 (94.4 mg, 0.0613 mmol) was added, followed by azeotropic distillation twice with ethyl acetate (2 mL). Under a nitrogen atmosphere, to the obtained residue was added ethyl acetate (1.6 mL) to prepare a solution, followed by the addition of molecular sieves 3A (100 mg) and stirring for 30 minutes. To the obtained solution was added DCI (15.9 mg, 0.135 mmol), followed by stirring at room temperature for 30 minutes. Furthermore, DCI (8 mg, 0.0677 mmol) was added, followed by stirring at room temperature for 18 hours. To the reaction solution was added 0.2 M iodine (ethyl acetate/pyridine/water solution, 0.613 mL), followed by stirring at room temperature for 10 minutes. To the reaction solution was added 1 M aqueous sodium thiosulfate (2 mL), followed by stirring and filtrating the molecular sieves while washing with ethyl acetate. The layers of the filtrate were separated, and the obtained organic layer was washed with saturated aqueous sodium bicarbonate (2 mL). Furthermore, it was washed with saturated brine, dried over magnesium sulfate, and concentrated under reduced pressure to obtain a crude product (168 mg). To the obtained crude product were added mercaptosuccinic acid (41.8 mg, 0.279 mmol), then ethyl acetate (1.7 mL) and THF (0.2 mL) to prepare a suspension. After cooling the reaction solution to 0°C, trifluoroacetic acid (0.135 mL, 1.70 mmol) was added, followed by stirring for 70 minutes while allowing the temperature to rise to room temperature. After cooling the reaction solution to 0°C, saturated aqueous sodium bicarbonate (2.37 mL) was added, followed by stirring while allowing the temperature to rise to room temperature, and then separating the layers. To the obtained organic layer was added THF, then washed with saturated brine. The obtained organic layer was dried over magnesium sulfate, concentrated, thereby obtaining a pale yellow solid (127 mg) containing compound 112 as the main product.
MS(ESI⁺): [M + 2H]²⁺ 1387.1904.

The above detailed description is merely illustrative of the objects and subject matter of the present invention, and is not intended to limit the appended claims. Various modifications and substitutions to the described embodiments, without departing from the appended claims, will be apparent to those skilled in the art from the teachings herein.

### [Industrial Applicability]

The present invention has provided a novel pseudo-solid-phase protecting group usable for the synthesis of oligonucleotides by liquid-phase synthesis, and a (poly)oligonucleotide to which the protecting group is bound. The present invention is useful for the synthesis of polyoligonucleotide(s).

## Claims

1. A compound represented by the following formula (1) or a salt thereof: 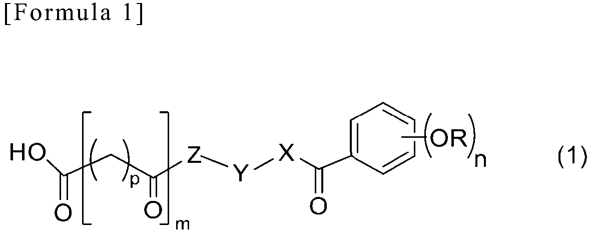 wherein,
R each independently represents an optionally substituted alkenyl group having 14 to 60 carbon atoms;
n represents 2 or 3, and the plurality of OR groups may be the same or different;
m represents 0 or 1;
p represents 1 or 2;
X is C, N, O or S, and when X is C, it represents CH₂ or may form, together with Y, a 5- to 7-membered carbocyclic or heterocyclic ring which may have a substituent, and when X is N, it represents N R₁ or may form, together with Y, a 5- to 7-membered heterocyclic ring which may have a substituent, wherein R₁ is H or a C1-6 alkyl group;
Y is a single bond or B(CH₂)ₜ* (wherein B is a 5- to 7-membered carbocyclic ring which may have a substituent, t is 0, 1, or 2, and * indicates a bond to X), or may form, together with X, a 5- to 7-membered carbocyclic or heterocyclic ring which may have a substituent;
Z is a single bond or (CH₂)ₛ (wherein s is an integer of 1 to 5), provided that when Y is a single bond, Z is not a single bond.

2. The compound or a salt thereof according to claim 1, which is represented by the following formula (2): 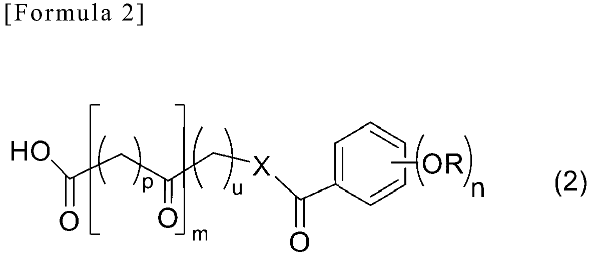 wherein,
R each independently represents an optionally substituted alkenyl group having 14 to 60 carbon atoms;
n represents 2 or 3, and the plurality of OR groups may be the same or different;
m represents 0 or 1;
p represents 1 or 2;
u represents an integer of 1 to 5;
X represents NR₁, O or S, wherein R₁ is H or a C1-6 alkyl group.

3. The compound or a salt thereof according to claim 2, wherein in the formula (2), m is 0 and X represents NR₁, O or S (wherein R₁ is H or a C1-6 alkyl group).

4. The compound or a salt thereof according to claim 2, wherein in the formula (2), m is 1 and X represents NR₁, O or S (wherein R₁ is H or a C1-6 alkyl group).

5. The compound or a salt thereof according to any one of claims 2 to 4, wherein in the formula (2), the OR groups on the benzene ring are in a 3,4,5-substitution, 2,4-substitution, or 2,3,4-substitution.

6. The compound or a salt thereof according to claim 1, which is represented by the following formula (3): 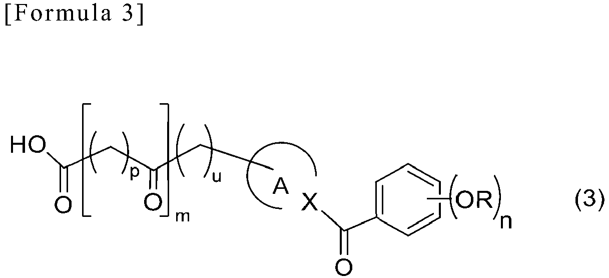 wherein,
R each independently represents an optionally substituted alkenyl group having 14 to 60 carbon atoms;
n represents 2 or 3, and the plurality of OR groups may be the same or different;
m represents 0 or 1;
p represents 1 or 2;
u represents 0 or an integer of 1 to 5;
ring A represents a 5- to 7-membered carbocyclic or heterocyclic ring which may have a substituent;
X represents C or N.

7. The compound or a salt thereof according to claim 6, wherein in the formula (3), m is 0, u is 0 or an integer of 1 to 3, and X represents C.

8. The compound or a salt thereof according to claim 7, wherein in the formula (3), ring A is benzene or cyclohexane which may have a substituent, and the substituent is a group selected from the group consisting of an optionally substituted hydroxyl group, an optionally substituted amino group, an optionally substituted alkyl group, a halogen atom, a nitro group, and a carbonyl group.

9. The compound or a salt thereof according to claim 6, wherein in the formula (3), u is **0, 1,** or 2, X represents N, and ring A containing X represents a 5- to 7-membered heterocyclic ring containing one nitrogen atom which may have a substituent, and the substituent is a group selected from the group consisting of an optionally substituted hydroxyl group, an optionally substituted amino group, an optionally substituted alkyl group, a halogen atom, a nitro group, and an optionally substituted carbonyl group.

10. The compound or a salt thereof according to claim 9, wherein in the formula (3), m is 0.

11. The compound or a salt thereof according to claim 9, wherein in the formula (3), ring A is pyrrolidine or piperidine which may have a substituent, and the substituent is a group selected from the group consisting of an optionally substituted hydroxyl group, an optionally substituted alkyl group, and an optionally substituted carbonyl group.

12. The compound or a salt thereof according to claim 6, wherein in the formula (3), the OR groups on the benzene ring are in a 3,4,5-substitution, 2,4-substitution, or 2,3,4-substitution.

13. The compound or a salt thereof according to claim 1, which is represented by the following formula (4): 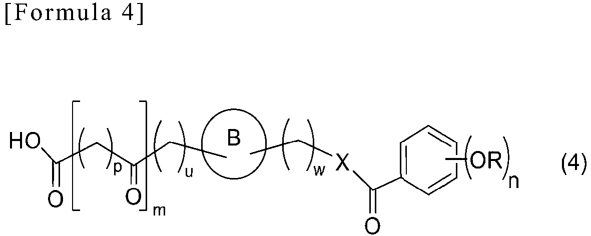 wherein,
R each independently represents an optionally substituted alkenyl group having 14 to 60 carbon atoms;
n represents 2 or 3, and the plurality of OR groups may be the same or different;
m represents 0 or 1;
p represents 1 or 2;
u represents 0 or an integer of 1 to 5;
w represents 0, 1, or 2;
ring B represents a carbocyclic ring having 5 to 7 carbon atoms which may have a substituent;
X represents NR₁, O or S, wherein R₁ is H or C H₃.

14. The compound or a salt thereof according to claim 13, wherein in the formula (4), m is 0.

15. The compound or a salt thereof according to claim 13, wherein in the formula (4), ring B is benzene or cyclohexane which may have a substituent, and the substituent is a group selected from the group consisting of an optionally substituted hydroxyl group, an optionally substituted amino group, an optionally substituted alkyl group, a halogen atom, a nitro group, and an optionally substituted carbonyl group.

16. The compound or a salt thereof according to claim 13, wherein in the formula (3), the OR groups on the benzene ring are in a 3,4,5-substitution, 2,4-substitution, or 2,3,4-substitution.

17. The compound or a salt thereof according to claim 1, wherein the compound represented by the formula (1) is a compound selected from the group consisting of compounds represented by the following formulae: 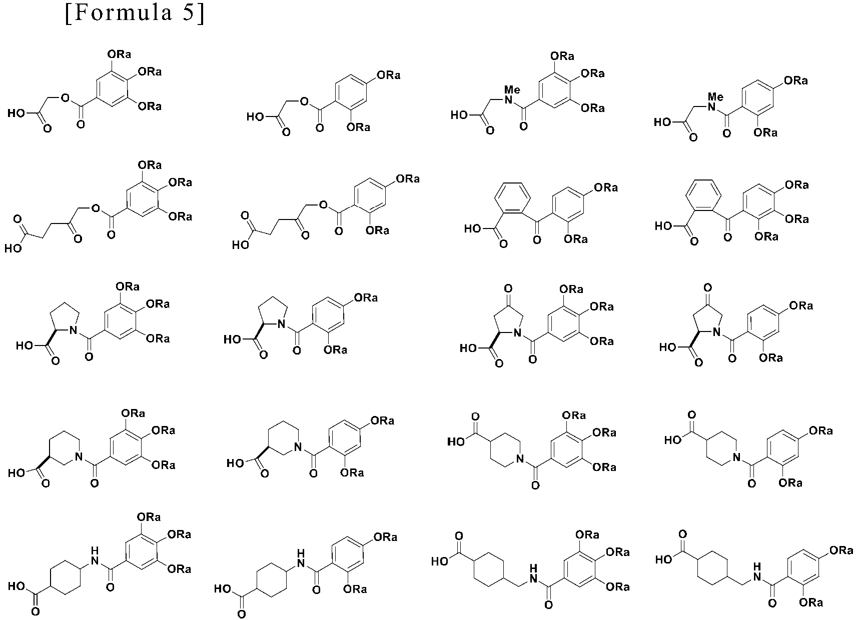 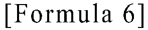 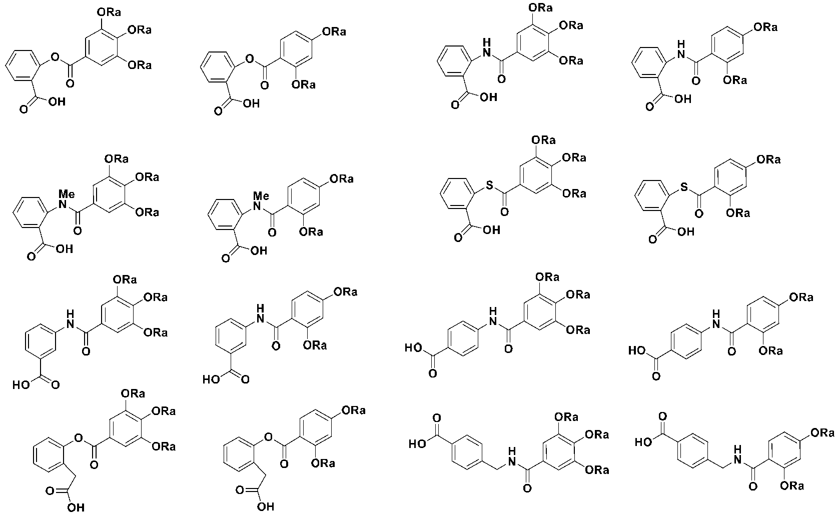 wherein Ra each independently represents an optionally substituted alkenyl group having 14 to 60 carbon atoms, and the plurality of ORa groups may be the same or different.

18. The compound or a salt thereof according to claim 17, wherein the Ra each independently represents an unsaturated hydrocarbon group derived from a compound selected from the group consisting of myristoleic acid (C14), palmitoleic acid (C16), sapienic acid (C16), oleic acid (C18), elaidic acid (C18), vaccenic acid (C18), gadoleic acid (C20), eicosenoic acid (C20), erucic acid (C22), nervonic acid (C24), linoleic acid (C18), eicosadienoic acid (C20), docosadienoic acid (C22), α- and γ-linolenic acids (C18), pinoleic acid (C18), eleostearic acid (C18), mead acid (C20), di-homo-γ-linolenic acid (C20), eicosatrienoic acid (C20), stearidonic acid (C18), arachidonic acid (C20), eicosatetraenoic acid (C20), adrenic acid (C22), bosseopentaenoic acid (C18), eicosapentaenoic acid (C20), osbond acid (C22), clupanodonic acid (C22), tetracosapentaenoic acid (C24), docosahexaenoic acid (C22), and nisinic acid (C24).

19. A nucleotide represented by the following formula (I): 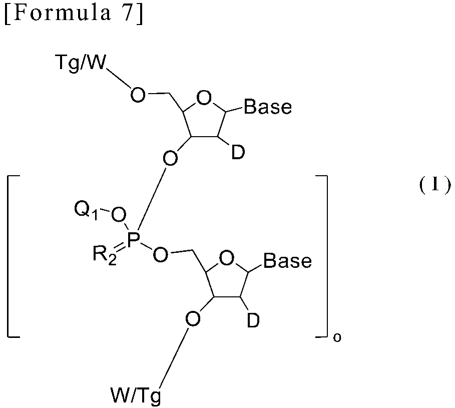 wherein,
o represents any integer of 0 or more;
o+1 Base each independently represents an optionally modified nucleic acid base;
o+1 D each independently represents a hydrogen atom, a halogen atom, or a hydroxyl group which may be protected, or may form a crosslink with the 4'-position carbon;
o Q₁ each independently represents a C1-4 alkyl group having an electron-withdrawing group, an allyl group, a C2-6 alkenyl group or a C6-10 aryl group having a cyano group, a nitro group or a halogen group, or a hydrogen atom;
W/Tg or Tg/W represents W or Tg, and when the 5' side is W, the 3' side is Tg, whereas when the 5' side is Tg, the 3' side is W, wherein
W represents a hydrogen atom or a temporary protecting group removable under acidic conditions or with a reagent containing a fluorine anion;
o R₂ each independently represents O or S; and
Tg represents a group represented by the following formula (5): 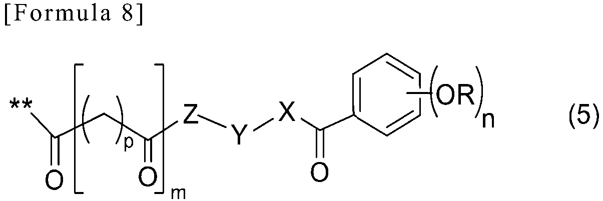 wherein,
R each independently represents an optionally substituted alkenyl group having 14 to 60 carbon atoms;
n represents 2 or 3, and the plurality of OR groups may be the same or different;
m represents 0 or 1;
p represents 1 or 2;
X is C, N, O or S, and when X is C, it represents CH₂ or may be combined with Y to form a 5- to 7-membered carbocyclic or heterocyclic ring which may have a substituent, and when X is N, it represents N R₁ or may form, together with Y, a 5- to 7-membered heterocyclic ring which may have a substituent, wherein R₁ is H or a C1-6 alkyl group;
Y is a single bond or B(CH₂)ₜ* (wherein B is a 5- to 7-membered carbocyclic ring which may have a substituent, t is 0, 1, or 2, and * indicates a bond to X), or may form, together with Y, a 5- to 7-membered carbocyclic or heterocyclic ring which may have a substituent;
Z is a single bond or (CH₂)ₛ, wherein s is an integer of 1 to 5, provided that when Y is a single bond, Z is not a single bond,
** indicates a bond to O, or
a nucleotide represented by the following formula (II): 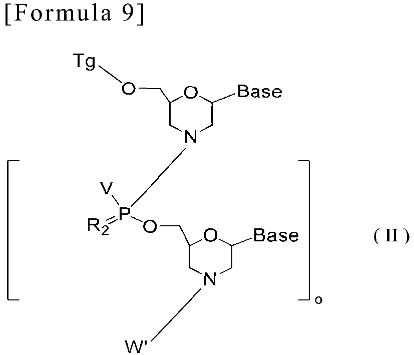 wherein,
o represents any integer of 0 or more;
o+1 Base each independently represents an optionally modified nucleic acid base;
o V each independently represents a C1-6 alkoxy group, a di(C1-6 alkyl)amino group, or a piperazino group in which the 4-position nitrogen is protected with a protecting group and which may be further substituted;
W' represents a hydrogen atom or a temporary protecting group removable under acidic conditions or with a reagent containing a fluorine anion;
o R₂ each independently represents O or S;
Tg represents a group represented by the following formula (5): 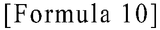 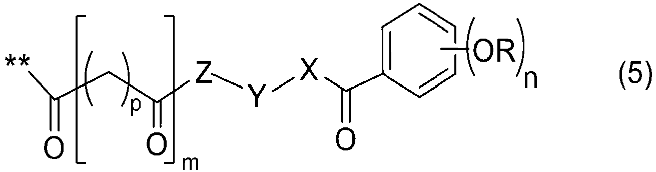 wherein,
R each independently represents an optionally substituted alkenyl group having 14 to 60 carbon atoms;
n represents 2 or 3, and the plurality of OR groups may be the same or different;
m represents 0 or 1;
p represents 1 or 2;
X is C, N, O or S, and when X is C, it represents CH₂ or may form, together with Y, a 5- to 7-membered carbocyclic or heterocyclic ring which may have a substituent, and when X is N, it represents NR₁ or may form, together with Y, a 5- to 7-membered heterocyclic ring which may have a substituent, wherein R₁ is H or a C1-6 alkyl group;
Y is be a single bond or B(CH₂)ₜ* (wherein B is a 5- to 7-membered carbocyclic ring which may have a substituent, t is 0, 1, or 2, and * indicates a bond to X), or may form, together with X, a 5- to 7-membered carbocyclic or heterocyclic ring which may have a substituent;
Z is a single bond or (CH₂)_{S}, wherein s is an integer of 1 to 5, provided that when Y is a single bond, Z is not a single bond,
** indicates a bond to O.

20. The nucleotide according to claim 19, wherein Tg in the formula (I) or formula (II) represents a group represented by the following formula (6): 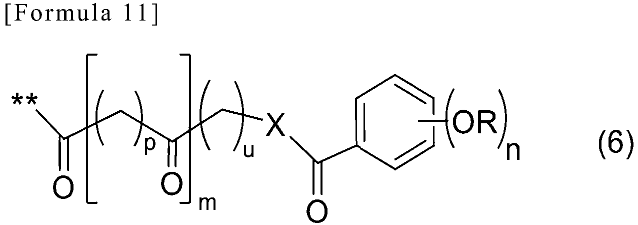 wherein,
R each independently represents an optionally substituted alkenyl group having 14 to 60 carbon atoms;
n represents 2 or 3, and the plurality of OR groups may be the same or different;
m represents 0 or 1;
p represents 1 or 2;
u represents an integer of 1 to 5;
X represents NR₁, O or S, wherein R₁ is H or a C1-6 alkyl group;
** indicates a bond to O.),
a group represented by the following formula (7): 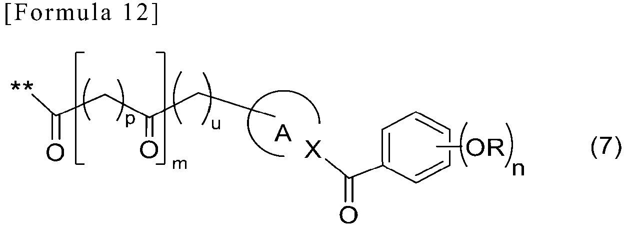 wherein,
R each independently represents an optionally substituted alkenyl group having 14 to 60 carbon atoms;
n represents 2 or 3;
m represents 0 or 1;
p represents 1 or 2;
u represents 0 or an integer of 1 to 5 (preferably 0 or an integer of 1 to 3, more preferably 0, 1, or 2);
ring A represents a 5- to 7-membered ring which may have a substituent;
X represents C or N;
** indicates a bond to O.), or
a group represented by the following formula (8): 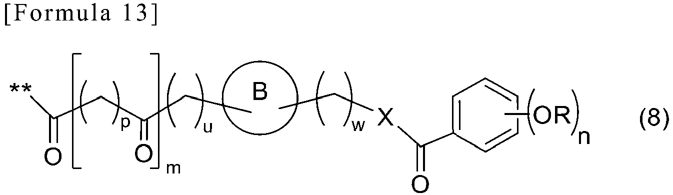 wherein,
R each independently represents an optionally substituted alkenyl group having 14 to 60 carbon atoms;
n represents 2 or 3, and the plurality of OR groups may be the same or different;
m represents 0 or 1;
p represents 1 or 2;
u represents 0 or an integer of 1 to 5;
w represents 0, 1, or 2;
ring B is a carbocyclic ring having 5 to 7 carbon atoms which may have a substituent;
X represents NR₁ , O or S, wherein R₁ is H or CH₃ ;
** indicates a bond to O.

21. A nucleotide represented by the following formula (V) or formula (VI): 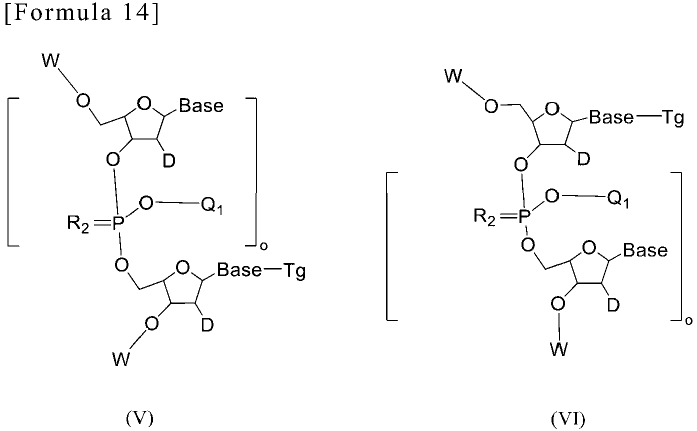 wherein,
o represents any integer of 0 or more (preferably an integer of 1 to 50);
o+1 Base each independently represent an optionally modified nucleic acid base;
o+1 D groups each independently represent a hydrogen atom, a halogen atom, or a hydroxyl group which may be protected, or may form a crosslink with the 4'-position carbon (preferably a crosslink of a Locked Nucleic Acid (LNA), an amide-bridged nucleic acid, a guanidine-bridged nucleic acid, or a spirocyclopropylene-bridged nucleic acid);
o Q₁ groups each independently represent a C1-4 alkyl group having an electron-withdrawing group (e.g., a cyano group, a nitro group, a 2-pyridyl group, a 4-pyridyl group, etc.), an allyl group, a C2-6 alkenyl group or a C6-10 aryl group having a cyano group, a nitro group or a halogen group, or a hydrogen atom;
W groups each independently represent a hydrogen atom or a temporary protecting group removable under acidic conditions or with a reagent containing a fluorine anion;
o R₂ groups each independently represent O or S;
Tg represents a group represented by the following formula (5): 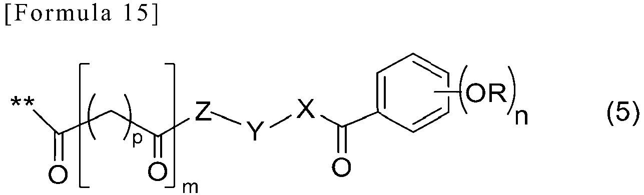 wherein,
R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
n represents 2 or 3, and the plurality of OR groups may be the same or different;
m represents 0 or 1;
p represents 1 or 2;
X is C, N, O or S, and when X is C, it represents CH₂ or may form, together with Y, a 5- to 7-membered carbocyclic or heterocyclic ring which may have a substituent, and when X is N, it represents N R₁ or may form, together with Y, a 5- to 7-membered heterocyclic ring which may have a substituent, wherein R₁ is H or a C1-6 alkyl group;
Y is a single bond or B(CH₂)ₜ* (wherein B is a 5- to 7-membered carbocyclic ring which may have a substituent, t is 0, 1, or 2, and * indicates a bond to X), or may form, together with X, a 5- to 7-membered carbocyclic or heterocyclic ring which may have a substituent;
Z is a single bond or (CH₂)_{S}, wherein s is an integer of 1 to 5 (preferably an integer of 1 to 3), provided that when Y is a single bond, Z is not a single bond,
** indicates a bond to Base, or
a nucleotide represented by the following formula (VII) or formula (VIII): 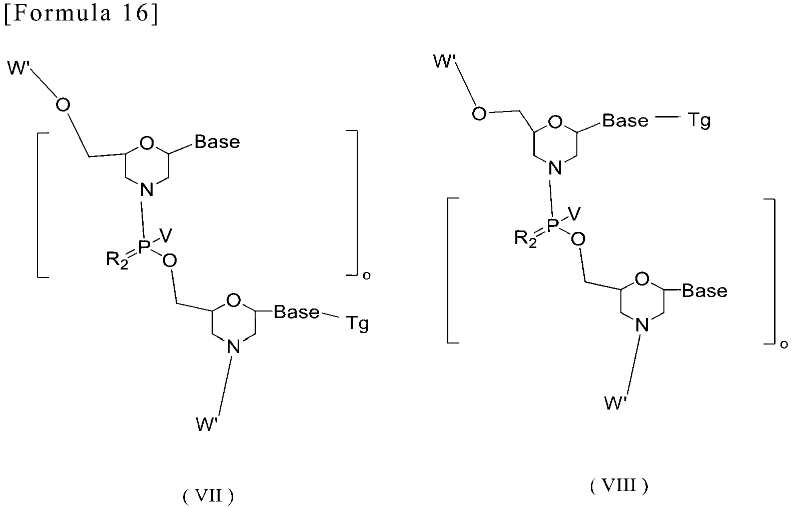 wherein,
o represents any integer of 0 or more (preferably an integer of 1 to 50);
o+1 Base each independently represents an optionally modified nucleic acid base;
o V each independently represents a C1-6 alkoxy group, a di(C1-6 alkyl)amino group, or a piperazino group in which the 4-position nitrogen is protected with a protecting group and which may be further substituted;
W' each independently represents a hydrogen atom or a temporary protecting group removable under acidic conditions or with a reagent containing a fluorine anion;
o R₂ each independently represents O or S;
Tg represents a group represented by the following formula (5): 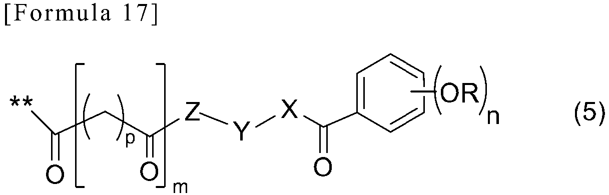 wherein,
R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
n represents 2 or 3, and the plurality of OR groups may be the same or different;
m represents 0 or 1;
p represents 1 or 2;
X is C, N, O or S, and when X is C, it represents CH₂ or may form, together with Y, a 5- to 7-membered carbocyclic or heterocyclic ring which may have a substituent, and when X is N, it represents N R₁ or may form, together with Y, a 5- to 7-membered heterocyclic ring which may have a substituent, wherein R₁ is H or a C1-6 alkyl group;
Y is a single bond or B(CH₂)ₜ* (wherein B is a 5- to 7-membered carbocyclic ring which may have a substituent, t is 0, 1, or 2, and * indicates a bond to X), or may form, together with X, a 5- to 7-membered carbocyclic or heterocyclic ring which may have a substituent;
Z is a single bond or (CH₂)_{S}, wherein s is an integer of 1 to 5 (preferably an integer of 1 to 3), provided that when Y is a single bond, Z is not a single bond,
** indicates a bond to Base.

22. The nucleotide according to claim 21, wherein Tg in the formula (V), the formula (VI), the formula (VII) or the formula (VIII) represents a group represented by the following formula (6): 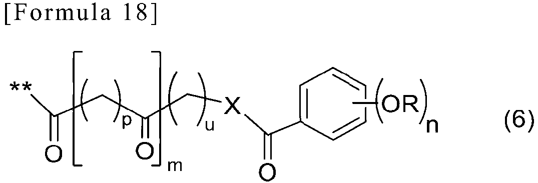 (In the formula,
R each independently represents an optionally substituted alkenyl group having 14 to 60 carbon atoms (preferably having 14 to 50 carbon atoms, more preferably having 10 to 40 carbon atoms, and still more preferably having 14 to 30 carbon atoms);
n represents 2 or 3, and the plurality of OR groups may be the same or different;
m represents 0 or 1;
p represents 1 or 2;
u represents an integer of 1 to 5 (preferably an integer of 1 to 3);
X represents NR₁, O or S, wherein R₁ is H or a C1-6 alkyl group;
** indicates a bond to Base.),
a group represented by the following formula (7): 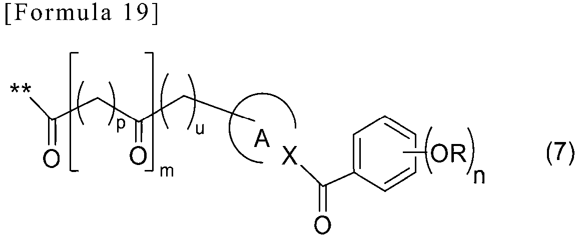 wherein,
R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
n represents 2 or 3, and the plurality of OR groups may be the same or different;
m represents 0 or 1;
p represents 1 or 2;
u represents 0 or an integer of 1 to 5 (preferably 0 or an integer of 1 to 3, more preferably 0, 1, or 2);
ring A represents a 5- to 7-membered ring which may have a substituent;
X represents C or N;
** indicates a bond to Base.), or
a group represented by the following formula (8): 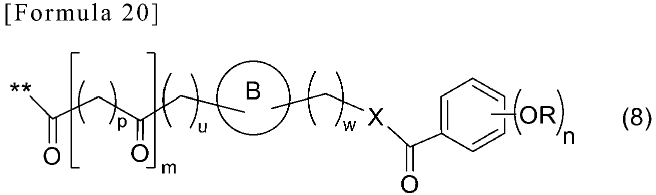 wherein,
R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
n represents 2 or 3, and the plurality of OR groups may be the same or different;
m represents 0 or 1;
p represents 1 or 2;
u represents 0 or an integer of 1 to 5 (preferably 0 or an integer of 1 to 3, more preferably 0, 1, or 2);
w represents 0, 1, or 2;
ring B represents a carbocyclic ring having 5 to 7 carbon atoms which may have a substituent;
X represents NR₁ , O or S, where R₁ is H or C H ₃ ;
** indicates a bond to Base.

23. The nucleotide according to any one of claims 19 to 22, wherein the temporary protecting group removable under acidic conditions in the formula (I), the formula (II), the formula (V), the formula (VI), the formula (VII) or the formula (VIII) is a group selected from the group consisting of a trityl group, a 9-(9-phenyl)xanthenyl group, a 9-phenylthioxanthenyl group, a di(C1-6 alkoxy)trityl group, a mono(C1-18 alkoxy)trityl group, and a silyl group substituted with an alkyl or allyl group, and the temporary protecting group removable with a reagent containing a fluorine anion is a silyl group substituted with an alkyl or allyl group, and the temporary protecting group removable under acidic conditions in the formula (II) is a group selected from the group consisting of a trityl group, a 9-(9-phenyl)xanthenyl group, a 9-phenylthioxanthenyl group, a di(C1-6 alkoxy)trityl group, a mono(C1-18 alkoxy)trityl group, and a silyl group substituted with an alkyl or allyl group.

24. The nucleotide according to any one of claims 19 to 22, wherein Base in the formula (I), (II), (V), (VI), (VII) or (VIII) is a pyrimidine base or a purine base which may be substituted by 1 to 3 substituents selected from the group consisting of a halogen atom, an alkyl group, an aralkyl group, an alkoxy group, an acyl group, an alkoxyalkyl group, a hydroxyl group, an amino group, a monoalkylamino group, a dialkylamino group, a carboxy group, a cyano group and a nitro group.

25. The nucleotide according to any one of claims 19 to 22, wherein D in the formula (I), (V) or (VI) is a hydroxyl group which may be protected by a methyl group, a benzyl group, a p-methoxybenzyl group, a tert-butyl group, a methoxymethyl group, a methoxyethyl group, a 2-tetrahydropyranyl group, an ethoxyethyl group, a cyanoethyl group, a cyanoethoxymethyl group, a phenylcarbamoyl group, a 1,1-dioxothiomorpholine-4-thiocarbamoyl group, an acetyl group, a pivaloyl group, a benzoyl group, a trimethylsilyl group, a triethylsilyl group, a triisopropylsilyl group, a tert-butyldimethylsilyl group (TBS), a [(triisopropylsilyl)oxy]methyl group or a 1-(4-chlorophenyl)-4-ethoxypiperidin-4-yl group.

26. A method for producing an oligonucleotide, comprising the following steps (A1)-(A3):
(A1) a step of reacting, in a nonpolar or polar solvent, an n-mer oligonucleotide (wherein n is any integer of 1 or more) in which the 3' - or 5'-position hydroxyl group is protected with a support protecting group and the 5'- or 3'-position hydroxyl group is protected with a temporary protecting group removable under acidic conditions or with a reagent containing a fluorine anion, with an acid and a cation scavenger, or with a reagent containing a fluorine anion to remove the temporary protecting group at the 5'- or 3'-position hydroxyl group, and then separating the n-mer oligonucleotide from impurities in the reaction solution;
(A2) a step of adding a p-mer oligonucleotide (p is any integer of 1 or more) in which the 3'-position hydroxyl group has been phosphoramiditized and the 5'-position hydroxyl group has been protected with a temporary protecting group removable under acidic conditions or with a reagent containing a fluorine anion, or in which the 5'-position hydroxyl group has been phosphoramiditized and the 3' - position hydroxyl group has been protected with a temporary protecting group removable under acidic conditions or with a reagent containing a fluorine anion, to a solution of the n-mer oligonucleotide obtained in step (A1) from which the temporary protecting group at the 5'-position hydroxyl group or the 3'-position hydroxyl group has been removed, thereby condensing the n-mer oligonucleotide and the p-mer oligonucleotide via the 5'-position hydroxyl group or the 3'-position hydroxyl group through a phosphite triester bond; and
(A3) a step of converting the phosphite triester bond of the n+p-mer oligonucleotide obtained in step (A2) into a phosphate triester bond or a thiophosphate triester bond using an oxidizing agent or a sulfurizing agent;
wherein the support protecting group is a group represented by the following formula (5): 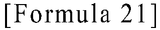 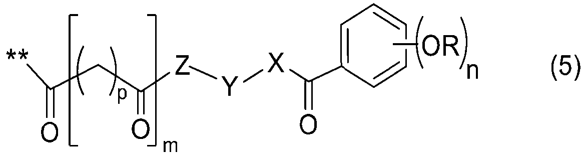 wherein,
R each independently represents an optionally substituted alkenyl group having 14 to 60 carbon atoms;
n represents 2 or 3, and the plurality of OR groups may be the same or different;
m represents 0 or 1;
p represents 1 or 2;
X is C, N, O or S, and when X is C, it represents CH₂ or may form, together with Y, a 5- to 7-membered carbocyclic or heterocyclic ring which may have a substituent, and when X is N, it represents N R₁ or may form, together with Y, a 5- to 7-membered heterocyclic ring which may have a substituent, wherein R₁ is H or a C1-6 alkyl group;
Y is a single bond or B(CH₂)ₜ* (wherein B is a 5- to 7-membered carbocyclic ring which may have a substituent, t is 0, 1, or 2, and * indicates a bond to X), or may form, together with X, a 5- to 7-membered carbocyclic or heterocyclic ring which may have a substituent;
Z is a single bond or (CH₂)_{S}, wherein s is an integer of 1 to 5, provided that when Y is a single bond, Z is not a single bond;
** indicates a bond to the 3'- or 5'-position hydroxyl group.).

27. The method for producing an oligonucleotide according to claim 26, wherein the support protecting group is a group represented by the following formula (6): 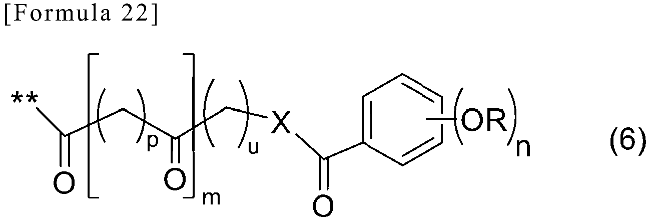 wherein,
R each independently represents an optionally substituted alkenyl group having 14 to 60 carbon atoms;
n represents 2 or 3, and the plurality of OR groups may be the same or different;
m represents 0 or 1;
p represents 1 or 2;
u represents an integer of 1 to 5;
X represents NR₁, O or S, wherein R₁ is H or a C1-6 alkyl group;
** indicates a bond to the 3'- or 5'-position hydroxyl group);
a group represented by the following formula (7): 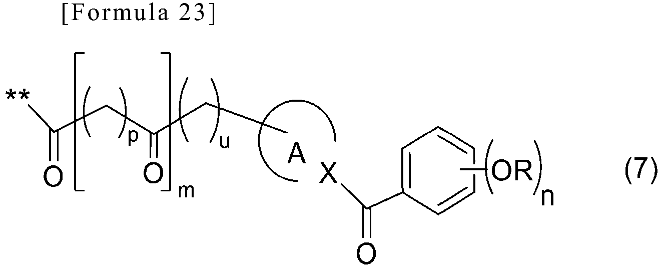 wherein,
R each independently represents an optionally substituted alkenyl group having 14 to 60 carbon atoms;
n represents 2 or 3, and the plurality of OR groups may be the same or different;
m represents 0 or 1;
p represents 1 or 2;
u represents 0 or an integer from 1 to 5;
ring A represents a 5- to 7-membered ring which may have a substituent;
X represents C or N;
** indicates a bond to the 3'- or 5'-position hydroxyl group.); or
a group represented by the following formula (8): 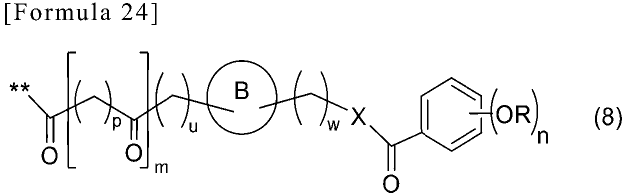 wherein,
R each independently represents an optionally substituted alkenyl group having 14 to 60 carbon atoms;
n represents 2 or 3, and the plurality of OR groups may be the same or different;
m represents 0 or 1;
p represents 1 or 2;
u represents 0 or an integer of 1 to 5;
w represents 0, 1, or 2;
ring B represents a carbocyclic ring having 5 to 7 carbon atoms which may have a substituent;
X represents NR₁ , O or S, wherein R₁ is H or C H ₃ ;
** indicates a bond to the 3'- or 5'-position hydroxyl group.

28. The method for producing an oligonucleotide according to claim 26 or claim 27, wherein in the step (A1), the step of separating the n-mer oligonucleotide from which the temporary protecting group has been removed from impurities in the reaction solution comprises neutralizing the reaction solution with a base, and then
(i) a step of isolating the n-mer oligonucleotide from which the temporary protecting group has been removed, or (ii) a step of washing the neutralized reaction solution with water to recover an organic layer.

29. The method according to claim 26 or claim 27, further comprising the following step (A4):
(A4) a step of adding a polar solvent to the reaction solution obtained in the step (A1) or (A3), to precipitate the n+p-mer oligonucleotide, and obtaining it by solid-liquid separation.

30. The method according to claim 29, further comprising the following step (A5):
(A5) a step of removing all protecting groups from the n+p-mer oligonucleotide obtained in the step (A4).

31. A method for producing a nucleotide, comprising a step of subjecting a nucleotide represented by the following formula (I): 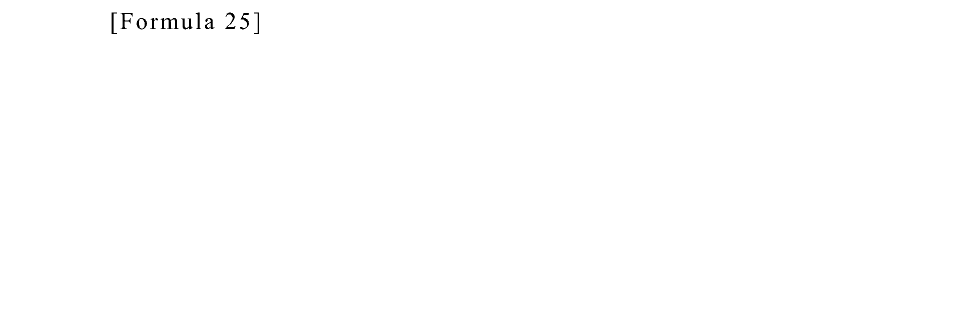 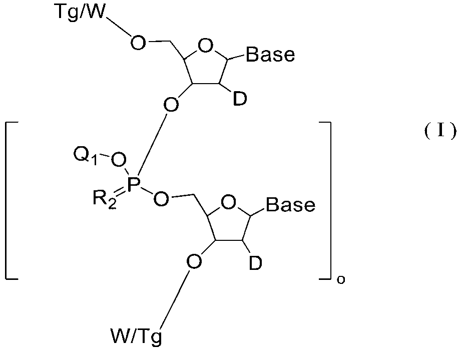 wherein,
o represents any integer of 0 or more;
o+1 Base each independently represents an optionally modified nucleic acid base;
o+1 D each independently represents a hydrogen atom, a halogen atom, or a hydroxyl group which may be protected, or may form a crosslink with the 4'-position carbon;
o Q₁ each independently represents a C1-4 alkyl group having an electron-withdrawing group, an allyl group, a C2-6 alkenyl group or a C6-10 aryl group having a cyano group, a nitro group or a halogen group, or a hydrogen atom;
W/Tg or Tg/W represents W or Tg, and when the 5' side is W, the 3' side is Tg, whereas when the 5' side is Tg, the 3' side is W, wherein
W represents a hydrogen atom or a temporary protecting group removable under acidic conditions or with a reagent containing a fluorine anion;
o R₂ each independently represents O or S; and
Tg represents a group represented by the following formula (5): 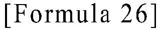 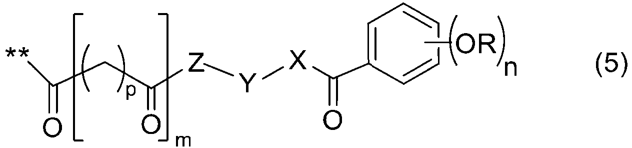 wherein,
R each independently represents an optionally substituted alkenyl group having 14 to 60 carbon atoms
n represents 2 or 3, and the plurality of OR groups may be the same or different;
m represents 0 or 1;
p represents 1 or 2;
X is C, N, O or S, and when X is C, it represents CH₂ or may form, together with Y, a 5- to 7-membered carbocyclic or heterocyclic ring which may have a substituent, and when X is N, it represents NR₁ or may form, together with Y, a 5- to 7-membered heterocyclic ring which may have a substituent, wherein R₁ is H or a C1-6 alkyl group;
Y is a single bond or B(CH₂)ₜ* (wherein B is a 5- to 7-membered carbocyclic ring which may have a substituent, t is 0, 1, or 2, and * indicates a bond to X), or may form, together with X, a 5- to 7-membered carbocyclic or heterocyclic ring which may have a substituent;
Z is a single bond or (CH₂)ₛ, wherein s is an integer of 1 to 5, provided that when Y is a single bond, Z is not a single bond;
** indicates a bond to O;
or a nucleotide represented by the following formula (II): 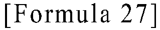 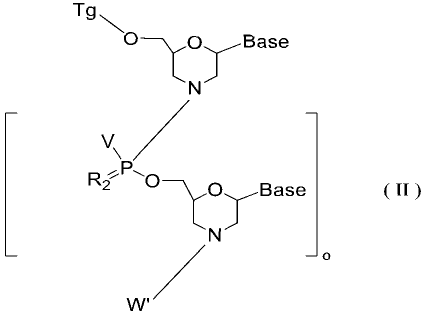 wherein,
o represents any integer of 0 or more;
o+1 Base each independently represents an optionally modified nucleic acid base;
o V each independently represents an alkoxy group having 1 to 6 carbon atoms, a di(C 1-6 alkyl)amino group, or a piperazino group in which the 4-position nitrogen is protected with a protecting group and which may be further substituted;
W' represents a hydrogen atom or a temporary protecting group removable under acidic conditions or with a reagent containing a fluorine anion;
o R₂ each independently represents O or S;
Tg represents a group represented by the following formula (5): 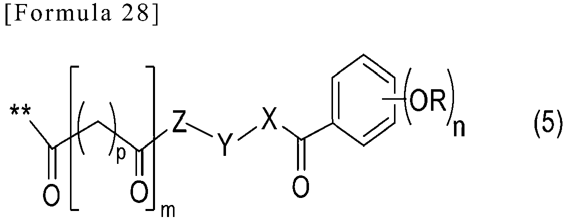 wherein,
R each independently represents an optionally substituted alkenyl group having 14 to 60 carbon atoms;
n represents 2 or 3, and the plurality of OR groups may be the same or different;
m represents 0 or 1;
p represents 1 or 2;
X is C, N, O or S, and when X is C, it represents CH₂ or may form, together with Y, a 5- to 7-membered carbocyclic or heterocyclic ring which may have a substituent, and when X is N, it represents N R₁ or may form, together with Y, a 5- to 7-membered heterocyclic ring which may have a substituent, wherein R₁ is H or a C1-6 alkyl group;
Y is a single bond or B(CH₂)ₜ* (wherein B is a 5- to 7-membered carbocyclic ring which may have a substituent, t is 0, 1, or 2, and * indicates a bond to X), or may form, together with X, a 5- to 7-membered carbocyclic or heterocyclic ring which may have a substituent;
Z is a single bond or (CH₂)_{S}, where s is an integer of 1 to 5, provided that when Y is a single bond, Z is not a single bond;
* indicates a bond to O,
to reduction treatment, reaction with hydrazine or alkylhydrazine, or hydrolysis, to remove the protecting group represented by the formula (5), to obtain a nucleotide represented by the formula (III): 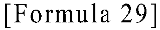 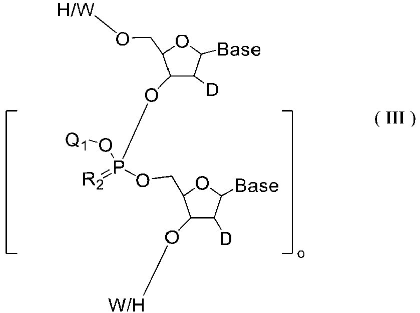
wherein, the definitions of Base, D, R₂, Q₁, and W are the same as those in the formula (I), and W/H represents W or hydrogen; when the 5' side is W, the 3' side is hydrogen, and when the 5' side is hydrogen, the 3' side is W,
or a nucleotide represented by the following formula (IV): 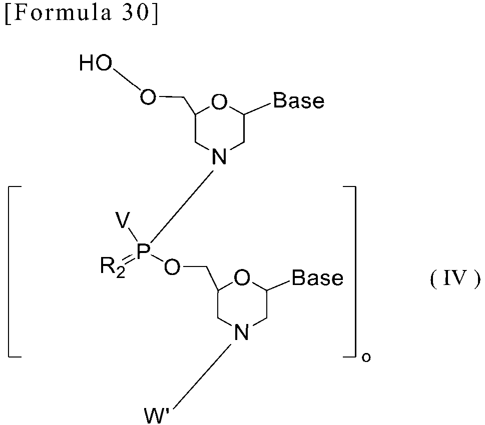 wherein, the definitions of Base, R₂, V, and W' are the same as those in the formula (II),

32. The production method according to claim 31, wherein the reduction treatment is performing using a boron-containing reducing agent, or both a boron-containing reducing agent and an amine.

33. A method for producing an oligonucleotide, comprising the following steps (B1) to (B3):
(B1) a step of reacting, in a non-polar or polar solvent, an n-mer oligonucleotide (n is any integer of 1 or more) in which a support protecting group is bound to a nucleic acid base at the 3' -terminal or a nucleic acid base at the 5'-terminal and the 3'-position hydroxyl group and the 5'-position hydroxyl group are protected with a temporary protecting group removable under acidic conditions or with a reagent containing a fluorine anion, with an acid and a cation scavenger, or with a reagent containing a fluorine anion (preferably pyridine hydrofluoride or tetrabutylammonium fluoride) to remove the temporary protecting group at only one of the 5'-position hydroxyl group or the 3'-position hydroxyl group, and then separating the n-mer oligonucleotide from impurities in the reaction solution;
(B2) a step of adding a p-mer oligonucleotide (p is any integer of 1 or more) in which the 3'-position hydroxyl group has been phosphoramiditized and the 5'-position hydroxyl group has been protected with a temporary protecting group removable under acidic conditions or with a reagent containing a fluorine anion, or in which the 5'-position hydroxyl group has been phosphoramiditized and the 3' - position hydroxyl group has been protected with a temporary protecting group removable under acidic conditions or with a reagent containing a fluorine anion, to a solution of the n-mer oligonucleotide obtained in step (B1) from which the temporary protecting group at the 5' -position hydroxyl group or the 3'-position hydroxyl group has been removed, thereby condensing the n-mer oligonucleotide and the p-mer oligonucleotide via the 5'-position hydroxyl group or the 3'-position hydroxyl group through a phosphite triester bond; and
(B3) a step of converting the phosphite triester bond of the n+p-mer oligonucleotide obtained in step (B2) into a phosphate triester bond or a thiophosphate triester bond using an oxidizing agent or a sulfurizing agent;
wherein the support protecting group is a group represented by the following formula (5): 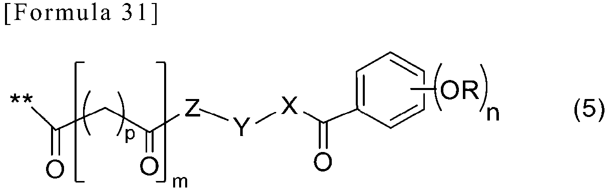 wherein,
R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
n represents 2 or 3, and the plurality of OR groups may be the same or different;
m represents 0 or 1;
p represents 1 or 2;
X is C, N, O or S, and when X is C, it represents CH₂ or may form, together with Y, a 5- to 7-membered carbocyclic or heterocyclic ring which may have a substituent, and when X is N, it represents N R₁ or may form, together with Y, a 5- to 7-membered heterocyclic ring which may have a substituent, wherein R₁ is H or a C1-6 alkyl group;
Y is a single bond or B(CH₂)ₜ* (wherein B is a - to 7-membered carbocyclic ring which may have a substituent, t is 0, 1, or 2, and * indicates a bond to X), or may form, together with X, a 5- to 7-membered carbocyclic or heterocyclic ring which may have a substituent;
Z is a single bond or (CH₂)_{S}, where s is an integer of 1 to 5 (preferably an integer of 1 to 3), provided that when Y is a single bond, Z is not a single bond;
** indicates a bond to a nucleic acid base.

34. The method for producing an oligonucleotide according to claim 33,
wherein the support protecting group is a group represented by the following formula (6): 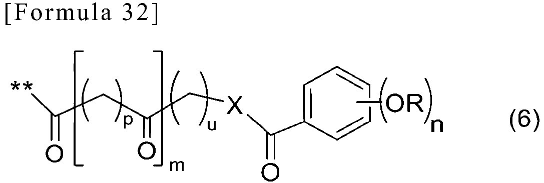 wherein,
R each independently represents an optionally substituted alkenyl group having 14 to 60 carbon atoms (preferably having 14 to 50 carbon atoms, more preferably having 10 to 40 carbon atoms, and still more preferably having 14 to 30 carbon atoms);
n represents 2 or 3, and the plurality of OR groups may be the same or different;
m represents 0 or 1;
p represents 1 or 2;
u represents an integer of 1 to 5 (preferably an integer of 1 to 3);
X represents NR₁, O or S, wherein R₁ is H or a C1-6 alkyl group;
** indicates a bond to a nucleic acid base.),
a group represented by the following formula (7): 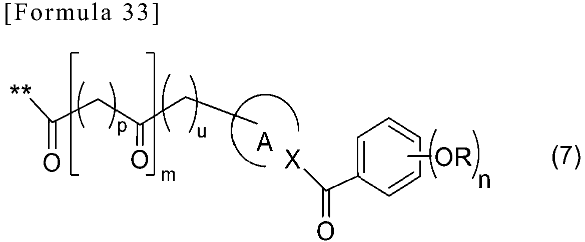 wherein,
R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
n represents 2 or 3, and the plurality of OR groups may be the same or different;
m represents 0 or 1;
p represents 1 or 2;
u represents 0 or an integer from 1 to 5 (preferably 0 or an integer from 1 to 3, more preferably 0, 1, or 2);
ring A represents a 5- to 7-membered ring which may have a substituent;
X represents C or N;
** indicates a bond to a nucleic acid base,
or a group represented by the following formula (8): 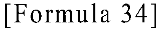 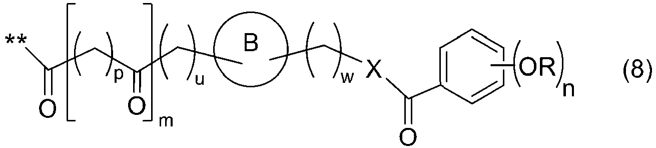 wherein,
R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
n represents 2 or 3, and the plurality of OR groups may be the same or different;
m represents 0 or 1;
p represents 1 or 2;
u represents 0 or an integer of 1 to 5 (preferably 0 or an integer of 1 to 3, more preferably 0, 1, or 2);
w represents 0, 1, or 2);
ring B represents a carbocyclic ring having 5 to 7 carbon atoms which may have a substituent;
X represents NR₁ , O or S, where R₁ is H or C H ₃ ;
** indicates a bond to a nucleic acid base.).

35. The method for producing an oligonucleotide according to claim 33 or claim 34, wherein in the step (B1), the step of separating the n-mer oligonucleotide from which the temporary protecting group has been removed from impurities in the reaction solution comprises neutralizing the reaction solution with a base, and then
(i) a step of isolating the n-mer oligonucleotide from which the temporary protecting group has been removed, or (ii) a step of washing the neutralized reaction solution with water to recover an organic layer.

36. The method according to claim 33 or claim 34, further comprising the following step (B4):
(B4) a step of adding a polar solvent to the reaction solution obtained in the step (B1) and/or (B3) to precipitate the n+p-mer oligonucleotide, and obtaining it by solid-liquid separation.

37. The method according to claim 36, further comprising the following step (B5):
(B5) a step of removing all protecting groups from the n+p-mer oligonucleotide obtained in the step (B4).

38. A method for producing a nucleotide, comprising a step of subjecting a nucleotide represented by the following formula (V) or the formula (VI): 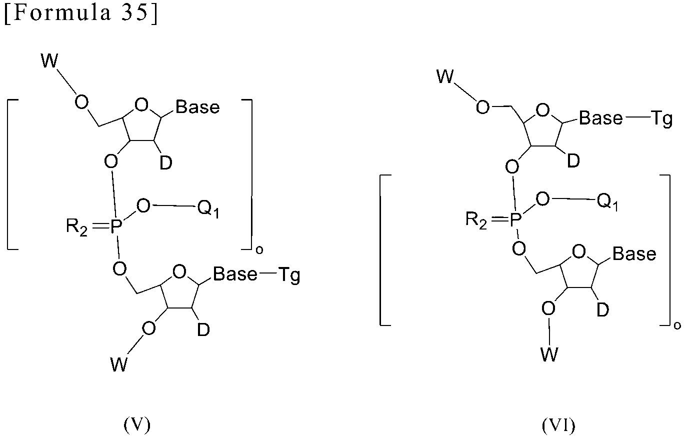 wherein,
o represents any integer of 0 or more (preferably an integer of 1 to 50);
o+1 Base each independently represents an optionally modified nucleic acid base;
o+1 D each independently represents a hydrogen atom, a halogen atom, or a hydroxyl group which may be protected, or may form a crosslink with the 4'-position carbon (preferably a crosslink of a Locked Nucleic Acid (LNA), an amide-bridged nucleic acid, a guanidine-bridged nucleic acid, or a spirocyclopropylene-bridged nucleic acid);
o Q₁ each independently represents a C1-4 alkyl group having an electron-withdrawing group (e.g., a cyano group, a nitro group, a 2-pyridyl group, a 4-pyridyl group, etc.), an allyl group, a C2-6 alkenyl group or a C6-10 aryl group having a cyano group, a nitro group, or a halogen group, or a hydrogen atom;
W each independently represents a hydrogen atom or a temporary protecting group removable under acidic conditions or with a reagent containing a fluorine anion;
o R₂ each independently represents O or S; and
Tg represents a group represented by the following formula (5): 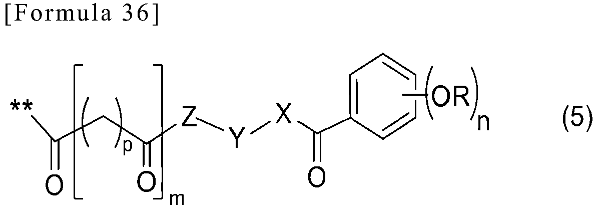 wherein,
R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
n represents 2 or 3, and the plurality of OR groups may be the same or different;
m represents 0 or 1;
p represents 1 or 2;
X is C, N, O or S, and when X is C, it represents CH₂ or may form, together with Y, a 5- to 7-membered carbocyclic or heterocyclic ring which may have a substituent, and when X is N, it represents N R₁ or may form, together with Y, a 5- to 7-membered heterocyclic ring which may have a substituent, wherein R₁ is H or a C1-6 alkyl group;
Y is a single bond or B(CH₂)ₜ* (wherein B is a - to 7-membered carbocyclic ring which may have a substituent, t is 0, 1, or 2, and * indicates a bond to X), or may form, together with X, a 5- to 7-membered carbocyclic or heterocyclic ring which may have a substituent;
Z is a single bond or (CH₂)ₛ, wherein s is an integer of 1 to 5 (preferably an integer of 1 to 3), provided that when Y is a single bond, Z is not a single bond;
** indicates a bond to O.)
or a nucleotide represented by the following formula (VII) or the formula (VIII): 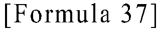 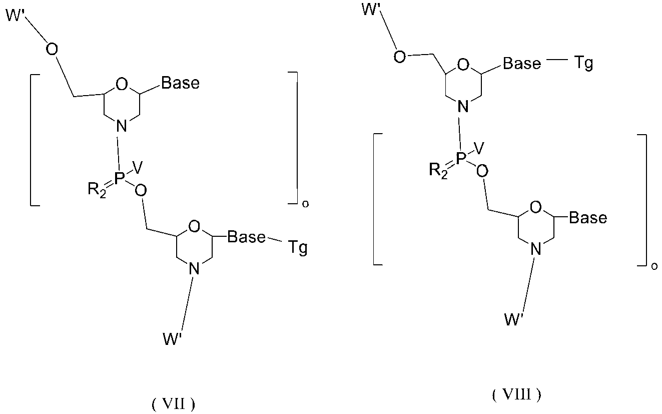 wherein,
o represents any integer of 0 or more (preferably an integer of 1 to 50);
o+1 Base each independently represents an optionally modified nucleic acid base;
o V each independently represents an alkoxy group having 1 to 6 carbon atoms, a di(C₁₋₆ alkyl)amino group, or a piperazino group in which the 4-position nitrogen is protected with a protecting group and which may be further substituted;
W' each independently represents a hydrogen atom or a temporary protecting group removable under acidic conditions or with a reagent containing a fluorine anion;
o R₂ each independently represents O or S;
Tg represents a group represented by the following formula (5): 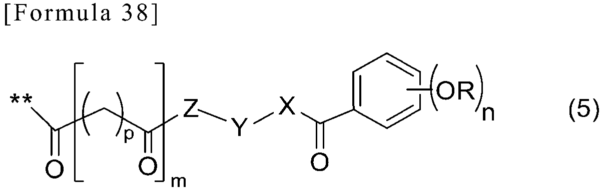 wherein,
R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
n represents 2 or 3, and the plurality of OR groups may be the same or different;
m represents 0 or 1;
p represents 1 or 2;
X is C, N, O or S, and when X is C, it represents CH₂ or may form, together with Y, a 5- to 7-membered carbocyclic or heterocyclic ring which may have a substituent, and when X is N, it represents N R₁ or may form, together with Y, a 5- to 7-membered heterocyclic ring which may have a substituent, wherein R₁ is H or a C1-6 alkyl group;
Y is a single bond or B(CH₂)ₜ* (wherein B is a 5- to 7-membered carbocyclic ring having 5 to 7 carbon atoms which may have a substituent, t is 0, 1, or 2, and * indicates a bond to X), or may form, together with X, a 5- to 7-membered carbocyclic or heterocyclic ring which may have a substituent;
Z is a single bond or (CH₂)_{S}, where s is an integer of 1 to 5 (preferably an integer of 1 to 3), provided that when Y is a single bond, Z is not a single bond;
** indicates a bond to Base,
to hydrolysis or ammolysis, to remove the protecting group represented by the formula (5), to obtain a nucleotide represented by the formula (IX): 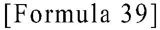 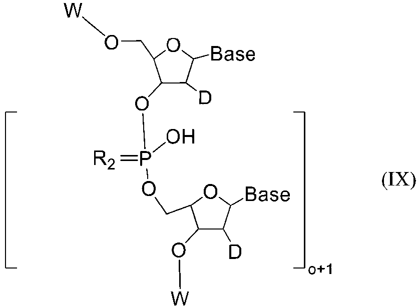
wherein, the definitions of Base, D, R₂, W and o are the same as those in the formula (V) or the formula (VI),
or the following formula (X): 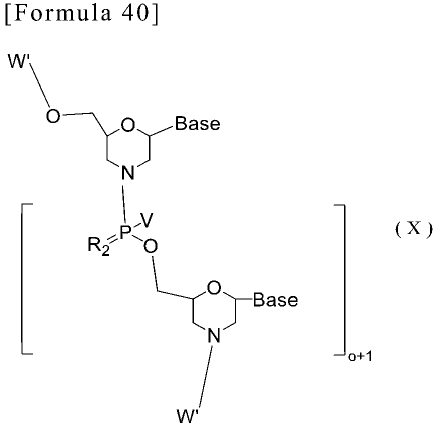 wherein, the definitions of Base, R₂, V, W', and o are the same as those in the formula (VII) or the formula (VIII).

39. A method for producing an oligonucleotide, comprising the following steps (C1) to (C4):
(C1) a step of obtaining an n-mer oligonucleotide from which the temporary protecting group at the 5'-position hydroxyl group has been removed and a p-mer oligonucleotide from which the temporary protecting group at the 3'-position hydroxyl group has been removed by carrying out the following steps (a) and (b), respectively:
(a) a step of reacting, in a nonpolar or polar solvent, an n-mer oligonucleotide (n is any integer of 1 or more) in which the 3' -position hydroxyl group is protected with a support protecting group and the 5'-position hydroxyl group is protected with a temporary protecting group removable under acidic conditions or with a reagent containing a fluorine anion, with an acid and a cation scavenger, or with a reagent containing a fluorine anion (preferably pyridine hydrofluoride or tetrabutylammonium fluoride) to remove the temporary protecting group at the 5' -position hydroxyl group, and then separating the n-mer oligonucleotide from impurities in the reaction solution; and
(b) a step of reacting, in a non-polar or polar solvent, a p-mer oligonucleotide (p is any integer of 1 or more) in which a support protecting group is bound to the 3'-terminal nucleic acid base and the 3'-position hydroxyl group and the 5'-position hydroxyl group are protected with temporary protecting groups removable under acidic conditions or with a reagent containing a fluorine anion, with an acid and a cation scavenger, or with a reagent containing a fluorine anion (preferably pyridine hydrofluoride or tetrabutylammonium fluoride) to remove only the temporary protecting group at the 3'-position hydroxyl group, and then separating the p-mer oligonucleotide from impurities in the reaction solution;
wherein the support protecting groups of (a) and (b) may be the same or different;
(C2) a step of phosphoramiditizing the 5'-position hydroxyl group of the n-mer oligonucleotide from which the temporary protecting group at the 5'-position hydroxyl group has been removed obtained in step (C1), or a step of phosphoramiditizing the 3'-position hydroxyl group of the p-mer oligonucleotide from which the temporary protecting group at the 3'-position hydroxyl group has been removed obtained in step (C1),
(C3) a step of adding the n-mer oligonucleotide whose 5'-position hydroxyl group has been phosphoramiditized obtained in step (C2) to a solution of the p-mer oligonucleotide from which the temporary protecting group at the 3'-position hydroxyl group has been removed obtained in step (C1), or adding the p-mer oligonucleotide whose 3'-position hydroxyl group has been phosphoramiditized obtained in step (C2) to a solution of the n-mer oligonucleotide from which the temporary protecting group at the 5'-position hydroxyl group has been removed obtained in step (C1), thereby condensing the n-mer oligonucleotide and the p-mer oligonucleotide through a phosphite triester bond; and
(C4) a step of converting the phosphite triester bond of the n+p-mer oligonucleotide obtained in step (C3) into a phosphate triester bond or a thiophosphate triester bond using an oxidizing agent or a sulfurizing agent;
wherein the support protecting group is a group represented by the following formula (5): 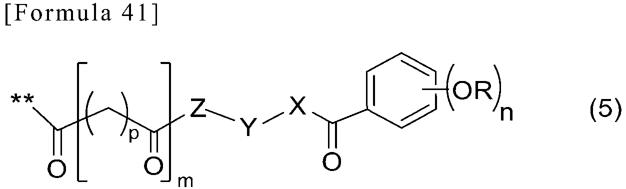 wherein,
R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
n represents 2 or 3, and the plurality of OR groups may be the same or different;
m represents 0 or 1;
p represents 1 or 2;
X is C, N, O or S, and when X is C, it represents CH₂ or may form, together with Y, a 5- to 7-membered carbocyclic or heterocyclic ring which may have a substituent, and when X is N, it represents N R₁ or may form, together with Y, a 5- to 7-membered heterocyclic ring which may have a substituent, wherein R₁ is H or a C1-6 alkyl group;
Y is a single bond or B(CH₂)ₜ* (wherein B is a 5- to 7-membered carbocyclic ring which may have a substituent, t is 0, 1, or 2, and * indicates a bond to X), or may form, together with X, a 5- to 7-membered carbocyclic or heterocyclic ring which may have a substituent;
Z is a single bond or (CH₂)_{S}, where s is an integer of 1 to 5 (preferably an integer of 1 to 3), provided that when Y is a single bond, Z is not a single bond;
** indicates a bond to the 3'-position hydroxyl group or the 3'-terminal nucleic acid base.

40. The method for producing an oligonucleotide according to claim 39, wherein the support protecting group is a group represented by the following formula (6): 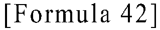 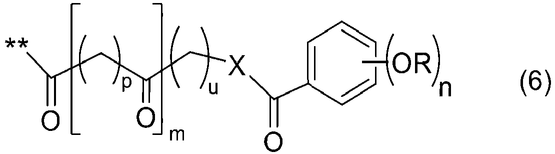 wherein,
R each independently represents an optionally substituted alkenyl group having 14 to 60 carbon atoms (preferably having 14 to 50 carbon atoms, more preferably having 10 to 40 carbon atoms, and still more preferably having 14 to 30 carbon atoms);
n represents 2 or 3, and the plurality of OR groups may be the same or different;
m represents 0 or 1;
p represents 1 or 2;
u represents an integer of 1 to 5 (preferably an integer of 1 to 3);
X represents NR₁, O or S, where R₁ is H or a C1-6 alkyl group;
** indicates a bond to the 3'-position hydroxyl group or the 3'-terminal nucleic acid base.),
a group represented by the following formula (7): 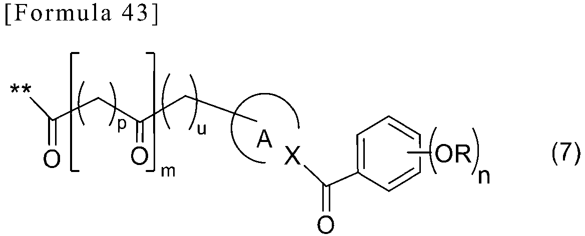 wherein,
R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
n represents 2 or 3, and the plurality of OR groups may be the same or different;
m represents 0 or 1;
p represents 1 or 2;
u represents 0 or an integer from 1 to 5 (preferably 0 or an integer from 1 to 3, more preferably 0, 1, or 2);
ring A represents a 5- to 7-membered ring which may have a sustituent;
X represents C or N;
** indicates a bond to the 3'-position hydroxyl group or the 3'-terminal nucleic acid base.),
or a group represented by the following formula (8): 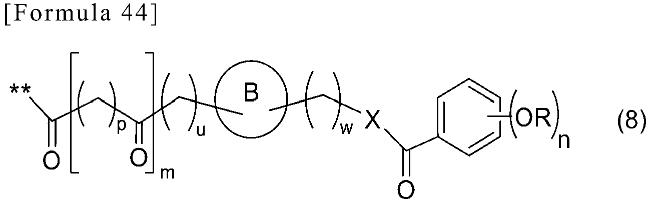 wherein,
R each independently represents an optionally substituted alkenyl group having 14 to 60 carbon atoms (preferably having 14 to 50 carbon atoms, more preferably having 14 to 40 carbon atoms, and still more preferably having 14 to 30 carbon atoms);
n represents 2 or 3, and the plurality of OR groups may be the same or different;
m represents 0 or 1;
p represents 1 or 2;
u represents 0 or an integer of 1 to 5 (preferably 0 or an integer of 1 to 3, more preferably 0, 1, or 2);
w represents 0, 1, or 2;
ring B represents a carbocyclic ring having 5 to 7 carbon atoms which may have a substituent;
X represents NR₁ , O or S, wherein R₁ is H or C H ₃ ;
** indicates a bond to the 3'-position hydroxyl group or the 3'-terminal nucleic acid base.

41. The method for producing an oligonucleotide according to claim 39 or claim 40, wherein in the step (C1), the step of separating the n-mer oligonucleotide from which the temporary protecting group has been removed from impurities in the reaction solution or the step of separating the p-mer oligonucleotide from which the temporary protecting group has been removed from impurities in the reaction solution comprises neutralizing the reaction solution with a base, and then
(i) a step of isolating the n-mer oligonucleotide or p-mer oligonucleotide from which the temporary protecting group has been removed, or (ii) a step of washing the neutralized reaction solution with water to recover an organic layer.

42. The method according to claim 39 or claim 40, further comprising the following step (C5):
(C5) a step of adding a polar solvent to the reaction solution obtained in the step (C1) and/or (C2) and/or (C4), to precipitate the n+p-mer oligonucleotide, and obtaining it by solid-liquid separation.

43. The method according to claim 42, further comprising the following step (C6):
(C6) a step of removing all protecting groups from the n+p-mer oligonucleotide obtained in the step (C5).
